# EUROPEAN PATENT APPLICATION

(11) **EP 4 782 441 A1**
(43) Date of publication of application: **29.07.2026**
(21) Application number: 25154353.4
(22) Date of filing: 28.01.2025
(51) Int. Cl.: C07D 401/14, C07D 405/14, C07D 471/04, C07D 487/04, C07D 519/00, A61P 33/00, A01N 43/40, A61K 31/44

(54) **SPIROCYCLE-SUBSTITUTED ETHYLSULFONE / SULFOXIMINE-PYRIDINE DERIVATIVES**

(71) Applicant: Boehringer Ingelheim Vetmedica GmbH, 55216 Ingelheim am Rhein (DE); Nihon Nohyaku Co., Ltd., Tokyo 104-8386 (JP)
(72) Inventor: KOOLMAN, Hannes Fiepko, 55216 Ingelheim am Rhein (DE); HERLÉ, Bart, 55216 Ingelheim am Rhein (DE)
(74) Representative: Lutze, Oliver

(57) **Abstract**

The present invention relates to new spirocycle-substituted ethyl-sulfon/sulfoximine-pyridine derivatives according to formula (I), (Ia) or (Ib), 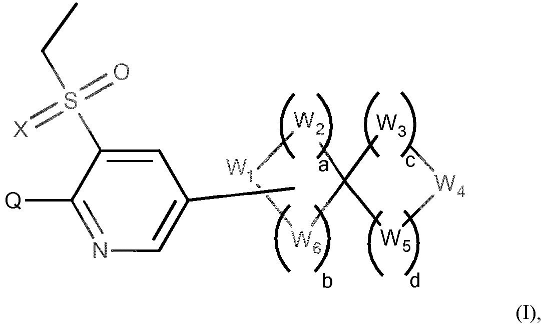 or 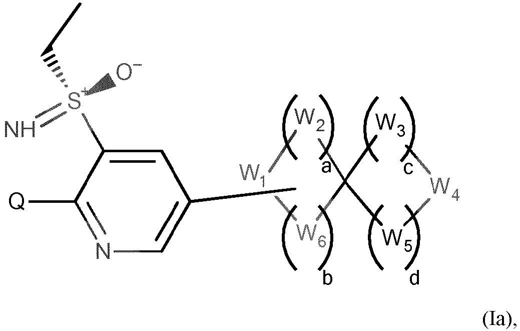 or 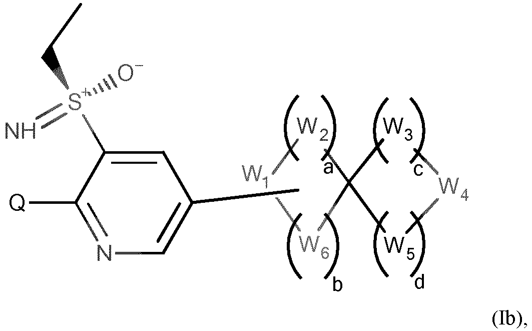 wherein the variables are as defined in the description and/or in the claims, which can be used as antiparasitic agents for the treatment, prevention and/or control of parasitic infections and/or infestations in/on animals as well as as crop protection agents.

## Description

### INCORPORATION BY REFERENCE

All references cited herein, are incorporated by reference herein, in their entirety.

### BACKGROUND OF THE INVENTION

### A. Field of the Invention

The present invention relates to the field of animal health and crop protection, in particular to new spirocycle-substituted ethyl-sulfon/sulfoximine-pyridine derivatives according to formula (I), (Ia) or (Ib) as antiparasitic and/or crop protection compounds as well as pharmaceutical compositions containing the same, and methods of using the same as antiparasitic agents for the treatment, prevention and/or control of parasitic infections and/or infestations in/on animals as well as as crop protection agents.

### B. Background and Description of the Related Art

Animals, such as mammals and birds are often susceptible to parasite infestations/infections. These parasites may be ectoparasites, such as insects and arachnids, and endoparasites such as filariae and other roundworms. Domesticated animals, such as cats and dogs, are often infested with one or more of the following ectoparasites: fleas (e.g. Ctenocephalides spp., such as Ctenocephalides felis and the like), ticks (e.g. Rhipicephalus spp., Ixodes spp., Dermacentor spp., Amblyoma spp., and the like), mites (e.g. Demodex spp., Sarcoptes spp., Otodectes spp., and the like), lice (e.g. Trichodectes spp., Cheyletiella spp., Linognathus spp. and the like), mosquitoes (Aedes spp., Culex spp., Anopheles spp. and the like) and flies (Hematobia spp., Musca spp., Stomoxys spp., Dematobia spp., Cocliomyia spp. and the like).

Fleas are a particular problem because not only do they adversely affect the health of the animal or human, but they also cause a great deal of psychological stress. Moreover, fleas are also vectors of pathogenic agents in animals and humans, such as dog tapeworm (Dipylidium caninum).

Similarly, ticks are also harmful to the physical and psychological health of the animal or human. However, the most serious problem associated with ticks is that they are the vector of pathogenic agents in both humans and animals. Major diseases which are caused by ticks include borrelioses (Lyme disease caused by Borrelia burgdorferi), babesioses (or piroplasmoses caused by Babesia spp.) and rickettsioses (also known as Rocky Mountain spotted fever). Ticks also release toxins which cause inflammation or paralysis in the host. Occasionally, these toxins are fatal to the host.

Likewise, farm animals are also susceptible to parasite infestations. For example, cattle are affected by a large number of parasites. A parasite which is very prevalent among farm animals is the tick genus Rhipicephalus, especially those of the species microplus (cattle tick), decoloratus and annulatus. Ticks, such as Rhipicephalus microplus (formerly Boophilus microplus), are particularly difficult to control because they live in the pasture where farm animals graze.

Currently available insecticidal and acaricidal treatments for animals do not always demonstrate good activity, good speed of action, or a long duration of action. Most treatments contain hazardous chemicals that can have serious consequences, including neurotoxicity and lethality from accidental ingestion. Persons applying these agents are generally advised to limit their exposure. Pet collars and tags have been utilized to overcome some problems, but these are susceptible to chewing, ingestion, and subsequent toxicological effects to the animal. Thus, current treatments achieve varying degrees of success, which depend partly on toxicity, method of administration, and efficacy. Additionally, some currently available agents are becoming ineffective due to parasitic resistance.

Further related art is as follows:

WO 2015/071180 discloses insecticidally active heterocyclic sulphur containing derivatives, to processes for their preparation, to compositions comprising those compounds, and to their use for controlling animal pests (including arthropods and in particular insects or representatives of the order Acarina).

WO 2016/030229 discloses pesticidally active heterocyclic derivatives with sulphur containing substituents.

WO 2016/039441 discloses agricultural and horticultural insecticide containing a novel imidazopyridazine compound or a salt thereof as an active ingredient, and a method for using the same.

WO 2016/071214 discloses polycyclic compounds and the agrochemically acceptable salts, stereoisomers, enantiomers, tautomers and N-oxides of those compounds, which can be used as insecticides and can be prepared in a manner known per se.

WO 2018/206348 discloses pesticidally active, in particular insecticidally active heterocyclic derivatives containing sulfur substituents, to processes for their preparation, to compositions comprising those compounds, and to their use for controlling animal pests, including arthropods and in particular insects or representatives of the order Acarina.

WO 2019/219689 discloses pesticidally active, in particular insecticidally active heterocyclic derivatives containing sulfoximine substituents, to processes for their preparation, to compositions comprising those compounds, and to their use for controlling animal pests, including arthropods and in particular insects or representatives of the order Acarina.

WO 2019/229089 discloses pesticidally active, in particular insecticidally active heterocyclic derivatives containing sulfur substituents, to processes for their preparation, to compositions comprising those compounds, and to their use for controlling animal pests, including arthropods and in particular insects or representatives of the order Acarina.

WO 2019/234158 discloses pesticidally active, in particular insecticidally active heterocyclic derivatives containing sulfoximine substituents, to processes for their preparation, to compositions comprising those compounds, and to their use for controlling animal pests, including arthropods and in particular insects or representatives of the order Acarina.

WO 2020/084075 discloses pesticidally active, in particular insecticidally active heterocyclic derivatives containing sulfoximine substituents, to processes for their preparation, to compositions comprising those compounds, and to their use for controlling animal pests, including arthropods and in particular insects or representatives of the order Acarina.

WO 2020/141136 discloses pesticidally active, in particular insecticidally active heterocyclic derivatives containing sulfur substituents, to processes for their preparation, to compositions comprising those compounds, and to their use for controlling animal pests, including arthropods and in particular insects or representatives of the order Acarina.

WO 2021/033141 discloses fused heterocyclic compounds as well as methods for their preparation and use of the heterocyclic compounds as a pest control agent.

WO 2021/085370 discloses azole derivatives or an agrochemically acceptable salt thereof and a pesticide characterized by comprising the same as an active ingredient.

WO 2021/204577 discloses imidazo-pyrimidone compounds as pesticides.

WO 2022/253841 discloses pesticidally active, in particular insecticidally active heterocyclic derivatives containing sulfoximine substituents, to processes for their preparation, to compositions comprising those compounds, and to their use for controlling animal pests, including arthropods and in particular insects or representatives of the order Acarina.

WO 2023/036934 discloses cyclopropyl-(hetero)aryl substituted ethylsulphonyl-pyridine derivatives, which can be used as antiparasitic agents for the treatment, prevention and/or control of parasitic infections and/or infestations in animals as well as crop protection agents.

WO 2024/175557 discloses cyclopropyl-(hetero)aryl substituted ethyl-sulfoximine-pyridine derivatives, which can be used as antiparasitic agents for the treatment, prevention and/or control of parasitic infections and/or infestations in/on animals as well as crop protection agents.

WO 2024/175558 discloses cyclopropyl-(hetero)aryl substituted ethyl-sulfone/sulfoximine-pyridine N-oxide derivatives, which can be used as antiparasitic agents for the treatment, prevention and/or control of parasitic infections and/or infestations in/on animals as well as crop protection agents.

WO 2024/175559 discloses tetrazole-substituted ethyl-sulfone/sulfoximine-pyridine derivatives, which can be used as antiparasitic agents for the treatment, prevention and/or control of parasitic infections and/or infestations in/on animals as well as crop protection agents.

Despite the availability of effective, broad spectrum antiparasitics, there remains a need for safer and more convenient, efficacious, and environmentally friendly products that will overcome the ever-present threat of resistance development. There is a need for improved antiparasitics, and in particular there is a need for improved insecticides and acaricides, particularly for use in animal health. Furthermore, there is a need for improved topical and oral products with convenient administration. Still further, there is a need for improved compositions which contains one or more active antiparasitics, which can be used to effectively treat against parasites. Such improvements would be particularly useful for the treatment of animals including companion animals (e.g., cats, dogs, llamas, and horses) and livestock (e.g., cattle, bison, swine, sheep, deer, elk, and goats).

Starting from the structurally closest prior art, i.e., compound "C-13" of WO 2021/204577, there is a need for improved antiparasitics that are for instance more potent/efficacious against in particular ectoparasites fleas and/or ticks.

### SUMMARY OF THE INVENTION

The present invention solves the problems inherent in the related art and provides a distinct advance in the state of the art.

In one aspect, the present invention relates to a compound of formula (I) or formula (Ia) or formula (Ib) 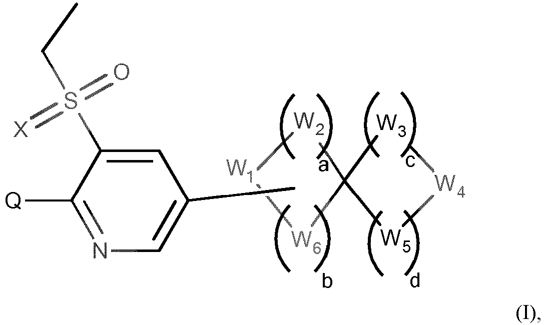 or 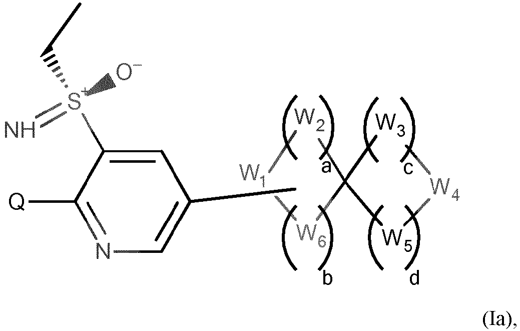 or 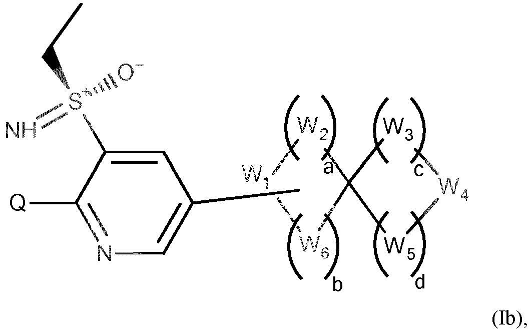 wherein:
X independently is O or NH, preferably O;
W₁ independently is C-Rₐ, if the central pyridine ring is attached via W₁, or C-R_{b}R_{c}, or N, if the central pyridine ring is attached via W₁, or N-R_{d};
W₂, W₆, if present, in each occurrence independently from each other are C-Rₑ, if the central pyridine ring is attached via a W₂ or a W₆, or C-R_{f}R_{g}, or N, if the central pyridine ring is attached via a W₂ or a W₆, or N-Rₕ;
W₃, W₅, if present, in each occurrence independently from each other are C-RᵢRⱼ, or (N=), or N-Rₖ, or O, or S(O)ᵤ, or S(O)ᵤ-R₁, or S(O)=N-Rₘ;
W₄ independently is C-RₙRₒ, or (N=), or N-Rₚ, or O, or S(O)ᵤ, or S(O)ᵤ-Rₚₚ, or S(O)=N-Rₚₚₚ;
a, b, c, d in each occurrence are independently from each other 0, 1, or 2, with the proviso that a+b and c+d independently from each other are 1, 2, 3 or 4;
u in each occurrence independently is 0, 1 or 2;
Rₐ, R_{b}, R_{c}, R_{d}, Rₑ, R_{f}, R_{g}, Rₕ, Rᵢ, Rⱼ, Rₖ, Rₗ, Rₘ, Rₙ, Rₒ, Rₚ, Rₚₚ, Rₚₚₚ, if present, in each occurrence independently from each other are hydrogen; halogen, such as F, Cl, Br, I; C₁-C₆-alkyl, such as CH₃; C₂-C₆-alkenyl, such as CH=CH₂; C₂-C₆-alkynyl, such as CCH; C₁-C₆-haloalkyl, such as CF₃, CHF₂, CH₂-CF₃, CH₂-CHF₂, CH₂-CH₂F, CF₂-CH₃, CH₂F, CF₂Cl, CF₂-CF₃, CF₂-CHF₂; C₂-C₆-haloalkenyl, such as CF=CH₂; C₂-C₆-haloalkynyl, such as CC-CHF₂; C(O)-(C₁-C₆-alkyl), such as C(O)-methyl; C(O)H; C(O)-(C₁-C₆-haloalkyl), such as C(O)-CF₂Cl; C(O)-(C₁-C₆-haloalkenyl), such as C(O)-CH=CHF; C(O)-(C₁-C₆-haloalkynyl), such as C(O)-CC-CHF₂; C(O)-N(Rₓ)(R_{y}), such as C(O)-NH₂; C(O)-N(CH₃)₂; C(O)-(C₃-C₈-cycloalkyl), such as C(O)-cyclopropyl; C(O)-C₃-C₈-cycloalkyl substituted with C₁-C₆-alkyl, such as C(O)-cyclopropyl-methyl; C(O)-C₃-C₈-cycloalkyl substituted with C₁-C₆-haloalkyl, such as C(O)-cyclopropyl-CH₂F; C(O)-C₃-C₈-cycloalkyl substituted with C₁-C₆-alkoxy, such as C(O)-cyclopropyl-OCH₃; C(O)-C₃-C₈-cycloalkyl substituted with C₁-C₆-haloalkoxy, such as C(O)-cyclopropyl-OCH₂F; C(S)-(C₁-C₆-alkyl), such as C(S)-methyl; C(S)-(C₁-C₆-haloalkyl), such as C(S)-CF₂Cl; C(S)-(C₁-C₆-haloalkenyl), such as C(S)-CH=CHF; C(S)-(C₁-C₆-haloalkynyl), such as C(S)-CC-CHF₂; C(S)-N(Rₓ)(R_{y}), such as C(S)-NH₂; C(S)-N(CH₃)₂; C(S)-(C₃-C₈-cycloalkyl), such as C(S)-cyclopropyl; C(S)-C₃-C₈-cycloalkyl substituted with C₁-C₆-alkyl, such as C(S)-cyclopropyl-methyl; C(S)-C₃-C₈-cycloalkyl substituted with C₁-C₆-haloalkyl, such as C(S)-cyclopropyl-CH₂F; C(S)-C₃-C₈-cycloalkyl substituted with C₁-C₆-alkoxy, such as C(S)-cyclopropyl-OCH₃; C(S)-C₃-C₈-cycloalkyl substituted with C₁-C₆-haloalkoxy, such as C(S)-cyclopropyl-OCH₂F; C₁-C₆-alkoxy, such as methoxy, ethoxy, propoxy; C₁-C₆-haloalkoxy, such as O-CF₃; C₃-C₈-cycloalkyl, such as cyclopropyl; (C₃-C₈-cycloalkyl)-OH, such as cyclopropyl-OH; (C₃-C₈-cycloalkyl)-CN, such as cyclopropyl-CN; (C₃-C₈-cycloalkyl)-C(O)ORᵥ; (C₃-C₈-cycloalkyl)-(C₁-C₆-haloalkyl); such as cyclopropyl-CH₂F; (C₃-C₈-cycloalkyl)-(C₁-C₆-haloalkenyl); such as cyclopropyl-CH=CHF; (C₃-C₈-cycloalkyl)-(C₁-C₆-haloalkynyl); such as cyclopropyl-CC-CHF₂; C₃-C₈-cycloalkoxy, such as cyclopropyloxy; C₃-C₈-cycloalkyl substituted with C₁-C₆-haloalkoxy, such as cyclopropyl-OCH₂F; OH; CN; C(O)ORᵥ; (C₁-C₆-alkyl)-OH, such as methyl-OH; (C₁-C₆-alkyl)-CN, such as methyl-CN; (C₁-C₆-alkyl)-C(O)OR_{y}; S(O)ᵣ-(C₁-C₆-alkyl), such as S-CH₃, S(O)-CH₃, S(O)₂-CH₃, S-ethyl, S(O)-ethyl, S(O)₂-ethyl; S(O)ₛ-(C₁-C₆-haloalkyl), such as S-CF₃, S(O)-CF₃, S(O)₂-CF₃; S(O)ₜ-R_{w}; S(O)₂-N(Rₓ)(R_{y}); S(O)-N(Rₓ)-C(O)-R_{y}, such as S(O)-NH-C(O)-CH₃; S(O)-(R_{w})=N-R_{z}; Si(Rₓ)(R_{y})(R_{z}); SH; SF₅; NO₂; N(Rₓ)(R_{y}), such as NH₂, NH-CH₃, N(CH₃)₂; S(O)(=N-Rₓ)-N(R_{y})(R_{z});
C-R_{b}R_{c}, C-R_{f}R_{g}, C-RᵢRⱼ, C-RₙRₒ in each occurrence independently from each other can also form C=O;
Q is selected from the group consisting of: Q1, Q2, Q3, wherein:
Q1 is an unsubstituted or substituted monocyclic, preferably 5- or 6-membered, heteroaryl or heterocyclyl containing one, two or three heteroatoms, which are each independently selected from the group consisting of N, O, S, S(O), S(O)₂; wherein preferably Q1 if substituted is substituted with one, two, three or four substituents each independently selected from the group consisting of: halogen, such as F, Cl, Br, I; C₁-C₆-alkyl, such as CH₃; C₂-C₆-alkenyl, such as CH=CH₂; C₂-C₆-alkynyl, such as CCH; C₁-C₆-haloalkyl, such as CF₃, CHF₂, CH₂-CF₃, CH₂-CHF₂, CH₂-CH₂F, CF₂-CH₃, CH₂F, CF₂Cl, CF₂-CF₃, CF₂-CHF₂; C₂-C₆-haloalkenyl, such as CF=CH₂; C₂-C₆-haloalkynyl, such as CC-CHF₂; C(O)-(C₁-C₆-alkyl), such as C(O)-methyl; C(O)H; C(O)-(C₁-C₆-haloalkyl), such as C(O)-CF₂Cl; C(O)-(C₁-C₆-haloalkenyl), such as C(O)-CH=CHF; C(O)-(C₁-C₆-haloalkynyl), such as C(O)-CC-CHF₂; C(O)-N(Rₓ)(R_{y}), such as C(O)-NH₂; C(O)-N(CH₃)₂; C(O)-(C₃-C₈-cycloalkyl), such as C(O)-cyclopropyl;
C(O)-C₃-C₈-cycloalkyl substituted with C₁-C₆-alkyl, such as C(O)-cyclopropyl-methyl; C(O)-C₃-C₈-cycloalkyl substituted with C₁-C₆-haloalkyl, such as C(O)-cyclopropyl-CH₂F; C(O)-C₃-C₈-cycloalkyl substituted with C₁-C₆-alkoxy, such as C(O)-cyclopropyl-OCH₃; C(O)-C₃-C₈-cycloalkyl substituted with C₁-C₆-haloalkoxy, such as C(O)-cyclopropyl-OCH₂F; C(S)-(C₁-C₆-alkyl), such as C(S)-methyl; C(S)-(C₁-C₆-haloalkyl), such as C(S)-CF₂Cl; C(S)-(C₁-C₆-haloalkenyl), such as C(S)-CH=CHF; C(S)-(C₁-C₆-haloalkynyl), such as C(S)-CC-CHF₂; C(S)-N(Rₓ)(R_{y}),
such as C(S)-NH₂; C(S)-N(CH₃)₂; C(S)-(C₃-C₈-cycloalkyl), such as C(S)-cyclopropyl; C(S)-C₃-C₈-cycloalkyl substituted with C₁-C₆-alkyl, such as C(S)-cyclopropyl-methyl; C(S)-C₃-C₈-cycloalkyl substituted with C₁-C₆-haloalkyl, such as C(S)-cyclopropyl-CH₂F; C(S)-C₃-C₈-cycloalkyl substituted with C₁-C₆-alkoxy, such as C(S)-cyclopropyl-OCH₃; C(S)-C₃-C₈-cycloalkyl substituted with C₁-C₆-haloalkoxy, such as C(S)-cyclopropyl-OCH₂F;
C₁-C₆-alkoxy, such as methoxy, ethoxy, propoxy; C₁-C₆-haloalkoxy, such as O-CF₃; C₃-C₈-cycloalkyl, such as cyclopropyl; (C₃-C₈-cycloalkyl)-OH, such as cyclopropyl-OH; (C₃-C₈-cycloalkyl)-CN, such as cyclopropyl-CN;
(C₃-C₈-cycloalkyl)-C(O)ORᵥ; (C₃-C₈-oalkyl)-(C₁-C₆-haloalkyl); such as cyclopropyl-CH₂F; (C₃-C₈-cycloalkyl)-(C₁-C₆-haloalkenyl); such as cyclopropyl-CH=CHF; (C₃-C₈-cycloalkyl)-(C₁-C₆-haloalkynyl); such as cyclopropyl-CC-CHF₂; C₃-C₈-cycloalkoxy, such as cyclopropyloxy; C₃-C₈-cycloalkyl substituted with C₁-C₆-haloalkoxy, such as cyclopropyl-OCH₂F; OH; CN; C(O)ORᵥ; (C₁-C₆-alkyl)-OH, such as methyl-OH; (C₁-C₆-alkyl)-CN, such as methyl-CN; (C₁-C₆-alkyl)-C(O)OR_{y}; S(O)ᵣ-(C₁-C₆-alkyl), such as S-CH₃, S(O)-CH₃, S(O)₂-CH₃, S-ethyl, S(O)-ethyl, S(O)₂-ethyl; S(O)ₛ-(C₁-C₆-haloalkyl), such as S-CF₃, S(O)-CF₃, S(O)₂-CF₃; S(O)ₜ-R_{w}; S(O)₂-N(Rₓ)(R_{y}); S(O)-N(Rₓ)-C(O)-R_{y}, such as S(O)-NH-C(O)-CH₃; S(O)-(R_{w})=N-R_{z}; Si(Rₓ)(R_{y})(R_{z}); SH; SF₅; NO₂; N(Rₓ)(R_{y}), such as NH₂, NH-CH₃, N(CH₃)₂; S(O)(=N-Rₓ)-N(R_{y})(R_{z});
Q2 is an unsubstituted or substituted fused bicyclic, preferably 9- or 10-membered, heteroaryl or heterocyclyl containing one, two, three, four or five heteroatoms, which are each independently selected from the group consisting of N, O, S, S(O), S(O)₂; wherein preferably Q2 if substituted is substituted with one, two, three, four, five or six substituents each independently selected from the group consisting of: halogen, such as F, Cl, Br, I; C₁-C₆-alkyl, such as CH₃; C₂-C₆-alkenyl, such as CH=CH₂; C₂-C₆-alkynyl, such as CCH; C₁-C₆-haloalkyl, such as CF₃, CHF₂, CH₂-CF₃, CH₂-CHF₂, CH₂-CH₂F, CF₂-CH₃, CH₂F, CF₂Cl, CF₂-CF₃, CF₂-CHF₂; C₂-C₆-haloalkenyl, such as CF=CH₂; C₂-C₆-haloalkynyl, such as CC-CHF₂; C(O)-(C₁-C₆-alkyl), such as C(O)-methyl; C(O)H; C(O)-(C₁-C₆-haloalkyl), such as C(O)-CF₂Cl; C(O)-(C₁-C₆-haloalkenyl), such as C(O)-CH=CHF; C(O)-(C₁-C₆-haloalkynyl), such as C(O)-CC-CHF₂; C(O)-N(Rₓ)(R_{y}), such as C(O)-NH₂; C(O)-N(CH₃)₂; C(O)-(C₃-C₈-cycloalkyl), such as C(O)-cyclopropyl; C(O)-C₃-C₈-cycloalkyl substituted with C₁-C₆-alkyl, such as C(O)-cyclopropyl-methyl; C(O)-C₃-C₈-cycloalkyl substituted with C₁-C₆-haloalkyl, such as C(O)-cyclopropyl-CH₂F; C(O)-C₃-C₈-cycloalkyl substituted with C₁-C₆-alkoxy, such as C(O)-cyclopropyl-OCH₃; C(O)-C₃-C₈-cycloalkyl substituted with C₁-C₆-haloalkoxy, such as C(O)-cyclopropyl-OCH₂F; C(S)-(C₁-C₆-alkyl), such as C(S)-methyl; C(S)-(C₁-C₆-haloalkyl), such as C(S)-CF₂Cl; C(S)-(C₁-C₆-haloalkenyl), such as C(S)-CH=CHF; C(S)-(C₁-C₆-haloalkynyl), such as C(S)-CC-CHF₂; C(S)-N(Rₓ)(R_{y}), such as C(S)-NH₂; C(S)-N(CH₃)₂; C(S)-(C₃-C₈-cycloalkyl), such as C(S)-cyclopropyl; C(S)-C₃-C₈-cycloalkyl substituted with C₁-C₆-alkyl, such as C(S)-cyclopropyl-methyl; C(S)-C₃-C₈-cycloalkyl substituted with C₁-C₆-haloalkyl, such as C(S)-cyclopropyl-CH₂F; C(S)-C₃-C₈-cycloalkyl substituted with C₁-C₆-alkoxy, such as C(S)-cyclopropyl-OCH₃; C(S)-C₃-C₈-cycloalkyl substituted with C₁-C₆-haloalkoxy, such as C(S)-cyclopropyl-OCH₂F; C₁-C₆-alkoxy, such as methoxy, ethoxy, propoxy; C₁-C₆-haloalkoxy, such as O-CF₃; C₃-C₈-cycloalkyl, such as cyclopropyl; (C₃-C₈-cycloalkyl)-OH, such as cyclopropyl-OH; (C₃-C₈-cycloalkyl)-CN, such as cyclopropyl-CN; (C₃-C₈-cycloalkyl)-C(O)ORᵥ; (C₃-C₈-cycloalkyl)-(C₁-C₆-haloalkyl); such as cyclopropyl-CH₂F; (C₃-C₈-cycloalkyl)-(C₁-C₆-haloalkenyl); such as cyclopropyl-CH=CHF; (C₃-C₈-cycloalkyl)-(C₁-C₆-haloalkynyl); such as cyclopropyl-CC-CHF₂; C₃-C₈-cycloalkoxy, such as cyclopropyloxy; C₃-C₈-cycloalkyl substituted with C₁-C₆-haloalkoxy, such as cyclopropyl-OCH₂F; OH; CN; C(O)ORᵥ; (C₁-C₆-alkyl)-OH, such as methyl-OH; (C₁-C₆-alkyl)-CN, such as methyl-CN; (C₁-C₆-alkyl)-C(O)OR_{y}; S(O)ᵣ-(C₁-C₆-alkyl), such as S-CH₃, S(O)-CH₃, S(O)₂-CH₃, S-ethyl, S(O)-ethyl, S(O)₂-ethyl; S(O)ₛ-(C₁-C₆-haloalkyl), such as S-CF₃, S(O)-CF₃, S(O)₂-CF₃; S(O)ₜ-R_{w}; S(O)₂-N(Rₓ)(R_{y}); S(O)-N(Rₓ)-C(O)-R_{y}, such as S(O)-NH-C(O)-CH₃; S(O)-(R_{w})=N-R_{z}; Si(Rₓ)(R_{y})(R_{z}); SH; SF₅; NO₂; N(Rₓ)(R_{y}), such as NH₂, NH-CH₃, N(CH₃)₂; S(O)(=N-Rₓ)-N(R_{y})(R_{z});
Q3 is an unsubstituted or substituted fused tricyclic, preferably 12- or 13-membered, heteroaryl or heterocyclyl containing one, two, three, four, five, six or seven heteroatoms, which are each independently selected from the group consisting of N, O, S, S(O), S(O)₂; wherein preferably Q3 if substituted is substituted with one, two, three, four, five, six, seven or eight substituents each independently selected from the group consisting of: halogen, such as F, Cl, Br, I; C₁-C₆-alkyl, such as CH₃; C₂-C₆-alkenyl, such as CH=CH₂; C₂-C₆-alkynyl, such as CCH; C₁-C₆-haloalkyl, such as CF₃, CHF₂, CH₂-CF₃, CH₂-CHF₂, CH₂-CH₂F, CF₂-CH₃, CH₂F, CF₂Cl, CF₂-CF₃, CF₂-CHF₂; C₂-C₆-haloalkenyl, such as CF=CH₂; C₂-C₆-haloalkynyl, such as CC-CHF₂; C(O)-(C₁-C₆-alkyl), such as C(O)-methyl; C(O)H; C(O)-(C₁-C₆-haloalkyl), such as C(O)-CF₂Cl; C(O)-(C₁-C₆-haloalkenyl), such as C(O)-CH=CHF; C(O)-(C₁-C₆-haloalkynyl), such as C(O)-CC-CHF₂; C(O)-N(Rₓ)(R_{y}), such as C(O)-NH₂; C(O)-N(CH₃)₂; C(O)-(C₃-C₈-cycloalkyl), such as C(O)-cyclopropyl; C(O)-C₃-C₈-cycloalkyl substituted with C₁-C₆-alkyl, such as C(O)-cyclopropyl-methyl; C(O)-C₃-C₈-cycloalkyl substituted with C₁-C₆-haloalkyl, such as C(O)-cyclopropyl-CH₂F; C(O)-C₃-C₈-cycloalkyl substituted with C₁-C₆-alkoxy, such as C(O)-cyclopropyl-OCH₃; C(O)-C₃-C₈-cycloalkyl substituted with C₁-C₆-haloalkoxy, such as C(O)-cyclopropyl-OCH₂F; C(S)-(C₁-C₆-alkyl), such as C(S)-methyl; C(S)-(C₁-C₆-haloalkyl), such as C(S)-CF₂Cl; C(S)-(C₁-C₆-haloalkenyl), such as C(S)-CH=CHF; C(S)-(C₁-C₆-haloalkynyl), such as C(S)-CC-CHF₂; C(S)-N(Rₓ)(R_{y}), such as C(S)-NH₂; C(S)-N(CH₃)₂; C(S)-(C₃-C₈-cycloalkyl), such as C(S)-cyclopropyl; C(S)-C₃-C₈-cycloalkyl substituted with C₁-C₆-alkyl, such as C(S)-cyclopropyl-methyl; C(S)-C₃-C₈-cycloalkyl substituted with C₁-C₆-haloalkyl, such as C(S)-cyclopropyl-CH₂F; C(S)-C₃-C₈-cycloalkyl substituted with C₁-C₆-alkoxy, such as C(S)-cyclopropyl-OCH₃; C(S)-C₃-C₈-cycloalkyl substituted with C₁-C₆-haloalkoxy, such as C(S)-cyclopropyl-OCH₂F; C₁-C₆-alkoxy, such as methoxy, ethoxy, propoxy; C₁-C₆-haloalkoxy, such as O-CF₃; C₃-C₈-cycloalkyl, such as cyclopropyl; (C₃-C₈-cycloalkyl)-OH, such as cyclopropyl-OH; (C₃-C₈-cycloalkyl)-CN, such as cyclopropyl-CN; (C₃-C₈-cycloalkyl)-C(O)ORᵥ; (C₃-C₈-cycloalkyl)-(C₁-C₆-haloalkyl); such as cyclopropyl-CH₂F; (C₃-C₈-cycloalkyl)-(C₁-C₆-haloalkenyl); such as cyclopropyl-CH=CHF; (C₃-C₈-cycloalkyl)-(C₁-C₆-haloalkynyl); such as cyclopropyl-CC-CHF₂; C₃-C₈-cycloalkoxy, such as cyclopropyloxy; C₃-C₈-cycloalkyl substituted with C₁-C₆-haloalkoxy, such as cyclopropyl-OCH₂F; OH; CN; C(O)ORᵥ; (C₁-C₆-alkyl)-OH, such as methyl-OH; (C₁-C₆-alkyl)-CN, such as methyl-CN; (C₁-C₆-alkyl)-C(O)OR_{y}; S(O)ᵣ-(C₁-C₆-alkyl), such as S-CH₃, S(O)-CH₃, S(O)₂-CH₃, S-ethyl, S(O)-ethyl, S(O)₂-ethyl; S(O)ₛ-(C₁-C₆-haloalkyl), such as S-CF₃, S(O)-CF₃, S(O)₂-CF₃; S(O)ₜ-R_{w}; S(O)₂-N(Rₓ)(R_{y}); S(O)-N(Rₓ)-C(O)-R_{y}, such as S(O)-NH-C(O)-CH₃; S(O)-(R_{w})=N-R_{z}; Si(Rₓ)(R_{y})(R_{z}); SH; SF₅; NO₂; N(Rₓ)(R_{y}), such as NH₂, NH-CH₃, N(CH₃)₂; S(O)(=N-Rₓ)-N(R_{y})(R_{z});
Rᵥ, R_{w}, Rₓ, R_{y}, R_{z} in each occurrence are independently from each other hydrogen; halogen, such as F, Cl, Br, I; C₁-C₆-alkyl, such as methyl, ethyl; C₂-C₆-alkenyl, such as CH=CH₂; C₂-C₆-alkynyl, such as CCH; C₁-C₆-haloalkyl, such as CF₃; CHF₂, CH₂-CF₃, CH₂-CHF₂, CH₂-CH₂F, CF₂-CH₃, CH₂F, CF₂Cl, CF₂-CF₃, CF₂-CHF₂; C₂-C₆-haloalkenyl, such as CF=CH₂; C₂-C₆-haloalkynyl, such as CC-CHF₂; C₁-C₆-haloalkyloxy, such as O-CF₃; C₃-C₈-cycloalkyl, such as cyclopropyl; (C₃-C₈-cycloalkyl)-OH, such as cyclopropyl-OH; (C₃-C₈-cycloalkyl)-CN, such as cyclopropyl-CN; (C₃-C₈-cycloalkyl)-C(O)OH; (C₃-C₈-cycloalkyl)-(C₁-C₆-haloalkyl); such as cyclopropyl-CH₂F; (C₃-C₈-cycloalkyl)-(C₁-C₆-haloalkenyl); such as cyclopropyl-CH=CHF; (C₃-C₈-cycloalkyl)-(C₁-C₆-haloalkynyl); such as cyclopropyl-CC-CHF₂; C₃-C₈-cycloalkoxy, such as cyclopropyloxy;
r, s, t in each occurrence independently from each other are 0, 1 or 2;
optionally, wherein at least one nitrogen atom is an N-oxide;
optionally, but preferably in case at least one nitrogen atom is an N-oxide, the at least one nitrogen atom being an N-oxide comprises the one of the central pyridine moiety;
optionally, but preferably in case at least one nitrogen atom is an N-oxide, the at least one nitrogen atom being an N-oxide is comprised within a six-membered heterocyclic moiety containing one or more nitrogen atoms of any one of Q1, Q2, Q3;
optionally, but preferably in case at least one nitrogen atom is an N-oxide, with the proviso that if X is present and is NH, the NH nitrogen atom is not an N-oxide;
optionally, but preferably in case at least one nitrogen atom is an N-oxide, with the further proviso that, if possible, and if any of Q1, Q2, Q3 is substituted, the at least one nitrogen atom being an N-oxide is not present in any potential substituents of any of Q1, Q2, Q3;
or a salt thereof.

In yet another aspect, the present invention also concerns a compound of formula (I), (Ia) or (Ib) as herein disclosed and/or claimed, wherein Q is Q1, Q2 or Q3, which independently are selected from the group consisting of: 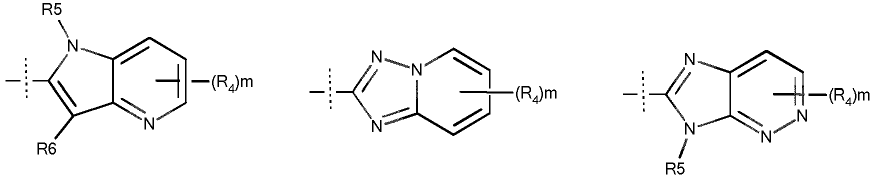 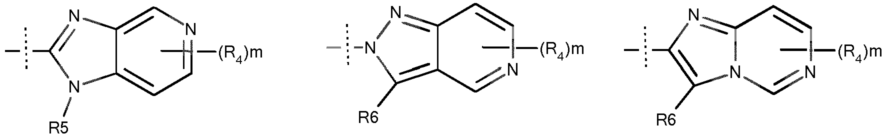 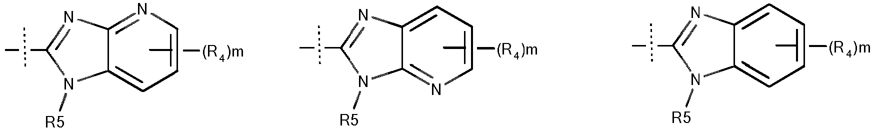 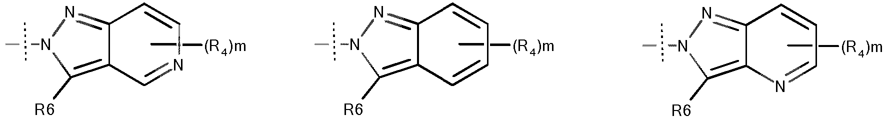 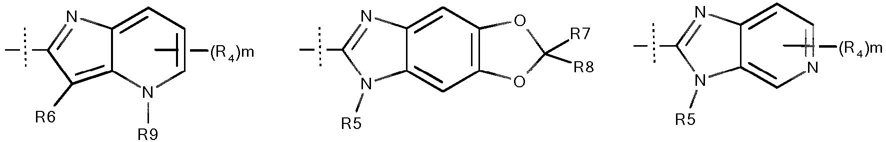 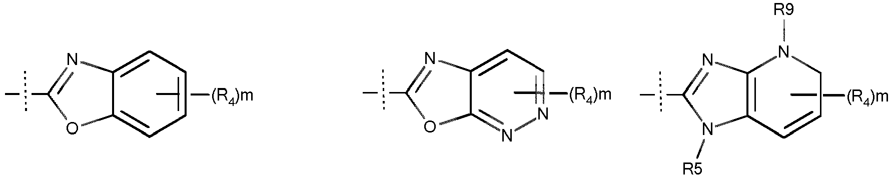 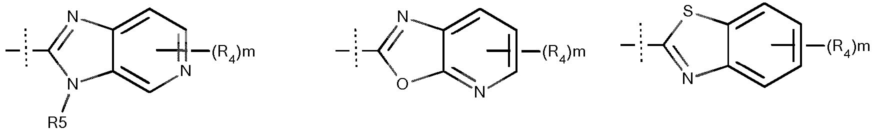 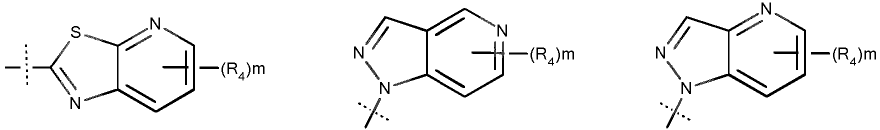 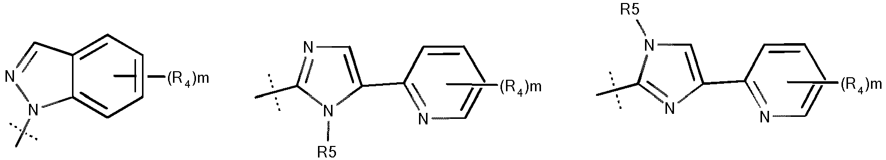 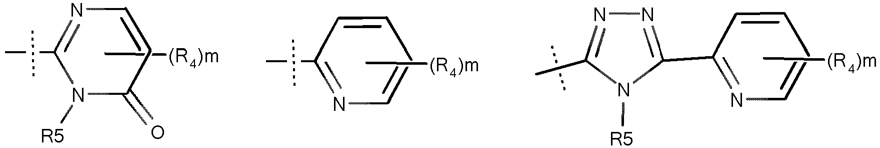 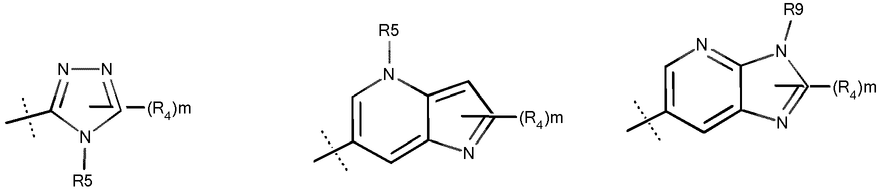 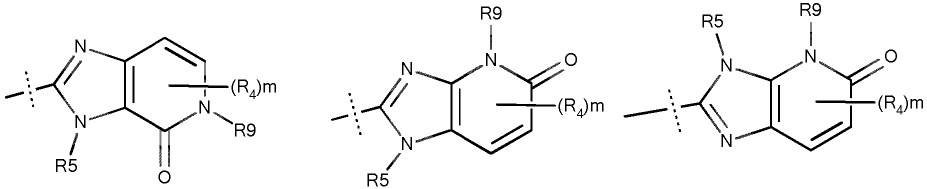 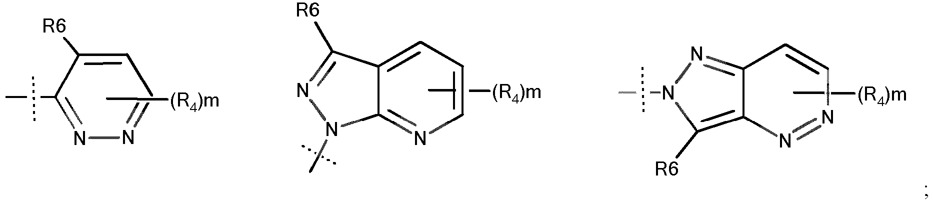 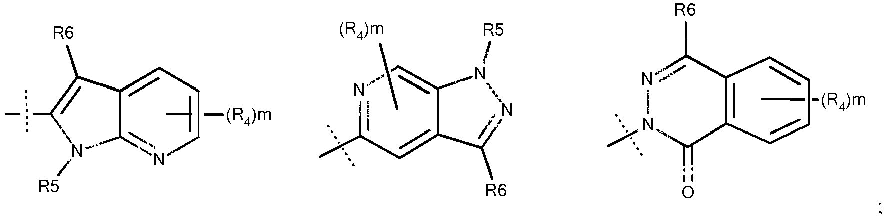 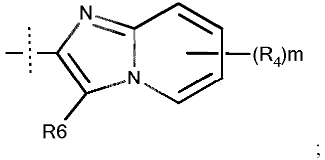 wherein:
R₄ in each occurrence independently is C₁-C₆-haloalkyl, such as CF₃, C₂F₅; CH₂-CF₂-CF₃; C₂-C₆-haloalkenyl, such as CH=CF-CF₃; C₁-C₆-haloalkoxy, such as O-CH₂-CF₃, O-CH₂-C₂F₅; S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; wherein R₄, if present once or more times, in each occurrence is independently attached to a C atom; and/or two R₄, if present, can form =O (provided that in such case there is no unsaturation at the corresponding ring C atom);
R5, R9 in each occurrence are independently hydrogen; C₁-C₆-alkyl, such as methyl, ethyl, isopropyl; C₂-C₆-alkenyl, such as ethenyl; C₁-C₆-haloalkyl, such as CF₃, CH₂-CHF₂, CH₂-CF₃, CHF₂, CH₂-CF₂-CF₃; C₃-C₈-cycloalkyl, such as cyclopropyl; C₃-C₈-cycloalkyl-alkyl, such as cyclopropyl-methyl; C₃-C₈-cycloalkyl substituted with one or more haloalkyl, such as cyclopropyl substituted with one or more CF₃; C₃-C₈-cycloalkyl substituted with one or more halogens, such as cyclopropyl substituted with one or more F; C(O)-(C₁-C₆-alkyl), such as C(O)-methyl; heterocyclyl, such as aziridinyl, oxetanyl; S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃;
R6, R7, R8 in each occurrence independently are hydrogen; halogen, such as F, Cl, Br, I;
m is 0, 1 or 2;
p, q in each occurrence independently are 0, 1 or 2;
or a salt thereof.

In yet another aspect, the present invention relates to a compound of formula (I) or formula (Ia) or formula (Ib) 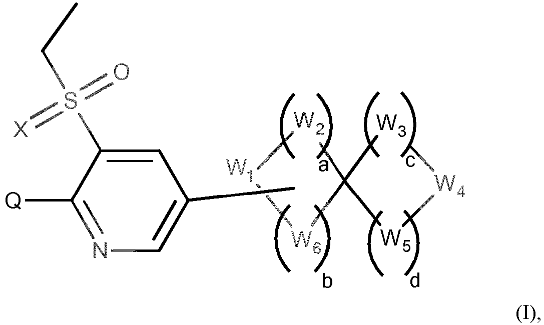 or 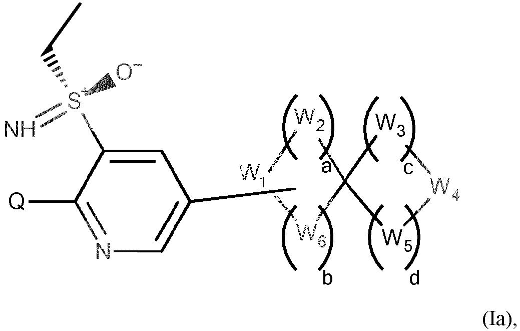 or 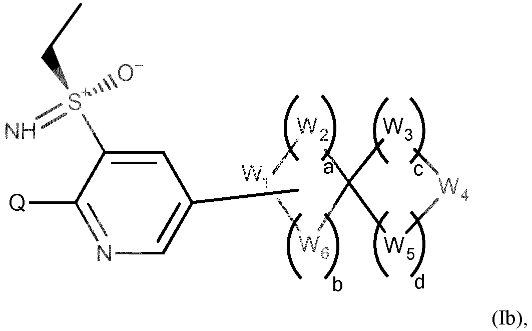 wherein:
X inpendently is O or NH, preferably O;
W₁ independently is C-Rₐ, if the central pyridine ring is attached via W₁, or C-R_{b}R_{c}, or N, if the central pyridine ring is attached via W₁, or N-Rₐ;
W₂, W₆, if present, in each occurrence independently from each other are C-Rₑ, if the central pyridine ring is attached via a W₂ or a W₆, or C-R_{f}R_{g}, or N, if the central pyridine ring is attached via a W₂ or a W₆, or N-Rₕ;
W₃, W₅, if present, in each occurrence independently from each other are C-RᵢRⱼ, or (N=), or N-Rₖ, or O, or S(O)ᵤ, or S(O)ᵤ-Rₗ, or S(O)=N-Rₘ;
W₄ independently is C-RₙRₒ, or (N=), or N-Rₚ, or O, or S(O)ᵤ, or S(O)ᵤ-Rₚₚ, or S(O)=N-Rₚₚₚ;
a, b, c, d in each occurrence are independently from each other 0, 1, or 2, with the proviso that a+b and c+d independently from each other are 1, 2, 3 or 4;
u in each occurrence independently is 0, 1 or 2;
Rₐ, R_{b}, R_{c}, R_{d}, Rₑ, R_{f}, R_{g}, Rₕ, Rᵢ, Rⱼ, Rₖ, Rₗ, Rₘ, Rₙ, Rₒ, Rₚ, Rₚₚ, Rₚₚₚ, if present, in each occurrence independently from each other are hydrogen; halogen, such as F, Cl, Br, I; C₁-C₆-alkyl, such as CH₃; C₂-C₆-alkenyl, such as CH=CH₂; C₂-C₆-alkynyl, such as CCH; C₁-C₆-haloalkyl, such as CF₃, CHF₂, CH₂-CF₃, CH₂-CHF₂, CH₂-CH₂F, CF₂-CH₃, CH₂F, CF₂Cl, CF₂-CF₃, CF₂-CHF₂; C₂-C₆-haloalkenyl, such as CF=CH₂; C₂-C₆-haloalkynyl, such as CC-CHF₂; C(O)-(C₁-C₆-alkyl), such as C(O)-methyl; C(O)H; C(O)-(C₁-C₆-haloalkyl), such as C(O)-CF₂Cl; C(O)-(C₁-C₆-haloalkenyl), such as C(O)-CH=CHF; C(O)-(C₁-C₆-haloalkynyl), such as C(O)-CC-CHF₂; C(O)-N(Rₓ)(R_{y}), such as C(O)-NH₂, C(O)-N(CH₃)₂; C(O)-(C₃-C₈-cycloalkyl), such as C(O)-cyclopropyl; C(O)-C₃-C₈-cycloalkyl substituted with C₁-C₆-alkyl, such as C(O)-cyclopropyl-methyl; C(O)-C₃-C₈-cycloalkyl substituted with C₁-C₆-haloalkyl, such as C(O)-cyclopropyl-CH₂F; C(O)-C₃-C₈-cycloalkyl substituted with C₁-C₆-alkoxy, such as C(O)-cyclopropyl-OCH₃; C(O)-C₃-C₈-cycloalkyl substituted with C₁-C₆-haloalkoxy, such as C(O)-cyclopropyl-OCH₂F; C(S)-(C₁-C₆-alkyl), such as C(S)-methyl; C(S)-(C₁-C₆-haloalkyl), such as C(S)-CF₂Cl; C(S)-(C₁-C₆-haloalkenyl), such as C(S)-CH=CHF; C(S)-(C₁-C₆-haloalkynyl), such as C(S)-CC-CHF₂; C(S)-N(Rₓ)(R_{y}), such as C(S)-NH₂, C(S)-N(CH₃)₂; C(S)-(C₃-C₈-cycloalkyl), such as C(S)-cyclopropyl; C(S)-C₃-C₈-cycloalkyl substituted with C₁-C₆-alkyl, such as C(S)-cyclopropyl-methyl; C(S)-C₃-C₈-cycloalkyl substituted with C₁-C₆-haloalkyl, such as C(S)-cyclopropyl-CH₂F; C(S)-C₃-C₈-cycloalkyl substituted with C₁-C₆-alkoxy, such as C(S)-cyclopropyl-OCH₃; C(S)-C₃-C₈-cycloalkyl substituted with C₁-C₆-haloalkoxy, such as C(S)-cyclopropyl-OCH₂F; C₁-C₆-alkoxy, such as methoxy, ethoxy, propoxy; C₁-C₆-haloalkoxy, such as O-CF₃; C₃-C₈-cycloalkyl, such as cyclopropyl; (C₃-C₈-cycloalkyl)-OH, such as cyclopropyl-OH; (C₃-C₈-cycloalkyl)-CN, such as cyclopropyl-CN; (C₃-C₈-cycloalkyl)-C(O)ORᵥ; (C₃-C₈-cycloalkyl)-(C₁-C₆-haloalkyl); such as cyclopropyl-CH₂F; (C₃-C₈-cycloalkyl)-(C₁-C₆-haloalkenyl); such as cyclopropyl-CH=CHF; (C₃-C₈-cycloalkyl)-(C₁-C₆-haloalkynyl); such as cyclopropyl-CC-CHF₂; C₃-C₈-cycloalkoxy, such as cyclopropyloxy; C₃-C₈-cycloalkyl substituted with C₁-C₆-haloalkoxy, such as cyclopropyl-OCH₂F; OH; CN; C(O)ORᵥ; (C₁-C₆-alkyl)-OH, such as methyl-OH; (C₁-C₆-alkyl)-CN, such as methyl-CN; (C₁-C₆-alkyl)-C(O)OR_{y}; S(O)ᵣ-(C₁-C₆-alkyl), such as S-CH₃, S(O)-CH₃, S(O)₂-CH₃, S-ethyl, S(O)-ethyl, S(O)₂-ethyl; S(O)ₛ-(C₁-C₆-haloalkyl), such as S-CF₃, S(O)-CF₃, S(O)₂-CF₃; S(O)ₜ-R_{w}; S(O)₂-N(Rₓ)(R_{y}); S(O)-(R_{w})=N-R_{z}; Si(Rₓ)(R_{y})(R_{z}); SH; SF₅; NO₂; N(Rₓ)(R_{y}), such as NH₂, NH-CH₃, N(CH₃)₂; S(O)(=N-Rₓ)-N(R_{y})(R_{z});
C-R_{b}R_{c}, C-R_{f}R_{g}, C-RᵢRⱼ, C-RₙRₒ in each occurrence independently from each other can form C=O;
r, s, t in each occurrence independently from each other are 0, 1 or 2;
Rᵥ, R_{w}, Rₓ, R_{y}, R_{z} in each occurrence are independently from each other hydrogen; halogen, such as F, Cl, Br, I; C₁-C₆-alkyl, such as methyl, ethyl; C₂-C₆-alkenyl, such as CH=CH₂; C₂-C₆-alkynyl, such as CCH; C₁-C₆-haloalkyl, such as CF₃; CHF₂, CH₂-CF₃, CH₂-CHF₂, CH₂-CH₂F, CF₂-CH₃, CH₂F, CF₂Cl, CF₂-CF₃, CF₂-CHF₂; C₂-C₆-haloalkenyl, such as CF=CH₂; C₂-C₆-haloalkynyl, such as CC-CHF₂; C₁-C₆-haloalkyloxy, such as O-CF₃; C₃-C₈-cycloalkyl, such as cyclopropyl; (C₃-C₈-cycloalkyl)-OH, such as cyclopropyl-OH; (C₃-C₈-cycloalkyl)-CN, such as cyclopropyl-CN; (C₃-C₈-cycloalkyl)-C(O)OH; (C₃-C₈-cycloalkyl)-(C₁-C₆-haloalkyl); such as cyclopropyl-CH₂F; (C₃-C₈-cycloalkyl)-(C₁-C₆-haloalkenyl); such as cyclopropyl-CH=CHF; (C₃-C₈-cycloalkyl)-(C₁-C₆-haloalkynyl); such as cyclopropyl-CC-CHF₂; C₃-C₈-cycloalkoxy, such as cyclopropyloxy;
optionally, wherein at least one nitrogen atom is an N-oxide;
optionally, but preferably in case at least one nitrogen atom is an N-oxide, the at least one nitrogen atom being an N-oxide comprises the one of the central pyridine moiety;
optionally, but preferably in case at least one nitrogen atom is an N-oxide, the at least one nitrogen atom being an N-oxide is comprised within a six-membered heterocyclic moiety containing one or more nitrogen atoms of any one of Q;
optionally, but preferably in case at least one nitrogen atom is an N-oxide, with the proviso that if X is present and is NH, the NH nitrogen atom is not an N-oxide;
optionally, but preferably in case at least one nitrogen atom is an N-oxide, with the further proviso that, if possible, and if any of Q is substituted, the at least one nitrogen atom being an N-oxide is not present in any potential substituents of any of Q;
   and 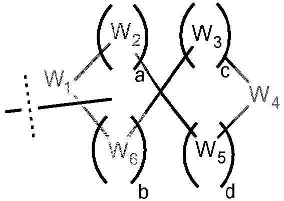 is 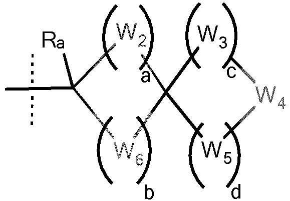 or 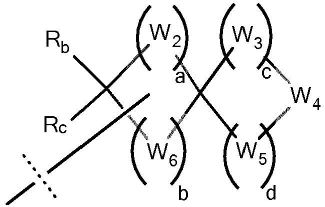 or 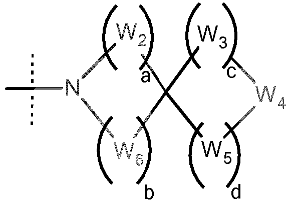 or 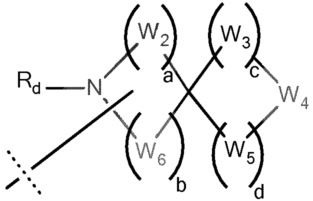 and
Q is 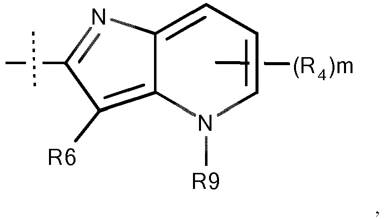
wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CHF₂" or "CF₂-CH₃";
R9 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, CH₂-CF₂-CF₃; C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R9 is "CHF₂" or "CH₂-CHF₂" or "ethyl" or "CH₂-cyclopropyl";
R6 is independently hydrogen, halogen, such as F, Cl, Br, I; in particular R6 is "hydrogen" or "F";
m is 0, 1 or 2, in particular m is 1;
p, q in each occurrence independently are 0, 1 or 2;
or Q is 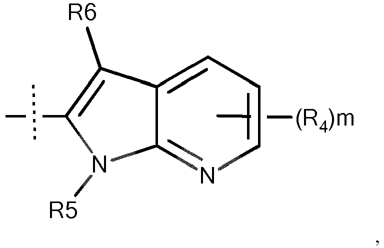
wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₂CF₃" or "CF₂-CH₃";
R5 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, CH₂-CF₂-CF₃; C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R5 is "hydrogen" or "methyl";
R6 is independently hydrogen, halogen, such as F, Cl, Br, I; in particular R6 is "hydrogen";
m is 0, 1 or 2, in particular m is 1;
p, q in each occurrence independently are 0, 1 or 2;
or Q is 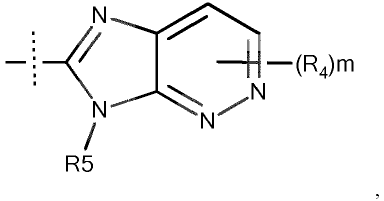
wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CHF₂" or "CF₂-cyclopropyl" or "CF₂-CF₃" or "CF₂-CH₂-CH₃" or "S-CF₃" or "S(O)₂-CF₃" or "CF₂-CH₃";
R5 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, CH₂-CF₂-CF₃; C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R5 is "methyl";
m is 0, 1 or 2, in particular m is 1;
p, q in each occurrence independently are 0, 1 or 2;
or Q is 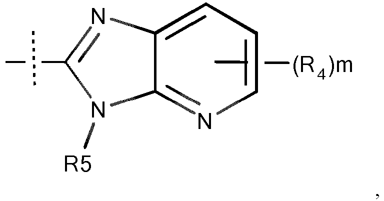
wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CHF₂" or "CF₂-CF₃" or "CF₂-cyclopropyl" or "CF₂-CH₂-CH₃" or "S(O)₂-CF₃" or "CF₂-CH₃";
R5 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, CH₂-CF₂-CF₃; C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl),
such as S-CF₃, S(O)-CF₃, S(O)₂-CF₃; in particular R5 is "methyl";
m is 0, 1 or 2, in particular m is 1;
p, q in each occurrence independently are 0, 1 or 2;
or Q is 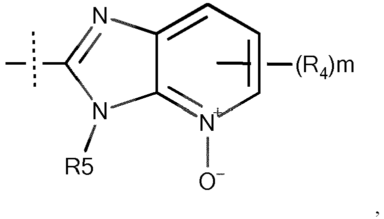
wherein:
R₄ in each occurrence is independently C₁-C₆-haloalkyl, such as CF₃, C₂F₅, C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl;
S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CHF₂" or "CF₂-CF₃" or "CF₂-cyclopropyl" or "CF₂-CH₂-CH₃" or "S(O)₂-CF₃" or "CF₂-CH₃";
R5 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, C₃-C₈-cycloalkyl, such as cyclopropyl, C₃-C₈-cycloalkylalkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R5 is "methyl";
m is 0, 1 or 2; in particular m is 1;
p, q in each occurrence independently are 0, 1 or 2;
or Q is 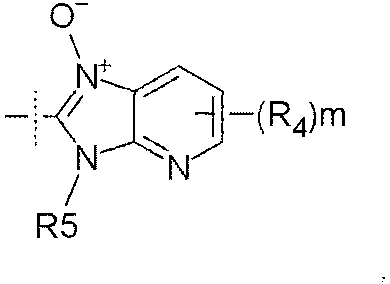
wherein:
R₄ in each occurrence is independently C₁-C₆-haloalkyl, such as CF₃, C₂F₅, C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CHF₂" or "CF₂-CF₃" or "CF₂-cyclopropyl" or "CF₂-CH₂-CH₃" or "S(O)₂-CF₃" or "CF₂-CH₃";
R5 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, C₃-C₈-cycloalkyl, such as cyclopropyl, C₃-C₈-cycloalkylalkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R5 is "methyl";
m is 0, 1 or 2; in particular m is 1;
p, q in each occurrence independently are 0, 1 or 2;
or Q is 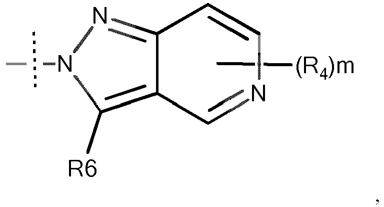
wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CF₂-CF₃" or "S-CF₃" or "S(O)-CF₃" or "S(O)₂-CF₃" or "CF₂-CH₃";
R6 is independently hydrogen, halogen, such as F, Cl, Br, I; in particular R6 is "hydrogen" or "F";
m is 0, 1 or 2, in particular m is 1;
p, q in each occurrence independently are 0, 1 or 2;
or wherein Q is 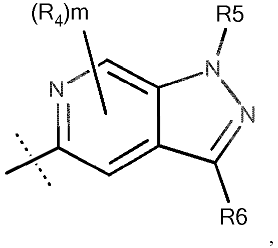
wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CF₂-CF₃" or "S-CF₃" or "S(O)-CF₃" or "S(O)₂-CF₃" or "CF₂-CH₃";
R5 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, CH₂-CF₂-CF₃; C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl),
such as S-CF₃, S(O)-CF₃, S(O)₂-CF₃; in particular R5 is "methyl" or "CH₂-CF₂-CF₃";
R6 is independently hydrogen, halogen, such as F, Cl, Br, I; in particular R6 is "hydrogen" or "F";
m is 0, 1 or 2, in particular m is 1;
p, q in each occurrence independently are 0, 1 or 2;
or Q is 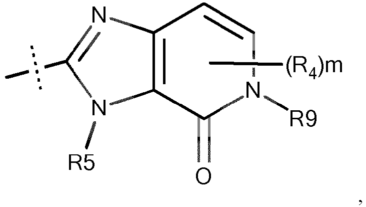
wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CHF₂" or "CF₂-CH₃";
R5 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, CH₂-CF₂-CF₃; C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R5 is "methyl" or "CH₂-CF₂-CF₃";
R9 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, CH₂-CF₂-CF₃; C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R9 is "CHF₂" or "CH₂-CHF₂" or "ethyl" or "cyclopropyl" or "CH₂-cyclopropyl";
R6 is independently hydrogen, halogen, such as F, Cl, Br, I; in particular R6 is "hydrogen" or "F";
m is 0, 1 or 2, in particular m is 1;
p, q in each occurrence independently are 0, 1 or 2;
or Q is 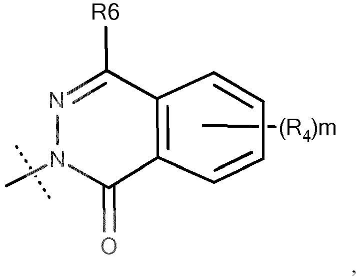
wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CF₂-CF₃" or "S-CF₃" or "S(O)-CF₃" or "S(O)₂-CF₃" or "CF₂-CH₃";
R6 is independently hydrogen, halogen, such as F, Cl, Br, I; in particular R6 is "hydrogen" or "F";
m is 0, 1 or 2, in particular m is 1;
p, q in each occurrence independently are 0, 1 or 2;
or Q is 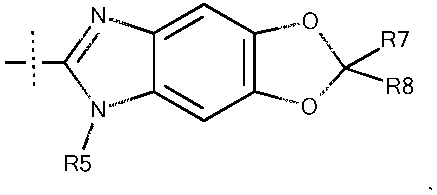
wherein:
R5 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, CH₂-CF₂-CF₃; C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R5 is "methyl" or "CH₂-CF₂-CF₃";
R7, R8 independently are hydrogen; halogen, such as F, Cl, Br, I; in particular R7, R8 are both "F";
p, q in each occurrence independently are 0, 1 or 2;
or Q is 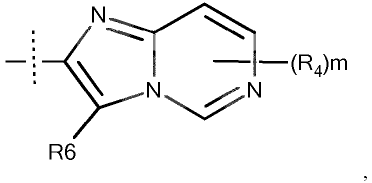
wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CF₂-CF₃" or "S-CF₃" or "S(O)-CF₃" or "S(O)₂-CF₃" or "CF₂-CH₃";
R6 is independently hydrogen, halogen, such as F, Cl, Br, I; in particular R6 is "hydrogen" or "F";
m is 0, 1 or 2, in particular m is 1;
p, q in each occurrence independently are 0, 1 or 2;
or Q is 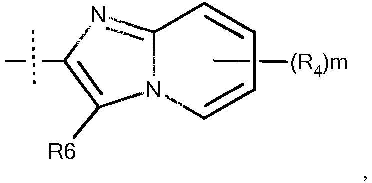
wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CF₂-CF₃" or "S-CF₃" or "S(O)-CF₃" or "S(O)₂-CF₃" or "CF₂-CH₃";
R6 is independently hydrogen, halogen, such as F, Cl, Br, I; in particular R6 is "hydrogen" or "F";
m is 0, 1 or 2, in particular m is 1;
p, q in each occurrence independently are 0, 1 or 2;
or Q is 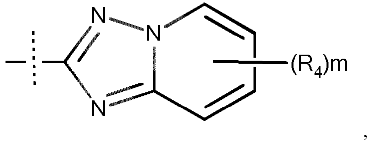
wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CF₂-CF₃" or "S-CF₃" or "S(O)-CF₃" or "S(O)₂-CF₃" or "CF₂-CH₃";
m is 0, 1 or 2, in particular m is 1;
p, q in each occurrence independently are 0, 1 or 2;
or Q is 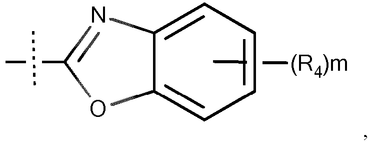
wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CF₂-CF₃" or "S-CF₃" or "S(O)-CF₃" or "S(O)₂-CF₃" or "CF₂-CH₃";
m is 0, 1 or 2, in particular m is 1;
p, q in each occurrence independently are 0, 1 or 2;
   and/or
wherein the optional at least one nitrogen atom being an N-oxide comprises the one of the central pyridine moiety selected from the group consisting of formula (II) or formula (IIa) or formula (IIb): 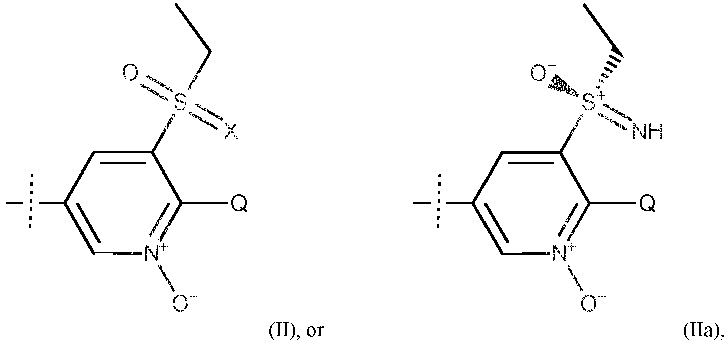 or 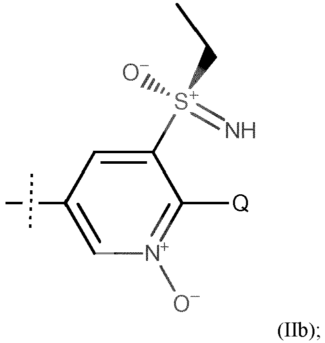
wherein X and Q independently are as defined as herein disclosed and/or claimed;
or a salt thereof.

In yet another aspect, the present invention further concerns a compound of formula (I), (Ia) or (Ib), preferably as herein disclosed and/or claimed, wherein the compound is selected from the group consisting of:
(R/S nomenclature was arbitrarily assigned according to SFC separation retention times, i.e. the enantiomer with the shorter SFC separation retention time was assigned "a", whereas the corresponding enantiomer with the long SFC separation retention time was assigned "b".)

| | |
|---|---|
| **1** | 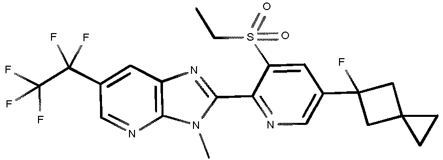 |
| **2** | 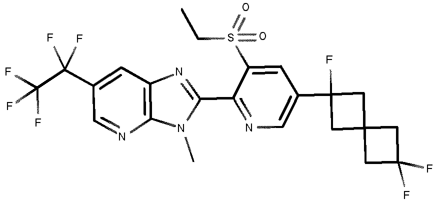 |
| **3** | 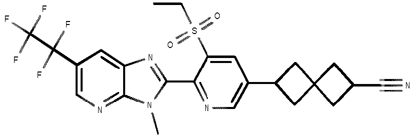 |
| **3a** | 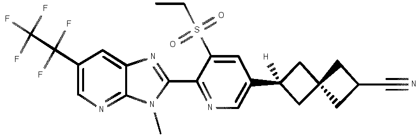 |
| **3b** | 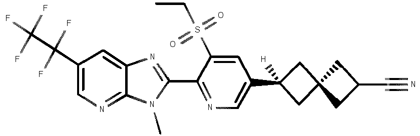 |
| **4** | 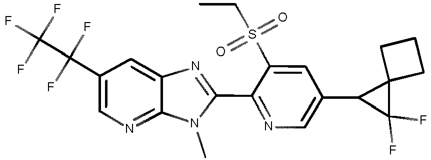 |
| **5** | 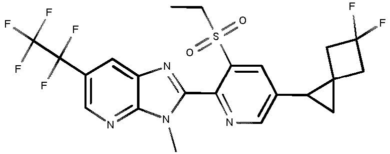 |
| **6** | 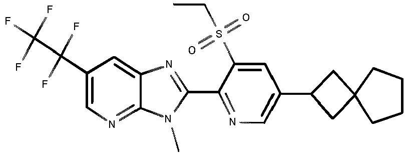 |
| **7** | 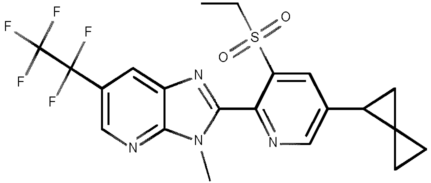 |
| **7a** | 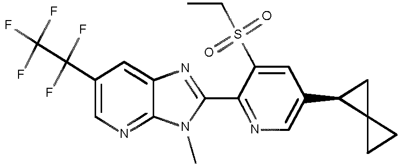 |
| **7b** | 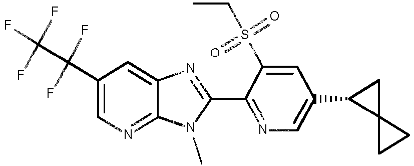 |
| **8** | 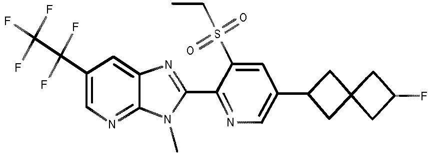 |
| **8a** | 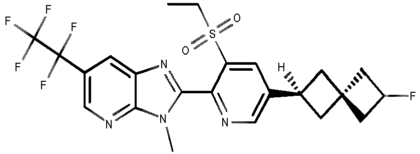 |
| **8b** | 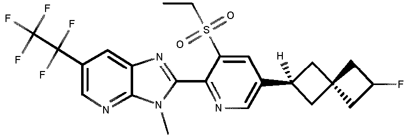 |
| **9** | 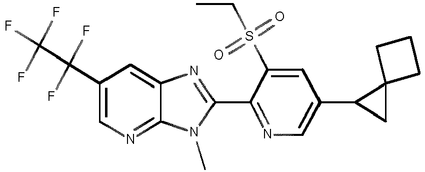 |
| **10** | 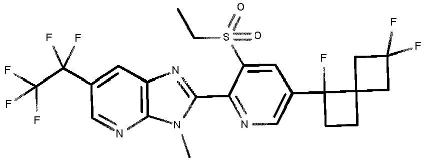 |
| **11** | 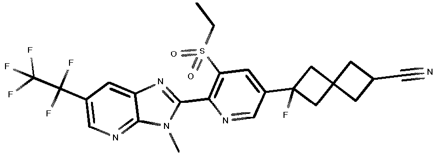 |
| **11a** | 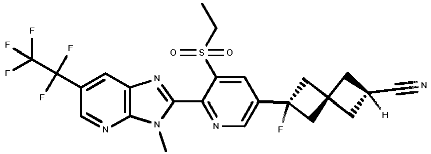 |
| **11b** | 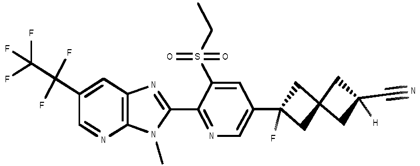 |
| **12** | 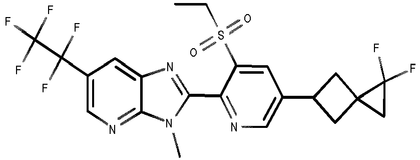 |
| **12a** | 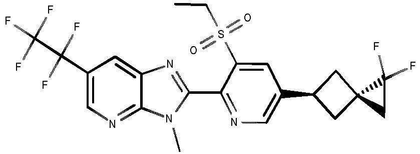 |
| **12b** | 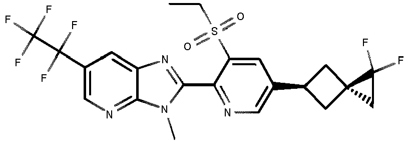 |
| **13** | 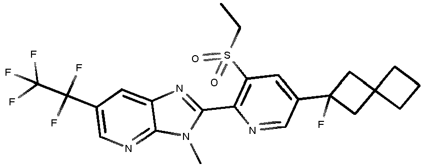 |
| **14** | 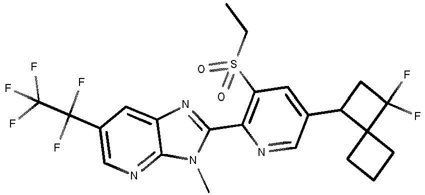 |
| **14a** | 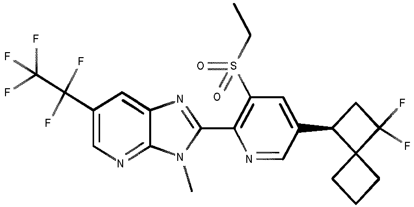 |
| **14b** | 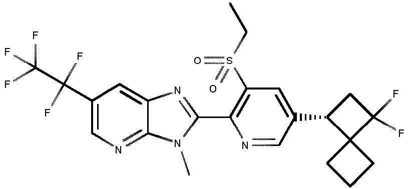 |
| **15** | 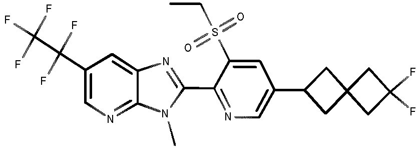 |
| **16** | 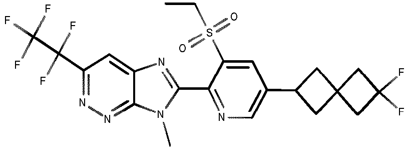 |
| **17** | 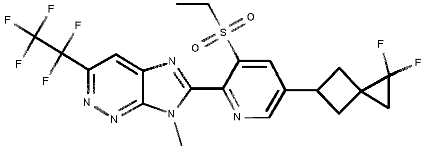 |
| **17a** | 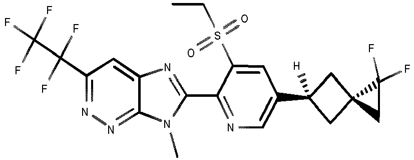 |
| **17b** | 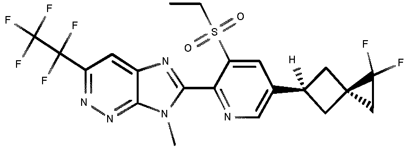 |
| **18** | 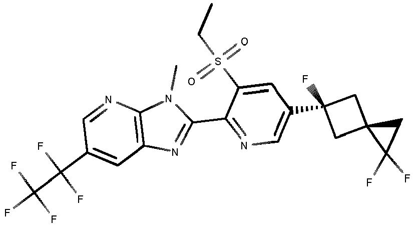 |
| **19** | 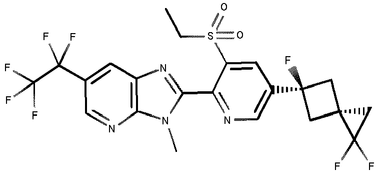 |
| **20** | 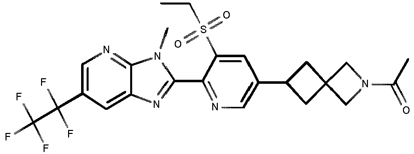 |
| **21** | 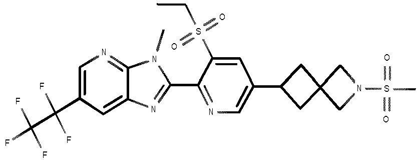 |
| **22** | 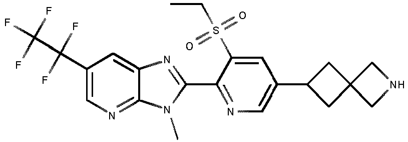 |
| **23** | 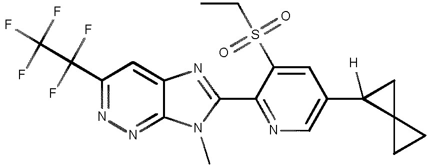 |
| **23a** | 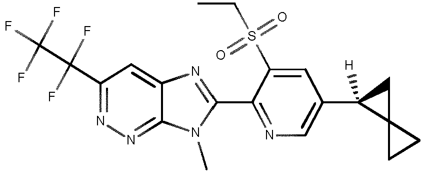 |
| **23b** | 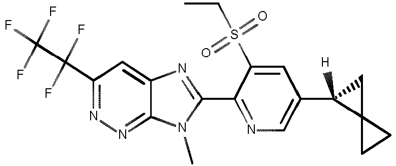 |
| **24** | 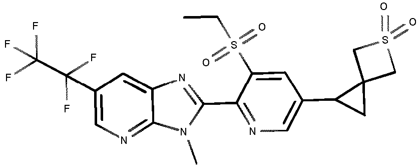 |
| **24a** | 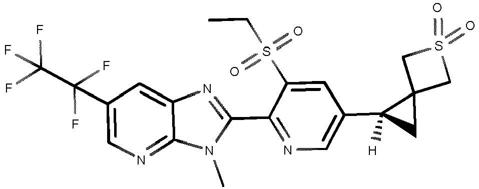 |
| **24b** | 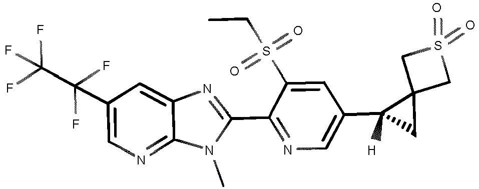 |
| **25** | 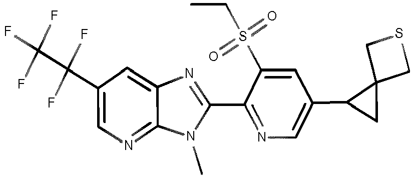 |
| **26** | 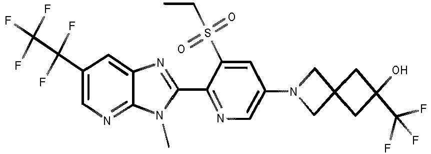 |
| **27** | 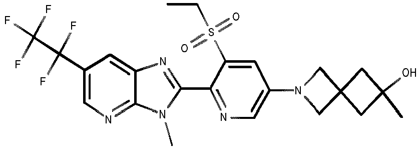 |
| **28** | 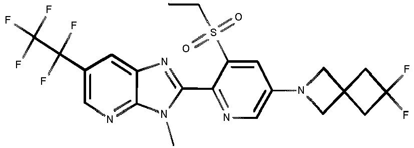 |
| **29** | 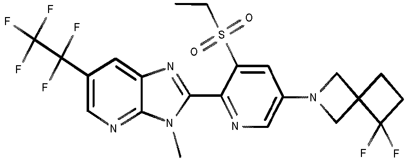 |
| **30** | 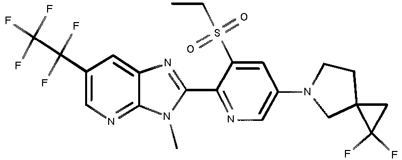 |
| **31** | 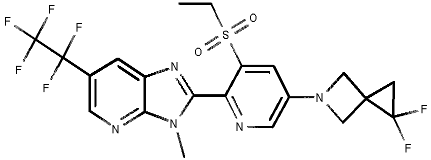 |
| **32** | 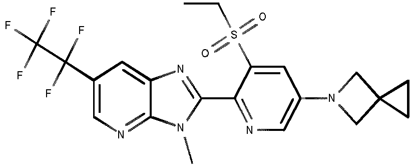 |
| **33** | 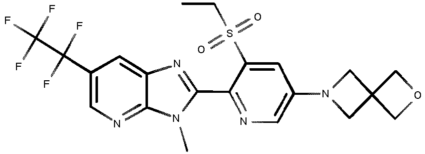 |
| **34** | 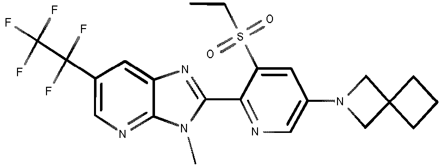 |
| **35** | 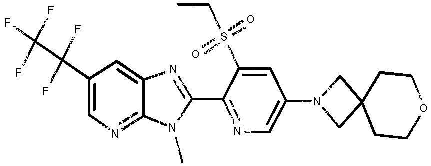 |
| **36** | 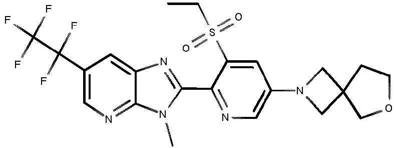 |
| **37** | 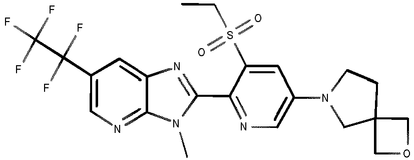 |
| **38** | 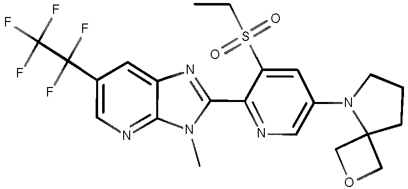 |
| **39** | 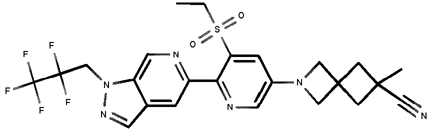 |
| **40** | 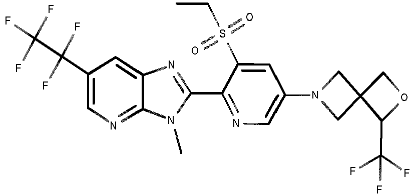 |
| **41** | 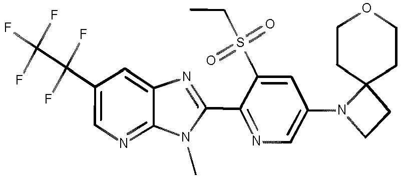 |
| **42** | 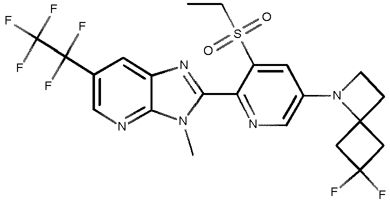 |
| **43** | 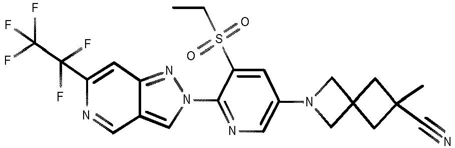 |
| **44** | 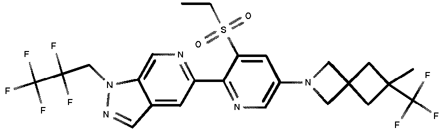 |
| **45** | 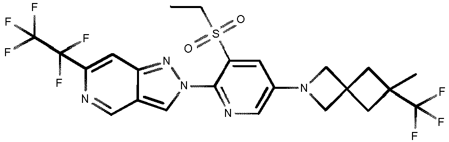 |
| **46** | 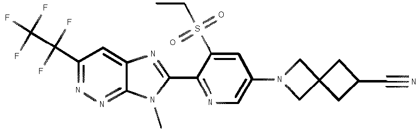 |
| **47** | 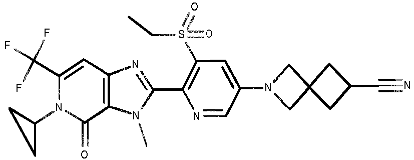 |
| **48** | 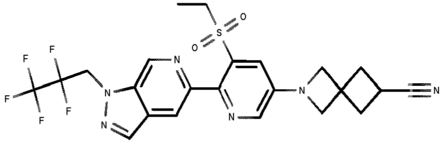 |
| **49** | 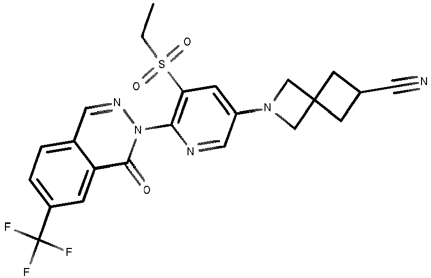 |
| **50** | 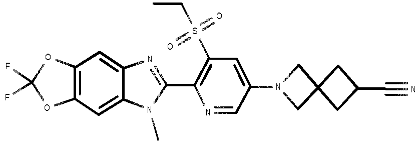 |
| **51** | 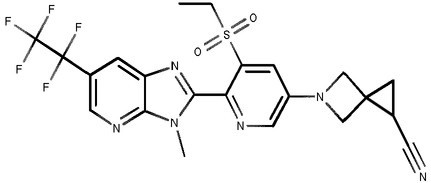 |
| **51a** | 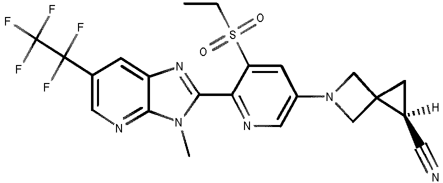 |
| **51b** | 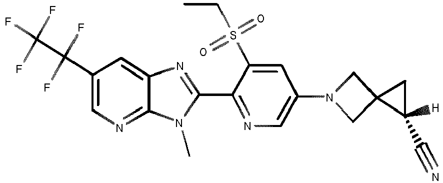 |
| **52** | 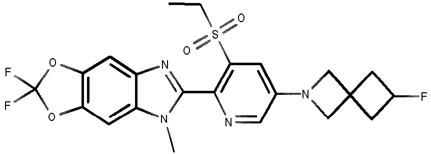 |
| **53** | 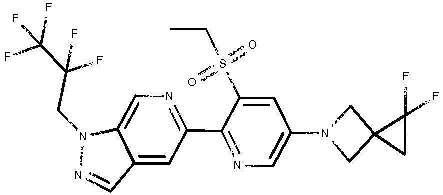 |
| **54** | 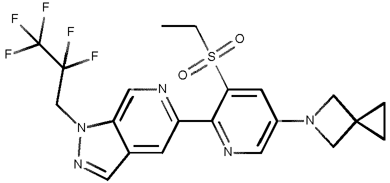 |
| **55** | 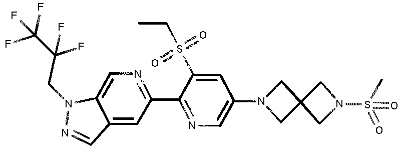 |
| **56** | 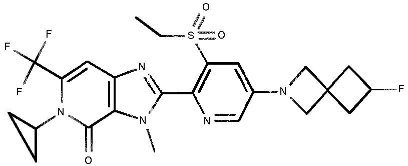 |
| **57** | 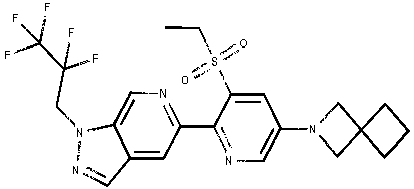 |
| **58** | 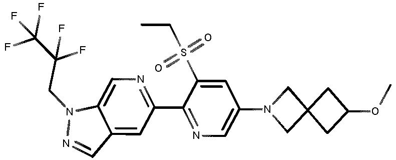 |
| **59** | 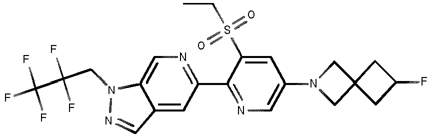 |
| **60** | 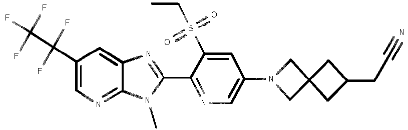 |
| **61** | 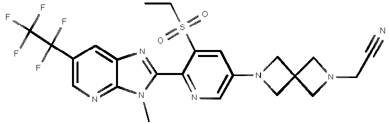 |
| **62** | 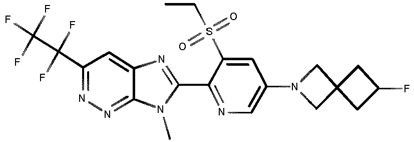 |
| **63** | 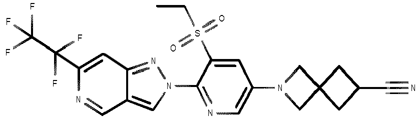 |
| **64** | 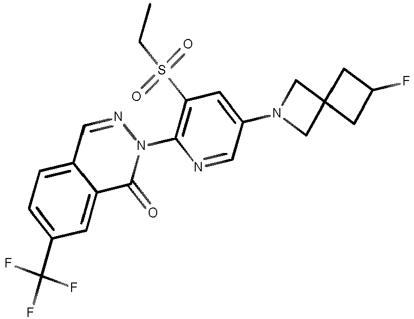 |
| **65** | 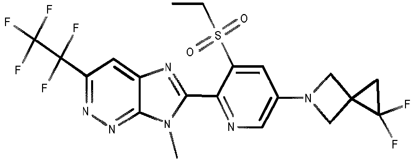 |
| **66** | 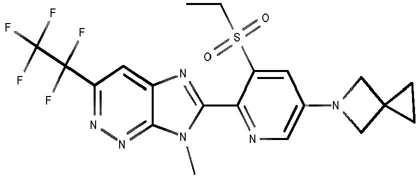 |
| **67** | 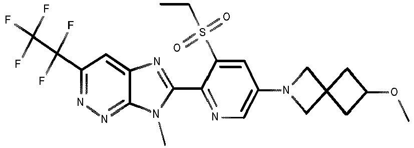 |
| **68** | 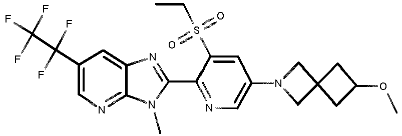 |
| **69** | 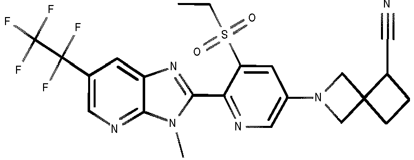 |
| **70** | 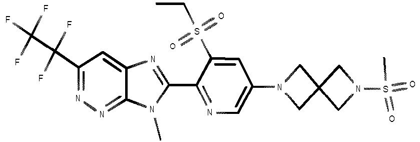 |
| **72** | 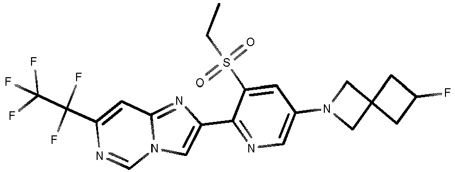 |
| **73** | 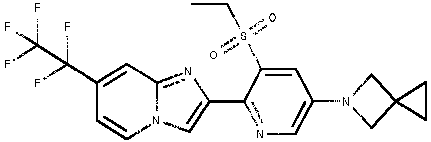 |
| **74** | 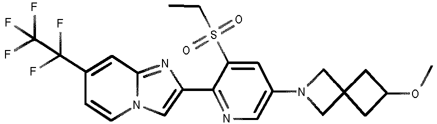 |
| **75** | 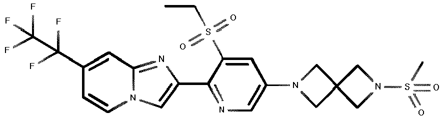 |
| **76** | 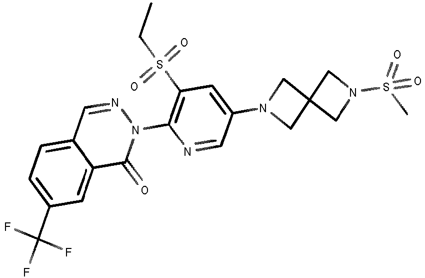 |
| **77** | 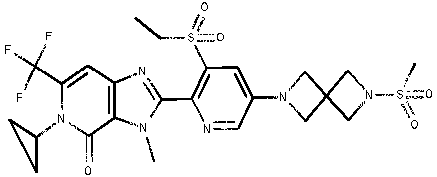 |
| **78** | 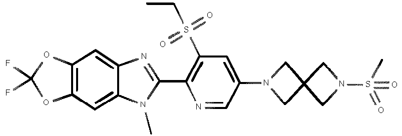 |
| **79** | 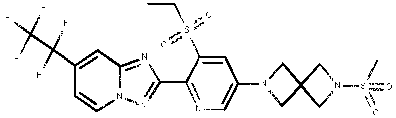 |
| **80** | 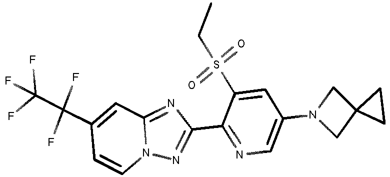 |
| **81** | 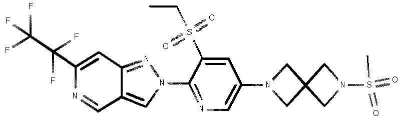 |
| **82** | 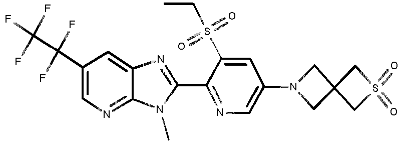 |
| **83** | 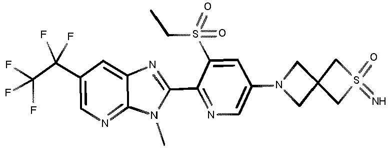 |
| **84** | 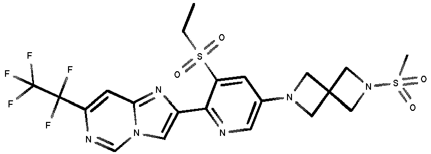 |
| **85** | 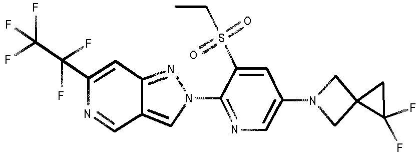 |
| **86** | 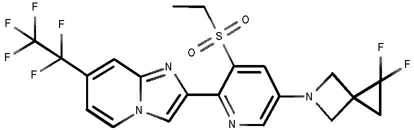 |
| **87** | 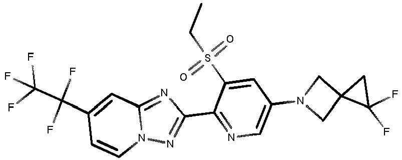 |
| **88** | 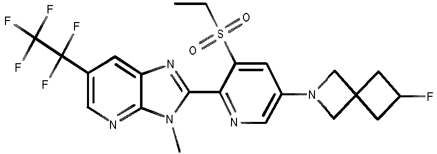 |
| **89** | 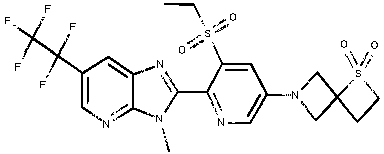 |
| **90** | 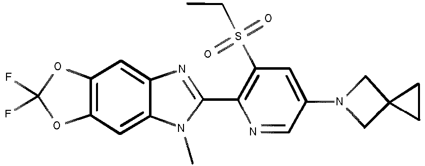 |
| **91** | 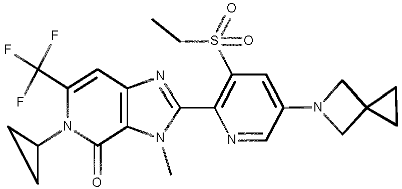 |
| **92** | 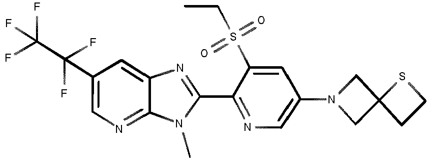 |
| **93** | 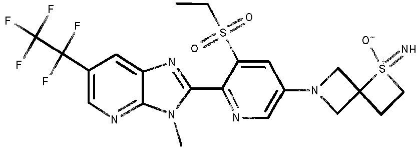 |
| **93a** | 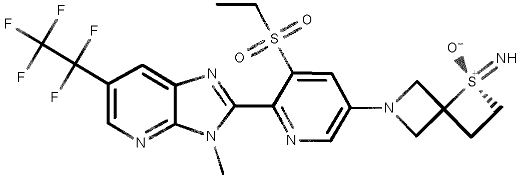 |
| **93b** | 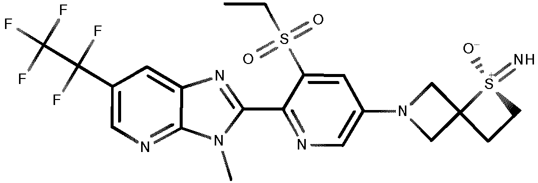 |
| **94** | 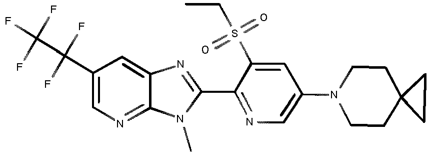 |
| **95** | 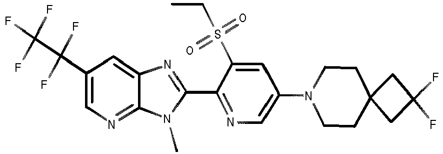 |
| **96** | 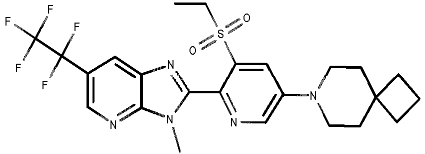 |
| **97** | 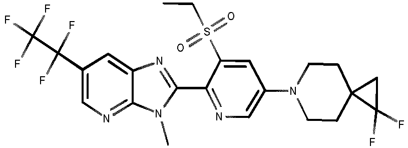 |
| **98** | 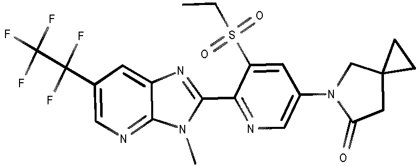 |
| **99** | 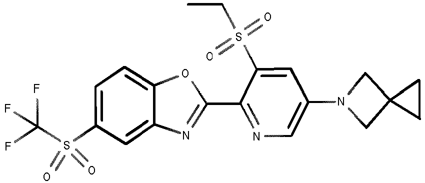 |
| **100** | 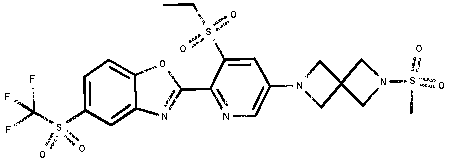 |
| **101** | 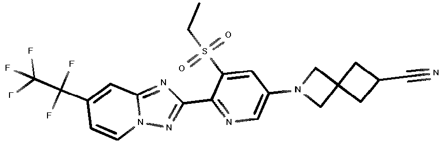 |
| **102** | 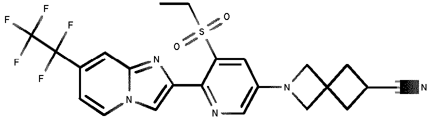 |
| **103** | 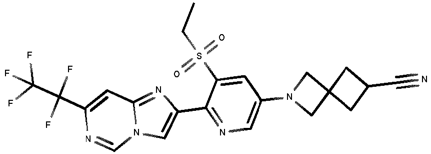 |
| **104** | 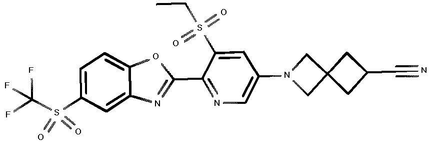 |
| **105** | 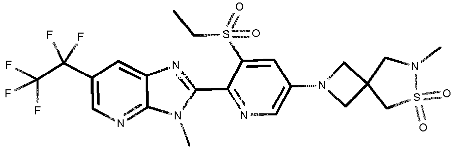 |
| **106** | 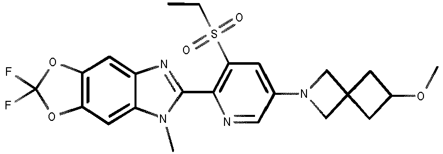 |
| **107** | 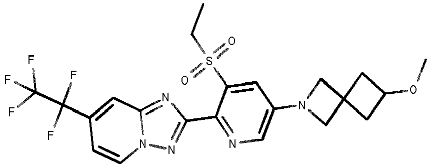 |
| **108** | 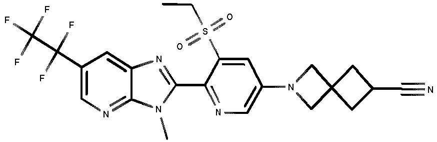 |
| **109** | 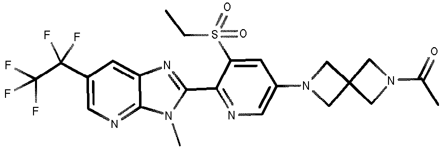 |
| **110** | 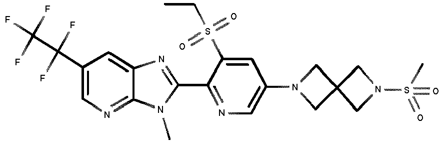 |
| **111** | 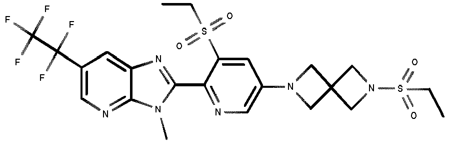 |
| **112** | 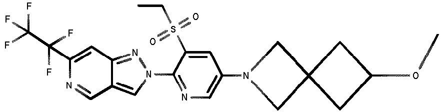 |
| **113** | 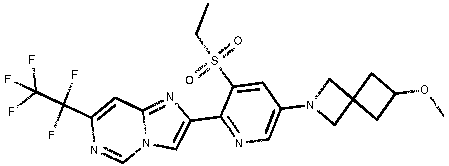 |
| **114** | 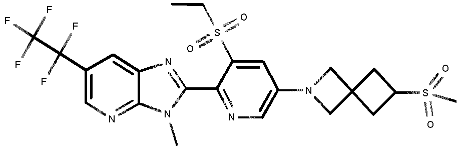 |
| **115** | 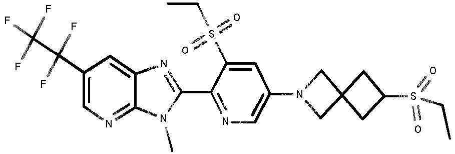 |
| **116** | 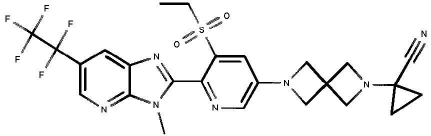 |
| **117** | 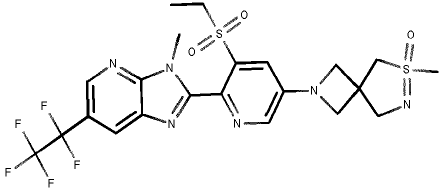 |
| **118** | 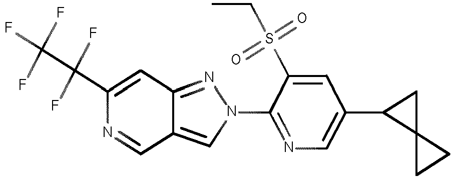 |
| **118a** | 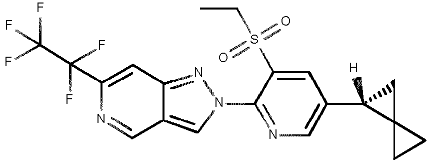 |
| **118b** | 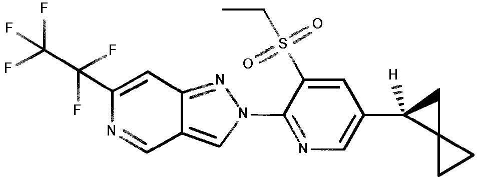 |
| **119** | 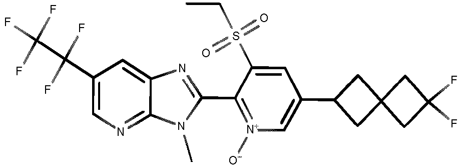 |
| **120** | 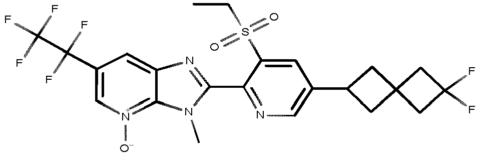 |
| **121** | 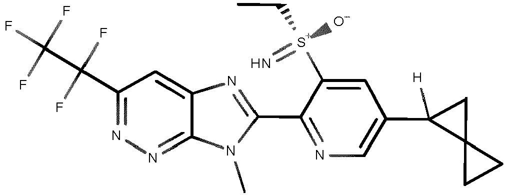 |
| **121a** | 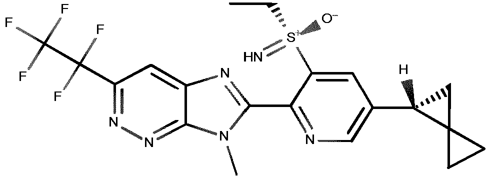 |
| **121b** | 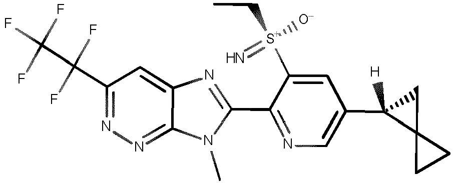 |
| **121c** | 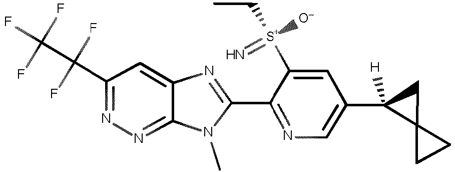 |
| **121d** | 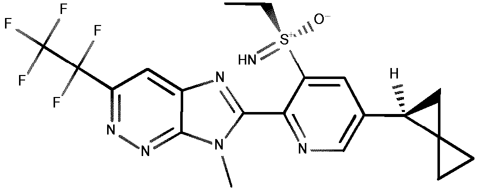 |
| **122** | 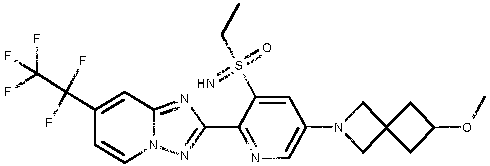 |
| **122a** | 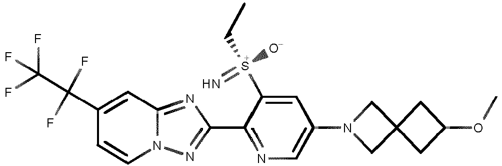 |
| **122b** | 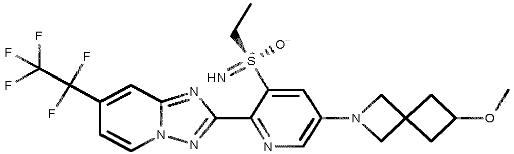 |

or a pharmaceutically acceptable salt thereof.

In yet another aspect, the present invention further concerns a pharmaceutical composition comprising one or more compound(s) of formula (I), (Ia) and/or (Ib) as herein disclosed and/or claimed or a pharmaceutically acceptable salt thereof and one or more pharmaceutically acceptable excipient(s).

In yet another aspect, the present invention further concerns a pharmaceutical composition consisting essentially of one or more compound(s) of formula (I), (Ia) and/or (Ib) as herein disclosed and/or claimed or a pharmaceutically acceptable salt thereof and one or more pharmaceutically acceptable excipient(s).

In yet another aspect, the present invention further concerns a pharmaceutical composition consisting of one or more compound(s) of formula (I), (Ia) and/or (Ib) as herein disclosed and/or claimed or a pharmaceutically acceptable salt thereof and one or more pharmaceutically acceptable excipient(s).

In yet another aspect, the present invention further concerns a pharmaceutical composition comprising one or more compound(s) of formula (I), (Ia) and/or (Ib) as herein disclosed and/or claimed or a pharmaceutically acceptable salt thereof, one or more additional pharmaceutically active agent(s), and one or more pharmaceutically acceptable excipient(s).

In yet another aspect, the present invention further concerns a pharmaceutical composition consisting essentially of one or more compound(s) of formula (I), (Ia) and/or (Ib) as herein disclosed and/or claimed or a pharmaceutically acceptable salt thereof, one or more additional pharmaceutically active agent(s), and one or more pharmaceutically acceptable excipient(s).

In yet another aspect, the present invention further concerns a pharmaceutical composition consisting of one or more compound(s) of formula (I), (Ia) and/or (Ib) as herein disclosed and/or claimed or a pharmaceutically acceptable salt thereof, one or more additional pharmaceutically active agent(s), and one or more pharmaceutically acceptable excipient(s).

In yet another aspect, the present invention further concerns a compound of formula (I), (Ia) or (Ib) as herein disclosed and/or claimed or a pharmaceutically acceptable salt thereof or a pharmaceutical composition as herein disclosed and/or claimed for use as a medicament, preferably for use as an antiparasitic medicament. A corresponding use of a compound of formula (I), (Ia) or (Ib) as herein disclosed and/or claimed or a pharmaceutically acceptable salt thereof or a pharmaceutical composition as herein disclosed and/or claimed for the preparation of a medicament, preferably an antiparasitic medicament, are also intended to be comprised by the present invention.

In yet another aspect, the present invention further concerns a compound of formula (I), (Ia) or (Ib) as herein disclosed and/or claimed or a pharmaceutically acceptable salt thereof or a pharmaceutical composition as herein disclosed and/or claimed for use in a method of treatment, prevention and/or control of a parasitic infection and/or infestation in/on an animal, preferably of an ectoparasitic infestation on an animal, more preferably of an infestation of fleas and/or ticks on an animal. A corresponding method of treatment, prevention and/or control of a parasitic infection and/or infestation in an animal, comprising administering an effective amount of compound of formula (I), (Ia) or (Ib) as herein disclosed and/or claimed or a pharmaceutically acceptable salt thereof or a pharmaceutical composition as herein disclosed and/or claimed to such animals, as well as the corresponding use of compound of formula (I), (Ia) or (Ib) as herein disclosed and/or claimed or a pharmaceutically acceptable salt thereof or a pharmaceutical composition as herein disclosed and/or claimed for the preparation of a medicament for the treatment, prevention and/or control of a parasitic infection and/or infestation in/on an animal, are also intended to be comprised by the present invention.

In yet another aspect, the present invention further concerns the use of a compound of formula (I), (Ia) or (Ib) as herein disclosed and/or claimed or a (pharmaceutically acceptable) salt thereof or a (pharmaceutical) composition comprising one or more compound(s) of formula (I), (Ia) or (Ib) as herein disclosed and/or claimed or a (pharmaceutically acceptable) salt thereof as a crop protection agent / for crop protection.

### DETAILED DESCRIPTION OF THE INVENTION

Generally, the present invention provides a compound of formula (I), (Ia) or (Ib) or a pharmaceutically acceptable salt thereof as herein disclosed and/or claimed as well as their corresponding pharmaceutical compositions, combinations and uses.

In a specific aspect, a compound of formula (I), (Ia) or (Ib) as herein disclosed and/or claimed is provided, wherein 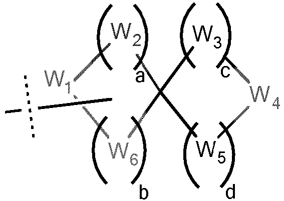 is independently selected from the group consisting of: 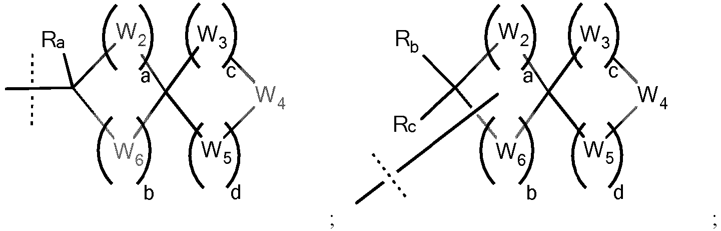 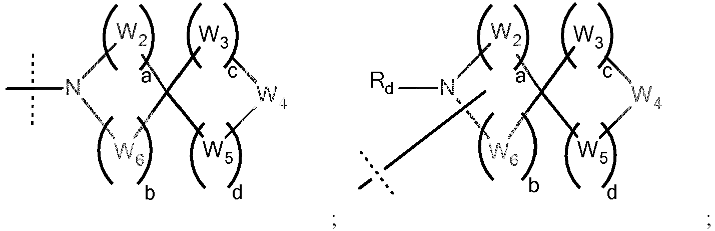 or a salt thereof.

In another specific aspect, a compound of formula (I), (Ia) or (Ib) as herein disclosed and/or claimed is provided, wherein Q is Q1, Q2 or Q3, which independently are selected from the group consisting of: 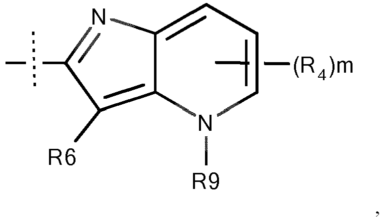 wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CHF₂" or "CF₂-CH₃";
R9 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, CH₂-CF₂-CF₃; C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R9 is "CHF₂" or "CH₂-CHF₂" or "ethyl" or "CH₂-cyclopropyl";
R6 is independently hydrogen, halogen, such as F, Cl, Br, I; in particular R6 is "hydrogen" or "F";
m is 0, 1 or 2, in particular m is 1;
p, q in each occurrence independently are 0, 1 or 2; 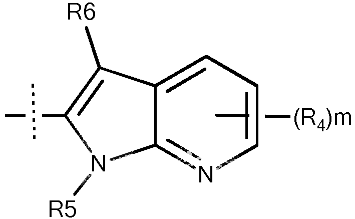 wherein:
   R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₂CF₃" or "CF₂-CH₃";
   R5 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, CH₂-CF₂-CF₃; C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl),
   such as S-CF₃, S(O)-CF₃, S(O)₂-CF₃; in particular R5 is "hydrogen" or "methyl";
   R6 is independently hydrogen, halogen, such as F, Cl, Br, I; in particular R6 is "hydrogen";
   m is 0, 1 or 2, in particular m is 1;
   p, q in each occurrence independently are 0, 1 or 2; 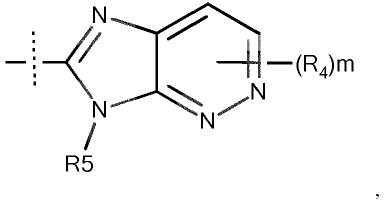 wherein:
      R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CHF₂" or "CF₂-cyclopropyl" or "CF₂-CF₃" or "CF₂-CH₂-CH₃" or "S-CF₃" or "S(O)₂-CF₃" or "CF₂-CH₃";
      R5 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, CH₂-CF₂-CF₃; C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R5 is "methyl";
      m is 0, 1 or 2, in particular m is 1;
      p, q in each occurrence independently are 0, 1 or 2; 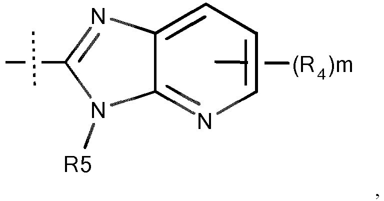 wherein:
         R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CHF₂" or "CF₂-CF₃" or "CF₂-cyclopropyl" or "CF₂-CH₂-CH₃" or "S(O)₂-CF₃" or "CF₂-CH₃";
         R5 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, CH₂-CF₂-CF₃; C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R5 is "methyl";
         m is 0, 1 or 2, in particular m is 1;
         p, q in each occurrence independently are 0, 1 or 2; 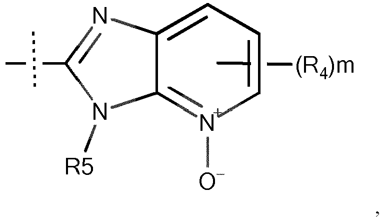 wherein:
            R₄ is independently C₁-C₆-haloalkyl, such as CF₃, C₂F₅, C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CHF₂" or "CF₂-CF₃" or "CF₂-cyclopropyl" or "CF₂-CH₂-CH₃" or "S(O)₂-CF₃" or "CF₂-CH₃";
            R5 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, C₃-C₈-cycloalkyl, such as cyclopropyl, C₃-C₈-cycloalkylalkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R5 is "methyl";
            m is 0, 1 or 2; in particular m is 1;
            p, q in each occurrence independently are 0, 1 or 2; 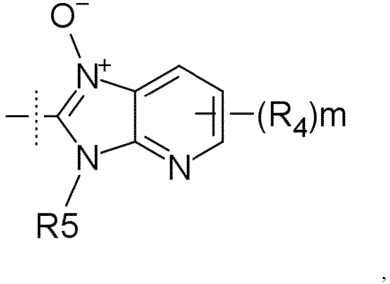 wherein:
               R₄ is independently C₁-C₆-haloalkyl, such as CF₃, C₂F₅, C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CHF₂" or "CF₂-CF₃" or "CF₂-cyclopropyl" or "CF₂-CH₂-CH₃" or "S(O)₂-CF₃" or "CF₂-CH₃";
               R5 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, C₃-C₈-cycloalkyl, such as cyclopropyl, C₃-C₈-cycloalkylalkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R5 is "methyl";
               m is 0, 1 or 2; in particular m is 1;
               p, q in each occurrence independently are 0, 1 or 2; 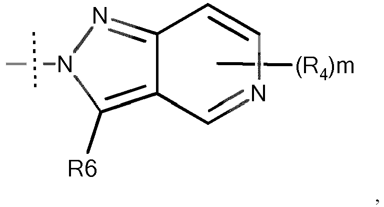 wherein:
                  R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CF₂-CF₃" or "S-CF₃" or "S(O)-CF₃" or "S(O)₂-CF₃" or "CF₂-CH₃";
                  R6 is independently hydrogen, halogen, such as F, Cl, Br, I; in particular R6 is "hydrogen" or "F";
                  m is 0, 1 or 2, in particular m is 1;
                  p, q in each occurrence independently are 0, 1 or 2; 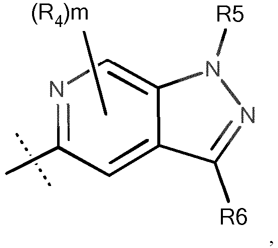 wherein:
                     R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CF₂-CF₃" or "S-CF₃" or "S(O)-CF₃" or "S(O)₂-CF₃" or "CF₂-CH₃";
                     R5 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, CH₂-CF₂-CF₃; C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R5 is "methyl" or "CH₂-CF₂-CF₃";
                     R6 is independently hydrogen, halogen, such as F, Cl, Br, I; in particular R6 is "hydrogen" or "F";
                     m is 0, 1 or 2, in particular m is 1;
                     p, q in each occurrence independently are 0, 1 or 2; 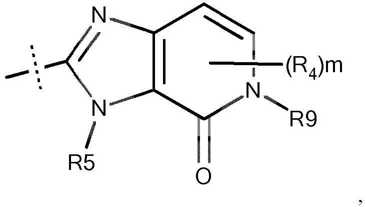 wherein:
                        R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CHF₂" or "CF₂-CH₃";
                        R5 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, CH₂-CF₂-CF₃; C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R5 is "methyl" or "CH₂-CF₂-CF₃";
                        R9 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, CH₂-CF₂-CF₃; C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R9 is "CHF₂" or "CH₂-CHF₂" or "ethyl" or "cyclopropyl" or "CH₂-cyclopropyl";
                        R6 is independently hydrogen, halogen, such as F, Cl, Br, I; in particular R6 is "hydrogen" or "F";
                        m is 0, 1 or 2, in particular m is 1;
                        p, q in each occurrence independently are 0, 1 or 2; 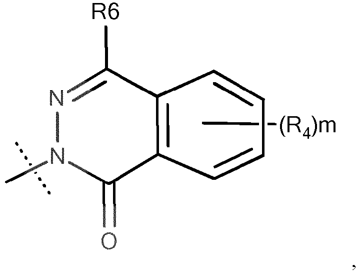 wherein:
                           R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CF₂-CF₃" or "S-CF₃" or "S(O)-CF₃" or "S(O)₂-CF₃" or "CF₂-CH₃";
                           R6 is independently hydrogen, halogen, such as F, Cl, Br, I; in particular R6 is "hydrogen" or "F";
                           m is 0, 1 or 2, in particular m is 1;
                           p, q in each occurrence independently are 0, 1 or 2; 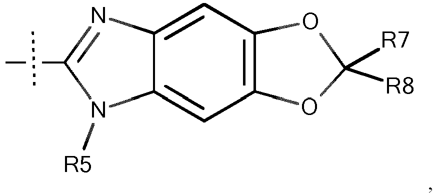 wherein:
                              R5 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, CH₂-CF₂-CF₃; C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R5 is "methyl" or "CH₂-CF₂-CF₃";
                              R7, R8 independently are hydrogen; halogen, such as F, Cl, Br, I; in particular R7, R8 are both "F";
                              p, q in each occurrence independently are 0, 1 or 2; 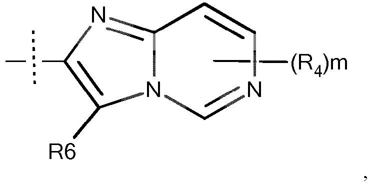 wherein:
                                 R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CF₂-CF₃" or "S-CF₃" or "S(O)-CF₃" or "S(O)₂-CF₃" or "CF₂-CH₃";
                                 R6 is independently hydrogen, halogen, such as F, Cl, Br, I; in particular R6 is "hydrogen" or "F";
                                 m is 0, 1 or 2, in particular m is 1;
                                 p, q in each occurrence independently are 0, 1 or 2; 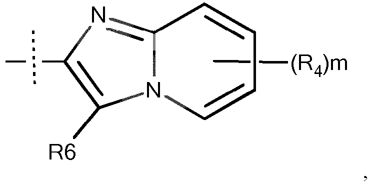 wherein:
                                    R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CF₂-CF₃" or "S-CF₃" or "S(O)-CF₃" or "S(O)₂-CF₃" or "CF₂-CH₃";
                                    R6 is independently hydrogen, halogen, such as F, Cl, Br, I; in particular R6 is "hydrogen" or "F";
                                    m is 0, 1 or 2, in particular m is 1;
                                    p, q in each occurrence independently are 0, 1 or 2; 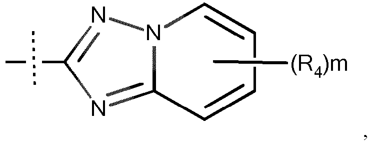 wherein:
                                       R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CF₂-CF₃" or "S-CF₃" or "S(O)-CF₃" or "S(O)₂-CF₃" or "CF₂-CH₃";
                                       m is 0, 1 or 2, in particular m is 1;
                                       p, q in each occurrence independently are 0, 1 or 2; 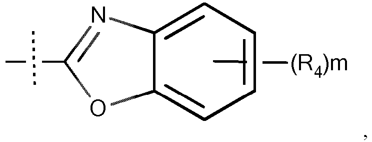 wherein:
                                          R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CF₂-CF₃" or "S-CF₃" or "S(O)-CF₃" or "S(O)₂-CF₃" or "CF₂-CH₃";
                                          m is 0, 1 or 2, in particular m is 1;
                                          p, q in each occurrence independently are 0, 1 or 2;
                                          or a salt thereof.

In yet another specific aspect, a compound of formula (I) or formula (Ia) or formula (Ib) as herein disclosed and/or claimed is provided, wherein the optional at least one nitrogen atom being an N-oxide comprises the one of the central pyridine moiety selected from the group consisting of formula (II) or formula (IIa) or formula (IIb): 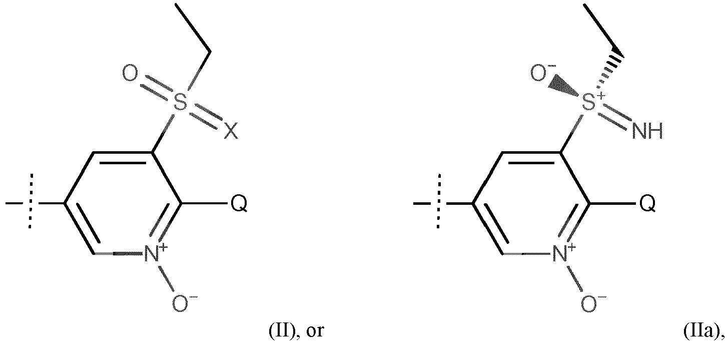 or 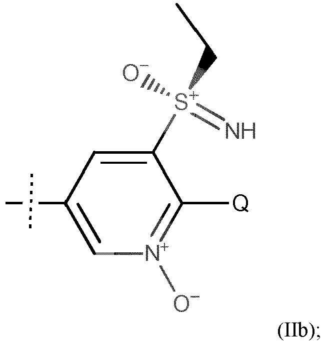 wherein X and Q independently are as defined as herein disclosed and/or claimed;
or a salt thereof.

### DEFINITIONS

Unless defined otherwise, all technical and scientific terms used herein have the same meaning as is commonly understood by one of skill in the art to which this invention belongs at the time of filing. Terms not specifically defined herein should be given the meanings that would be given to them by one of skill in the art in light of the disclosure and the context. The meaning and scope of terms should be clear; however, in the event of any latent ambiguity, definitions provided herein take precedent over any dictionary or extrinsic definition. Further, unless otherwise required by context, singular terms shall include pluralities and plural terms shall include the singular. Herein, the use of "or" means "and/or" unless stated otherwise. Furthermore, the use of the term "including", as well as other forms such as "includes" and "included" is not limiting. As used in the specification, however, unless specified to the contrary, the following terms have the meaning indicated and the following conventions are adhered to.

In the groups, radicals, or moieties defined herein, the number of carbon atoms is often specified preceding the group, for example, C₁₋₆-alkyl means an alkyl group or radical having 1 to 6 carbon atoms. In general, in groups like HO, H₂N, (O)S, (O)₂S, NC (cyano), HOOC, F₃C or the like, the skilled artisan can see the radical attachment point(s) to the molecule from the free valences of the group itself. For combined groups comprising two or more subgroups, the last-named subgroup is the radical attachment point, for example, the substituent "aryl-C₁₋₃-alkyl" means an aryl group which is bound to a C₁₋₃-alkyl-group, the latter of which is bound to the core or to the group to which the substituent is attached.

In case a compound of the present invention is depicted in the form of a chemical name and as a formula, in case of any discrepancy the formula shall prevail. An asterisk may be used in sub-formulas to indicate the bond which is connected to the core molecule as defined.

The numeration of the atoms of a substituent starts with the atom which is closest to the core or to the group to which the substituent is attached. For example, the term "3-carboxypropyl-group" represents the following substituent: 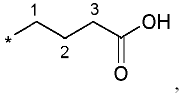 wherein the carboxy group is attached to the third carbon atom of the propyl group. The terms "1-methylpropyl-", "2,2-dimethylpropyl-" or "cyclopropylmethyl-" group represent the following groups: 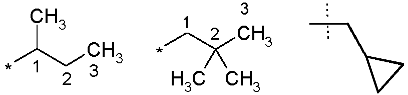 The asterisk or 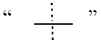 may be used in sub-formulas to indicate the bond which is connected to the core molecule as defined.

The term "substituted" as used herein, means that one or more hydrogens on the designated atom are replaced by a group selected from a defined group of substituents, provided that the designated atom's normal valence is not exceeded, and that the substitution results in a stable compound. Likewise, the term "substituted" may be used in connection with a chemical moiety instead of a single atom, e.g., "substituted alkyl", "substituted aryl" or the like.

Unless specifically indicated, throughout the specification and the appended claims, a given chemical formula or name shall encompass tautomers and all stereo, optical and geometrical isomers (e.g. enantiomers, diastereomers, E/Z isomers etc.) and racemates thereof as well as mixtures in different proportions of the separate enantiomers, mixtures of diastereomers, or mixtures of any of the foregoing forms where such isomers and enantiomers exist, as well as solvates thereof such as for instance hydrates.

Unless specifically indicated, also "pharmaceutically acceptable salts" as defined in more detail below shall encompass solvates thereof such as for instance hydrates.

In general, substantially pure stereoisomers can be obtained according to synthetic principles known to a person skilled in the field, e.g., by separation of corresponding mixtures, by using stereochemically pure starting materials and/or by stereoselective synthesis. It is known in the art how to prepare optically active forms, such as by resolution of racemic forms or by synthesis, e.g., starting from optically active starting materials and/or by using chiral reagents.

Enantiomerically pure compounds of this invention or intermediates may be prepared via asymmetric synthesis, for example by preparation and subsequent separation of appropriate diastereomeric compounds or intermediates which can be separated by known methods (e.g. by chromatographic separation or crystallization) and/or by using chiral reagents, such as chiral starting materials, chiral catalysts or chiral auxiliaries.

Further, it is known to the person skilled in the art how to prepare enantiomerically pure compounds from the corresponding racemic mixtures, such as by chromatographic separation of the corresponding racemic mixtures on chiral stationary phases; or by resolution of a racemic mixture using an appropriate resolving agent, e.g. by means of diastereomeric salt formation of the racemic compound with optically active acids or bases, subsequent resolution of the salts and release of the desired compound from the salt; or by derivatization of the corresponding racemic compounds with optically active chiral auxiliary reagents, subsequent diastereomer separation and removal of the chiral auxiliary group; or by kinetic resolution of a racemate (e.g. by enzymatic resolution); by enantioselective crystallization from a conglomerate of enantiomorphous crystals under suitable conditions; or by (fractional) crystallization from a suitable solvent in the presence of an optically active chiral auxiliary.

The phrase "pharmaceutically acceptable" is employed herein to refer to those compounds, materials, compositions, and/or dosage forms which are, within the scope of sound medical judgment, suitable for use in contact with the tissues of animals without excessive toxicity, irritation, allergic response, or other problem or complication, and commensurate with a reasonable benefit/risk ratio.

As used herein, "pharmaceutically acceptable salt" refers to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like.

For example, such salts include salts from benzenesulfonic acid, benzoic acid, citric acid, ethanesulfonic acid, fumaric acid, gentisic acid, hydrobromic acid, hydrochloric acid, maleic acid, malic acid, malonic acid, mandelic acid, methanesulfonic acid, 4-methyl-benzenesulfonic acid, phosphoric acid, salicylic acid, succinic acid, sulfuric acid and tartaric acid. Further pharmaceutically acceptable salts can be formed with cations from ammonia, L-arginine, calcium, 2,2'-iminobisethanol, L-lysine, magnesium, N-methyl-D-glucamine, potassium, sodium and tris(hydroxymethyl)-aminomethane.

The pharmaceutically acceptable salts of the present invention can be synthesized from the parent compound which contains a basic or acidic moiety by conventional chemical methods. Generally, such salts can be prepared by reacting the free acid or base forms of these compounds with a sufficient amount of the appropriate base or acid in water or in an organic diluent such as ether, ethyl acetate, ethanol, isopropanol, or acetonitrile, or a mixture thereof.

Salts of other acids than those mentioned above which for example are useful for purifying or isolating the compounds of the present invention (e.g. trifluoro acetate salts,) also comprise a part of the invention.

The term "halogen" denotes fluorine, chlorine, bromine and iodine.

The term "C₁₋ₙalkyl", wherein n is an integer selected from 2, 3, 4, 5 or 6, either alone or in combination with another radical, denotes an acyclic, saturated, branched or linear hydrocarbon radical with 1 to n C atoms. For example the term C₁₋₅alkyl embraces the radicals H₃C, H₃CCH₂, H₃CCH₂CH₂, H₃CCH(CH₃), H₃CCH₂CH₂CH₂, H₃CCH₂CH(CH₃), H₃CCH(CH₃)CH₂, H₃CC(CH₃)₂, H₃CCH₂CH₂CH₂CH₂, H₃CCH₂CH₂CH(CH₃), H₃CCH₂CH(CH₃)CH₂, H₃CCH(CH₃)CH₂CH₂, H₃CCH₂C(CH₃)₂, H₃CC(CH₃)₂CH₂, H₃CCH(CH₃)CH(CH₃) and H₃CCH₂CH(CH₂CH₃).

The term "C₁₋ₙ-alkylene", wherein n is an integer selected from 2, 3, 4, 5 or 6, either alone or in combination with another radical, denotes an acyclic, saturated, branched or linear chain divalent alkyl radical containing from 1 to n carbon atoms. For example, the term C₁₋₄alkylene includes CH₂, CH₂CH₂, CH(CH₃), CH₂CH₂CH₂, C(CH₃)₂, CH(CH₂CH₃), CH(CH₃)CH₂, CH₂CH(CH₃), CH₂CH₂CH₂CH₂, CH₂CH₂CH(CH₃), CH(CH₃)CH₂CH₂, CH₂CH(CH₃)CH₂, CH₂C(CH₃)₂, C(CH₃)₂CH₂, CH(CH₃)CH(CH₃), CH₂CH(CH₂CH₃), CH(CH₂CH₃)CH₂, CH(CH₂CH₂CH₃) , CH(CH(CH₃))₂ and C(CH₃)(CH₂CH₃).

The term "C₂₋ₘ-alkenyl" is used for a group "C₂₋ₘ-alkyl", wherein m is an integer selected from 3, 4, 5 or 6, if at least two carbon atoms of said group are bonded to each other by a double bond.

The term "C₂₋ₘ-alkenylene" is used for a group "C₂₋ₘ-alkylene", wherein m is an integer selected from 3, 4, 5 or 6, if at least two carbon atoms of said group are bonded to each other by a double bond.

The term "C₂₋ₘ-alkynyl" is used for a group "C₂₋ₘ-alkyl", wherein m is an integer selected from 3, 4, 5 or 6, if at least two carbon atoms of said group are bonded to each other by a triple bond.

The term "C₂₋ₘ-alkynylene" is used for a group "C₂₋ₘ-alkylene", wherein m is an integer selected from 3, 4, 5 or 6, if at least two of those carbon atoms of said group are bonded to each other by a triple bond.

The term "C₃₋ₖcycloalkyl", wherein k is an integer selected from 4, 5, 6, 7 or 8, either alone or in combination with another radical, denotes a cyclic, saturated, unbranched hydrocarbon radical with 3 to k C atoms. For example, the term C₃₋₇cycloalkyl includes cyclopropyl, cyclobutyl, cyclopentyl, cyclohexyl and cycloheptyl.

The term "C₃₋ₖcycloalkenyl", wherein k is an integer selected from 4, 5, 6, 7 or 8, either alone or in combination with another radical, denotes a cyclic, unsaturated, but non-aromatic, unbranched hydrocarbon radical with 3 to k C atoms, at least two of which are bonded to each other by a double bond. For example, the term C₃₇cycloalkenyl includes cyclopropenyl, cyclobutenyl, cyclopentenyl, cyclopentadienyl, cyclohexenyl, cyclohexadienyl, cycloheptenyl, cycloheptadienyl and cycloheptatrienyl.

The term "halo" added to an "alkyl", "alkylene", "alkenyl", "alkenylene", "alkynyl", "alkynylene", "cycloalkyl", "cycloalkenyl" or "alkoxy" group defines an alkyl, alkylene, alkenyl, alkenylene, alkynyl, alkynylene, cycloalkyl, cycloalkenyl or alkoxy group, wherein one or more hydrogen atoms are replaced by a halogen atom selected from among fluorine, chlorine, bromine or iodine. For example C₁-C₄-haloalkyl includes, but is not limited to, chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl and the like.

The term "fluoroalkyl" as used herein refers to an alkyl in which one or more of the hydrogen atoms is replaced with fluorine atoms, for example difluoromethyl, trifluoromethyl, 1-fluoroethyl, 2-fluoroethyl, 2,2-difluoroethyl, 2,2,2-trifluoroethyl, 1,1,2,2-tetrafluoroethyl or pentafluoroethyl.

The term "alkoxy" refers to an alkyl-O-, wherein alkyl is as defined above. Similarly, the terms "alkenyloxy", "alkynyloxy", "haloalkoxy", "haloalkenyloxy", "haloalkynyloxy", "cycloalkoxy", "cycloalkenyloxy", "halocycloalkoxy", and "halocycloalkenyloxy" refer to the groups alkenyl-O-, alkynyl-O-, haloalkyl-O-, haloalkenyl-O-, haloalkynyl-O-, cycloalkyl-O-, cycloalkenyl-O-, halocycloalkyl-O-, and halocycloalkenyl-O-, respectively, wherein alkenyl, alkynyl, haloalkyl, haloalkenyl, haloalkynyl, cycloalkyl, cycloalkenyl, halocycloalkyl, and halocycloalkenyl are as defined herein. Examples of C₁-C₆-alkoxy include, but are not limited to, methoxy, ethoxy, OCH₂-C₂H₅, OCH(CH₃)₂, n-butoxy, OCH(CH₃)-C₂H₃, OCH₂-CH(CH₃)₂, OC(CH₃)₃, n-pentoxy, 1-methylbutoxy, 2-methylbutoxy, 3-methylbutoxy, 1,1-dimethylpropoxy, 1,2-dimethylpropoxy, 2,2-dimethyl-propoxy, 1-ethylpropoxy, n-hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy, 1-ethyl-2-methylpropoxy and the like.

The term "carbocyclyl" or "carbocycle", either alone or in combination with another radical, means a mono, bi or tricyclic ring structure consisting of 3 to 14 carbon atoms. The term "carbocyclyl" or "carbocycle" refers to fully saturated, partially saturated or aromatic ring systems. The term "carbocyclyl" or "carbocycle" encompasses fused, bridged and spirocyclic systems. Examples include:

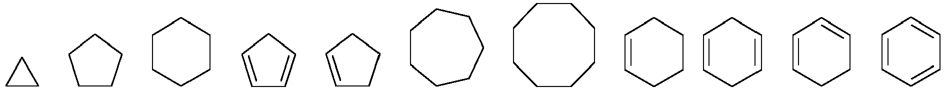

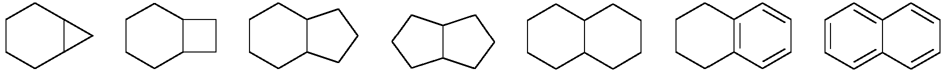

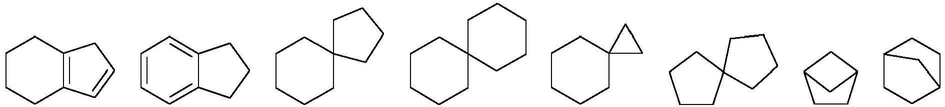

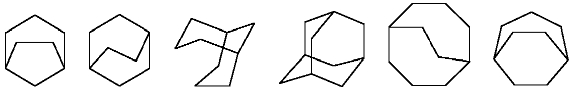

The term "aryl", either alone or in combination with another radical, denotes a carbocyclic aromatic monocyclic group containing 6 carbon atoms, which is optionally further fused to a second five- or six-membered, carbocyclic group which is aromatic, fully saturated or partially saturated. The term "aryl" includes, but is not limited to, phenyl, indanyl, indenyl, naphthyl, anthracenyl, phenanthrenyl, tetrahydronaphthyl and dihydronaphthyl.

The term "aralkyl" refers to an aryl group that is bonded to the parent compound through a diradical alkylene bridge, (-CH₂-)ₙ, where n is 1-6 and where "aryl" is as defined herein.

The term "heterocyclyl" or "heterocycle" means a saturated or unsaturated mono- or polycyclic ring system optionally comprising aromatic rings, containing one or more heteroatoms selected from N, O, S, S(O) or S(O)₂ consisting of 3 to 14 ring atoms, wherein none of the heteroatoms is part of the aromatic ring. The term "heterocyclyl" or "heterocycle" is intended to include all possible isomeric forms. Thus, the term "heterocyclyl" or "heterocycle" includes the following exemplary structures (not depicted as radicals as each form is optionally attached through a covalent bond to any atom so long as appropriate valences are maintained):

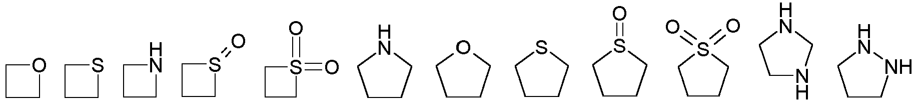

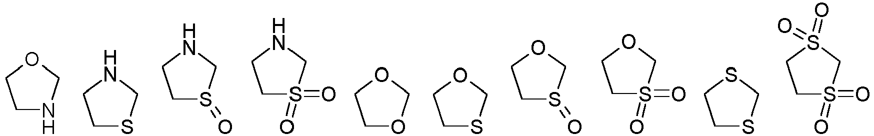

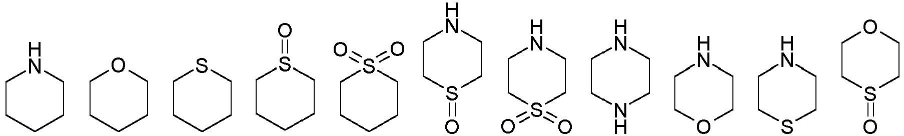

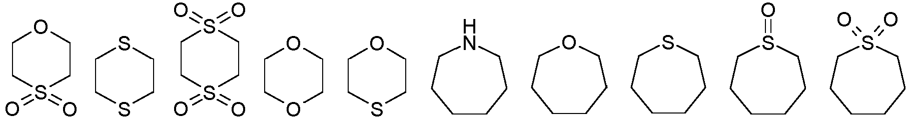

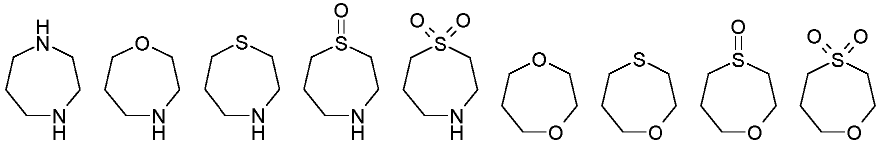

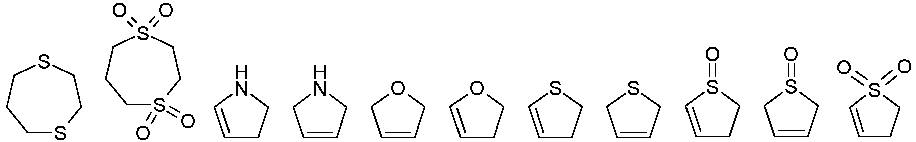

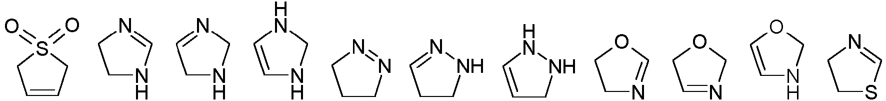

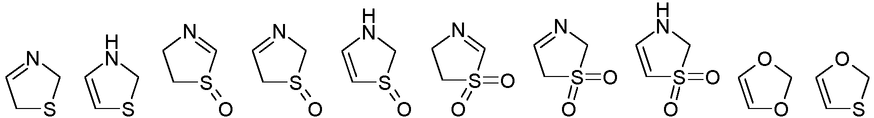

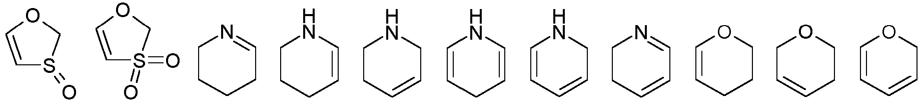

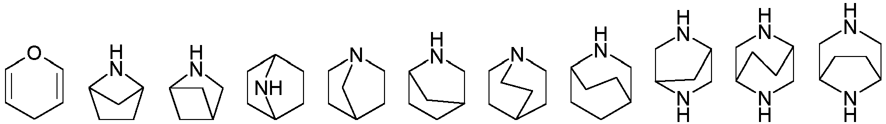

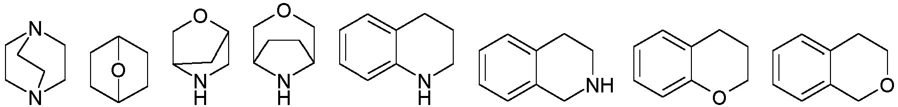

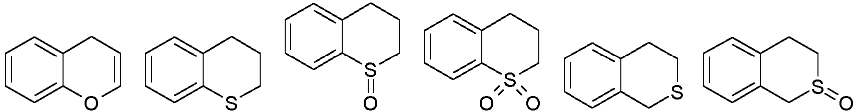

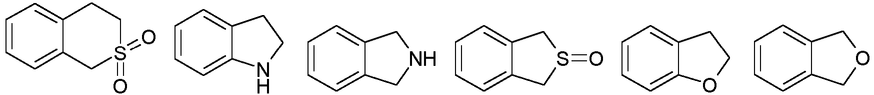

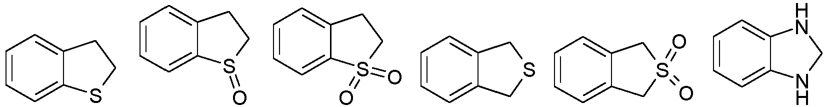

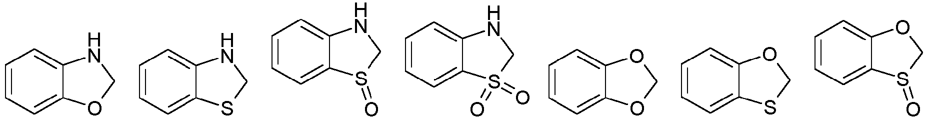

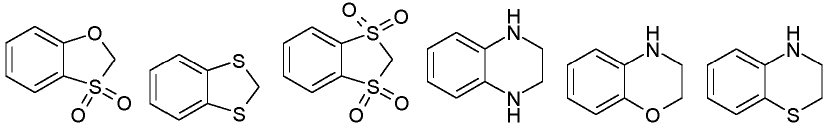

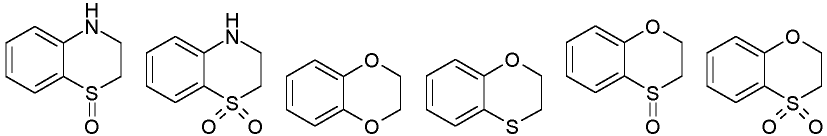

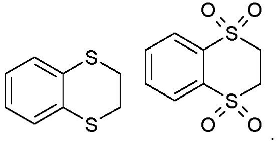

The term "heteroaryl" means a mono- or polycyclic ring system, comprising at least one aromatic ring, containing one or more heteroatoms selected from N, O, S, S(O) or S(O)₂, consisting of 5 to 14 ring atoms, wherein at least one of the heteroatoms is part of an aromatic ring. The term "heteroaryl" is intended to include all the possible isomeric forms. Thus, the term "heteroaryl" includes the following exemplary structures (not depicted as radicals as each form is optionally attached through a covalent bond to any atom so long as appropriate valences are maintained):

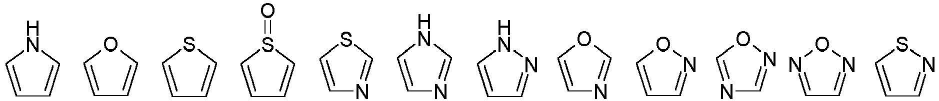

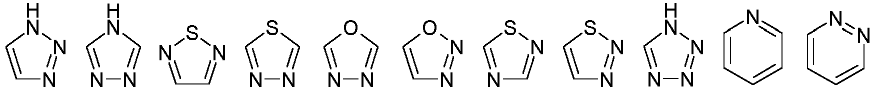

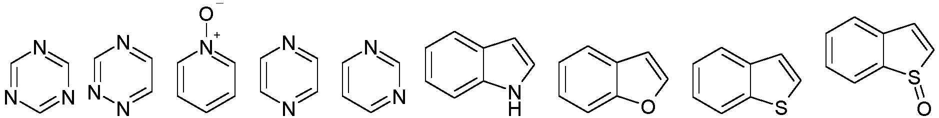

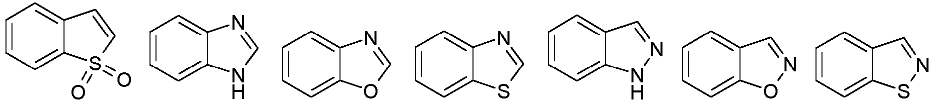

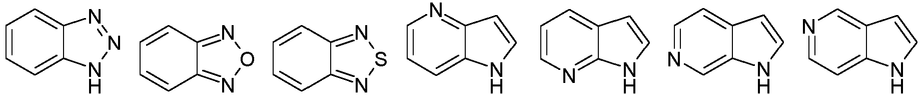

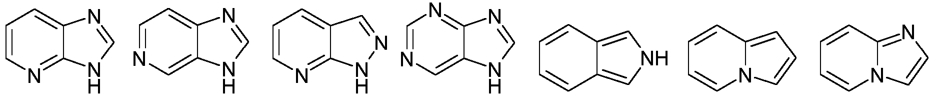

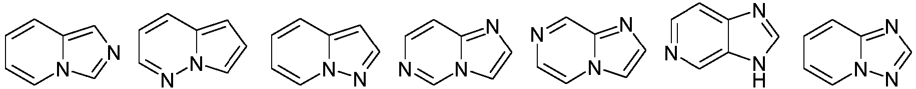

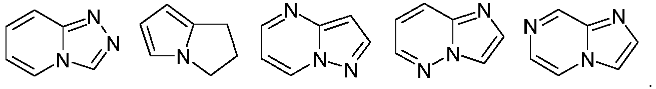

Many of the terms given herein may be used repeatedly in the definition of a formula or group and in each case have one of the meanings given herein, independently of one another.

The term "bicyclic ring systems" means groups consisting of 2 joined cyclic substructures including spirocyclic, fused, and bridged ring systems.

The term "tricyclic ring systems" means groups consisting of 3 joined cyclic substructures including spirocyclic, fused, and bridged ring systems.

As used herein, the term "prevention" in connection with "parasitic infections and/or infestations in/on animals" means prophylactical treatment of a given animal. For instance, the action of stopping endoparasitic infections or ectoparasitic infestations from establishing, usually for a defined post-treatment time interval, such as the protective period, i.e. the time period, usually expressed in days or weeks after the treatment, that a product will kill a newly internalized endoparasite, thereby preventing endoparasitic (re-)infection from developing in the animal, or prevent re-infestation of the animal by the ectoparasite (sometimes also referred to as the prophylactic period or the persistent efficacy period), is a non-limiting example of such a prevention.

As used herein, the term "control" in connection with "parasitic infections and/or infestations in animals" means that the parasitic infection and/or infestation is ameliorated or improved, sustainedly reduced in incidence and/or prevented from worsening as regards the animal.

### PHARMACEUTICAL COMPOSITIONS

The pharmaceutical compositions of the invention comprise effective amounts of compounds of the invention or salts thereof, including those of formula (I), (Ia) and/or (Ib), in combination with an acceptable carrier or diluent. The pharmaceutical compositions may be in a variety of solid and liquid forms which are suitable for various methods of application or administration to an animal. For example, the pharmaceutical compositions comprising the one or more compounds of the invention may be in compositions suitable for oral administration, injectable administration, including subcutaneous and parenteral administration, topical administration (e.g. spot-on or pour-on), including dermal or subdermal administration.

Suitable dosage forms for oral administration include dietary supplements, troches, lozenges, chewables (e.g. chewable tablets or soft chews), tablets, hard or soft capsules, boluses, emulsions, aqueous or oily suspensions, aqueous or oily solutions, oral drench compositions, dispersible powders or granules, premixes, syrups or elixirs, enteric compositions or pastes. Pharmaceutical compositions intended for oral use may be prepared according to any method known in the art for the manufacture of pharmaceutical compositions. Suitable tablets may be obtained, for example, by mixing one or more compounds of the invention, including those of formula (I), (Ia) and/or (Ib), with known excipients, for example inert diluents, carriers, disintegrants, adjuvants, surfactants, binders and/or lubricants.

In one embodiment of the invention, a soft chewable veterinary composition is provided comprising an effective amount of at least one compound of formula (I), (Ia) and/or (Ib) in a pharmaceutically acceptable carrier.

In some embodiments, the compositions may be in the form of a sterile injectable composition. The injectable composition may be a solution or a suspension. This suspension may be formulated according to the known art using those suitable dispersing or wetting agents and suspending agents. The sterile injectable preparation may also be a sterile injectable solution or suspension in a non-toxic parenterally acceptable diluent or solvent.

Implant compositions that may be used to deliver the compounds of the invention are commonly subcutaneously delivered to an animal. In addition, external wearable devices such as collars, pendants, ear tags, and the like, may be used to deliver the compounds of the invention to an animal.

Suitable topical spot-on or pour-on pharmaceutical composition comprise a pharmaceutically effective amount of at least one compound of the invention, including those of formula (I), (Ia) and/or (Ib), in a pharmaceutically acceptable carrier. The pharmaceutically acceptable carrier for the topical compositions of the invention may include, but are not limited to, solvents and solvent mixtures, permeation enhancers, surfactants, antioxidants, pH stabilizers, preservatives and crystallization inhibitors known in the art.

### METHODS OF TREATMENT

The compositions of the invention are intended to be administered to an animal including, but not limited to, mammals, birds and fish. Examples of mammals include but are not limited to humans, cattle, sheep, goats, llamas, alpacas, pigs, horses, donkeys, dogs, cats and other livestock or domestic mammals. Examples of birds include turkeys, chickens, ostriches and other livestock or domestic birds. In one embodiment, the invention provides a use of the compound to protect companion animals such as dogs and cats from endoparasites. In another embodiment, the compound of the invention may be used to protect equine animals from parasites. The compound may also be used to protect livestock animals.

The present invention is directed to compounds of formula (I), (Ia) or (Ib), which are useful in the treatment, prevention and/or control of parasitic infections and/or infestations in animals.

Accordingly, the present invention relates to a compound of formula (I), (Ia) or (Ib) for use as a medicament, including, but not limited to, for use as an antiparasitic medicament.

Furthermore, the present invention relates to the use of a compound of formula (I), (Ia) or (Ib) for the treatment, prevention and/or control of parasitic infections and/or infestations in animals.

In a further aspect the present invention relates to a compound of formula (I), (Ia) or (Ib) for use in the treatment, prevention and/or control of parasitic infections and/or infestations in animals.

In a further aspect the present invention relates to the use of a compound of formula (I), (Ia) or (Ib) for the preparation of a medicament for the treatment, prevention and/or control of parasitic infections and/or infestations in animals.

In a further aspect of the present invention the present invention relates to methods for the treatment, prevention and/or control of parasitic infections and/or infestations in animals, which methods comprise the administration of a pharmaceutically effective amount of one or more compounds of formula (I), (Ia) or (Ib) or pharmaceutical compositions of the invention to an animal / animal patient in need thereof.

The compounds of the present invention are highly effective for the treatment, prevention and/or control of external and/or internal parasites in animals including mammals, fish and birds. The compounds of the invention may be used to treat cats, dogs, horses, chicken, pigs, sheep and cattle, but also humans, with the aim of substantially ridding these hosts of ectoparasites and/or endoparasites. Mammals, which can be treated, include, but are not limited to, humans, cats, dogs, cattle, cows, bison, deer, goats, horses, llamas, camels, pigs, sheep and yaks.

In one embodiment of the present invention, the mammals treated are humans, cats or dogs. In another embodiment of the present invention, the mammals treated are horses. In yet another embodiment of the present invention, the mammals treated are livestock animals, such as cattle or sheep. In yet another embodiment, the compounds of the invention may be used to treat fish.

The dose range of the compounds of formula (I), (Ia) or (Ib) applicable per day is usually from 0.001 mg/kg to 1,000 mg/kg of body weight for animals.

The actual pharmaceutically effective amount or therapeutic dosage will usually depend on factors known by those skilled in the art such as age and weight of the animal patient, route of administration and severity of disease. In any case the compounds of the invention will be administered at dosages and in a manner which allows a pharmaceutically effective amount to be delivered based upon the animal patient's unique condition.

The agricultural and horticultural insecticidal and acaricidal agent comprising the compounds of formulae (I), (Ia) and/or (Ib) of the present invention or a salt thereof as an active ingredient has a remarkable control effect on pests which damage lowland crops, field crops, fruit trees, vegetables, other crops, ornamental flowering plants, etc. The desired effect can be obtained when the agricultural and horticultural insecticidal and acaricidal agent is applied to nursery facilities for seedlings, paddy fields, fields, fruit trees, vegetables, other crops, ornamental flowering plants, etc. and their seeds, paddy water, foliage, cultivation media such as soil, or the like around the expected time of pest infestation, i.e., before the infestation or upon the confirmation of the infestation. In particularly preferable embodiments, the application of the agricultural and horticultural insecticidal and acaricidal agent utilizes so-called penetration and translocation. That is, nursery soil, soil in transplanting holes, plant foot, irrigation water, cultivation water in hydroponics, or the like is treated with the agricultural and horticultural insecticidal and acaricidal agent to allow crops, ornamental flowering plants, etc. to absorb the compound of the present invention through the roots via soil or otherwise.

Examples of useful plants to which the agricultural and horticultural insecticidal and acaricidal agent of the present invention can be applied include, but are not particularly limited to, cereals (e.g., rice, barley, wheat, rye, oats, corn, etc.), legumes (e.g., soybeans, azuki beans, broad beans, green peas, kidney beans, peanuts, etc.), fruit trees and fruits (e.g., apples, citrus fruits, pears, grapes, peaches, plums, cherries, walnuts, chestnuts, almonds, bananas, etc.), leaf and fruit vegetables (e.g., cabbages, tomatoes, spinach, broccoli, lettuce, onions, green onions (chives and Welsh onions), green peppers, eggplants, strawberries, pepper crops, okra, Chinese chives, etc.), root vegetables (e.g., carrots, potatoes, sweet potatoes, taros, Japanese radishes, turnips, lotus roots, burdock roots, garlic, Chinese scallions, etc.), crops for processing (e.g., cotton, hemp, beet, hops, sugarcane, sugar beet, olives, rubber, coffee, tobacco, tea, etc.), gourds (e.g., Japanese pumpkins, cucumbers, watermelons, oriental sweet melons, melons, etc.), pasture grass (e.g., orchardgrass, sorghum, timothy, clover, alfalfa, etc.), lawn grass (e.g., Korean lawn grass, bent grass, etc.), spice and aromatic crops and ornamental crops (e.g., lavender, rosemary, thyme, parsley, pepper, ginger, etc.), ornamental flowering plants (e.g., chrysanthemum, rose, carnation, orchid, tulip, lily, etc.), garden trees (e.g., ginkgo trees, cherry trees, Japanese aucuba, etc.) and forest trees (e.g., *Abies sachalinensis, Picea jezoensis,* pine, yellow cedar, *Japanese cedar,* hinoki cypress, eucalyptus, etc.).

The above-mentioned "plants" also include plants provided with herbicide tolerance by a classical breeding technique or a gene recombination technique. Examples of such herbicide tolerance include tolerance to HPPD inhibitors, such as isoxaflutole; ALS inhibitors, such as imazethapyr and thifensulfuron-methyl; EPSP synthase inhibitors, such as glyphosate; glutamine synthetase inhibitors, such as glufosinate; acetyl-CoA carboxylase inhibitors, such as sethoxydim; or other herbicides, such as bromoxynil, dicamba and 2,4-D.

Examples of the plants provided with herbicide tolerance by a classical breeding technique include varieties of rapeseed, wheat, sunflower and rice tolerant to the imidazolinone family of ALS-inhibiting herbicides such as imazethapyr, and such plants are sold under the trade name of Clearfield (registered trademark). Also included is a variety of soybean provided with tolerance to the sulfonyl urea family of ALS-inhibiting herbicides such as thifensulfuron-methyl by a classical breeding technique, and this is sold under the trade name of STS soybean. Also included are plants provided with tolerance to acetyl-CoA carboxylase inhibitors such as trione oxime herbicides and aryloxy phenoxy propionic acid herbicides by a classical breeding technique, for example, SR corn and the like.

Plants provided with tolerance to acetyl-CoA carboxylase inhibitors are described in Proc. Natl. Acad. Sci. USA, 87, 7175-7179 (1990), and the like. Further, acetyl-CoA carboxylase mutants resistant to acetyl-CoA carboxylase inhibitors are reported in Weed Science, 53, 728-746 (2005), and the like, and by introducing the gene of such an acetyl-CoA carboxylase mutant into plants by a gene recombination technique, or introducing a resistance-conferring mutation into acetyl-CoA carboxylase of plants, plants tolerant to acetyl-CoA carboxylase inhibitors can be engineered. Alternatively, by introducing a nucleic acid causing base substitution mutation into plant cells (a typical example of this technique is chimeraplasty technique (Gura T. 1999. Repairing the Genome's Spelling Mistakes. Science 285: 316-318)) to allow site-specific substitution mutation in the amino acids encoded by an acetyl-CoA carboxylase gene, an ALS gene or the like of plants, plants tolerant to acetyl-CoA carboxylase inhibitors, ALS inhibitors or the like can be engineered. The agricultural and horticultural insecticidal and acaricidal agent of the present invention can be applied to these plants as well.

Further, exemplary toxins expressed in genetically modified plants include insecticidal proteins of Bacillus cereus or *Bacillus popilliae; Bacillus thuringiensis* δ-endotoxins, such as Cry1Ab, Cry1Ac, Cry1F, Cry1Fa2, Cry2Ab, Cry3A, Cry3Bb1 and Cry9C, and other insecticidal proteins, such as VIP1, VIP2, VIP3 and VIP3A; nematode insecticidal proteins; toxins produced by animals, such as scorpion toxins, spider toxins, bee toxins and insect-specific neurotoxins; toxins of filamentous fungi; plant lectins; agglutinin; protease inhibitors, such as trypsin inhibitors, serine protease inhibitors, patatin, cystatin and papain inhibitors; ribosome inactivating proteins (RIP), such as ricin, maize RIP, abrin, luffin, saporin and bryodin; steroid metabolizing enzymes, such as 3-hydroxy steroid oxidase, ecdysteroid-UDP-glucosyltransferase and cholesterol oxidase; ecdysone inhibitors; HMG-CoA reductase; ion channel inhibitors, such as sodium channel inhibitors and calcium channel inhibitors; juvenile hormone esterase; diuretic hormone receptors; stilbene synthase; bibenzyl synthase; chitinase; and glucanase.

Due to the toxins contained in such genetically modified plants, the plants exhibit resistance to pests, in particular, Coleopteran insect pests, Hemipteran insect pests, Dipteran insect pests, Lepidopteran insect pests and nematodes. The above-described technologies and the agricultural and horticultural insecticidal and acaricidal agent of the present invention can be used in combination or used systematically.

In order to control target pests, the agricultural and horticultural insecticidal and acaricidal agent of the present invention, with or without appropriate dilution or suspension in water etc., is applied to plants potentially infested with the target insect pests or nematodes in an amount effective for the control of the insect pests or nematodes. For example, in order to control insect pests and nematodes that may damage crop plants such as fruit trees, cereals and vegetables, foliar application and seed treatment such as dipping, dust coating and calcium peroxide coating can be performed. Further, treatment of soil or the like may also be performed to allow plants to absorb agrochemicals through their roots. Examples of such treatment include whole soil incorporation, planting row treatment, bed soil incorporation, plug seedling treatment, planting hole treatment, plant foot treatment, top-dressing, treatment of nursery boxes for paddy rice, and submerged application. In addition, application to culture media in hydroponics, smoking treatment, trunk injection and the like can also be performed. Further, the agricultural and horticultural insecticidal and acaricidal agent of the present invention, with or without appropriate dilution or suspension in water etc., can be applied to sites potentially infested with pests in an amount effective for the control of the pests. For example, it can be directly applied to stored grain pests, house pests, sanitary pests, forest pests, etc., and also be used for coating of residential building materials, for smoking treatment, or as a bait formulation.

Exemplary methods of seed treatment include dipping of seeds in a diluted or undiluted fluid of a liquid or solid formulation for the permeation of agrochemicals into the seeds; mixing or dust coating of seeds with a solid or liquid formulation for the adherence of the formulation onto the surfaces of the seeds; coating of seeds with a mixture of an agrochemical and an adhesive carrier such as resins and polymers; and application of a solid or liquid formulation to the vicinity of seeds at the same time as seeding.

The term "seed" in the above-mentioned seed treatment refers to a plant body which is in the early stages of cultivation and used for plant propagation. The examples include, in addition to a so-called seed, a plant body for vegetative propagation, such as a bulb, a tuber, a seed potato, a bulbil, a propagule, a discoid stem and a stem used for cuttage.

The term "soil" or "cultivation medium" in the method of the present invention for using an agricultural and horticultural insecticide refers to a support medium for crop cultivation, in particular a support medium which allows crop plants to spread their roots therein, and the materials are not particularly limited as long as they allow plants to grow. Examples of the support medium include what is called soils, seedling mats and water, and specific examples of the materials include sand, pumice, vermiculite, diatomite, agar, gelatinous substances, high-molecular-weight substances, rock wool, glass wool, wood chip and bark.

Exemplary methods of the application to crop foliage or to stored grain pests, house pests, sanitary pests, forest pests, etc. include application of a liquid formulation, such as an emulsifiable concentrate and a flowable, or a solid formulation, such as a wettable powder and a water-dispersible granule, after appropriate dilution in water; dust application; and smoking.

Exemplary methods of soil application include application of a water-diluted or undiluted liquid formulation to the foot of plants, nursery beds for seedlings, or the like; application of a granule to the foot of plants, nursery beds for seedlings, or the like; application of a dust, a wettable powder, a water-dispersible granule, a granule or the like onto soil and subsequent incorporation of the formulation into the whole soil before seeding or transplanting; and application of a dust, a wettable powder, a water-dispersible granule, a granule or the like to planting holes, planting rows or the like before seeding or planting.

To nursery boxes for paddy rice, for example, a dust, a water-dispersible granule, a granule or the like can be applied, although the suitable formulation may vary depending on the application timing, in other words, depending on the cultivation stage such as seeding time, greening period and planting time. A formulation such as a dust, a water-dispersible granule and a granule may be mixed with nursery soil. For example, such a formulation is incorporated into bed soil, covering soil or the whole soil. Simply, nursery soil and such a formulation may be alternately layered.

In the application to paddy fields, a solid formulation, such as a jumbo, a pack, a granule and a water-dispersible granule, or a liquid formulation, such as a flowable and an emulsifiable concentrate, is applied usually to flooded paddy fields. In a rice planting period, a suitable formulation, as it is or after mixed with a fertilizer, may be applied onto soil or injected into soil. In addition, an emulsifiable concentrate, a flowable or the like may be applied to the source of water supply for paddy fields, such as a water inlet and an irrigation device. In this case, treatment can be accomplished with the supply of water and thus achieved in a labor-saving manner.

In the case of field crops, their seeds, cultivation media in the vicinity of their plants, or the like may be treated in the period of seeding to seedling culture. In the case of plants of which the seeds are directly sown in the field, in addition to direct seed treatment, plant foot treatment during cultivation is preferable. Specifically, the treatment can be performed by, for example, applying a granule onto soil, or drenching soil with a formulation in a water-diluted or undiluted liquid form. Another preferable treatment is incorporation of a granule into cultivation media before seeding.

In the case of culture plants to be transplanted, preferable examples of the treatment in the period of seeding to seedling culture include, in addition to direct seed treatment, drench treatment of nursery beds for seedlings with a formulation in a liquid form; and granule application to nursery beds for seedlings. Also included are treatment of planting holes with a granule; and incorporation of a granule into cultivation media in the vicinity of planting points at the time of fix planting.

The amount of the active ingredient compound in the agricultural and horticultural insecticidal and acaricidal agent of the present invention can be adjusted as needed, and basically, the amount of the active ingredient compound is appropriately selected from the range of 0.01 to 90 parts by weight in 100 parts by weight of the agricultural and horticultural insecticide. For example, in the case where the agricultural and horticultural insecticide is a dust, a granule, an emulsifiable concentrate or a wettable powder, it is suitable that the amount of the active ingredient compound is 0.01 to 50 parts by weight (0.01 to 50% by weight relative to the total weight of the agricultural and horticultural insecticidal and acaricidal agent).

The application rate of the agricultural and horticultural insecticidal and acaricidal agent of the present invention may vary with various factors, for example, the purpose, the target pest, the growing conditions of crops, the tendency of pest infestation, the weather, the environmental conditions, the dosage form, the application method, the application site, the application timing, etc., but basically, the application rate of the active ingredient compound is appropriately selected from the range of 0.001 g to 10 kg, and preferably 0.01 g to 1 kg per 10 ares depending on the purpose.

### VETERINARY PARASITES

In one embodiment, the compounds and pharmaceutical compositions of the invention may be used for treating, controlling and/or preventing an endoparasitic infection in an animal by one or more of the following parasite genera: *Aelurostrongylus, Acanthocheilonema, Ancylostoma, Angiostrongylus, Anoplocephala, Ascaridia, Ascaris, Babesia, Besnoitia, Brugia, Bunostomum, Caballonema, Capillaria, Chabertia, Cooperia, Crenosoma, Coronocyclus, Craterostomum, Crenosoma, Cryptosporidium, Cyathostomum, Cylicocyclus, Cylicodontophorus, Cylicostephanus, Cylindropharynx, Cystocaulus, Cystoisospora, Cytauxzoon, Dictyocaulus, Dipetalonema, Diphyllobothrium (Dibothriocephalus), Diplopylidium, Dipylidium, Dioctophyme, Dirofilaria, Dracunculus, Draschia, Echinococcus, Eimeria, Enterobius, Fasciola, Filaroides, Giardia, Gyalocephalus, Habronema, Haemonchus, Hammondia, Heligmosomoides, Hepatozoon, Heterakis, Histomonas, Hsiungia, Hymenolepis, Hyostrongylus, Isospora (Cytoisospora), Joyeuxiella, Leishmania, Linguatula, Litomosoides, Macracanthorynchus, Mansonella, Mesocestoides, Metastrongylus, Moniezia, Necator, Nematodirus, Neospora, Nippostrongylus, Oesophagostomum, Onchocerca, Opisthorchis, Oslerus, Ostertagia, Oxyuris, Parafilaria, Parapoteriostomum, Parascaris, Petrovinema, Pneumonyssoides, Pneumonyssus, Poteriostomum, Probstmayria, Protostrongylus, Schistosoma, Skrjabinodentus, Spirocerca, Spirometra, Spirura, Strongyloides, Strongylus, Taenia, Teladorsagia, Theileria, Thelazia, Toxascaris, Toxocara, Toxoplasma, Trichinella, Trichostrongylus, Trichuris, Tridentoinfundibulum, Triodontophorus, Tritrichomonas, Troglostrongylus, Trypanosoma, Uncinaria, Wuchereria.*

In one embodiment, the compounds and compositions of the invention may be used to treat, control and/or prevent an endoparasitic infection in an animal by one or more of the following parasites: *Aelurostrongylus abstrusus, Ancylostoma braziliense, Ancylostoma caninum, Ancylostoma ceylanicum, Ancylostoma tubaeforme, Angiostrongylus vasorum, Anoplocephala magna, Anoplocephala perfoliata, Ascaris suum, Babesia bigemina, Babesia bovis, Babesia canis, Babesia divergens, Babesia gisoni, Babesia major, Babesia rossi, Babesia vogeli, Babesia vulpes, Brugia buckleyi, Brugia ceylonensis, Brugia malayi, Brugia pahangi, Brugia patei, Bunostomum phlebotomum, Bunostomum trigonocephalum, Caballonema longicapsulatum, Capillaria aerophila, Capillaria anatis, Capillaria bovis, Capillaria contorta, Capillaria feliscati, Capillaria obsignata, Chabertia ovina, Clonorchis sinensis, Cooperia curticei, Cooperia oncophora, Cooperia pectinata, Cooperia punctata, Cooperia surnabada, Coronocyclus coronatus, Coronocyclus labiatus, Coronocyclus labratus, Craterostomum acuticaudatum, Crenosoma vulpis, Cyathostomum alveatum, Cyathostomum catinatum, Cyathostomum paternatum, Cyathostomum tetracanthum, Cylicocyclus ashworthi, Cylicocyclus brevicapsulatus, Cylicocyclus elongatus, Cylicocyclus insigniae, Cylicocyclus leptosomatum, Cylicocyclus nassatus, Cylicocyclus radiatus, Cylicocyclus ultrajectinus, Cylicodontophorus bicoronatus, Cylicodontophorus mettami, Cylicostephanus asymetricus, Cylicostephanus bidentatus, Cylicostephanus calicatus, Cylicostephanus goldi, Cylicostephanus hybridus, Cylicostephanus longibursatus, Cylicostephanus minutus, Cylindropharynx brevicauda, Cylindropharynx intermedia, Cylindropharynx longicauda, Cystocaulus oreatus, Cytauxzoon felis, Cytstoisispora burrowsi, Cytstoisispora canis, Cytstoisispora felis, Cytstoisispora ohioensis, Cytstoisispora rivolta, Cytstoisospora suis, Dictyocaulus arnfieldi, Dictyocaulus filaria, Dictyocaulus viviparus, Dipetalonema caudispina, Dipetalonema freitasi, Dipetalonema gracile, Dipetalonema robini, Dipetalonema yatesi, Diphyllobothrium latum, Dipylidium caninum, Dirofilaria hongkongensis, Dirofilaria immitis, Dirofilaria repens, Dracunculus insignis, Dracunculus lutrae, Dracunculus medinensis, Draschia megastoma, Echinococcus granulosus, Echinococcus multilocularis, Eimeria acervuline, Eimeria adenoids, Eimeria auburnensis, Eimeria bovis, Eimeria brunetti, Eimeria crandallis, Eimeria dispersa, Eimeria gallopavonis, Eimeria hagani, Eimeria maxima, Eimeria meleagrimitis, Eimeria mitavi, Eimeria necatrix, Eimeria ninakohlyakimovae, Eimeria ovina, Eimeria ovinoidalis, Eimeria praecox, Eimeria tenella, Eimeria zuernii, Fasciola gigantica, Fasciola hepatica, Fascioloides magna, Filaroides hirthi, Filaroides milksi, Filaroides osleri, Giardia duodenalis (syn. Giardia lamblia, Giardia intestinalis), Gyalocephalus capitatus, Habronema microstoma, Habronema muscae, Haemonchus contortus, Haemonchus placei, Haemonchus similis, Heterakis gallinarum, Histomonas meleagridis, Hsiungia triciliata, Hyostrongylus rubidus, Joyeuxiella pasquali, Leishmania infantum, Metastrongylus apri, Metastrongylus pudendotectus, Metastrongylus salmi, Moniezia benedeni, Moniezia expansa, Muellerius capillaris, Necator americanus, Nematodirus battus, Nematodirus filicollis, Nematodirus spathiger, Nippostrongylus brasiliensis, Nippostrongylos smalesae, Oesophagodontus robustus, Oesophagostomum bifurcum, Oesophagostomum columbianum, Oesophagostomum dendatum, Oesophagostomum quadrispinulatum, Oesophagostomum radiatum, Oesophagostomum venulosum, Onchocerca cervicalis, Onchocerca lupi, Opisthorchis felineus, Opisthorchis viverrine, Oslerus osleri, Ostertagia circumcincta, Ostertagia lyrata, Ostertagia ostertagi, Oxyuris equi, Oxyuris vermicularis, Paranoplocephala mamillana, Parascaris equorum, Parascaris univalens, Petrovinema poculatum, Poteriostomum imparidentatum, Probstmayria vivipara*, *Schistosoma indicum, Schistosoma leiperi, Schistosoma nasale, Schistosoma spindale, Spirocerca lupi, Spirometra felis, Strongylus edentatus, Strongylus equinus, Strongylus vulgaris, Strongyoides papillosus, Strongyloides ransomi, Strongyloides westeri, Taenia crassiceps, Taenia hydatigena*, *Taenia multiceps, Taenia ovis, Taenia pisiformis, Taenia saginata, Taenia serialis, Taenia solium, Taenia taeniaeformis, Teladorsagia circumcincta, Teladorsagia davtiani, Teladorsagia trifurcate*, *Theileria annulate, Theileria parva, Thelazia callipaeda, Thelazia californiensis, Toxascaris leonine, Toxocara canis, Toxocara cati (syn Toxocara mystax), Toxoplasma gondii, Trichostrongylus axei, Trichostrongylus colubriformis, Trichostrongylus rugatus, Trichostrongylus vitrinus, Triodontophorus brevicauda, Triodontophorus minor, Triodontophorus nipponicus, Triodontophorus serratus, Triodontophorus tenuicollis, Tritrichomonas blagburni (syn. Tritrichomonas foetus), Trichinella britovi, Trichinella papue, Trichinella spiralis, Trichuris campanula, Trichuris serrata, Trichuris vulpis, Trichuris suis, Troglostrongylus brevoir, Troglostrongylus subcrenatus, Troglostrongylus wilsoni, Trypanosoma brucei, Trypanosoma congolense, Trypanosoma evansi, Trypanosoma vivax* and *Uncinaria stenocephala.*

In one embodiment, the compounds and compositions of the invention may be used to treat, control and/or prevent an endoparasitic infection in a canine animal, including a dog, by one or more of *Ancylostoma braziliense, Ancylostoma caninum, Angiostrongylus vasorum, Babesia canis, Babesia gisoni, Babesia rossi, Babesia vogeli, Babesia vulpes, Crenosoma vulpis, Cytstoisispora burrowsi, Cytstoisispora canis, Cytstoisispora ohioensis, Diphyllobothrium latum, Dipylidium caninum, Dirofilaria hongkongensis, Dirofilaria immitis, Dirofilaria repens, Echinococcus granulosus, Echinococcus multilocularis, Filaroides hirthi, Filaroides milksi, Filaroides osleri, Giardia duodenalis (syn. Giardia lamblia, Giardia intestinalis), Leishmania infantum, Necator americanus, Onchocerca lupi, Oslerus osleri, Spirocerca lupi, Taenia crassiceps, Taenia hydatigena*, *Taenia multiceps, Taenia ovis, Taenia pisiformis, Taenia serialis, Thelazia callipaeda, Thelazia californiensis, Toxascaris leonine, Toxocara canis, Trichinella britovi, Trichuris vulpis* and *Uncinaria stenocephala..*

In another embodiment, the compounds and compositions of the invention may be used to treat, control and/or prevent an endoparasitic infection in a feline animal, including a cat, by one or more of *Aelurostrongylus abstrusus, Ancylostoma braziliense, Ancylostoma ceylanicum, Ancylostoma tubaeforme, Capillaria aerophila, Capillaria feliscati, Clonorchis sinensis, Cytauxzoon felis, Cytstoisispora felis, Cytstoisispora rivolta, Diphyllobothrium latum, Dipylidium caninum, Dirofilaria hongkongensis, Dirofilaria immitis, Dirofilaria repens, Echinococcus multilocularis, Giardia duodenalis (syn. Giardia lamblia, Giardia intestinalis), Joyeuxiella pasquali, Necator americanus, Opisthorchis felineus, Opisthorchis viverrine, Spirometra felis, Taenia taeniaeformis, Toxascaris leonine, Toxocara cati, Trichuris campanula, Trichuris serrata, Tritrichomonas blagburni (syn. Tritrichomonas foetus), Troglostrongylus brevoir, Troglostrongylus subcrenatus, Troglostrongylus wilsoni* and *Uncinaria stenocephala.*

In another embodiment, the compounds and compositions of the invention may be used to treat, control and/or prevent an endoparasitic infection in an equine animal by one or more of *Anoplocephala magna, Anoplocephala perfoliate, Cooperia curticei, Coronocyclus coronatus, Coronocyclus labiatus, Coronocyclus labratus, Craterostomum acuticaudatum, Cyathostomum alveatum, Cyathostomum catinatum, Cyathostomum paternatum, Cyathostomum tetracanthum, Cylicocyclus ashworthi, Cylicocyclus brevicapsulatus, Cylicocyclus elongatus, Cylicocyclus insigniae, Cylicocyclus leptosomatum, Cylicocyclus nassatus, Cylicocyclus radiatus, Cylicocyclus ultrajectinus, Dictyocaulus arnfieldi, Draschia megastoma, Gyalocephalus capitatus, Habronema microstoma, Habronema muscae, Haemonchus contortus, Nematodirus battus, Oesophagodontus robustus, Onchocerca cervicalis, Ostertagia circumcincta, Oxyuris equi, Paranoplocephala mamillana, Parascaris equorum, Parascaris univalens, Petrovinema poculatum, Poteriostomum imparidentatum, Probstmayria vivipara*, *Strongyloides westeri, Strongylus edentates, Strongylus equinus, Strongylus vulgaris, Trichinella britovi, Trichostrongylus axei, Trichostrongylus colubriformis, Triodontophorus brevicauda, Triodontophorus minor, Triodontophorus nipponicus, Triodontophorus serratus* and *Triodontophorus tenuicollis.*

In one embodiment, the compounds and compositions of the invention may be used to treat, control and/or prevent an endoparasitic infection in a ruminant animal by one or more of *Babesia bigemina, Babesia bovis, Babesia divergens, Babesia major, Bunostomum phlebotomum, Bunostomum trigonocephalum, Capillaria bovis, Chabertia ovina, Cooperia curticei, Cooperia oncophora, Cooperia pectinata, Cooperia punctata, Cooperia surnabada, Cystocaulus oreatus, Dictyocaulus filaria, Dictyocaulus viviparus, Eimeria auburnensis, Eimeria bovis, Eimeria crandallis, Eimeria ninakohlyakimovae, Eimeria ovina, Eimeria ovinoidalis, Eimeria zuernii, Fasciola gigantica*, *Fasciola hepatica, Fascioloides magna, Haemonchus contortus, Haemonchus placei, Haemonchus similis, Hsiungia triciliata, Moniezia benedeni, Moniezia expansa, Muellerius capillaris, Nematodirus filicollis, Nematodirus spathiger, Oesophagostomum columbianum, Oesophagostomum radiatum, Oesophagostomum venulosum, Ostertagia lyrata, Ostertagia ostertagi, Schistosoma indicum, Schistosoma leiperi, Schistosoma nasale, Schistosoma spindale, Strongyoides papillosus, Taenia saginata, Teladorsagia circumcincta, Teladorsagia davtiani, Teladorsagia trifurcate*, *Theileria annulate, Theileria parva, Toxoplasma gondii, Trichostrongylus axei, Trichostrongylus colubriformis, Trichostrongylus rugatus, Trichostrongylus vitrinus, Trypanosoma brucei, Trypanosoma congolense, Trypanosoma evansi* and *Trypanosoma vivax.*

In another embodiment, the compounds and compositions of the invention may be used to treat, control and/or prevent an endoparasitic infection in poultry by one or more of *Capillaria anatis, Capillaria contorta, Capillaria obsignata, Eimeria acervuline, Eimeria adenoids, Eimeria brunetti, Eimeria dispersa, Eimeria gallopavonis, Eimeria hagani, Eimeria maxima, Eimeria meleagrimitis, Eimeria mitavi, Eimeria necatrix, Eimeria praecox, Eimeria tenella, Heterakis gallinarum* and *Histomonas meleagridis.*

In another embodiment, the compounds and compositions of the invention may be used to treat, control and/or prevent an endoparasitic infection in swine by one or more of *Ascaris suum, Cytstoisospora suis, Hyostrongylus rubidus, Metastrongylus apri, Metastrongylus pudendotectus, Metastrongylus salmi, Oesophagostomum bifurcum*, *Oesophagostomum dendatum, Oesophagostomum quadrispinulatum, Strongyloides ransomi, Taenia solium, Trichinella britovi, Trichinella papue, Trichinella spiralis* and *Trichuris suis.*

In one embodiment, the compounds and pharmaceutical compositions of the invention may be used for treating, controlling and/or preventing an ectoparasitic infestation of one or more of the following parasite genera: *Acarapis, Aedes, Alphitobius, Anopheles, Amblyomma, Bovicola, Calliphora, Ceratophyllus, Cheyletiella, Chorioptes, Chrysomya, Chrysops, Cochliomyia, Ctenocephalides, Culex, Culicoides, Damalinia, Demodex, Dermacentor, Dermanyssus, Dermatobia, Diachlorus, Echidnophaga, Felicola, Gasterophilus, Gliricola, Glossina, Gyropus, Haemagogus, Haemaphysalis, Haematobia, Haematopinus, Haematopota, Heterodoxus, Hippobosca*, *Holakartikos, Hyalomma, Hypoderma, Ixodes, Linognathus, Lucilia, Lutzomyia, Lynxacarus, Melophagus, Menopon, Musca, Notoedres, Oestrus, Ornithonyssus, Otobius, Otodectes, Pediculus, Phlebotomus, Pneumonyssoides, Psorergates, Psoroptes, Pulex, Rhipicephalus (syn. Boophilus), Sarcoptes, Simulium, Solenoptes, Stomoxys, Tabanus, Trichodectes, Trimenopan, Trixacarus, Trombicula, Tunga, Varroa, Werneckiella* and *Wohlfahrtia, Xenopsylla.*

In another embodiment, the compounds and compositions of the invention may be used to treat, control and/or prevent an infestation by one or more of the following ectoparasites: *Aedes aegypti, Aedes albopictus, Aedes serratus, Alphitobius diaperinus, Anopheles gambiae, Amblyomma americanum, Ambylomma aureolatum, Amblyomma cajennense, Amblyomma maculatum, Ambylomma oblongoguttatum, Ambylomma ovale, Ambylomma parvum, Amblyomma sculptum, Ambylomma triste, Amblyomma variegatum, Bovicola bovis, Bovicola caprae, Bovicola limbatus, Bovicola ovis, Calliflora stygia, Ceratophyllus gallinae, Cheyletiella blakei, Cheyletiella parasitovorax, Cheyletiella yasguri, Chorioptes bovis, Chorioptes texanus, Chrysomya rufifacies, Cochliomyia hominivorax, Ctenocephalides canis, Ctenocephalides felis, Culex pipiens, Culicoides actoni, Culicoides brevitarsis, Culicoides fulvus, Culicoides imicola, Culicoides insignis, Culicoides nubeculosus, Culicoides variipennis, Damalinia bovis, Demodex brevis, Demodex canis, Demodex catis, Demodex cornei, Demodex follicolorum, Demodex gatoi, Demodex injai, Dermacentor andersoni, Dermacentor marginatus, Dermacentor occidentalis, Dermacentor reticulatus, Dermacentor variabilis, Dermanyssus gallinae, Dermatobia hominis, Echidnophaga gallinacea, Felicola subrostratus, Gasterophilus pecorum, Gasterophilus intestinalis, Gasterophilus nasalis, Gasterophilus haemorrhoidalis, Gasterophilus inermis, Gliricola porcelli, Glossina morsitans, Glossina brevipalpis, Glossina tachinoides, Glossina fuscipes, Glossina palpalis, Glossina pallidipes, Glossina austeni, Gyropus ovalis, Haemagogus janthinomys, Haemagogus leucocelaenus, Haemaphysalis longicornis, Haemaphysalis punctata, Haematobia irritans, Haematobia irritans exigua, Haematobia stimulans, Haematopinus eurysternus, Haematopinus quadripertusis, Holakartikos crassipes, Hyalomma anatolicum, Hyalomma dromedarii, Hyalomma lusitanicum, Hyalomma marginatum, Hyalomma truncatum, Hypoderma bovis, Hypoderma lineatum, Hypoderma diana, Hypoderma actaeon, Hypoderma tarandi, Ixodes gibbosus, Ixodes hexagonus, Ixodes holocyclus, Ixodes pacificus*, *Ixodes persulcatus, Ixodes ricinus, Ixodes scapularis, Linognathus africanus, Linognathus ovillus, Linognathus pedalis, Linognathus setosus, Linognathus stenopsis, Linognathus vituli, Lucilia cuprina, Lucilia sericata, Lutzomyia longipalpis, Lutzomyia shannoni, Lutzomyia anthophora, Lynxacarus radovskyi, Musca autumnalis, Notoedres cati, Oestrus ovis, Ornithonyssus bursae, Ornithonyssus sylviarum, Otobius megnini, Otodectes cynotis, Phlebotomus argentipes, Phlebotomus papatasi, Pneumonyssoides caninum, Przhevalskiana silenus, Psorergates ovis, Psoroptes cervinus, Psoroptes cuniculi, Psoroptes equi, Psoroptes natalensis, Psoroptes ovis, Pulex simulans, Pulex irritans, Rhipicephalus annulatus, Rhipicephalus appenidiculatus, Rhipicephalus bursa, Rhipicephalus decoloratus, Rhipicephalus microplus, Rhipicephalus sanguineus, Rhipicephalus turanicus, Sarcoptes anthracis, Sarcoptes scabiei, Sarcoptes scabiei var. canis, Solenopotes capillatus, Stomoxys calcitrans, Trichodectes canis, Trimenopan hispidum, Trixacarus caviae, Trixacarus diversus, Werneckiella equi, Wohlfahrtia magnifica* and *Xenopsylla cheopis.*

In another embodiment, the compounds and compositions of the invention may be used to treat, control and/or prevent an ectoparasitic infestation by one or more of the following flea species: *Ceratophyllus gallinae, Ctenocephalides canis, Ctenocephalides felis, Echidnophaga gallinacea, Pulex simulans, Pulex irritans* and *Xenopsylla cheopis.*

In another embodiment, the compounds and compositions of the invention may be used to treat, control and/or prevent an ectoparasitic infestation by one or more of the following tick species: *Amblyomma americanum, Ambylomma aureolatum, Amblyomma cajennense, Amblyomma maculatum, Ambylomma oblongoguttatum, Ambylomma ovale, Ambylomma parvum, Amblyomma sculptum, Ambylomma triste, Ambylomma triguttatum, Ambylomma triguttatum, Amblyomma variegatum, Dermacentor andersoni, Dermacentor marginatus, Dermacentor occidentalis, Dermacentor reticulatus, Dermacentor variabilis, Haemaphysalis elliptica, Haemaphysalis flava, Haemaphysalis elliptica, Haemaphysalis flava, Haemaphysalis longicornis, Haemaphysalis punctata, Hyalomma anatolicum, Hyalomma dromedarii, Hyalomma lusitanicum, Hyalomma marginatum, Hyalomma truncatum, Ixodes gibbosus, Ixodes hexagonus, Ixodes holocyclus, Ixodes, ovatus, Ixodes pacificus*, *Ixodes persulcatus, Ixodes ricinus, Ixodes scapularis, Otobius megnini, Rhipicephalus annulatus, Rhipicephalus appenidiculatus, Rhipicephalus bursa, Rhipicephalus decoloratus, Rhipicephalus microplus, Rhipicephalus sanguineus* and *Rhipicephalus turanicus.*

In another embodiment, the compounds and compositions of the invention may be used to treat, control and/or prevent an ectoparasitic infestation by one or more of the following fly species: *Calliflora stygia, Cochliomyia hominivorax, Chrysomya rufifacies, Culicoides actoni, Culicoides brevitarsis, Culicoides fulvus, Culicoides imicola, Culicoides insignis, Culicoides nubeculosus, Culicoides variipennis, Dermatobia hominis, Gasterophilus pecorum, Gasterophilus intestinalis, Gasterophilus nasalis, Gasterophilus haemorrhoidalis, Gasterophilus inermis, Glossina morsitans, Glossina brevipalpis, Glossina tachinoides, Glossina fuscipes, Glossina palpalis, Glossina pallidipes, Glossina austeni,, Haematobia irritans, Haematobia irritans exigua, Haematobia stimulans, Hypoderma bovis, Hypoderma lineatum, Hypoderma diana, Hypoderma actaeon, Hypoderma tarandi, Lucilia cuprina, Lucilia sericata, Lutzomyia longipalpis, Lutzomyia shannoni, Lutzomyia anthophora, Musca autumnalis, Oestrus ovis, Phlebotomus argentipes, Phlebotomus papatasi, Stomoxys calcitrans* and *Wohlfahrtia magnifica.*

In another embodiment, the compounds and compositions of the invention may be used to treat, control and/or prevent an ectoparasitic infestation by one or more of the following mite species: *Chorioptes bovis, Chorioptes texanus, Cheyletiella blakei, Cheyletiella parasitovorax, Cheyletiella yasguri, Demodex brevis, Demodex canis, Demodex catis, Demodex cornei, Demodex follicolorum, Demodex gatoi, Dermanyssus gallinae, Demodex injai, Lynxacarus radovskyi, Notoedres cati, Ornithonyssus bursae, Ornithonyssus sylviarum, Otodectes cynotis, Pneumonyssoides caninum, Psorergates ovis, Psoroptes cervinus, Psoroptes cuniculi, Psoroptes equi, Psoroptes natalensis, Psoroptes ovis, Sarcoptes anthracis, Sarcoptes scabiei* and *Sarcoptes scabiei var. canis.*

In another embodiment, the compounds and compositions of the invention may be used to treat, control and/or prevent an ectoparasitic infestation by one or more of the following lice species: *Bovicola bovis, Bovicola caprae, Bovicola limbatus, Bovicola ovis, Damalinia bovis, Felicola subrostratus, Gliricola porcelli, Gyropus ovalis, Haematopinus eurysternus, Haematopinus quadripertusis, Holakartikos crassipes, Linognathus africanus, Linognathus ovillus, Linognathus pedalis, Linognathus setosus, Linognathus stenopsis, Linognathus vituli, Solenopotes capillatus, Trichodectes canis, Trimenopan hispidum, Trixacarus caviae, Trixacarus diversus* and *Werneckiella equi.*

In yet another embodiment, the compounds and compositions of the invention may be used to treat, control and/or prevent an ectoparasitic infestation in an animal by one or more of the following mosquito species: *Aedes aegypti, Aedes albopictus, Aedes serratus, Anopheles gambiae, Culex pipiens*, *Haemagogus janthinomys* and *Haemagogus leucocelaenus.*

The herein disclosed list is not exhaustive and other ectoparasites are well known in the art to be harmful to animals and humans. These include, for example migrating dipteran larvae.

In each aspect of the invention, the compounds and pharmaceutical compositions of the invention can be applied against a single pest or combinations thereof.

### AGRICULTURAL AND HORTICULTURAL PESTS AND NEMATODES

The agricultural and horticultural insecticidal and acaricidal agent comprising the compound of formula (I), (Ia) and/or (Ib) of the present invention or a salt thereof as an active ingredient is suitable for controlling a variety of pests which may damage paddy rice, fruit trees, vegetables, other crops and ornamental flowering plants. The target pests are, for example, agricultural and forest pests, horticultural pests, stored grain pests, sanitary pests, other pests such as nematodes, or mites, etc.

Examples of the above pests or nematodes include the following.

Examples of the species of the order Lepidoptera include Parasa consocia, Anomis mesogona, Papilio xuthus, Matsumuraeses azukivora, Ostrinia scapulalis, Spodoptera exempta, Hyphantria cunea, Ostrinia furnacalis, Pseudaletia separata, Tinea translucens, Bactra furfurana, Parnara guttata, Marasmia exigua, Parnara guttata, Sesamia inferens, Brachmia triannulella, Monema flavescens, Trichoplusia ni, Pleuroptya ruralis, Cystidia couaggaria, Lampides boeticus, Cephonodes hylas, Helicoverpa armigera, Phalerodonta manleyi, Eumeta japonica, Pieris brassicae, Malacosoma neustria testacea, Stathmopoda masinissa, Cuphodes diospyrosella, Archips xylosteanus, Agrotis segetum, Tetramoera schistaceana, Papilio machaon hippocrates, Endoclyta sinensis, Lyonetia prunifoliella, Phyllonorycter ringoneella, Cydia kurokoi, Eucoenogenes aestuosa, Lobesia botrana, Latoia sinica, Euzophera batangensis, Phalonidia mesotypa, Spilosoma imparilis, Glyphodes pyloalis, Olethreutes mori, Tineola bisselliella, Endoclyta excrescens, Nemapogon granellus, Synanthedon hector, Cydia pomonella, Plutella xylostella, Cnaphalocrocis medinalis, Sesamia calamistis, Scirpophaga incertulas, Pediasia teterrellus, Phthorimaea operculella, Stauropus fagi persimilis, Etiella zinckenella, Spodoptera exigua, Palpifer sexnotata, Spodoptera mauritia, Scirpophaga innotata, Xestia c-nigrum, Spodoptera depravata, Ephestia kuehniella, Angerona prunaria, Clostera anastomosis, Pseudoplusia includens, Matsumuraeses falcana, Helicoverpa assulta, Autographa nigrisigna, Agrotis ipsilon, Euproctis pseudoconspersa, Adoxophyes orana, Caloptilia theivora, Homona magnanima, Ephestia elutella, Eumeta minuscula, Clostera anachoreta, Heliothis maritima, Sparganothis pilleriana, Busseola fusca, Euproctis subflava, Biston robustum, Heliothis zea, Aedia leucomelas, Narosoideus flavidorsalis, Viminia rumicis, Bucculatrix pyrivorella, Grapholita molesta, Spulerina astaurota, Ectomyelois pyrivorella, Chilo suppressalis, Acrolepiopsis sapporensis, Plodia interpunctella, Hellula undalis, Sitotroga cerealella, Spodoptera litura, a species of the family Tortricidae (Eucosma aporema), Acleris comariana, Scopelodes contractus, Orgyia thyellina, Spodoptera frugiperda, Ostrinia zaguliaevi, Naranga aenescens, Andraca bipunctata, Paranthrene regalis, Acosmeryx castanea, Phyllocnistis toparcha, Endopiza viteana, Eupoecillia ambiguella, Anticarsia gemmatalis, Cnephasia cinereipalpana, Lymantria dispar, Dendrolimus spectabilis, Leguminivora glycinivorella, Maruca testulalis, Matsumuraeses phaseoli, Caloptilia soyella, Phyllocnistis citrella, Omiodes indicata, Archips fuscocupreanus, Acanthoplusia agnata, Bambalina sp., Carposina niponensis, Conogethes punctiferalis, Synanthedon sp., Lyonetia clerkella, Papilio helenus, Colias erate poliographus, Phalera flavescens, the species of the family Pieridae such as Pieris rapae crucivora and Pieris rapae, Euproctis similis, Acrolepiopsis suzukiella, Ostrinia nubilalis, Mamestra brassicae, Ascotis selenaria, Phtheochroides clandestina, Hoshinoa adumbratana, Odonestis pruni japonensis, Triaena intermedia, Adoxophyes orana fasciata, Grapholita inopinata, Spilonota ocellana, Spilonota lechriaspis, Illiberis pruni, Argyresthia conjugella, Caloptilia zachrysa, Archips breviplicanus, Anomis flava, Pectinophora gossypiella, Notarcha derogata, Diaphania indica, Heliothis virescens and Earias cupreoviridis.

Examples of the species of the order Hemiptera include Nezara antennata, Stenotus rubrovittatus, Graphosoma rubrolineatum, Trigonotylus coelestialium, Aeschynteles maculatus, Creontiades pallidifer, Dysdercus cingulatus, Chrysomphalus ficus, Aonidiella aurantii, Graptopsaltria nigrofuscata, Blissus leucopterus, Icerya purchasi, Piezodorus hybneri, Lagynotomus elongatus, Thaia subrufa, Scotinophara lurida, Sitobion ibarae, Stariodes iwasakii, Aspidiotus destructor, Taylorilygus pallidulus, Myzus mumecola, Pseudaulacaspis prunicola, Acyrthosiphon pisum, Anacanthocoris striicornis, Ectometopterus micantulus, Eysarcoris lewisi, Molipteryx fuliginosa, Cicadella viridis, Rhopalosophum rufiabdominalis, Saissetia oleae, Trialeurodes vaporariorum, Aguriahana quercus, Lygus spp., Euceraphis punctipennis, Andaspis kashicola, Coccus pseudomagnoliarum, Cavelerius saccharivorus, Galeatus spinifrons, Macrosiphoniella sanborni, Aonidiella citrina, Halyomorpha mista, Stephanitis fasciicarina, Trioza camphorae, Leptocorisa chinensis, Trioza quercicola, Uhlerites latius, Erythroneura comes, Paromius exiguus, Duplaspidiotus claviger, Nephotettix nigropictus, Halticiellus insularis, Perkinsiella saccharicida, Psylla malivorella, Anomomeura mori, Pseudococcus longispinis, Pseudaulacaspis pentagona, Pulvinaria kuwacola, Apolygus lucorum, Togo hemipterus, Toxoptera aurantii, Saccharicoccus sacchari, Geoica lucifuga, Numata muiri, Comstockaspis perniciosa, Unaspis citri, Aulacorthum solani, Eysarcoris ventralis, Bemisia argentifolii, Cicadella spectra, Aspidiotus hederae, Liorhyssus hyalinus, Calophya nigridorsalis, Sogatella furcifera, Megoura crassicauda, Brevicoryne brassicae, Aphis glycines, Leptocorisa oratorius, Nephotettix virescens, Uroeucon formosanum, Cyrtopeltis tennuis, Bemisia tabaci, Lecanium persicae, Parlatoria theae, Pseudaonidia paeoniae, Empoasca onukii, Plautia stali, Dysaphis tulipae, Macrosiphum euphorbiae, Stephanitis pyrioides, Ceroplastes ceriferus, Parlatoria camelliae, Apolygus spinolai, Nephotettix cincticeps, Glaucias subpunctatus, Orthotylus flavosparsus, Rhopalosiphum maidis, Peregrinus maidis, Eysarcoris parvus, Cimex lectularius, Psylla abieti, Nilaparvata lugens, Psylla tobirae, Eurydema rugosum, Schizaphis piricola, Psylla pyricola, Parlatoreopsis pyri, Stephanitis nashi, Dysmicoccus wistariae, Lepholeucaspis japonica, Sappaphis piri, Lipaphis erysimi, Neotoxoptera formosana, Rhopalosophum nymphaeae, Edwardsiana rosae, Pinnaspis aspidistrae, Psylla alni, Speusotettix subfusculus, Alnetoidia alneti, Sogatella panicicola, Adelphocoris lineolatus, Dysdercus poecilus, Parlatoria ziziphi, Uhlerites debile, Laodelphax striatellus, Eurydema pulchrum, Cletus trigonus, Clovia punctata, Empoasca sp., Coccus hesperidum, Pachybrachius luridus, Planococcus kraunhiae, Stenotus binotatus, Arboridia apicalis, Macrosteles fascifrons, Dolycoris baccarum, Adelphocoris triannulatus, Viteus vitifolii, Acanthocoris sordidus, Leptocorisa acuta, Macropes obnubilus, Cletus punctiger, Riptortus clavatus, Paratrioza cockerelli, Aphrophora costalis, Lygus disponsi, Lygus saundersi, Crisicoccus pini, Empoasca abietis, Crisicoccus matsumotoi, Aphis craccivora, Megacopta punctatissimum, Eysarcoris guttiger, Lepidosaphes beckii, Diaphorina citri, Toxoptera citricidus, Planococcus citri, Dialeurodes citri, Aleurocanthus spiniferus, Pseudococcus citriculus, Zyginella citri, Pulvinaria citricola, Coccus discrepans, Pseudaonidia duplex, Pulvinaria aurantii, Lecanium corni, Nezara viridula, Stenodema calcaratum, Rhopalosiphum padi, Sitobion akebiae, Schizaphis graminum, Sorhoanus tritici, Brachycaudus helichrysi, Carpocoris purpureipennis, Myzus persicae, Hyalopterus pruni, Aphis farinose yanagicola, Metasalis populi, Unaspis yanonensis, Mesohomotoma camphorae, Aphis spiraecola, Aphis pomi, Lepidosaphes ulmi, Psylla mali, Heterocordylus flavipes, Myzus malisuctus, Aphidonuguis mali, Orientus ishidai, Ovatus malicolens, Eriosoma lanigerum, Ceroplastes rubens and Aphis gossypii.

Examples of the species of the order Coleoptera include Xystrocera globosa, Paederus fuscipes, Eucetonia roelofsi, Callosobruchus chinensis, Cylas formicarius, Hypera postica, Echinocnemus squameus, Oulema oryzae, Donacia provosti, Lissorhoptrus oryzophilus, Colasposoma dauricum, Euscepes postfasciatus, Epilachna varivestis, Acanthoscelides obtectus, Diabrotica virgifera virgifera, Involvulus cupreus, Aulacophora femoralis, Bruchus pisorum, Epilachna vigintioctomaculata, Carpophilus dimidiatus, Cassida nebulosa, Luperomorpha tunebrosa, Phyllotreta striolata, Psacothea hilaris, Aeolesthes chrysothrix, Curculio sikkimensis, Carpophilus hemipterus, Oxycetonia jucunda, Diabrotica spp., Mimela splendens, Sitophilus zeamais, Tribolium castaneum, Sitophilus oryzae, Palorus subdepressus, Melolontha japonica, Anoplophora malasiaca, Neatus picipes, Leptinotarsa decemlineata, Diabrotica undecimpunctata howardi, Sphenophorus venatus, Crioceris quatuordecimpunctata, Conotrachelus nenuphar, Ceuthorhynchidius albosuturalis, Phaedon brassicae, Lasioderma serricorne, Sitona japonicus, Adoretus tenuimaculatus, Tenebrio molitor, Basilepta balyi, Hypera nigrirostris, Chaetocnema concinna, Anomala cuprea, Heptophylla picea, Epilachna vigintioctopunctata, Diabrotica longicornis, Eucetonia pilifera, Agriotes spp., Attagenus unicolor japonicus, Pagria signata, Anomala rufocuprea, Palorus ratzeburgii, Alphitobius laevigatus, Anthrenus verbasci, Lyctus brunneus, Tribolium confusum, Medythia nigrobilineata, Xylotrechus pyrrhoderus, Epitrix cucumeris, Tomicus piniperda, Monochamus alternatus, Popillia japonica, Epicauta gorhami, Sitophilus zeamais, Rhynchites heros, Listroderes costirostris, Callosobruchus maculatus, Phyllobius armatus, Anthonomus pomorum, Linaeidea aenea and Anthonomus grandis.

Examples of the species of the order Diptera include Culex pipiens pallens, Pegomya hyoscyami, Liriomyza huidobrensis, Musca domestica, Chlorops oryzae, Hydrellia sasakii, Agromyza oryzae, Hydrellia griseola, Hydrellia griseola, Ophiomyia phaseoli, Dacus cucurbitae, Drosophila suzukii, Rhacochlaena japonica, Muscina stabulans, the species of the family Phoridae such as Megaselia spiracularis, Clogmia albipunctata, Tipula aino, Phormia regina, Culex tritaeniorhynchus, Anopheles sinensis, Hylemya brassicae, Asphondylia sp., Delia platura, Delia antiqua, Rhagoletis cerasi, Culex pipiens molestus Forskal, Ceratitis capitata, Bradysia agrestis, Pegomya cunicularia, Liriomyza sativae, Liriomyza bryoniae, Chromatomyia horticola, Liriomyza chinensis, Culex quinquefasciatus, Aedes aegypti, Aedes albopictus, Liriomyza trifolii, Liriomyza sativae, Dacus dorsalis, Dacus tsuneonis, Sitodiplosis mosellana, Meromuza nigriventris, Anastrepha ludens and Rhagoletis pomonella.

Examples of the species of the order Hymenoptera include Pristomyrmex pungens, the species of the family Bethylidae, Monomorium pharaonis, Pheidole noda, Athalia rosae, Dryocosmus kuriphilus, Formica fusca japonica, the species of the subfamily Vespinae, Athalia infumata infumata, Arge pagana, Athalia japonica, Acromyrmex spp., Solenopsis spp., Arge mali and Ochetellus glaber.

Examples of the species of the order Orthoptera include Homorocoryphus lineosus, Gryllotalpa sp., Oxya hyla intricata, Oxya yezoensis, Locusta migratoria, Oxya japonica, Homorocoryphus jezoensis and Teleogryllus emma.

Examples of the species of the order Thysanoptera include Selenothrips rubrocinctus, Stenchaetothrips biformis, Haplothrips aculeatus, Ponticulothrips diospyrosi, Thrips flavus, Anaphothrips obscurus, Liothrips floridensis, Thrips simplex, Thrips nigropilosus, Heliothrips haemorrhoidalis, Pseudodendrothrips mori, Microcephalothrips abdominalis, Leeuwenia pasanii, Litotetothrips pasaniae, Scirtothrips citri, Haplothrips chinensis, Mycterothrips glycines, Thrips setosus, Scirtothrips dorsalis, Dendrothrips minowai, Haplothrips niger, Thrips tabaci, Thrips alliorum, Thrips hawaiiensis, Haplothrips kurdjumovi, Chirothrips manicatus, Frankliniella intonsa, Thrips coloratus, Franklinella occidentalis, Thrips palmi, Frankliniella lilivora and Liothrips vaneeckei.

Examples of the species of the order Acari include Leptotrombidium akamushi, Tetranychus ludeni, Dermacentor variabilis, Tetranychus truncatus, Ornithonyssus bacoti, Demodex canis, Tetranychus viennensis, Tetranychus kanzawai, the species of the family Ixodidae such as Rhipicephalus sanguineus, Cheyletus malaccensis, Tyrophagus putrescentiae, Dermatophagoides farinae, Latrodectus hasseltii, Dermacentor taiwanensis, Acaphylla theavagrans, Polyphagotarsonemus latus, Aculops lycopersici, Ornithonyssus sylvairum, Tetranychus urticae, Eriophyes chibaensis, Sarcoptes scabiei, Haemaphysalis longicornis, Ixodes scapularis, Tyrophagus similis, Cheyletus eruditus, Panonychus citri, Cheyletus moorei, Brevipalpus phoenicis, Octodectes cynotis, Dermatophagoides ptrenyssnus, Haemaphysalis flava, Ixodes ovatus, Phyllocoptruta citri, Aculus schlechtendali, Panonychus ulmi, Amblyomma americanum, Dermanyssus gallinae, Rhyzoglyphus robini and Sancassania sp.

Examples of the species of the order Isoptera include Reticulitermes miyatakei, Incisitermes minor, Coptotermes formosanus, Hodotermopsis japonica, Reticulitermes sp., Reticulitermes flaviceps amamianus, Glyptotermes kushimensis, Coptotermes guangzhoensis, Neotermes koshunensis, Glyptotermes kodamai, Glyptotermes satsumensis, Cryptotermes domesticus, Odontotermes formosanus, Glyptotermes nakajimai, Pericapritermes nitobei and Reticulitermes speratus.

Examples of the species of the order Blattodea include Periplaneta fuliginosa, Blattella germanica, Blatta orientalis, Periplaneta brunnea, Blattella lituricollis, Periplaneta japonica and Periplaneta americana.

Examples of the species of the phylum Nematoda include Nothotylenchus acris, Aphelenchoides besseyi, Pratylenchus penetrans, Meloidogyne hapla, Meloidogyne incognita, Globodera rostochiensis, Meloidogyne javanica, Heterodera glycines, Pratylenchus coffeae, Pratylenchus neglectus and Tylenchus semipenetrans.

Examples of the species of the phylum Mollusca include such as Pomacea canaliculata, Achatina fulica, Meghimatium bilineatum, Lehmannina valentiana, Limax flavus and Acusta despecta sieboldiana.

### COMBINATION THERAPIES

The pharmaceutical compositions comprising the compounds of the invention, including those of formula (I), (Ia) and/or (Ib), may also include other pharmaceutical active agents.

In one embodiment of the invention, one or more arylpyrazole compounds, such as a phenylpyrazole insecticide known in the art, may be combined with the compounds of the invention, including those of formula (I) in the pharmaceutical compositions of the invention. Phenylpyrazole insecticides act by blocking glutamate-activated chloride channels (GABA_{A} gated chloride) in insects.

In another embodiment of the invention, one or more macrocyclic lactones, which act as an acaricide, anthelmintic agent and/or insecticide, can be added to the pharmaceutical compositions of the invention. Macrocyclic lactones include both avermectins and milbemycins active agents.

In another embodiment of the invention, a composition comprising one or more compounds of the invention in combination with one or more molecules of a class of insecticides known as insect growth regulators (IGRs) is provided. Compounds belonging to this group are well known to the practitioner and represent a wide range of different chemical classes. These compounds all act by interfering with the development or growth of the insect pests.

In one embodiment, the IGR is a compound that mimics juvenile hormone (juvenile hormone mimic).

In another embodiment, the IGR compound is a chitin synthesis inhibitor. Chitin synthesis inhibitors act by interfering with the insect molting process.

In yet another embodiment of the invention, adulticide insecticides and acaricides can also be added to the pharmaceutical compositions of the invention. In some embodiments, the pharmaceutical compositions of the invention may include one or more antinematodal agents. Antinematodal agents (e.g. nematicides or nematocides) are active against parasitic worms such as roundworms.

In other embodiments, the pharmaceutical compositions of the invention may include one or more antitrematodal agents. Antitrematodal agents are active against trematodes, including parasitic flatworms known as flukes such as *Clonorhis sinensis, Fasciola hepatica* and *Opisthorchis* species.

One or more anticestodal compounds may also be advantageously used in the pharmaceutical compositions of the invention. Anticestodal compounds are active agents that are active against cestodes known as tapeworms.

In yet other embodiments, the pharmaceutical compositions of the invention may include one or more other active agents that are effective against arthropod parasites.

An antiparasitic agent that can be combined with one or more compounds of the invention to form a pharmaceutical composition can be one or more biologically active peptides or proteins including, but not limited to, anthelmintic cyclic depsipeptides, which act at the neuromuscular junction by stimulating presynaptic receptors belonging to the secretin receptor family resulting in the paralysis and death of parasites. Anthelmintic cyclic depsipeptides include anthelmintic cyclooctadepsipeptides known in the art.

In another embodiment, the pharmaceutical compositions of the invention may comprise one or more active agents from the neonicotinoid class of pesticides. The neonicotinoids bind and inhibit insect specific nicotinic acetylcholine receptors.

In another embodiment, the compositions of the invention may advantageously include one or more insecticidal and/or isoxazoline active agents known in the art that are potent inhibitors of gamma-aminobutyric acid (GABA)-gated chloride channels. Isoxazoline active agents are, among others, highly effective against ectoparasites, such as fleas and ticks.

In another embodiment of the invention, nodulisporic acid and/or its derivatives (a class of known acaricidal, anthelmintic, anti-parasitic and insecticidal agents) may be added to the pharmaceutical compositions of the invention.

In another embodiment, one or more anthelmintic compounds of the amino acetonitrile class (AAD) of compounds may be added to the pharmaceutical compositions of the invention. In another embodiment, one or more aryloazol-2-yl cyanoethylamino compounds may be included in the pharmaceutical compositions.

The pharmaceutical compositions of the invention may also be combined with one or more paraherquamide compounds and/or derivatives of these compounds. The paraherquamide family of compounds is a known class of compounds that include a spirodioxepino indole core with activity against certain parasites. In addition, the structurally related marcfortine family of compounds are also known and may be combined with the pharmaceutical compositions of the invention.

In another embodiment of the invention, the pharmaceutical compositions may include one or more spinosyn active agents produced by the soil actinomycete Saccharopolyspora spinosa or a semi-synthetic spinosoid active agent. The spinosyns are typically referred to as factors or components A, B, C, D, E, F, G, H, J, K, L, M, N, 0, P, Q, R, S, T, U, V, W, or Y, and any of these components, or a combination thereof, may be used in the pharmaceutical compositions of the invention.

### CHEMICAL SYNTHESIS

The compounds according to the present invention and their intermediates may be obtained using methods of synthesis which are known to the one skilled in the art and described in the literature of organic synthesis. Preferably, the compounds are obtained in analogous fashion to the methods of preparation explained more fully hereinafter, in particular as described in the experimental section. In some cases, the order in carrying out the reaction steps may be varied. Variants of the reaction methods that are known to the one skilled in the art but not described in detail here may also be used.

The general processes for preparing the compounds according to the invention will become apparent to the one skilled in the art studying the following schemes. Starting materials may be prepared by methods that are described in the literature or herein, or may be prepared in an analogous or similar manner. Any functional groups in the starting materials or intermediates may be protected using conventional protecting groups. These protecting groups may be cleaved again at a suitable stage within the reaction sequence using methods familiar to the one skilled in the art.

The Examples that follow are intended to illustrate the present invention without restricting it. The terms "ambient temperature" and "room temperature" are used interchangeably and designate a temperature of about 20 °C. The compounds of formulae (I), (Ia) and (Ib) or pharmaceutically acceptable salts thereof may be prepared by adopting one of the following reaction schemes. The starting materials for their preparation can be prepared by methods known per se and as described in the literature or are an intermediate of any of the other schemes detailed herein. Although the following subject matter is described in some detail by way of illustration and example for purposes of clarity of understanding, it will be understood by those skilled in the art that certain changes and modifications can be practiced within the scope of the Examples.

**Abbreviations:**

| | |
|---|---|
| ACN | acetonitrile |
| AcOH | Acetic acid |
| Alox | aluminium oxide |
| Aq. | aqueous |
| °C | degree celsius |
| CyH/CH | cyclohexane |
| conc. | concentrated |
| CV | column volumes |
| DCC | N,N'-dicyclohexylmethanediimine |
| DCM | dichloro methane |
| 1,2-DCE | 1,2-dichloro ethane |
| DEE | diethyl ether |
| DIAD | Diisopropyl azodicarboxylate |
| DIPE | diisopropylether |
| DIPEA/DIEA | N,N-diisopropylethylamine |
| 1,2-DME | 1,2-dimethyl ether |
| DMF | *N,N*-dimethylformamide |
| DMSO | dimethyl sulfoxide |
| ESI-MS | electrospray ionisation mass spectrometry |
| Et₂O | diethylether |
| EtOAc/EE/EA | ethyl acetate |
| ex | example |
| eq, equiv | equivalent |
| h, hr | hour |
| HAc | acetic acid |
| HATU | *N,N,N',N'*-tetramethyl-*O*-(7-azabenzotriazol-1-yl)uranium hexafluorophosphate |
| HCl | hydrochlorid acid |
| HNO₃ | nitric acid |
| HOAc | acetic acid |
| HPLC | high performance liquid chromatography |
| K₃PO₄ | tripotassium phosphate |
| LED | light emitting diode |
| LiHMDS | lithium-*bis*(trimethylsilyl)amide |
| LiOH | lithium hydroxide |
| LR | Lawesson's reagent |
| MeOH | methanol |
| NaH | sodium hydride |
| NaHMDS | Sodium bis(trimethylsilyl)amide |
| NaHCO₃ | sodium bicarbonate |
| Na₂SO₄ | sodium sulfate |
| NBS | *N*-bromosuccinimide |
| NClS | *N*-chlorosuccinimide |
| NH₃ | ammonia |
| NH₄Cl | ammonium chloride |
| NIS | *N*-iodosuccinimide |
| NMP | *N*-methyl-2-pyrrolidone |
| mCPBA | 3-Chloroperoxybenzoic acid |
| min | minute |
| mL | milliliter |
| Pd/C | palladium on activated carbon |
| Pd(dppf)Cl₂ | [1,1'-*bis*(diphenylphosphino)ferrocene]dichloropalladium(II) |
| PE | petroleum ether |
| PtO₂ | platinum dioxide |
| RT | room temperature (about 20 °C) |
| Rₜ | retention time |
| sat. | saturated |
| TEA | triethylamine |
| TFA | trifluoroacetic acid |
| THF | tetrahydrofuran |
| TLC | thin-layer chromatography on SiO₂ |

### EXAMPLES

The following examples are included to demonstrate preferred embodiments of the invention. It should be appreciated by those of skill in the art that the techniques disclosed in the examples which follow represent techniques discovered by the inventors to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### EXAMPLE 1: Chemical Syntheses

### Synthesis of 5-bromo-3-(ethanesulfonyl)-2-[7-methyl-3-(1,1,2,2,2-pentafluoroethyl)-7H-imidazo[4,5-c]pyridazin-6-yl]pyridine

### Synthesis of N-methyl-6-(1,1,2,2,2-pentafluoroethyl)pyridazin-3-amine

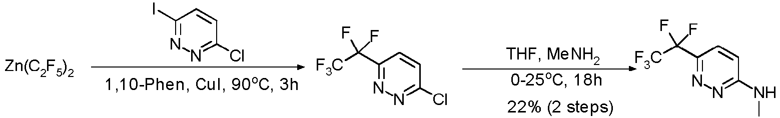

Into a 5-L 3-necked round-bottom flask, purged and maintained with an inert atmosphere of nitrogen, a solution of bis(1,1,2,2,2-pentafluoroethyl)zinc (60.0 g, 197.7 mmol, 1.0 equiv) in NMP (2.0 mL), CuI (18.8 g, 98.9 mmol, 0.5 equiv), 1,10-phenanthroline (17.8 g, 98.9 mmol, 0.5 equiv), 3-chloro-6-iodopyridazine (47.6 g, 197.8 mmol, 1 equiv) was placed. The resulting solution was stirred for 3 hr at 90 degrees C. The mixture was used in the next step without any other purification.

Into a 5-L 3-necked round-bottom flask, was placed a solution of 3-chloro-6-(1,1,2,2,2-pentafluoroethyl)pyridazine (60.0 g, 258.0 mmol, 1.0 equiv) in NMP (2 L), THF (80.0 mL). This was followed by the addition of MeNH₂ in EtOH (200 mL, 10 equiv, 35%) at 0 degrees C. The resulting solution was stirred for 18 hr at 25 degrees C. The solids were filtered out. The resulting solution was extracted with 3x800 mL of ethyl acetate. The organic layer was washed with 5 x 800 mL of brine. The resulting mixture was concentrated. The residue was applied onto a silica gel column with ethyl acetate/petroleum ether (1:1). This resulted in 13 g (22%) of N-methyl-6-(1,1,2,2,2-pentafluoroethyl)pyridazin-3-amine as a yellow solid.

### Synthesis of 4-bromo-N-methyl-6-(1,1,2,2,2-pentafluoroethyl)pyridazin-3-amine

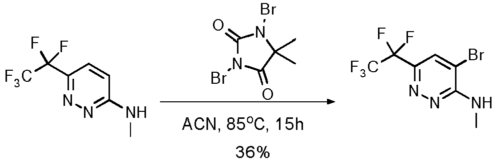

Into a 1-L round-bottom flask, a solution of N-methyl-6-(1,1,2,2,2-pentafluoroethyl)pyridazin-3-amine (29.0 g, 127.7 mmol, 1.0 equiv) was placed in ACN (400 mL), along with 1,3-Dibromo-5,5-dimethylhydantoin (80.3 g, 280.9 mmol, 2.2 equiv). The resulting solution was stirred for 15 hr at 85 degrees C. The reaction was then quenched by the addition of 500 mL of 10% NaHSO₃. The resulting solution was extracted with 2 x 500 mL of ethyl acetate. The mixture was washed with 2x500 mL of brine. The mixture was then concentrated. The residue was applied onto a silica gel column with ethyl acetate/petroleum ether (1:5-1:1). This resulted in 14.0 g (36%) of 4-bromo-N-methyl-6-(1,1,2,2,2-pentafluoroethyl)pyridazin-3-amine as a light yellow solid.

### Synthesis of N3-methyl-6-(1,1,2,2,2-pentafluoroethyl)pyridazine-3,4-diamine

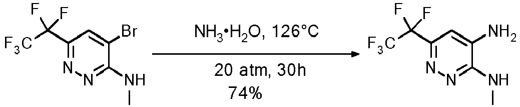

Into a 1-L pressure tank reactor, purged and maintained with an inert atmosphere of nitrogen, was placed 4-bromo-*N*-methyl-6-(1,1,2,2,2-pentafluoroethyl)pyridazin-3-amine (17.0 g, 55.5 mmol, 1.0 equiv), NH₃•H₂O (300 mL). The resulting solution was stirred for 30 hr at 126 degrees C under 20 atm N₂ atmosphere. The resulting mixture was concentrated. The residue was applied onto a silica gel column with ethyl acetate/petroleum ether (1:0). This resulted in 10.0 g (74%) of N3-methyl-6-(1,1,2,2,2-pentafluoroethyl)pyridazine-3,4-diamine as a yellow solid.

### Synthesis of 5-bromo-3-(ethylsulfanyl)-N-[3-(methylamino)-6-(1,1,2,2,2-pentafluoroethyl)pyridazin-4-yl]pyridine-2-carboxamide

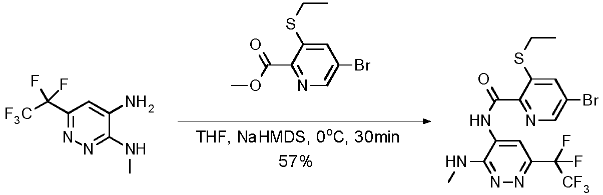

Into a 250-mL round-bottom flask, purged and maintained with an inert atmosphere of nitrogen, was placed a solution of methyl 5-bromo-3-(ethylsulfanyl)pyridine-2-carboxylate (9.0 g, 32.6 mmol, 1.0 equiv) in THF (150 mL), N3-methyl-6-(1,1,2,2,2-pentafluoroethyl)pyridazine-3,4-diamine (8.3 g, 34.2 mmol, 1.1 equiv). This was followed by the addition of NaHMDS (29.3 mL, 58.6 mmol, 1.8 equiv, 2M) dropwise with stirring at 0 degrees C. The resulting solution was stirred for 30 min at 0 degrees C. The reaction was then quenched by the addition of 500 mL of 10% NH₄Cl. The resulting solution was extracted with 2x250 mL of ethyl acetate. The organic layer was concentrated, applied onto a silica gel column with ethyl acetate/petroleum ether (1:10-1:3). This resulted in 9.0 g (57%) of 5-bromo-3-(ethylsulfanyl)-N-[3-(methylamino)-6-(1,1,2,2,2-pentafluoroethyl)pyridazin-4-yl]pyridine-2-carboxamide as a yellow solid.

### Synthesis of 5-bromo-3-(ethylsulfanyl)-2-[7-methyl-3-(1,1,2,2,2-pentafluoroethyl)-7H-imidazo[4,5-c]pyridazin-6-yl]pyridine

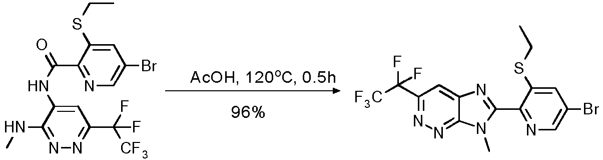

Into a 250-mL round-bottom flask, was placed 5-bromo-3-(ethylsulfanyl)-N-[3-(methylamino)-6- (1,1,2,2,2-pentafluoroethyl)pyridazin-4-yl]pyridine-2-carboxamide (6.5 g, 13.36 mmol, 1.0 equiv), AcOH (100 mL). The resulting solution was stirred for 0.5 hr at 120 degrees C. The resulting mixture was concentrated. The resulting solution was diluted with 200 mL of EA. The resulting mixture was washed with 2 x 50 mL of 10% NaHCO₃. The resulting mixture was washed with 2x50 mL of brine. The resulting mixture was concentrated. The residue was applied onto a silica gel column with ethyl acetate/petroleum ether (1:10-1:3). This resulted in 6 g (96%) of 5-bromo-3-(ethylsulfanyl)-2-[7-methyl-3-(1,1,2,2,2- pentafluoroethyl)imidazo[4,5-c]pyridazin-6-yl]pyridine as a light yellow solid.

### Synthesis of 5-bromo-3-(ethanesulfonyl)-2-[7-methyl-3-(1,1,2,2,2-pentafluoroethyl)-7H-imidazo [4,5-c] pyridazin-6-yl] pyridine

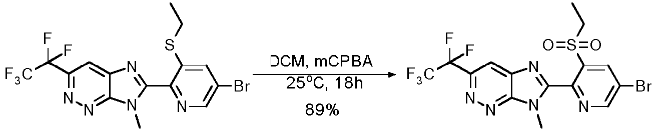

Into a 250-mL round-bottom flask, was placed a solution of 5-bromo-3-(ethylsulfanyl)-2- [7-methyl-3-(1,1,2,2,2-pentafluoroethyl)imidazo[4,5-c]pyridazin-6-yl]pyridine (8.0 g, 17.1 mmol, 1.0 equiv) in DCM (180 mL). This was followed by the addition of mCPBA (11.8 g, 68.3 mmol, 4.0 equiv) at 0 degrees C. The resulting solution was stirred for 10 hr at 0-25 degrees C. The resulting mixture was concentrated. The residue was applied onto a silica gel column with ethyl acetate/petroleum ether (1:4). This resulted in 7.6 g (89%) of 5-bromo-3-(ethanesulfonyl)-2-[7-methyl-3-(1,1,2,2,2-pentafluoroethyl) imidazo[4,5-c]pyridazin-6-yl]pyridine as a yellow solid.

### Synthesis of 5-bromo-3-(ethanesulfonyl)-2-[3-methyl-6-(1,1,2,2,2-pentafluoroethyl)imidazo[4,5-b]pyridin-2-yl]pyridine

### Synthesis of 2-chloro-5-(1,1,2,2,2-pentafluoroethyl)pyridine

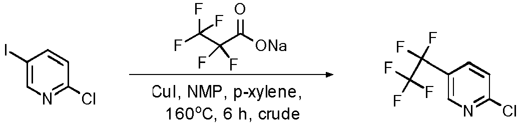

Into a 500-mL 3-necked round-bottom flask, purged and maintained with an inert atmosphere of nitrogen, was placed 2-chloro-5-iodopyridine (20.0 g, 83.5 mmol, 1.0 equiv), sodium pentafluoropropanoate (77.7 g, 417.6 mmol, 5.0 equiv), CuI (47.7 g, 250.5 mmol, 3.0 equiv), NMP (84.0 mL), p-Xylene (84.0 mL). The resulting solution was stirred for 6 hr at 160 degrees C. The reaction mixture was cooled to room temperature. The reaction was then quenched by the addition of 200 mL of water. The resulting solution was extracted with 5x300 mL of MTBE. The resulting mixture was washed with 1x500 mL of brine. The mixture was dried over anhydrous sodium sulfate and concentrated. This resulted in 35.0 g (crude) of 2-chloro-5-(1,1,2,2,2-pentafluoroethyl)pyridine as a brown liquid.

### Synthesis of N-methyl-5-(1,1,2,2,2-pentafluoroethyl)pyridin-2-amine

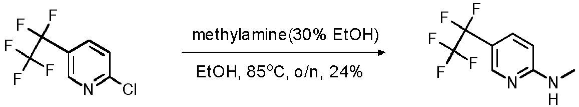

Into a 1000-mL pressure tank reactor, was placed 2-chloro-5-(1,1,2,2,2-pentafluoroethyl)pyridine (24 g, 1.0 equiv, crude), methylamine ethanol solution (50 mL) and EtOH (300 mL). The resulting solution was stirred overnight at 85 degrees C. The reaction mixture was cooled to room temperature. The resulting mixture was concentrated. The residue was applied onto a silica gel column with ethyl acetate/petroleum ether (1:10-1:4). This resulted in 3.8 g of N-methyl-5-(1,1,2,2,2-pentafluoroethyl)pyridin-2-amine as an off-white solid.

### Synthesis of N-methyl-3-nitro-5-(1,1,2,2,2-pentafluoroethyl)pyridin-2-amine

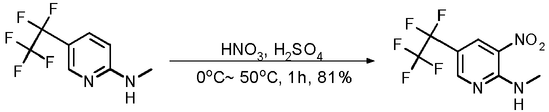

Into a 250-mL 3-necked round-bottom flask, purged and maintained with an inert atmosphere of nitrogen, was placed N-methyl-5-(1,1,2,2,2-pentafluoroethyl)pyridin-2-amine (3.8 g, 16.8 mmol, 1.0 equiv), H₂SO₄ (80 mL). This was followed by the addition of HNO₃ (2.4 g, 25.1 mmol, 1.5 equiv, 65%) dropwise with stirring at 0 degrees C. The resulting solution was stirred for 1 hr at 50 degrees C. The reaction mixture was cooled to room temperature. The reaction was then quenched by the addition of 300 mL of water/ice. The pH value of the solution was adjusted to 8-9 with Na₂CO₃. The resulting solution was extracted with 3 x 100 mL of ethyl acetate. The resulting mixture was washed with 1 x 100 mL of brine. The mixture was dried over anhydrous sodium sulfate and concentrated. The residue was applied onto a silica gel column with ethyl acetate/petroleum ether (1:10-1:5). This resulted in 3.7 g (81%) of N-methyl-3-nitro-5-(1,1,2,2,2-pentafluoroethyl)pyridin-2-amine as a yellow solid.

### Synthesis of N2-methyl-5-(1,1,2,2,2-pentafluoroethyl)pyridine-2,3-diamine

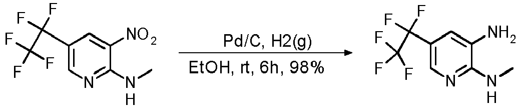

Into a 250-mL round-bottom flask, was placed N-methyl-3-nitro-5-(1,1,2,2,2-pentafluoroethyl)pyridin-2-amine (3.20 g, 11.8 mmol, 1.0 equiv), EtOH (70 mL), Pd/C (640 mg). The flask was evacuated and flushed three times with nitrogen, followed by flushing with hydrogen. The resulting mixture was stirred 6 h at room temperature under an atmosphere of hydrogen. The solids were filtered out. The resulting mixture was concentrated. This resulted in 2.8 g (98%) of N2-methyl-5-(1,1,2,2,2-pentafluoroethyl)pyridine-2,3-diamine as an off-white solid.

### Synthesis of 5-bromo-3-(ethylsulfanyl)-N-[2-(methylamino)-5-(1,1,2,2,2-pentafluoroethyl)pyridin-3-yl]pyridine-2-carboxamide

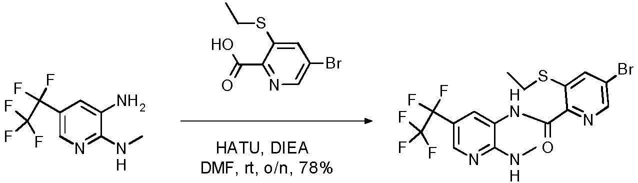

Into a 250-mL 3-necked round-bottom flask, purged and maintained with an inert atmosphere of nitrogen, was placed N2-methyl-5-(1,1,2,2,2-pentafluoroethyl)pyridine-2,3-diamine (3.2 g, 13.2 mmol, 1.0 equiv), 5-bromo-3-(ethylsulfanyl)pyridine-2-carboxylic acid (3.8 g, 14.6 mmol, 1.1 equiv), HATU (7.6 g, 19.9 mmol, 1.5 equiv), DMF (70.0 mL), DIEA (5.1 g, 39.8 mmol, 3.0 equiv). The resulting solution was stirred overnight at room temperature. The reaction was then quenched by the addition of 200 mL of water. The resulting solution was extracted with 3 x 100 mL of ethyl acetate. The resulting mixture was washed with 1 x 100 mL of brine. The mixture was dried over anhydrous sodium sulfate and concentrated. The residue was applied onto a silica gel column with ethyl acetate/petroleum ether (1:10-1:3). This resulted in 5.0 g (78%) of 5-bromo-3-(ethylsulfanyl)-N-[2-(methylamino)-5-(1,1,2,2,2-pentafluoroethyl)pyridin-3-yl]pyridine-2-carboxamide as an off-white solid.

### Synthesis of 5-bromo-3-(ethylsulfanyl)-2-[3-methyl-6-(1,1,2,2,2-pentafluoroethyl)imidazo[4,5-b]pyridin-2-yl]pyridine

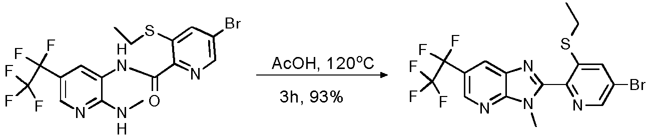

Into a 250-mL round-bottom flask purged and maintained with an inert atmosphere of nitrogen, was placed 5-bromo-3-(ethylsulfanyl)-N-[2-(methylamino)-5-(1,1,2,2,2-pentafluoroethyl)pyridin-3-yl]pyridine-2-carboxamide (5.0 g, 10.3 mmol, 1.0 equiv), AcOH (100 mL). The resulting solution was stirred for 3 h at 120 degrees C. The reaction mixture was cooled to room temperature. The resulting mixture was concentrated. The residue was applied onto a silica gel column with ethyl acetate/petroleum ether (1:10-1:3). This resulted in 4.5 g (93%) of 5-bromo-3-(ethylsulfanyl)-2-[3-methyl-6-(1,1,2,2,2-pentafluoroethyl)imidazo[4,5-b]pyridin-2-yl]pyridine as an off-white solid.

### Synthesis of 5-bromo-3-(ethanesulfonyl)-2-[3-methyl-6-(1,1,2,2,2-pentafluoroethyl)imidazo[4,5-b]pyridin-2-yl]pyridine

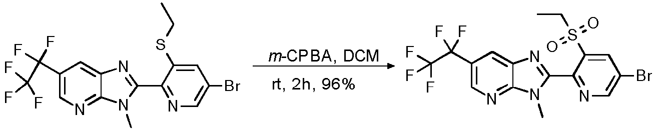

Into a 250-mL 3-necked round-bottom flask purged and maintained with an inert atmosphere of nitrogen, was placed 5-bromo-3-(ethylsulfanyl)-2-[3-methyl-6-(1,1,2,2,2-pentafluoroethyl)imidazo[4,5-b]pyridin-2-yl] pyridine (4.5 g, 9.6 mmol, 1.0 equiv), DCM (90 mL). This was followed by the addition of mCPBA (4.3 g, 21.1 mmol, 2.2 equiv, 85%), in portions at room temperature. The resulting solution was stirred for 2 hr at room temperature. The resulting mixture was washed with 1x50 ml of 10%Na₂SO₃ and 1x50 mL of 10%NaHCO₃, then 1x50 mL of brine. The organic phase was collected and dried over anhydrous sodium sulfate and concentrated. The residue was applied onto a silica gel column with ethyl acetate/petroleum ether (1:10-1:3). This resulted in 4.6 g (96%) of 5-bromo-3-(ethanesulfonyl)-2-[3-methyl-6-(1,1,2,2,2-pentafluoroethyl)imidazo[4,5-b]pyridin-2-yl]pyridine as an off-white solid.

### Synthesis of 11-[5-bromo-3-(ethanesulfonyl)pyridin-2-yl]-5,5-difluoro-12-methyl-4,6-dioxa-10,12-diazatricyclo[7.3.0.0^[3,7]]dodeca-1,3(7),8,10-tetraene

### Synthesis of 2,2-difluoro-1,3-benzodioxol-5-amine

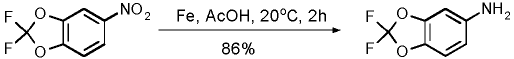

Into a 250-mL round-bottom flask, was placed 2,2-difluoro-5-nitro-1,3-benzodioxole (10.0 g, 49 mmol, 1.0 equiv), AcOH (100 mL), Fe (8.3 g, 148 mmol, 3.0 equiv). The resulting solution was stirred for 2 hr at 20 degrees C. The resulting mixture was concentrated. The resulting solution was diluted with 200 mL of H₂O. The pH value of the solution was adjusted to 9 with Na₂CO₃ (Sat.). The resulting solution was extracted with 3 x 100 mL of ethyl acetate. The organic phase dried over anhydrous sodium sulfate and concentrated. The residue was applied onto a silica gel column with ethyl acetate/petroleum ether (1/1). The collected fractions were combined and concentrated. This resulted in 8.1 g (86%) of 2,2-difluoro-1,3-benzodioxol-5-amine as yellow oil.

### Synthesis of N-(2,2-difluoro-1,3-benzodioxol-5-yl)acetamide

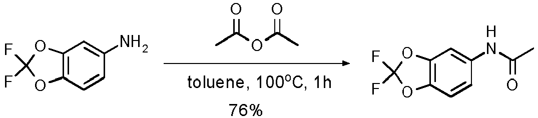

Into a 250-mL round-bottom flask, was placed 2,2-difluoro-1,3-benzodioxol-5-amine (7.6 g, 44 mmol, 1.0 equiv), toluene (80 mL), acetic anhydride (6.7 g, 66 mmol, 1.5 equiv). The resulting solution was stirred for 1 hr at 100 degrees C in an oil bath. The resulting mixture was concentrated. The crude product was purified by recrystallization from PE. This resulted in 8.23 g (76%) of N-(2,2-difluoro-1,3-benzodioxol-5-yl)acetamide as a white solid.

### Synthesis of N-(2,2-difluoro-6-nitro-1,3-benzodioxol-5-yl)acetamide

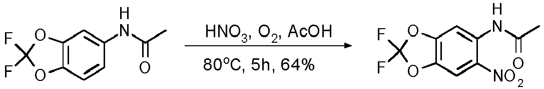

Into a 50-mL round-bottom flask, purged and maintained with an inert atmosphere of oxygen, placed N-(2,2-difluoro-1,3-benzodioxol-5-yl)acetamide (7.0 g, 33 mmol, 1.0 equiv), AcOH (70 mL), HNO₃ (7.2 g, 114 mmol, 3.5 equiv). The resulting solution was stirred for 5 hr at 80 degrees C in an oil bath. The reaction mixture was cooled. The resulting mixture was concentrated. The resulting solution was diluted with 500 mL of EA. The resulting mixture was washed with 3x250 mL of Na₂CO₃ (aq, 2M). The organic phase was dried over anhydrous sodium sulfate and concentrated. The residue was applied onto a silica gel column with ethyl acetate/petroleum ether (1/5). The collected fractions were combined and concentrated. This resulted in 5.4 g (64%) of N-(2,2-difluoro-6-nitro-1,3-benzodioxol-5-yl)acetamide as a yellow solid.

### Synthesis of N-(2,2-difluoro-6-nitro-1,3-benzodioxol-5-yl)-N-methylacetamide

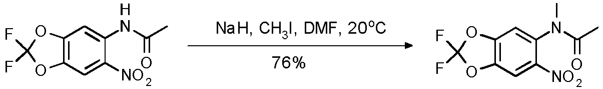

Into a 50-mL 3-necked round-bottom flask, was placed N-(2,2-difluoro-6-nitro-1,3-benzodioxol-5-yl)acetamide (5.0 g, 19.2 mmol, 1.0 equiv), DMF (20 mL), 0 degrees C was added NaH (1.15 g, 29 mmol, 1.5 equiv, 60%). The resulting solution was stirred for 10 min at 20 degrees C. Then methyl iodide (4.1 g, 29 mmol, 1.5 equiv) was added at 0 degrees C. The resulting solution was stirred for 1 hr at 20 degrees C. The reaction was then quenched by the addition of 30 mL of water and diluted with 600 mL of EA. The resulting mixture was washed with 3x300 mL of water. The organic phase was dried over anhydrous sodium sulfate and concentrated. The residue was applied onto a silica gel column with ethyl acetate/petroleum ether (1/2). The collected fractions were combined and concentrated. This resulted in 4.0 g (76%) of N-(2,2-difluoro-6-nitro-1,3-benzodioxol-5-yl)-N-methylacetamide as a yellow solid.

### Synthesis of 2,2-difluoro-N-methyl-6-nitro-1,3-benzodioxol-5-amine

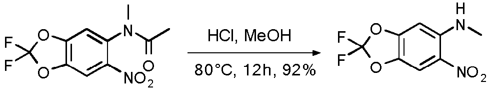

Into a 50-mL round-bottom flask, was placed N-(2,2-difluoro-6-nitro-1,3-benzodioxol-5-yl)-N-methylacetamide (3.6 g, 13 mmol, 1.0 equiv), MeOH (30 mL), HCl (10 mL). The resulting solution was stirred for 12 hr at 80 degrees C in an oil bath. The reaction mixture was cooled. The resulting mixture was concentrated. The resulting solution was diluted with 500 mL of EA. The resulting mixture was washed with 3x250 mL of Na₂CO₃ (aq, 2M). The organic phase was dried over anhydrous sodium sulfate and concentrated. The residue was applied onto a silica gel column with ethyl acetate/petroleum ether (1/4). The collected fractions were combined and concentrated. This resulted in 2.8 g (92%) of 2,2-difluoro-N-methyl-6-nitro-1,3-benzodioxol-5-amine as a yellow solid.

### Synthesis of 2,2-difluoro-N5-methyl-1,3-benzodioxole-5,6-diamine

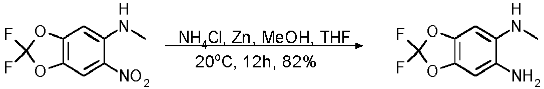

Into a 250-mL round-bottom flask, was placed 2,2-difluoro-N-methyl-6-nitro-1,3-benzodioxol-5-amine (2.8 g, 12 mmol, 1.0 equiv), THF (60 mL), MeOH (60 mL), Zn (5.5 g, 84 mmol, 7.0 equiv), NH₄Cl (6. 5 g, 120 mmol, 10 equiv). The resulting solution was stirred for 12 hr at 20 degrees C. The solids were filtered out. The filtrate was concentrated. The residue was applied onto a silica gel column with ethyl acetate/petroleum ether (1/2). The collected fractions were combined and concentrated. This resulted in 2.0 g (82%) of 2,2-difluoro-N5-methyl-1,3-benzodioxole-5,6-diamine as a brown solid.

### Synthesis of 5-bromo-N-[2,2-difluoro-6-(methylamino)-1,3-benzodioxol-5-yl]-3-(ethylsulfanyl)pyridine-2-carboxamide

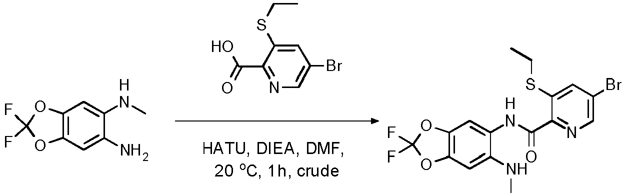

Into a 50-mL round-bottom flask, was placed 2,2-difluoro-N5-methyl-1,3-benzodioxole-5,6-diamine (350 mg, 1.7 mmol, 1.0 equiv), 5-bromo-3-(ethylsulfanyl)pyridine-2-carboxylic acid (454 mg, 1.7 mmol, 1.0 equiv), DCM (10 mL), DIEA (448 mg, 3.5 mmol, 2.0 equiv), HATU (790 mg, 2.1 mmol, 1.2 equiv). The resulting solution was stirred for 1 hr at 20 degrees C. The resulting solution was diluted with 50 mL of EA. The resulting mixture was washed with 3x20 mL of H₂O and 1x20 mL of brine. The organic phase was dried over anhydrous sodium sulfate and concentrated. This resulted in 1.75 g of 5-bromo-N-[2,2-difluoro-6-(methylamino)-1,3-benzodioxol-5-yl]-3-(ethylsulfanyl)pyridine-2-carboxamide as black oil.

### Synthesis of 11-[5-bromo-3-(ethylsulfanyl)pyridin-2-yl]-5,5-difluoro-12-methyl-4,6-dioxa-10,12-diazatricyclo[7.3.0.0^[3,7]]dodeca-1,3(7),8,10-tetraene

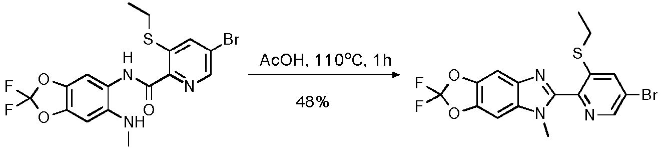

Into a 50-mL round-bottom flask, was placed 5-bromo-N-[2,2-difluoro-6-(methylamino)-1,3-benzodioxol-5-yl]-3-(ethylsulfanyl)pyridine-2-carboxamide (1.75 g, 3.9 mmol, 1.0 equiv), AcOH (20 mL). The resulting solution was stirred for 1 hr at 110 degrees C. The mixture was concentrated. The residue was applied onto a silica gel column with ethyl acetate/petroleum ether (1/4). The collected fractions were combined and concentrated. This resulted in 810 mg (48%) of 11-[5-bromo-3-(ethylsulfanyl)pyridin-2-yl]-5,5-difluoro-12-methyl-4,6-dioxa-10,12-diazatricyclo[7.3.0.0^[3,7]]dodeca-1,3(7),8,10-tetraene as light yellow oil.

### Synthesis of 11-[5-bromo-3-(ethanesulfonyl)pyridin-2-yl]-5,5-difluoro-12-methyl-4,6 dioxa-10,12-diazatricyclo[7.3.0.0^[3,7]]dodeca-1,3(7),8,10-tetraene

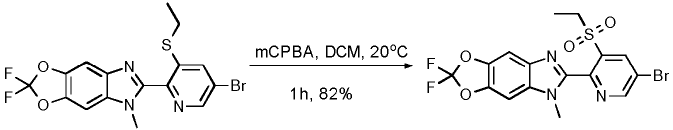

Into a 50-mL round-bottom flask, was placed 11-[5-bromo-3-(ethylsulfanyl)pyridin-2-yl]-5,5-difluoro-12-methyl-4,6-dioxa-10,12-diazatricyclo[7.3.0.0^[3,7]]dodeca-1,3(7),8,10-tetraene (700 mg, 1.6 mmol, 1.0 equiv), DCM (14 mL), m-CPBA (564 mg, 3.3 mmol, 2.0 equiv). The resulting solution was stirred for 1 hr at 20 degrees C. The resulting mixture was concentrated. The residue was applied onto a silica gel column with ethyl acetate/petroleum ether (1/3). The collected fractions were combined and concentrated. This resulted in 705 mg (82%) of 11-[5-bromo-3-(ethanesulfonyl)pyridin-2-yl]-5,5-difluoro-12-methyl-4,6-dioxa-10,12-diazatricyclo[7.3.0.0^[3,7]]dodeca-1,3(7),8,10-tetraene as a white solid.

### Synthesis of 2-[5-bromo-3-(ethanesulfonyl)pyridin-2-yl]-5-trifluoromethanesulfonyl-1,3-benzoxazole core

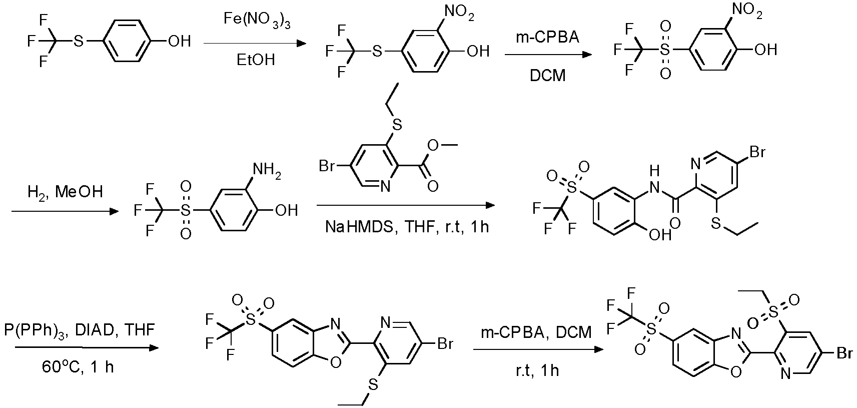

### Synthesis of 2-nitro-4-[(trifluoromethyl)sulfanyl]phenol

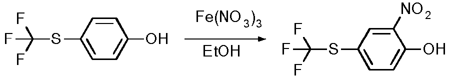

Into a 1000-mL round-bottom flask, was placed EtOH (500 mL, 10.9 mol, 55.7 equiv), 4-[(trifluoromethyl)sulfanyl]phenol (30 g, 154 mmol, 1.0 equiv), Fe(NO₃)₄ (29.9 g, 123 mmol, 0.8 equiv). The resulting solution was stirred overnight at 80 degrees C. The resulting mixture was concentrated under vacuum. The residue was applied onto a silica gel column with ethyl acetate/petroleum ether (1:5). This resulted in 30 g (81%) of 2-nitro-4-[(trifluoromethyl)sulfanyl]phenol as a yellow solid.

### Synthesis of 2-nitro-4-trifluoromethanesulfonylphenol

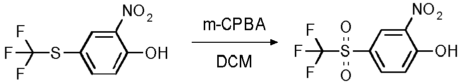

Into a 1000-mL round-bottom flask, was placed DCM (600 mL, 9.4 mol, 75.2 equiv), 2-nitro-4-[(trifluoromethyl)sulfanyl]phenol (30 g, 125 mmol, 1.0 equiv), m-CPBA (110 g, 637 mmol, 5.0 equiv). The resulting solution was stirred overnight at 50 degrees C. The solids were filtered out. The resulting mixture was concentrated under vacuum. The crude product was purified by Flash-Prep-HPLC with the following conditions (IntelFlash-1): Column, C18 silica gel; mobile phase, H₂O:ACN=70:30 increasing to H₂O:ACN=30:70 within 20 min; Detector, 254 nm. This resulted in 30 g (88%) of 2-nitro-4-trifluoromethanesulfonylphenol as light-yellow oil.

### Synthesis of 2-amino-4-trifluoromethanesulfonylphenol

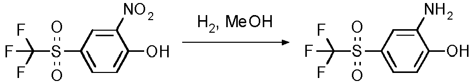

Into a 500-mL round-bottom flask, was placed MeOH (200 mL, 4.9 mol, 53.6 equiv), 2-nitro-4-trifluoromethanesulfonylphenol (25 g, 92.2 mmol, 1.0 equiv), Pd/C (20. g, 188 mmol, 2.0 equiv). To the above H₂(g) was introduced in. The resulting solution was stirred overnight at room temperature. The solids were filtered out. The filtrate was concentrated under vacuum. This resulted in 16 g (72%) of 2-amino-4-trifluoromethanesulfonylphenol as a yellow solid.

### Synthesis of 5-bromo-3-(ethylsulfanyl)-N-(2-hydroxy-5-trifluoromethanesulfonylphenyl) pyridine-2-carboxamide

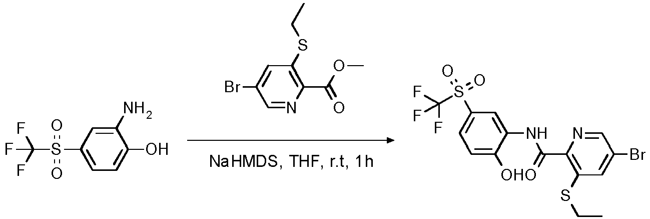

Into a 500-mL 3-necked round-bottom flask purged and maintained with an inert atmosphere of nitrogen, was placed methyl 5-bromo-3-(ethylsulfanyl)pyridine-2-carboxylate (10 g, 0.04 mmol, 1.0 equiv), THF (200 mL), 2-amino-4-trifluoromethanesulfonylphenol (8.7 g, 0.04 mmol, 1.0 equiv). This was followed by the addition of NaHMDS (36 mL) dropwise with stirring at 0 degrees C. The resulting solution was stirred for 1 hr at room temperature. The resulting solution was diluted with 40 mL of 2 M HCl. The resulting solution was extracted with 2x200 mL of ethyl acetate and the organic layers combined and dried over anhydrous sodium sulfate and concentrated. The crude product was purified by Prep-Flash with the following conditions: Column, C18 silica gel; mobile phase, 0.1% FA in water and ACN (40% ACN increasing to 70% within 14 min). Detector, UV 254 nm, 220 nm. This resulted in 5.3 g (27%) of 5-bromo-3-(ethylsulfanyl)-N-(2-hydroxy-5-trifluoromethanesulfonylphenyl)pyridine-2-carboxamide as a brown solid.

### Synthesis of 2-[5-bromo-3-(ethylsulfanyl)pyridin-2-yl]-5-trifluoromethanesulfonyl-1,3-benzoxazole

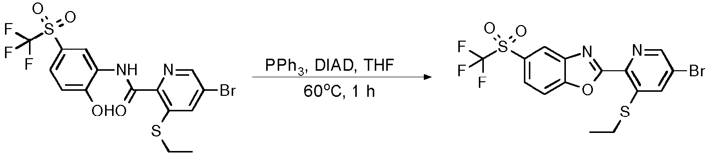

Into a 250-mL 3-necked round-bottom flask purged and maintained with an inert atmosphere of nitrogen, was placed 5-bromo-3-(ethylsulfanyl)-N-(2-hydroxy-5-trifluoromethanesulfonylphenyl)pyridine-2-carboxamide (4.8 g, 9.8 mmol, 1.0 equiv), THF (96 mL), PPh₃ (10.4 g, 39 mmol, 4 equiv), DIAD (8.0 g, 40 mmol, 4.0 equiv). The resulting solution was stirred for 1 hr at 60 degrees C. The crude product was purified by Prep-Flash with the following conditions: Column, C18 silica gel; mobile phase, 0.1% TFA in water and ACN (60% ACN increasing to 95% within 15 min). Detector, UV 254 nm, 220 nm. This resulted in 4 g (74%) of 2-[5-bromo-3-**(ethylsulfanyl)pyridin-2-yl]-5-trifluoromethanesulfonyl-1,3-benzoxazole** as a yellow solid.

### Synthesis of 2-[5-bromo-3-(ethanesulfonyl)pyridin-2-yl]-5-trifluoromethanesulfonyl-1,3-benzoxazole

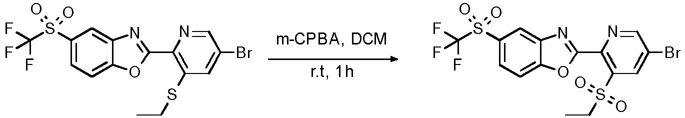

Into a 250-mL 3-necked round-bottom flask, was placed 2-[5-bromo-3-(ethylsulfanyl)pyridin-2-yl]-5-trifluoromethanesulfonyl-1,3-benzoxazole (4.0 g, 8.6 mmol, 1.0 equiv), DCM (80 mL). This was followed by the addition of m-CPBA (5.9 g, 34 mmol, 4 equiv) in several batches at 0 degrees C. The resulting solution was stirred for 1 hr at room temperature. The resulting solution was diluted with 100 mL of water. The resulting solution was extracted with 200 mL of dichloromethane and the organic layers combined. The resulting mixture was washed with 2x200 ml of NaHCO₃. The mixture was dried over anhydrous magnesium sulfate and concentrated. The residue was applied onto a silica gel column with ethyl acetate/petroleum ether (0-20%). This resulted in 3.7 g (85%) of 2-[5-bromo-3-(ethanesulfonyl)pyridin-2-yl]-5-trifluoromethanesulfonyl-1,3-benzoxazole as a white solid.

### Synthesis of 2-[5-bromo-3-(ethanesulfonyl) pyridin-2-yl]-5-cyclopropyl-3-methyl-6-(trifluoromethyl)imidazo[4,5-c]pyridin-4-one

### Synthesis of N-[2-oxo-6-(trifluoromethyl)pyran-3-yl]benzamide

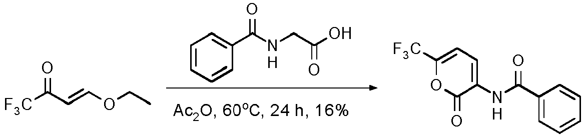

Into a 1000-mL round-bottom flask, was placed (3E)-4-ethoxy-1,1,1-trifluorobut-3-en-2-one (94 g, 559.1 mmol, 1.0 equiv), hippuric acid (100 g, 559 mmol, 1.0 equiv), Ac₂O (50 mL). The resulting solution was stirred for 24 hr at 60 degrees C. The resulting mixture was concentrated. The crude product was re-crystallized from PE:EA in the ratio of 3:1. This resulted in 25 g (16%) of N-[2-oxo-6-(trifluoromethyl)pyran-3-yl]benzamide as a red solid.

### Synthesis of N-[1-cyclopropyl-6-hydroxy-2-oxo-6-(trifluoromethyl)-5H-pyridin-3-yl]benzamide

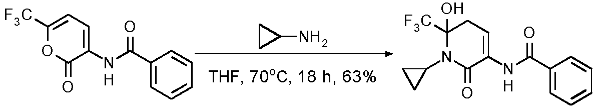

Into a 250-mL pressure tank reactor, was placed N-[2-oxo-6-(trifluoromethyl)pyran-3-yl]benzamide (21 g, 74 mmol, 1.0 equiv), aminocyclopropane (5.1 g, 89 mmol, 1.2 equiv), THF (200 mL). The resulting solution was stirred for 18 hr at 70 degrees C. The resulting mixture was concentrated. The residue was applied onto a silica gel column with ethyl acetate/petroleum ether (1:2). This resulted in 16 g (63%) of N-[1-cyclopropyl-6-hydroxy-2-oxo-6-(trifluoromethyl)-5H-pyridin-3-yl]benzamide as a yellow solid.

### Synthesis of 3-amino-1-cyclopropyl-6-(trifluoromethyl)pyridin-2-one

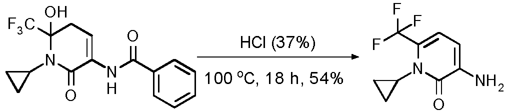

Into a 250-mL round-bottom flask, was placed N-[1-cyclopropyl-6-hydroxy-2-oxo-6-(trifluoromethyl)-5H-pyridin-3-yl]benzamide (14.0 g, 41.0 mmol, 1.0 equiv), HCl (140 mL, 37%). The resulting solution was stirred for 18 hr at 100 degrees C. The resulting solution was diluted with 150 mL of H₂O. The solids were filtered out. The resulting solution was extracted with 3x200 mL of ethyl acetate and the organic layers combined. The resulting mixture was washed with 2x200 mL of brine. The mixture was dried over anhydrous sodium sulfate and concentrated. This resulted in 4.8 g (53%) of 3-amino-1-cyclopropyl-6-(trifluoromethyl)pyridin-2-one as a grey solid.

### Synthesis of 1-cyclopropyl-N-methyl-2-methylidene-6-(trifluoromethyl)pyridin-3-amine

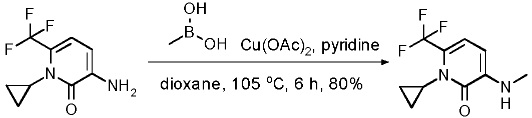

Into a 250-mL 3-necked round-bottom flask, was placed 1-cyclopropyl-2-methylidene-6-(trifluoromethyl)pyridin-3-amine (3.5 g, 16 mmol, 1.0 equiv), Cu(OAc)₂ (7.3 g, 40 mmol, 2.5 equiv), pyridine (4.4 g, 56 mmol, 3.5 equiv), dioxane (200 mL), methylboronic acid (2.4 g, 40 mmol, 2.5 equiv). The resulting solution was stirred for 20 min at room temperature. The resulting solution was allowed to react, with stirring, for an additional 6 hr at 105 degrees C. The solids were filtered out. The filtrate was concentrated. The residue was applied onto a silica gel column with ethyl acetate/petroleum ether (1:2). This resulted in 3.0 g (80%) of 1-cyclopropyl-N-methyl-2-methylidene-6-(trifluoromethyl)pyridin-3-amine as a yellow solid.

### Synthesis of 1-cyclopropyl-3-(methylamino)-4-nitro-6-(trifluoromethyl)pyridin-2-one

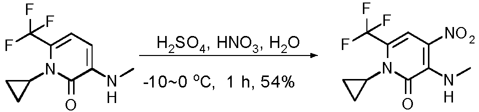

Into a 100-mL 3-necked round-bottom flask, was placed 1-cyclopropyl-3-(methylamino)-6-(trifluoromethyl)pyridin-2-one (1.85 g, 8.0 mmol, 1.0 equiv), H₂SO₄ (15 mL). This was followed by the addition of H₂O (6.0 mL) dropwise with stirring at 0 degrees. To this was added HNO₃ (0.4 mL) dropwise with stirring at - 10 degrees C. The resulting solution was stirred for 1 hr at -10~0 degrees C. The reaction was then quenched by the addition of 150 mL of water/ice. The solids were collected by filtration. The solid was dried in an oven under reduced pressure. This resulted in 1.2 g (54%) of 1-cyclopropyl-3-(methylamino)-4-nitro-6-(trifluoromethyl)pyridin-2-one as a yellow solid.

### Synthesis of 4-amino-1-cyclopropyl-3-(methylamino)-6-(trifluoromethyl)pyridin-2-one

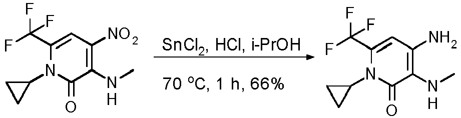

Into a 250-mL round-bottom flask, was placed 1-cyclopropyl-3-(methylamino)-4-nitro-6-(trifluoromethyl)pyridin-2-one (1.7 g, 6.1 mmol, 1.0 equiv), isopropyl alcohol (50 mL), SnCl₂ (4.2 g, 22.2 mmol, 3.6 equiv), HCl (5 mL, 37%). The resulting solution was stirred for 1 hr at 70 degrees C. The reaction was then quenched by the addition of 200 mL of water/ice. The pH value of the solution was adjusted to 12 with NaOH (6 mol/L). The resulting solution was extracted with 3 x 100 mL of ethyl acetate and the organic layers combined. The resulting mixture was washed with 2 x 200 mL of brine. The mixture was dried over anhydrous sodium sulfate. This resulted in 1 g (66%) of 4-amino-1-cyclopropyl-3-(methylamino)-6-(trifluoromethyl)pyridin-2-one as a yellow solid.

### Synthesis of 5-bromo-N-[1-cyclopropyl-3-(methylamino)-2-oxo-6-(trifluoromethyl)pyridin-4-yl]-3-(ethylsulfanyl)pyridine-2-carboxamide

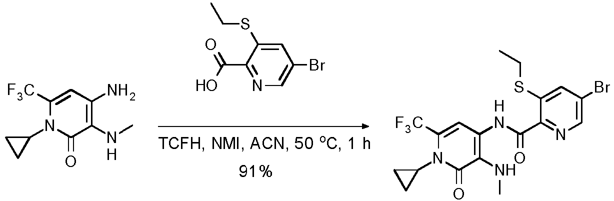

Into a 250-mL round-bottom flask, was placed 5-bromo-3-(ethylsulfanyl)pyridine-2-carboxylic acid (424 mg, 1.6 mmol, 1.0 equiv), ACN (40 mL), TCFH (500 mg, 1.8 mmol, 1.1 equiv), NMI (465 mg, 5.7 mmol, 3.5 equiv), 4-amino-1-cyclopropyl-3-(methylamino)-6-(trifluoromethyl)pyridin-2-one (400 mg, 1.6 mmol, 1.0 equiv). The resulting solution was stirred for 1 hr at 50 degrees C. The resulting mixture was concentrated. The residue was solvent with 50 ml of EA. The organic phase was washed with 3x20 mL of water and 1x20 mL of brine. The organic phase was dried over anhydrous sodium sulfate and concentrated. This resulted in 0.72 g (91%) of 5-bromo-N-[1-cyclopropyl-3- (methylamino)-2-oxo-6-(trifluoromethyl)pyridin-4-yl]-3-(ethylsulfanyl)pyridine-2-carboxamide as a yellow solid.

### Synthesis of 2-[5-bromo-3-(ethylsulfanyl)pyridin-2-yl]-5-cyclopropyl-3-methyl-6-(trifluoromethyl)imidazo[4,5-c]pyridin-4-one

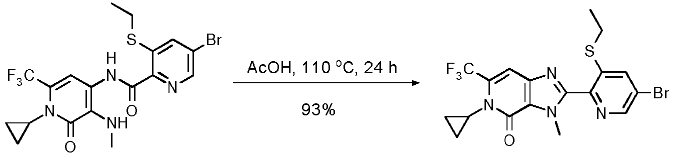

Into a 250-mL round-bottom flask, was placed 5-bromo-N-[1-cyclopropyl-3-(methylamino)-2-oxo -6-(trifluoromethyl)pyridin-4-yl]-3-(ethylsulfanyl)pyridine-2-carboxamide (0.67 g, 1.4 mmol, 1.0 equiv), AcOH (35 mL). The resulting solution was stirred for 24 hr at 110 degrees C. The reaction mixture was cooled to room temperature with a water/ice bath. The reaction was poured onto 200 mL of saturated aqueous NaHCO₃. The resulting solution was extracted with 3 x 50 mL of ethyl acetate and the organic layers combined. The organic layer was washed with 1 x 50 ml of brine. The organic phase was dried over anhydrous sodium sulfate and concentrated. The residue was applied onto a silica gel column with ethyl acetate/petroleum ether (1:4). This resulted in 0.6 g (93%) of 2-[5-bromo-3-(ethylsulfanyl)pyridin-2-yl]-5-cyclopropyl-3-methyl-6-(trifluoromethyl)imidazo[4,5-c]pyridin-4-one as a light yellow solid.

### Synthesis of 2-[5-bromo-3-(ethanesulfonyl)pyridin-2-yl]-5-cyclopropyl-3-methyl-6-(trifluoromethyl)imidazo[4,5-c]pyridin-4-one

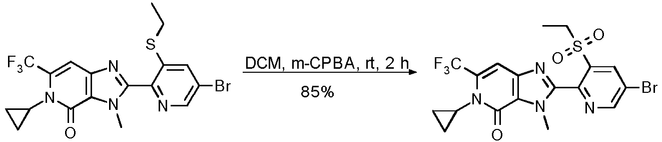

Into a 50-mL round-bottom flask, was placed 2-[5-bromo-3-(ethylsulfanyl)pyridin-2-yl]-5-cyclopropyl -3-methyl-6-(trifluoromethyl)imidazo[4,5-c]pyridin-4-one (0.55 g, 1.2 mmol, 1.0 equiv), DCM (11 mL), m-CPBA (0.4 g, 2.3 mmol, 2.0 equiv). The resulting solution was stirred for 2 hr at room temperature. The resulting mixture was washed with 1x20 ml of NaHCO₃ (sat.) and 1x20 mL of brine. The organic layer was dried over anhydrous sodium sulfate and concentrated. The residue was applied onto a silica gel column with ethyl acetate/petroleum ether (1:1). This resulted in 0.5 g (85%) of 2-[5-bromo-3-(ethanesulfonyl)pyridin-2-yl]-5-cyclopropyl-3-methyl-6-(trifluoromethyl)imidazo[4,5-c]pyridin-4-one as a white solid.

### Synthesis of 5-(5-bromo-3-ethylsulfonyl-2-pyridyl)-1-(2,2,3,3,3-pentafluoropropyl)pyrazolo[3,4-c]pyridine

### Synthesis of afford 5-bromo-1-(2,2,3,3,3-pentafluoropropyl)pyrazolo[3,4-c]pyridine

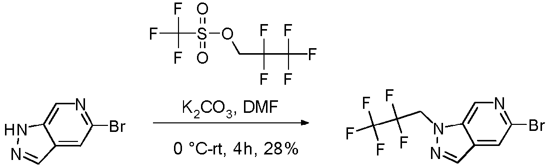

To a stirred solution of commercially available 5-bromo-1H-pyrazolo[3,4-c]pyridine (1.0 eq, 60.0 g, 303.0 mmol) in DMF (600 mL) were added K₂CO₃ (1.0 eq, 41.8 g, 303.0 mmol) and 2,2,3,3,3-pentafluoropropyl trifluoromethanesulfonate (1.0 eq, 85.5 g, 303.0 mmol) at 0 °C under a nitrogen atmosphere. The resulting mixture was stirred for 4 h at room temperature under a nitrogen atmosphere. The resulting mixture was filtered, the filter cake was washed with EtOAc (3 x 100 mL). The reaction was quenched with water/ice at room temperature. The resulting mixture was extracted with EtOAc (3 x 1 L). The combined organic layers were washed with water (3 x 1 L), dried over anhydrous Na₂SO₄. After filtration, the filtrate was concentrated under reduced pressure. The residue was purified by silica gel column chromatography, eluted with PE / EA (10:1) to afford 5-bromo-1-(2,2,3,3,3-pentafluoropropyl)pyrazolo[3,4-c]pyridine (29.0 g,84.9 mmol, 28% yield) and 5-bromo-2-(2,2,3,3,3-pentafluoropropyl)pyrazolo[3,4-c]pyridine (20.0 g,59.4 mmol, 19% yield) as an orange solid.

### Synthesis of trimethyl-[1-(2,2,3,3,3-pentafluoropropyl)pyrazolo[3,4-c]pyridin-5-yl]stannane

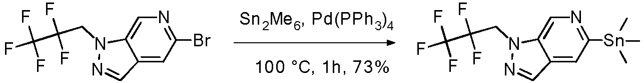

To a stirred solution of 5-bromo-1-(2,2,3,3,3-pentafluoropropyl)pyrazolo[3,4-c]pyridine (1.0 eq, 30.0 g, 90.9 mmol) in toluene (300 mL) were added Pd(PPh₃)₄ (0.4 eq, 42.0 g, 36.4 mmol) and Sn₂Me₆ (2.0 eq, 59.6 g, 182.0 mmol) at room temperature under a nitrogen atmosphere. The resulting mixture was stirred for 1 h at 100 °C under a nitrogen atmosphere. The mixture was allowed to cool down to room temperature. The resulting mixture was concentrated under reduced pressure. The residue was purified by silica gel column chromatography, eluted with PE / EA (10:1) to afford trimethyl-[1-(2,2,3,3,3-pentafluoropropyl)pyrazolo[3,4-c]pyridin-5-yl]stannane (30.0 g, 66.6 mmol, 73% yield) as a yellow oil.

### Synthesis of 5-(5-bromo-3-fluoro-2-pyridyl)-1-(2,2,3,3,3-pentafluoropropyl)pyrazolo[3,4-c]pyridine

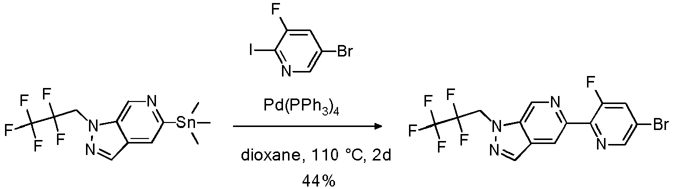

To a stirred solution of 5-bromo-3-fluoro-2-iodo-pyridine (1.0 eq, 10.9 g, 36.2 mmol) and trimethyl-[1-(2,2,3,3,3-pentafluoropropyl)pyrazolo[3,4-c]pyridin-5-yl]stannane (1.0 eq, 15.0 g, 36.2 mmol) in dioxane (300 mL) were added Pd(PPh₃)₄ (0.4 eq, 16.8 g, 14.5 mmol) at room temperature under a nitrogen atmosphere. The resulting mixture was stirred for 2 days at 110 °C under a nitrogen atmosphere. The mixture was allowed to cool down to room temperature. The resulting mixture was concentrated under reduced pressure. The residue was purified by silica gel column chromatography, eluted with PE / EA (5:1) to afford 5-(5-bromo-3-fluoro-2-pyridyl)-1-(2,2,3,3,3-pentafluoropropyl)pyrazolo[3,4-c]pyridine (8.0 g, 16.1 mmol, 44% yield) as a white solid.

### Synthesis of 5-(5-bromo-3-ethylsulfanyl-2-pyridyl)-1-(2,2,3,3,3-pentafluoropropyl)pyrazolo[3,4-c]pyridine

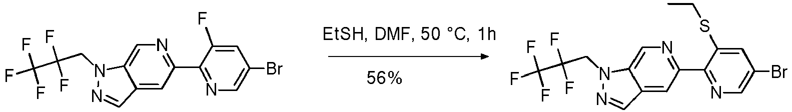

To a stirred solution of 5-(5-bromo-3-fluoro-2-pyridyl)-1-(2,2,3,3,3-pentafluoropropyl)pyrazolo[3,4-c]pyridine (1.0 eq, 8.0 g, 18.8 mmol) in DMF (80 mL) was added ethanethiol (3.0 eq, 3.5 g, 56.5 mmol) at room temperature under a nitrogen atmosphere. The resulting mixture was stirred for 1 h at 50 °C under a nitrogen atmosphere. The mixture was allowed to cool down to room temperature. The reaction was quenched by the addition of Water/Ice (80 mL) at room temperature. The resulting mixture was extracted with EtOAc (3 x 100 mL). The combined organic layers were washed with water (3 x 100 mL), dried over anhydrous Na₂SO₄. After filtration, the filtrate was concentrated under reduced pressure. The residue was purified by silica gel column chromatography, eluted with PE / EA (5:1) to afford 5-(5-bromo-3-ethylsulfanyl-2-pyridyl)-1-(2,2,3,3,3-pentafluoropropyl)pyrazolo[3,4-c]pyridine (5.0 g, 10.5 mmol, 56% yield) as an off-white solid.

### Synthesis of 5-(5-bromo-3-ethylsulfonyl-2-pyridyl)-1-(2,2,3,3,3-pentafluoropropyl)pyrazolo[3,4-c]pyridine

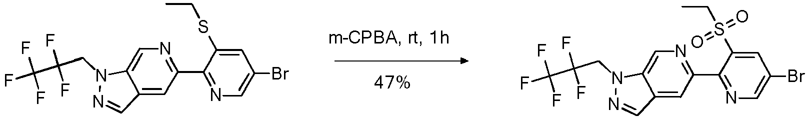

To a stirred solution of 5-(5-bromo-3-ethylsulfanyl-2-pyridyl)-1-(2,2,3,3,3-pentafluoropropyl)pyrazolo[3,4-c]pyridine (1.0 eq, 10.0 g, 21.4 mmol) in DCM (200 mL) was added m-CPBA (3.3 eq, 12.0 g, 69.6 mmol) at room temperature under a nitrogen atmosphere. The resulting mixture was stirred for 1 h at room temperature under a nitrogen atmosphere. The reaction was quenched with sat. sodium hyposulfite (aq.) at 0°C. The resulting mixture was extracted with DCM (3 x 200 mL). The combined organic layers were washed with water (3x200 mL), dried over anhydrous Na₂SO₄. After filtration, the filtrate was concentrated under reduced pressure. The residue was purified by silica gel column chromatography, eluted with PE / EA (5:1) to afford 5-(5-bromo-3-ethylsulfonyl-2-pyridyl)-1-(2,2,3,3,3-pentafluoropropyl)pyrazolo[3,4-c]pyridine (5.0 g, 10.1 mmol, 47% yield) as an off-white solid.

### Synthesis of 2-(5-bromo-3-ethylsulfonyl-2-pyridyl)-7-(trifluoromethyl)phthalazin-1-one

### Synthesis of 7-(trifluoromethyl)-2H-phthalazin-1-one

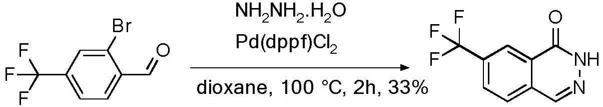

Into a 250 mL pressure tank reactor were added commercially available 2-bromo-4-(trifluoromethyl)benzaldehyde (1.0 eq, 10.0 g, 39.5 mmol), NH₂NH₂.H₂O (5.0 eq, 9.9 g, 198 mmol), Pd(dppf)Cl₂ (0.1 eq, 3.2 g, 3.9 mmol), dioxane (200 mL) at room temperature. The reaction mixture was stirred for 30 min at room temperature. Then the mixture was stirred for 2 h at 100 °C under a carbon monoxide atmosphere with 20 atm. The reaction was monitored by LCMS. The resulting mixture was concentrated under reduced pressure. The residue was purified by silica gel column chromatography, eluted with PE / THF (1:1) to afford 7-(trifluoromethyl)-2H-phthalazin-1-one (3.5 g, 13.4 mmol, 34% yield) as a yellow solid.

### Synthesis of 5-bromo-2-chloro-3-ethylsulfanyl-pyridine

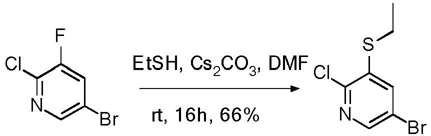

A solution of commercially available 5-bromo-2-chloro-3-fluoro-pyridine (1.0 eq, 300.0 g, 1426 mmol), ethanethiol (1.2 eq, 106.3 g, 1711 mmol) and Cs₂CO₃ (1.5 eq, 696.8 g, 2138 mmol) in DMF (6 L) was stirred for 16 h at room temperature under a nitrogen atmosphere. The resulting mixture was diluted with brine (15 L). The resulting mixture was extracted with EtOAc (3 x 5 L). The combined organic layers were washed with brine (4 x 25 L), dried over anhydrous Na₂SO₄. After filtration, the filtrate was concentrated under reduced pressure. The residue was purified by silica gel column chromatography, eluted with PE / EA (10:1) to afford 5-bromo-2-chloro-3-ethylsulfanyl-pyridine (270.0 g, 951 mmol, 67% yield) as a brown oil.

### Synthesis of 5-bromo-2-chloro-3-ethylsulfonyl-pyridine

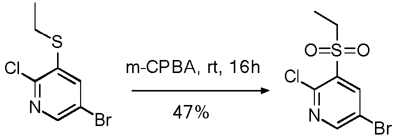

A solution of 5-bromo-2-chloro-3-ethylsulfanyl-pyridine (1.0 eq, 35.0 g, 139 mmol) and m-CPBA (3.0 eq, 71.7 g, 416 mmol) in DCM (700 mL) was stirred overnight at room temperature under a nitrogen atmosphere. The reaction was quenched with sat. sodium hyposulfite (aq.) at room temperature. The resulting mixture was extracted with CH₂Cl₂ (3 x 500 mL). The combined organic layers were washed with water (3x300 mL), dried over anhydrous Na₂SO₄. After filtration, the filtrate was concentrated under reduced pressure. The residue was purified by silica gel column chromatography, eluted with PE / THF (1:1) to afford 5-bromo-2-chloro-3-ethylsulfonyl-pyridine (38.0 g, 116 mmol, 84% yield) as a white solid.

### Synthesis of 2-(5-bromo-3-ethylsulfonyl-2-pyridyl)-7-(trifluoromethyl)phthalazin-1-one

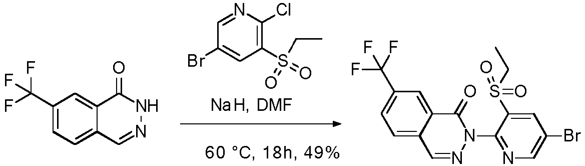

To a solution of 7-(trifluoromethyl)-2H-phthalazin-1-one (1.0 eq, 2.0 g, 7.7 mmol) in DMF (40 mL) was added NaH (2.0 eq, 0.4 g, 15.3 mmol) at 0 degrees C. The mixture was stirred for 30 min at 0 degrees. Then 5-bromo-2-chloro-3-ethylsulfonyl-pyridine (2.5 eq, 5.4 g, 19.1 mmol) was added and the mixture was allowed to warm to 60 °C and stirred overnight. The reaction was quenched by the addition of sat. NH₄Cl (aq.) (100 mL). The resulting mixture was extracted with EtOAc (2 x 100 mL). The combined organic layers were washed with brine (3 x 100 mL), dried over anhydrous Na₂SO₄. After filtration, the filtrate was concentrated under reduced pressure. The residue was purified by silica gel column chromatography, eluted with PE / THF (1:1) to afford 2-(5-bromo-3-ethylsulfonyl-2-pyridyl)-7-(trifluoromethyl)phthalazin-1-one (2.0 g, 3.8 mmol, 49% yield) as a yellow solid.

### Synthesis of 2-(5-bromo-3-ethylsulfonyl-2-pyridyl)-7-(1,1,2,2,2-pentafluoroethyl)imidazo[1,2-a]pyridine

### Synthesis of 5-bromo-3-(ethylsulfanyl)pyridine-2-carbonitrile

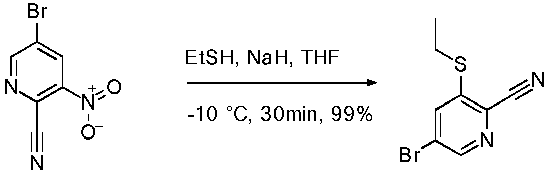

Into a 2 L 4-necked round-bottom flask, commercially available 5-bromo-3-nitropyridine-2-carbonitrile (100 g, 438.6 mmol, 1 equiv), THF (1 L) and ethanethiol (26 g, 416.9 mmol, 0.95 equiv) were added at room temperature. A solution of NaH (21.1 g, 879.2 mmol, 2 equiv) was added under a nitrogen atmosphere in portions at -10°C. The resulting mixture was stirred at -10 °C for 30 min under a nitrogen atmosphere. The reaction was quenched by the addition of Water/Ice (1 L) at room temperature. The resulting mixture was extracted with EtOAc (3 x 1 L). The combined organic layers were washed with brine (3 x 1 L), dried over anhydrous Na₂SO₄. After filtration, the filtrate was concentrated under reduced pressure. The residue was purified by trituration with PE/EA (10/1, 500 mL). The precipitated solids were collected by filtration and washed with PE (3 x 100 mL). This resulted in 5-bromo-3-(ethylsulfanyl)pyridine-2-carbonitrile (74 g, 99%) as a yellow solid.

### Synthesis of 1-[5-bromo-3-(ethylsulfanyl)pyridin-2-yl]ethan-1-one

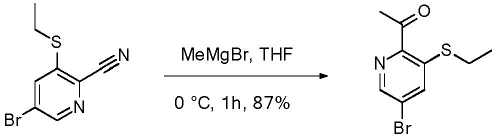

Into a 1 L 3-necked round-bottom flask, 5-bromo-3-(ethylsulfanyl)pyridine-2-carbonitrile (55 g, 224.2 mmol, 1 equiv) and THF (550 mL) were added at room temperature. A solution of bromo(methyl)magnesium (152 mL, 456 mmol, 2.0 equiv) was added dropwise at 0 °C for 20 min under a nitrogen atmosphere. The resulting mixture was stirred at 0 °C for 1 h under a nitrogen atmosphere. The reaction was quenched by the addition of HCl/ice (1M, 500 mL) at room temperature. The resulting mixture was extracted with EtOAc (3 x 500 mL). The combined organic layers were washed with brine (3 x 500 mL), dried over anhydrous Na₂SO₄. After filtration, the filtrate was concentrated under reduced pressure. The residue was purified by trituration with PE/EA (10/1, 500 mL). The precipitated solids were collected by filtration and washed with PE (3 x 100 mL). This resulted in 1-[5-bromo-3-(ethylsulfanyl)pyridin-2-yl]ethan-1-one (52 g, 78%) as a yellow solid.

### Synthesis of 1-[5-bromo-3-(ethanesulfonyl)pyridin-2-yl]ethanone

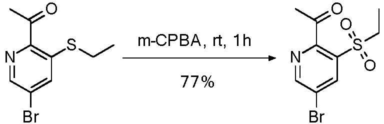

Into a 1 L 3-necked round-bottom flask, 1-[5-bromo-3-(ethylsulfanyl)pyridin-2-yl]ethanone (49 g, 185.7 mmol, 1 equiv) and DCM (490 mL) were added at room temperature. A solution of m-CPBA (98 g, 567.9 mmol, 3.1 equiv) was added under a nitrogen atmosphere in portions at 0°C. The resulting mixture was stirred at room temperature for 1 h under a nitrogen atmosphere. The mixture was then washed with saturated K₂CO₃ (aq.) (2 x 500 mL), and the combined organic layers were dried over anhydrous Na₂SO₄. After filtration, the filtrate was concentrated under reduced pressure. The residue was purified by trituration with PE/EA (10/1, 500 mL). The precipitated solids were collected by filtration and washed with PE (2 x 50 mL). This resulted in 1-[5-bromo-3-(ethanesulfonyl)pyridin-2-yl]ethanone (43 g, 77%) as a yellow solid.

### Synthesis of 2-bromo-1-[5-bromo-3-(ethanesulfonyl)pyridin-2-yl]ethan-1-one

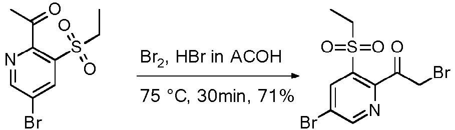

Into a 1 L 3-necked round-bottom flask, Br₂ (23 g, 143.9 mmol, 0.98 equiv), AcOH (520 mL), 1-[5-bromo-3-(ethanesulfonyl)pyridin-2-yl]ethanone (44 g, 146.5 mmol, 1 equiv) and HBr in AcOH (88 mL, 33%) were added at room temperature. The resulting mixture was stirred at 75 °C for 30 min under a nitrogen atmosphere. The mixture was then diluted with EtOAc (300 mL) and neutralized to pH 7 with saturated NaHCO₃ (aq.). The resulting mixture was extracted with EtOAc (3 x 300 mL). The combined organic layers were washed with brine (3 x 300 mL), dried over anhydrous Na₂SO₄. After filtration, the filtrate was concentrated under reduced pressure. The residue was purified by trituration with PE/EA (10/1, 200 mL). The precipitated solids were collected by filtration and washed with PE (2 x 50 mL). This resulted in 2-bromo-1-[5-bromo-3-(ethanesulfonyl)pyridin-2-yl]ethan-1-one (43 g, 71%, 90% purity) as a yellow solid.

### Synthesis of 4-(1,1,2,2,2-pentafluoroethyl)pyridine-2-carboxylic acid

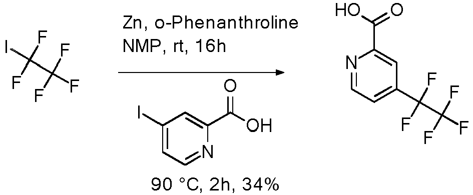

Into a 10 L vessel was added 1,1,1,2,2-pentafluoro-2-iodoethane (2.0 eq, 197.5 g, 803 mmol) to NMP (2 L). Under a nitrogen atmosphere at ice bath cooling, zinc (26.3 g, 401.6 mmol, 1.0 equiv) was added as powder to the mixture. The mixture was stirred at room temperature overnight. A mixture of 4-iodopyridine-2-carboxylic acid (1.0 eq, 100.0 g, 402 mmol), o-Phenanthroline (0.2 eq, 14.5 g, 80.3 mmol) and CuI (0.2 eq, 15.3 g, 80.3 mmol) in NMP (2 L) was stirred at 90 °C for 2 h under nitrogen atmosphere. The mixture was allowed to cool down to room temperature. The precipitated solids were collected by filtration and washed with EA (3x10 mL). To afford 4-(1,1,2,2,2-pentafluoroethyl)pyridine-2-carboxylic acid (78.0 g,137 mmol, 34 % yield) as a white solid.

### Synthesis of afford tert-butyl N-[4-(1,1,2,2,2-pentafluoroethyl)-2-pyridyl]carbamate

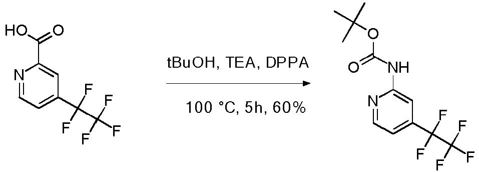

To a stirred mixture of 4-(1,1,2,2,2-pentafluoroethyl)pyridine-2-carboxylic acid (1.0 eq, 25.0 g, 104 mmol) in dioxane (500 mL) and tert-Butanol (500 mL) were added TEA (1.5 eq, 15.7 g, 156 mmol) and DPPA (1.3 eq, 37.0 g, 135 mmol) in portions at room temperature under nitrogen atmosphere. The resulting mixture was stirred at 100 °C for 5 h under nitrogen atmosphere. The mixture was allowed to cool down to room temperature. The resulting mixture was washed with 3 x 10 mL of water. The resulting mixture was extracted with EtOAc (3 x 100mL). The combined organic layers were washed with brine (3 x 10 mL), dried over anhydrous Na₂SO₄. After filtration, the filtrate was concentrated under reduced pressure. The residue was purified by silica gel column chromatography, eluted with PE / EA (5:1) to afford tert-butyl N-[4-(1,1,2,2,2-pentafluoroethyl)-2-pyridyl]carbamate (20.0 g, 63.0 mmol, 60 % yield) as a yellow oil.

### Synthesis of 4-(1,1,2,2,2-pentafluoroethyl)pyridin-2-amine

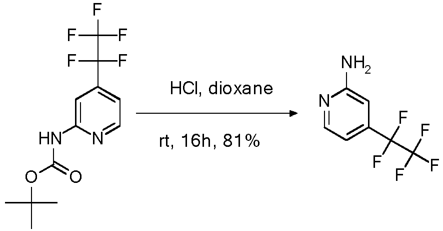

A mixture of tert-butyl N-[4-(1,1,2,2,2-pentafluoroethyl)-2-pyridyl]carbamate (1.0 eq, 20.0 g, 64.1 mmol) in HCl in dioxane was stirred at room temperature for overnight under nitrogen atmosphere. The mixture was basifed to pH 7 with saturated Na₂CO₃ (aq.). The resulting mixture was extracted with EtOAc (3 x 10mL). The combined organic layers were washed with brine (3 x 10 mL), dried over anhydrous Na₂SO₄. After filtration, the filtrate was concentrated under reduced pressure. to afford 4-(1,1,2,2,2-pentafluoroethyl)pyridin-2-amine (12.0 g, 52.2 mmol, 81 % yield) as a yellow oil.

### Synthesis of 2-(5-bromo-3-ethylsulfonyl-2-pyridyl)-7-(1,1,2,2,2-pentafluoroethyl)imidazo[1,2-a]pyridine

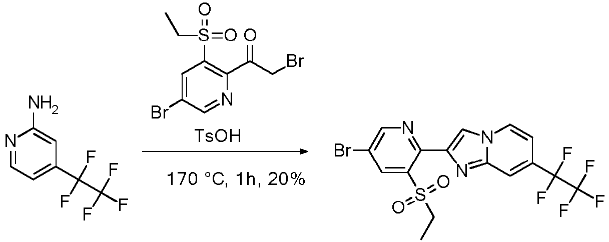

A mixture of 4-(1,1,2,2,2-pentafluoroethyl)pyridin-2-amine (1.0 eq, 10.0 g, 47.1 mmol), 2-bromo-1-(5-bromo-3-ethylsulfonyl-2-pyridyl)ethanone (1.2 eq, 21.0 g, 56.6 mmol) and TsOH (0.2 eq, 1.6 g, 9.4 mmol) ) in NMP (100 mL) was stirred at 170 °C for 1 h under nitrogen atmosphere. The mixture was allowed to cool down to room temperature. The resulting mixture was washed with 3 x 10 mL of water. The resulting mixture was extracted with EtOAc (3 x 10mL). The combined organic layers were washed with brine (3 x 4 mL), dried over anhydrous Na₂SO₄. After filtration, the filtrate was concentrated under reduced pressure. The residue was purified by silica gel column chromatography, eluted with PE / EA (5:1), to afford 2-(5-bromo-3-ethylsulfonyl-2-pyridyl)-7-(1,1,2,2,2-pentafluoroethyl)imidazo[1,2-a]pyridine (5.03 g, 9.9 mmol, 21 % yield) as a yellow solid.

### Synthesis of 5-bromo-3-(ethanesulfonyl)-2-[7-(1,1,2,2,2-pentafluoroethyl)imidazo[1,2-c]pyrimidin-2-yl]pyridine

### Synthesis of 6-(1,1,2,2,2-pentafluoroethyl)pyrimidin-4-amine

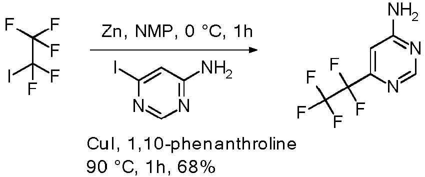

Into a 1 L 3-necked round-bottom flask were added Zn (5.1 g, 77.8 mmol, 1.0 equiv) and NMP (400 mL) at room temperature. A solution of 1,1,1,1,1-pentafluoro-2-iodoethyne (38.3 g, 155.7 mmol, 2 equiv) were added at 0 °C under nitrogen atmosphere. The mixture was stirred at room temperature for 1 h under nitrogen atmosphere. A solution of 6-iodopyrimidin-4-amine (20 g, 77.8 mmol, 1 equiv), CuI (3.5 g, 18.4 mmol, 0.2 equiv) and 1,10-phenanthroline (3.3 g, 18.3 mmol, 0.2 equiv) in 50 mL NMP were added at room temperature under nitrogen atmosphere. The resulting mixture was stirred at 90 °C for 1 h under nitrogen atmosphere. The mixture was allowed to cool down to room temperature. The resulting mixture was diluted with water (300 mL). The resulting mixture was extracted with EtOAc (3 x300 mL). The combined organic layers were washed with brine (3 x 300 mL), dried over anhydrous Na₂SO₄. After filtration, the filtrate was concentrated under reduced pressure. The residue was purified by silica gel column chromatography, eluted with PE / EA (1:1) to afford 6-(1,1,2,2,2-pentafluoroethyl)pyrimidin-4-amine (12 g, 68%) as a yellow solid.

### Synthesis of 5-bromo-3-(ethanesulfonyl)-2-[7-(1,1,2,2,2-pentafluoroethyl)imidazo[1,2-c]pyrimidin-2-yl]pyridine

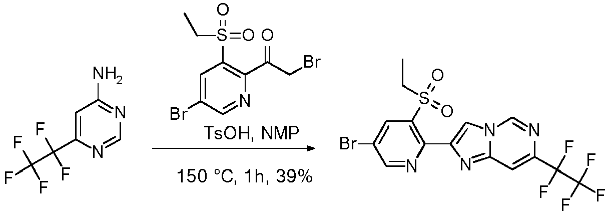

Into a 250 mL 3-necked round-bottom flask were added 6-(1,1,2,2,2-pentafluoroethyl)pyrimidin-4-amine (8.0 g, 35.4 mmol, 1 equiv, 94%), NMP (80 mL), 2-bromo-1-[5-bromo-3-(ethanesulfonyl)pyridin-2-yl]ethanone (18 g, 43.8 mmol, 1.2 equiv, 90%) and TsOH (1.3 g, 4.2 mmol, 0.1 equiv) at room temperature. The resulting mixture was stirred at 150 °C for 1 h under a nitrogen atmosphere. The mixture was allowed to cool down to room temperature. The resulting mixture was concentrated under reduced pressure. The residue was purified by reversed-phase flash chromatography with the following conditions: column, C18 silica gel; mobile phase, MeCN in Water (0.1% NH₃.H₂O), 20% to 100% gradient in 10 min; detector, UV 254 nm. This resulted in 5-bromo-3-(ethanesulfonyl)-2-[7-(1,1,2,2,2-pentafluoroethyl)imidazo[1,2-c]pyrimidin-2-yl]pyridine (6.9 g, 39%) as a brown-yellow solid.

### Synthesis of 2-(5-bromo-3-ethylsulfonyl-2-pyridyl)-7-(1,1,2,2,2-pentafluoroethyl)-[1,2,4]triazolo[1,5-a]pyridine

### Synthesis of 2-chloro-4-(1,1,2,2,2-pentafluoroethyl)pyridine

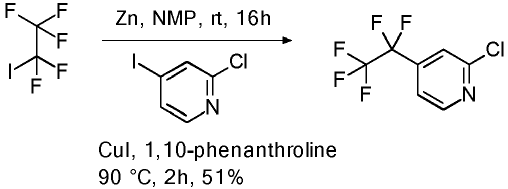

To a stirred mixture of 1,1,1,2,2-pentafluoro-2-iodoethane (8.0 eq, 493.0 g, 2005 mmol) in 1-methylpyrrolidin-2-one (1500 mL) was added Zinc (4.0 eq, 13 mL, 1002 mmol) at room temperature under a nitrogen atmosphere. The resulting mixture was stirred at room temperature overnight under a nitrogen atmosphere. A mixture of 2-chloro-4-iodo-pyridine (1 eq, 60 g, 251 mmol) and CuI (0.2 eq, 9.5 g, 50.1 mmol) and o-Phenanthroline (9.0 g, 50.1 mmol, 0.2 equiv) in 20 mL NMP was to the above reaction at room temperature under a nitrogen atmosphere. The resulting mixture was stirred at 90 °C for 2 h under a nitrogen atmosphere. The mixture was allowed to cool down to room temperature. The reaction was poured into water at room temperature. The resulting mixture was extracted with MTBE (2 x 800 mL). The combined organic layers were washed with brine (2 x 50 mL), dried over anhydrous Na₂SO₄. After filtration, the filtrate was concentrated under reduced pressure. The residue was purified by silica gel column chromatography, eluted with PE / EA (10:1) to afford 2-chloro-4-(1,1,2,2,2-pentafluoroethyl)pyridine (40.0 g,130 mmol, 52% yield) as a light yellow oil.

### Synthesis of 5-bromo-3-ethylsulfanyl-N'-hydroxy-pyridine-2-carboxamidine

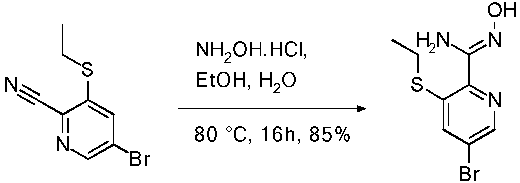

To a stirred mixture of 5-bromo-3-ethylsulfanyl-pyridine-2-carbonitrile (1.0 eq, 20 g, 82.3 mmol; prepared as described above) in EtOH (200 mL) and H₂O (50 mL) was added hydroxylamine hydrochloride (3.0 eq, 17.0 g, 247 mmol) at room temperature. The resulting mixture was stirred at 80 °C overnight. The mixture was allowed to cool down to room temperature. The resulting mixture was diluted with water (500 mL). The resulting mixture was extracted with EtOAc (2 x 300 mL). The combined organic layers were washed with brine (2 x 50 mL), dried over anhydrous Na₂SO₄. After filtration, the filtrate was concentrated under reduced pressure. The crude product 5-bromo-3-ethylsulfanyl-N'-hydroxy-pyridine-2-carboxamidine (22 g, 70.3 mmol, 86% yield) was used in the next step directly without further purification.

### Synthesis of 2-(5-bromo-3-ethylsulfanyl-2-pyridyl)-7-(1,1,2,2,2-pentafluoroethyl)-[1,2,4]triazolo[1,5-a]pyridine

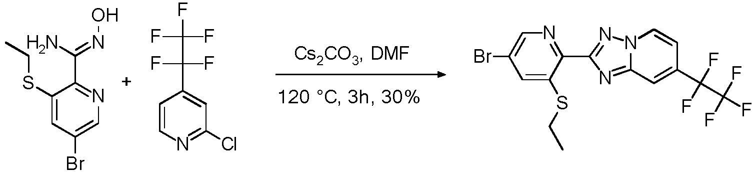

To a stirred mixture of 5-bromo-3-ethylsulfanyl-N'-hydroxy-pyridine-2-carboxamidine (1.0 eq, 20.0 g, 72.4 mmol) and 2-chloro-4-(1,1,2,2,2-pentafluoroethyl)pyridine (3.0 eq, 50.3 g, 217 mmol) in DMF (400 mL) was added Cs₂CO₃ (3.0 eq, 70.8 g, 217 mmol) at room temperature under a nitrogen atmosphere. The resulting mixture was stirred at 120 °C for 3 h under a nitrogen atmosphere. The mixture was allowed to cool down to room temperature. The resulting mixture was diluted with water (500 mL). The resulting mixture was extracted with EtOAc (2 x 500 mL). The combined organic layers were washed with brine (2 x 100 mL), dried over anhydrous Na₂SO₄. After filtration, the filtrate was concentrated under reduced pressure. The residue was purified by silica gel column chromatography, eluted with PE / EA (5:1) to afford 2-(5-bromo-3-ethylsulfanyl-2-pyridyl)-7-(1,1,2,2,2-pentafluoroethyl)-[1,2,4]triazolo[1,5-a]pyridine (11.0 g, 21.8 mmol, 30% yield) as an off-white solid.

### Synthesis of 2-(5-bromo-3-ethylsulfonyl-2-pyridyl)-7-(1,1,2,2,2-pentafluoroethyl)-[1,2,4]triazolo [1,5-a] pyridine

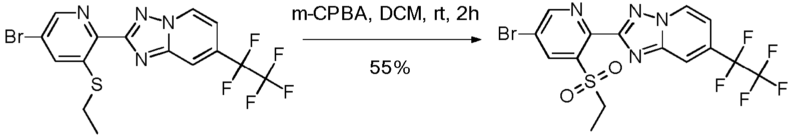

To a stirred mixture of 2-(5-bromo-3-ethylsulfanyl-2-pyridyl)-7-(1,1,2,2,2-pentafluoroethyl)-[1,2,4]triazolo[1,5-a]pyridine (1.0 eq, 10 g, 22.1 mmol) in DCM (200 mL) was added m-CPBA (3.0 eq, 11.4 g, 66.2 mmol) at room temperature under a nitrogen atmosphere. The resulting mixture was stirred at room temperature for 2 h under a nitrogen atmosphere. The resulting mixture was diluted with water (300 mL). The resulting mixture was extracted with EtOAc (2 x 300 mL). The combined organic layers were washed with aq. Na₂CO₃ (2 x 50 mL), dried over anhydrous Na₂SO₄. After filtration, the filtrate was concentrated under reduced pressure. The residue was purified by reversed-phase flash chromatography with the following conditions: column, C18 silica gel; mobile phase, MeCN in Water (0.1% NH₃.H₂O), 15% to 90% gradient in 20 min; detector, UV 254 nm to afford 2-(5-bromo-3-ethylsulfonyl-2-pyridyl)-7-(1,1,2,2,2-pentafluoroethyl)-[1,2,4]triazolo[1,5-a]pyridine (6.3 g, 12.3 mmol, 56% yield) as an off-white solid.

**General description methods for the separation of chiral compounds:** Racemic mixtures of compounds were separated into their respective pure enantiomers employing the general methods described below. In cases, the methods listed are also used to separate compounds with syn- and anti- configuration of substituents along a spiro-bicyclic ring system.

| **Method** | **Device** | **Column** | **Detection wavelength** | **Solvent** | **Pressure / temp** | **Flowrate** |
|---|---|---|---|---|---|---|
| **A** | Sepiatec 1 Prep SFC100 | Chiral Art^{®} Cellulose-SC 20x250mm, 5 µm | 220 nm | scCO₂ 90% / IPA + 20mM NH₃ 10% | 200 bar / 40°C | 60ml/min |
| **B** | Sepiatec PrepSFC50_2 | Chiralpak ^{®} IG 10x250 mm, 5 µm | 220 nm | scCO₂ 90% / IPA + 20mM NH₃ 10% | 150 bar / 40 °C | 15 ml/min |
| **C** | Sepiatec 2 Prep SFC 100 | Chiralpak ^{®} IG 20x250 mm, 5 µm | 220 nm | scCO₂ 80% / MeOH + 20mM NH₃ 20% | 150 bar / 40°C | 60 **ml/min** |
| **D** | Sepiatec 1 Prep SFC 100 | Lux^{®} Cellulose-2 21.2x250 mm, 5 µm | 220 nm | scCO₂ 95% / IPA + 20mM NH₃ 5% | 150 bar / 40°C | 60 **ml/min** |
| **E** | Sepiatec PrepSFC50 | Chiralpak^{®} IG 10x250 mm, 5 µm | 220 nm | scCO₂ 93% / IPA + 20mM NH₃ 7% | 150 bar / 40°C | 20 **ml/min** |
| **F** | Sepiatec 2 Prep SFC 100 | Chiralpak ^{®} IG 20x250 mm, 5 µm | 220 nm | scCO₂ 95% / MeOH + 20mM NH₃ 5% | 150 bar / 40°C | 60 ml/min |
| **G** | Sepiatec basic | Lux^{®} Cellulose-4 10x250 mm, 5 µm | 220 nm | scCO₂ 90% / IPA + 20mM NH₃ 10% | 150 bar / 40 °C | 15 ml/min |
| **H** | Sepiatec PrepSFC50_2 | Chiral Airt*) Cellulose-SC 10x250mm, 5 µm | 220 nm | scCO₂ 80% / MeOH + 20mM NH₃ 20% | 150 bar / 40 °C | 15 ml/min |
| **I** | Shimadzu LC-20AD | Chiralpak^{®} IC, 2x250 mm, 5 µm | 254 nm | n-Hexane-DCM 3:1 / Ethanol (20mM NH₃) | 100 bar / 25°C | 25 ml/min |
| **J** | Agilent 1260 Infinity II SFC | Chiralpak ^{®} IG 4.6x250 mm, 5 µm | 220 nm | scCO₂ 85% / EtOH + 20mM NH₃ 15% | 150 bar / 40 °C | 4 ml/min |

R/S nomenclature was arbitrarily assigned according to SFC separation retention times, i.e. the enantiomer with the shorter SFC separation retention time was assigned "a", whereas the corresponding enantiomer with the longer SFC separation retention time was assigned "b".

### General procedure I for the synthesis of compounds as demonstrated by the synthesis of 5-{5,5-difluorospiro[2.3]hexan-1-yl}-3-(ethanesulfonyl)-2-[3-methyl-6-(1,1,2,2,2-pentafluoroethyl)-3H-imidazo[4,5-b]pyridin-2-yl]pyridine (Compound 5):

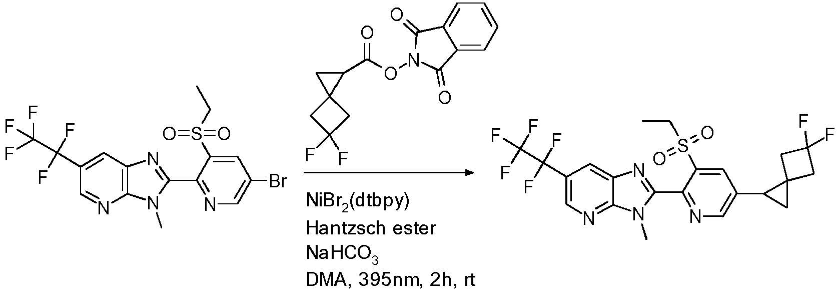

To a stirred solution of 2-(5-bromo-3-ethylsulfanyl-2-pyridyl)-3-methyl-6-(1,1,2,2,2-pentafluoroethyl)imidazo[4,5-b]pyridine (1.0 eq, 90 mg, 0.175 mmol) in 3.5 mL DMA was added 1,3-dioxo-2,3-dihydro-1H-isoindol-2-yl 5,5-difluorospiro[2.3]hexane-1-carboxylat (2.2 eq., 120 mg, 0.39 mmol) under Argon. Subsequently, 4,4'-di-tert-butyl-2,2'-bipyridine; dibromonickel (2.1 equiv, 42 mg, 0.08 mmol), 3,5-diethyl 2,6-dimethyl-1,4-dihydropyridine-3,5-dicarboxylate (2 equiv, 93 mg, 0.36 mmol) and NaHCO₃ (4 equiv, 63 mg, 0.75 mmol) were added under Argon and the vial capped under Argon. The vial was irradiated at a wavelength of 395 nm with a 14W LED for 2 hours at rt. The resulting mixture was diluted with EtOAc and washed with water and brine. The organic layer was filtered over Celite and evaporated. The crude residue was purified by HPLC to yield 5-{5,5-difluorospiro[2.3]hexan-1-yl}-3-(ethanesulfonyl)-2-[3-methyl-6-(1,1,2,2,2-pentafluoroethyl)-3H-imidazo[4,5-b]pyridin-2-yl]pyridine (31 mg, 0.05 mmol, 33%) as a solid.

### General procedure II for the synthesis of active ester intermediates employed in the synthesis of compounds, by the example of the synthesis of 1,3-dioxo-2,3-dihydro-1H-isoindol-2-yl 5,5-difluorospiro[2.3]hexane-1-carboxylate employed in the synthesis of 5-{5,5-difluorospiro[2.3]hexan-1-yl}-3-(ethanesulfonyl)-2-[3-methyl-6-(1,1,2,2,2-pentafluoroethyl)-3H-imidazo[4,5-b]pyridin-2-yl]pyridine (Compound 5)

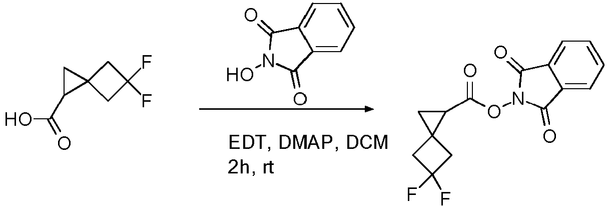

To a stirred solution of 5,5-difluorospiro[2.3]hexane-1-carboxylic acid (1.0 eq, 209 mg, 1.2 mmol) in 3.5 mL DCM was added *N*-Hydroxyphthalimid (1.0 equiv, 200 mg, 1.2 mmol). Then, 1-Ethyl-3-(3-dimethylaminopropyl)carbodiimide-hydrochloride (1.05 equiv, 240mg, 1.25 mmol) and DMAP (0.1 equiv, 15 mg, 0.12 mmol) were added and the mixture stirred for 2 h at room temperature. After evaporation the mixture was purified directly via silica gel column chromatography, eluted with cyclohexane / EA (0-50%) to afford 1,3-dioxo-2,3-dihydro-1H-isoindol-2-yl 5,5-difluorospiro[2.3]hexane-1-carboxylate (120mg, 0.38 mmol, 32%) as solid.

The following compounds were synthesized employing the general procedures I and II as described for compound 5 above analogously by employing commercial starting materials: Compounds 3, 4, 6, 7, 8, 9, 12, 15, 16, 17, 23, 25, 118. A person skilled in the art recognizes that the yield of such reaction can be optimized by modifying the source of catalyst, ligand, base and solvent as well as reaction time and temperature.

The following compounds were obtained by separating the enantiomers of the described racemic compounds using the respective chiral purification methods as listed above:

| **Compounds** | **Chiral separation method** |
|---|---|
| 12a, 12b | B |
| 7a, 7b | D |
| 3a, 3b | C |
| 17a, 17b | B |
| 8a, 8b | E |
| 23a, 23b | B |
| 51a, 51b | F |
| 93a, 93b | G |
| 118a, 118b | H |

The following compounds were synthesized with procedures deviating from the general procedure:

### Synthesis of 3-(ethanesulfonyl)-2-[3-methyl-6-(1,1,2,2,2-pentafluoroethyl)-3H-imidazo[4,5-b]pyridin-2-yl]-5-[(3s,5s)-1,1,5-trifluorospiro[2.3]hexan-5-yl]pyridine (Compound 18) and 3-(ethanesulfonyl)-2-[3-methyl-6-(1,1,2,2,2-pentafluoroethyl)-3H-imidazo[4,5-b]pyridin-2-yl]-5-[(3s,5s)-1,1,5-trifluorospiro[2.3]hexan-5-yl]pyridine (Compound 19):

### Synthesis of 5-[5-ethylsulfanyl-6-[3-methyl-6-(1,1,2,2,2-pentafluoroethyl)imidazo[4,5-b]pyridin-2-yl]-3-pyridyl]-2,2-difluoro-spiro[2.3]hexan-5-ol

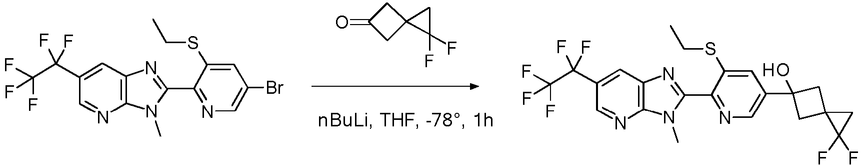

To a stirred solution of 2-(5-bromo-3-ethylsulfanyl-2-pyridyl)-3-methyl-6-(1,1,2,2,2-pentafluoroethyl)imidazo[4,5-b]pyridine (1.0 eq, 500 mg, 1.1 mmol) in THF (10 mL) were added n-BuLi in hexanes (2.0 eq, 0.9 mL, 2.1 mmol) and commercially available 2,2-difluorospiro[2.3]hexan-5-one (2.0 eq, 283 mg, 2.1 mmol) at -78 °C under a nitrogen atmosphere. The resulting mixture was stirred for 1 h at -78°C under a nitrogen atmosphere. The reaction was quenched by the addition of sat. NH4Cl (aq.) (20 mL) at -78 °C. The resulting mixture was extracted with DCM (3 x 50 mL). The combined organic layers were washed with brine (1 x 50 mL), dried over anhydrous Na₂SO₄. After filtration, the filtrate was concentrated under reduced pressure. The residue was purified by silica gel column chromatography, eluted with PE / EA (1:1) to afford 5-[5-ethylsulfanyl-6-[3-methyl-6-(1,1,2,2,2-pentafluoroethyl)imidazo[4,5-b]pyridin-2-yl]-3-pyridyl]-2,2-difluoro-spiro[2.3]hexan-5-ol (540 mg, 79% yield) as yellow oil.

### Synthesis 3-(ethylsulfanyl)-2-[3-methyl-6-(1,1,2,2,2-pentafluoroethyl)-3H-imidazo[4,5-b]pyridin-2-yl]-5-[(3r,5r)-1,1,5-trifluorospiro[2.3]hexan-5-yl]pyridine and 3-(ethylsulfanyl)-2-[3-methyl-6-(1,1,2,2,2-pentafluoroethyl)-3H-imidazo[4,5-b]pyridin-2-yl]-5-[(3s,5s)-1,1,5-trifluorospiro[2.3]hexan-5-yl]pyridine

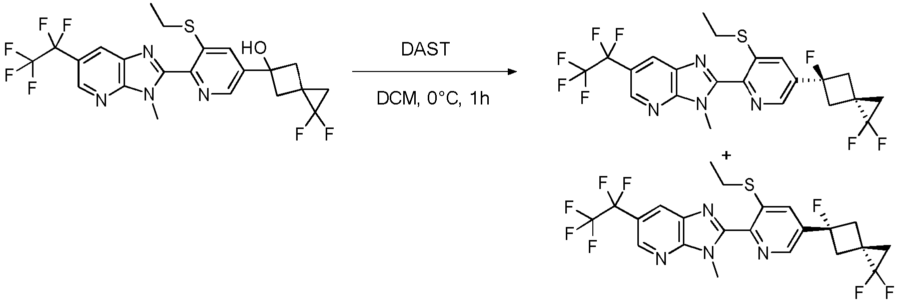

To a stirred solution of 5-[5-ethylsulfanyl-6-[3-methyl-6-(1,1,2,2,2-pentafluoroethyl)imidazo[4,5-b]pyridin-2-yl]-3-pyridyl]-2,2-difluoro-spiro[2.3]hexan-5-ol (1.0 eq, 500 mg, 0.8 mmol) in DCM (10 mL) was added DAST (1.0 eq, 48 mg, 0.8 mmol) at 0 °C for 1 h. The resulting mixture was stirred for 1 h at 0°C. The reaction was quenched by the addition of sat. NH₄Cl (aq.) (10 mL) at 0 °C. The resulting mixture was extracted with DCM (3 x 50 mL). The combined organic layers were washed with brine (1 x 50 mL), dried over anhydrous Na₂SO₄. After filtration, the filtrate was concentrated under reduced pressure. The residue was purified by silica gel column chromatography, eluted with PE / EA (1:1) to afford 3-(ethylsulfanyl)-2-[3-methyl-6-(1,1,2,2,2-pentafluoroethyl)-3H-imidazo[4,5-b]pyridin-2-yl]-5-[(3r,5r)-1,1,5-trifluorospiro[2.3]hexan-5-yl]pyridine (112 mg, 0.2 mmol, 24% yield) as yellow oil and 3-(ethylsulfanyl)-2-[3-methyl-6-(1,1,2,2,2-pentafluoroethyl)-3H-imidazo[4,5-b]pyridin-2-yl]-5-[(3s,5s)-1,1,5-trifluorospiro[2.3]hexan-5-yl]pyridine (224 mg, 0.4 mmol, 45% yield) as yellow oil.

### Synthesis of 3-(ethanesulfonyl)-2-[3-methyl-6-(1,1,2,2,2-pentafluoroethyl)-3H-imidazo[4,5-b]pyridin-2-yl]-5-[(3s,5s)-1,1,5-trifluorospiro[2.3]hexan-5-yl]pyridine (Compound 18)

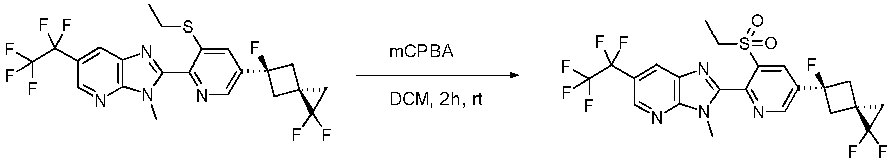

To a stirred solution of 2-[3-ethylsulfanyl-5-(2,2,5-trifluorospiro[2.3]hexan-5-yl)-2-pyridyl]-3-methyl-6-(1,1,2,2,2-pentafluoroethyl)imidazo[4,5-b]pyridine (1.0 eq, 112 mg, 0.2 mmol) in DCM (2 mL) was added 3-chlorobenzene-1-carboperoxoic acid (13.5 eq, 431 mg, 2.5 mmol). The resulting mixture was stirred for 2 h at room temperature. The resulting mixture was concentrated under reduced pressure. The crude product was purified by Prep-HPLC with the following conditions (Column: XBride Prep OBD Column 19 x 150mm 8um; Mobile Phase A: water (0.05% NH₃.H₂O), Mobile Phase B: MeCN; Flow rate: 20 mL/min; Gradient: 10% B to 40% B in 12 min, 30% B; Wave Length: 220 nm) to afford 3-(ethanesulfonyl)-2-[3-methyl-6-(1,1,2,2,2-pentafluoroethyl)-3H-imidazo[4,5-b]pyridin-2-yl]-5-[(3s,5s)-1,1,5-trifluorospiro[2.3]hexan-5-yl]pyridine (58 mg, 0.1 mmol, 55% yield) as a white solid.

### Synthesis of 3-(ethanesulfonyl)-2-[3-methyl-6-(1,1,2,2,2-pentafluoroethyl)-3H-imidazo[4,5-b]pyridin-2-yl]-5-[(3s,5s)-1,1,5-trifluorospiro[2.3]hexan-5-yl]pyridine (Compound 19)

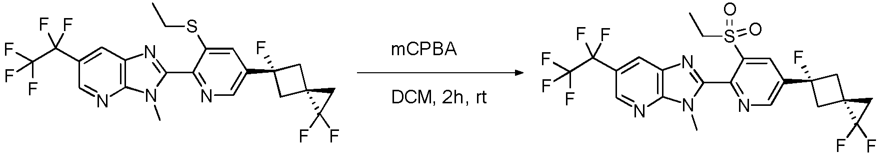

To a stirred solution of 2-[3-ethylsulfairyl-5-(2,2,5-trifluorospiro[2.3]hexan-5-yl)-2-pyridyl]-3-methyl-6-(1,1,2,2,2-pentafluoroethyl)imidazo[4,5-b]pyridine (1.0 eq, 112 mg, 0.2 mmol) in DCM (2 mL) was added 3-chlorobenzene-1-carboperoxoic acid (13.5 eq, 431 mg, 2.5 mmol). The resulting mixture was stirred for 2 h at room temperature. The resulting mixture was concentrated under reduced pressure. The crude product was purified by Prep-HPLC with the following conditions (Column: XBride Prep OBD Column 19 x 150mm 8um; Mobile Phase A: water (0.05% NH₃.H₂O), Mobile Phase B: MeCN; Flow rate: 20 mL/min; Gradient: 10% B to 40% B in 12 min, 30% B; Wave Length: 220 nm) to afford 3-(ethanesulfonyl)-2-[3-methyl-6-(1,1,2,2,2-pentafluoroethyl)-3H-imidazo[4,5-b]pyridin-2-yl]-5-[(3s,5s)-1,1,5-trifluorospiro[2.3]hexan-5-yl]pyridine (58 mg, 0.1 mmol, 55% yield) as a white solid.

The following compounds were synthesized analogously as described above:
**3-(ethanesulfonyl)-5-{5-fluorospiro[2.3]hexan-5-yl}-2-[3-methyl-6-(1,1,2,2,2-pentafluoroethyl)-3H-imidazo[4,5-b]pyridin-2-yl]pyridine (Compound 1)**
**3-(ethanesulfonyl)-2-[3-methyl-6-(1,1,2,2,2-pentafluoroethyl)-3H-imidazo[4,5-b]pyridin-2-yl]-5-{2,6,6-trifluorospiro[3.3]heptan-2-yl}pyridine (Compound 2)**
**3-(ethanesulfonyl)-2-[3-methyl-6-(1,1,2,2,2-pentafluoroethyl)-3H-imidazo[4,5-b]pyridin-2-yl]-5-{1,6,6-trifluorospiro[3.3]heptan-1-yl}pyridine (Compound 10).**

The following compound was also synthesized with procedures deviating from the general procedures:

### Synthesis of 3-(ethanesulfonyl)-5-{2-fluorospiro[3.3]heptan-2-yl}-2-[3-methyl-6-(1,1,2,2,2-pentafluoroethyl)-3H-imidazo[4,5-b]pyridin-2-yl]pyridine (Compound 13):

### Synthesis of 2-[5-(ethanesulfonyl)-6-[3-methyl-6-(1,1,2,2,2-pentafluoroethyl)-3H-imidazo[4,5-b]pyridin-2-yl]pyridin-3-yl]spiro[3.3]heptan-2-ol

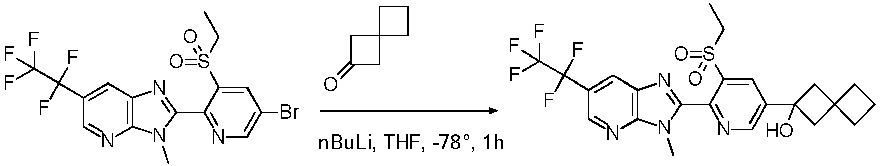

To a stirred solution of 5-bromo-3-(ethanesulfonyl)-2-[3-methyl-6-(1,1,2,2,2-pentafluoroethyl)-3H-imidazo[4,5-b]pyridin-2-yl]pyridine (1.0 eq, 400 mg, 0.8 mmol) in THF (10 mL) and commercially available spiro[3.3]heptan-2-one (2.5 eq, 220 mg, 2.0 mmol) at -78 °C under a nitrogen atmosphere, n-BuLi in hexanes (2.0 eq, 0.8 mL, 2.0 mmol) was added dropwise. The resulting mixture was stirred for 10 min at -78°C under a nitrogen atmosphere. The reaction was quenched by the addition of saturated NH₄Cl (aq.) (20 mL) at -78 °C. The resulting mixture was extracted with DCM (3 x 50 mL). The combined organic layers were washed with brine (1 x 50 mL), dried over anhydrous Na₂SO₄. After filtration, the filtrate was concentrated under reduced pressure. The residue was purified by silica gel column chromatography, eluted with a cyclohexane / EA gradient to afford 2-[5-(ethanesulfonyl)-6-[3-methyl-6-(1,1,2,2,2-pentafluoroethyl)-3H-imidazo[4,5-b]pyridin-2-yl]pyridin-3-yl]spiro[3.3]heptan-2-ol (138 mg, 32% yield) as a yellow oil.

### Synthesis of 3-(ethanesulfonyl)-5-{2-fluorospiro[3.3]heptan-2-yl}-2-[3-methyl-6-(1,1,2,2,2-pentafluoroethyl)-3H-imidazo[4,5-b]pyridin-2-yl]pyridine (Compound 13):

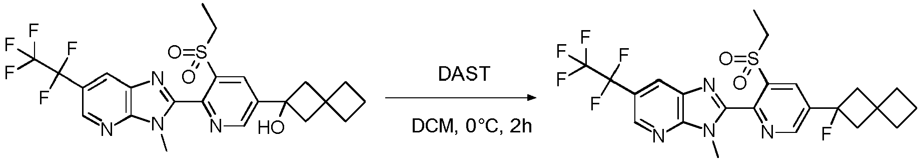

To a stirred solution of 2-[5-(ethanesulfonyl)-6-[3-methyl-6-(1,1,2,2,2-pentafluoroethyl)-3H-imidazo[4,5-b]pyridin-2-yl]pyridin-3-yl]spiro[3.3]heptan-2-ol (1.0 eq, 130 mg, 0.2 mmol) in DCM (2 mL), DAST (1.0 eq, 48 mg, 0.3 mmol) was added at 0 °C and stirred for 2 h. The reaction was quenched by the addition of saturated NH₄Cl (aq.) (10 mL) at 0 °C. The resulting mixture was extracted with DCM (3 x 50 mL). The combined organic layers were washed with brine (1 x 50 mL), dried over anhydrous Na₂SO₄. After filtration, the filtrate was concentrated under reduced pressure. The residue was purified by preparative HPLC to afford 3-(ethanesulfonyl)-5-{2-fluorospiro[3.3]heptan-2-yl}-2-[3-methyl-6-(1,1,2,2,2-pentafluoroethyl)-3H-imidazo[4,5-b]pyridin-2-yl]pyridine (21 mg, 0.1 mmol, 16% yield) as a solid.

The following compound was synthesized analogously to the above-mentioned compound:

### 6-[5-(ethanesulfonyl)-6-[3-methyl-6-(1,1,2,2,2-pentafluoroethyl)-3H-imidazo[4,5-b]pyridin-2-yl]pyridin-3-yl]-6-fluorospiro[3.3]heptane-2-carbonitrile (Compound 11)

The following compound was also synthesized with procedures deviating from the general procedure:

### Synthesis of 5-{3,3-difluorospiro[3.3]heptan-1-yl}-3-(ethanesulfonyl)-2-[3-methyl-6-(1,1,2,2,2-pentafluoroethyl)-3H-imidazo[4,5-b]pyridin-2-yl]pyridine (Compound 14):

### Synthesis of 3-[5-(ethanesulfonyl)-6-[3-methyl-6-(1,1,2,2,2-pentafluoroethyl)-3H-imidazo[4,5-b]pyridin-2-yl]pyridin-3-yl]spiro[3.3]heptan-1-one

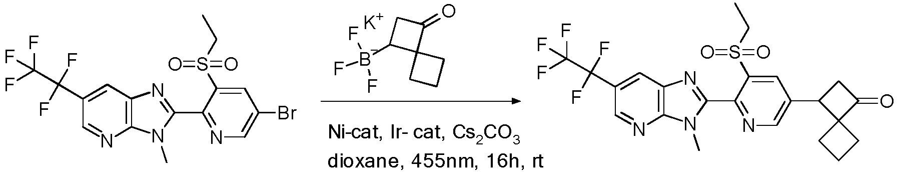

A 5 mL vial was charged with 2-(5-bromo-3-ethylsulfanyl-2-pyridyl)-3-methyl-6-(1,1,2,2,2-pentafluoroethyl)imidazo[4,5-b]pyridine (1.0 eq, 100 mg, 0.2 mmol), potassium 3-trifluoroborate-spiro[3.3]heptan-1-one (1.7 eq, 75 mg, 0.3 mmol), cesium carbonate (2.3 eq, 150 mg, 0.5 mmol), [Ni(DTBBPY)(H₂O)₄]Cl₂ (0.2 eq, 15 mg, 0.03 mmol) and Ir{dFCF₃ppy}₂(bpy)]PF₆ (1.7 eq, 35 mg, 0.03 mmol). Under argon, 2 mL of 1,4-dioxane were added and the mixture degassed and the vial sealed. The vial was irradiated with 455 nm at room temperature for 16 h. The mixture was evaporated, and the residue directly purified by silica gel chromatography (cyclohexane:ethyl acetate 0-100%) to afford 3-[5-(ethanesulfonyl)-6-[3-methyl-6-(1,1,2,2,2-pentafluoroethyl)-3H-imidazo[4,5-b]pyridin-2-yl]pyridin-3-yl]spiro[3.3]heptan-1-one (39 mg, 0.07 mmol, 36%) as a brown solid.

### Synthesis of 5-{3,3-difluorospiro[3.3]heptan-1-yl}-3-(ethanesulfonyl)-2-[3-methyl-6-(1,1,2,2,2-pentafluoroethyl)-3H-imidazo[4,5-b]pyridin-2-yl]pyridine (Compound 14):

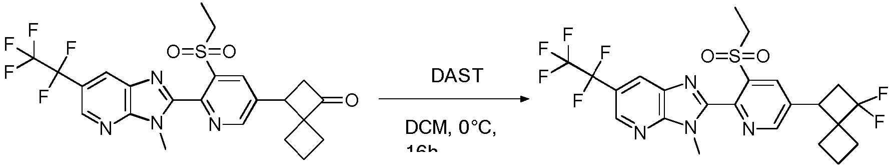

To a stirred solution of 3-[5-(ethanesulfonyl)-6-[3-methyl-6-(1,1,2,2,2-pentafluoroethyl)-3H-imidazo[4,5-b]pyridin-2-yl]pyridin-3-yl]spiro[3.3]heptan-1-one (1.0 eq, 39 mg, 0.07 mmol) in DCM (2 mL) was added DAST (2.5 eq, 0.3 g, 1.9 mmol) at 0 °C and the mixture was stirred overnight, wanning to room temperature. The reaction was quenched by the addition of sat. NH₄Cl (aq.) (10 mL) at 0 °C. The resulting mixture was extracted with DCM (3 x 50 mL). The combined organic layers were washed with brine (1 x 50 mL), dried over anhydrous Na₂SO₄. After filtration, the filtrate was concentrated under reduced pressure. The residue was purified by preparative HPLC to afford 5-{3,3-difluorospiro[3.3]heptan-1-yl}-3-(ethanesulfonyl)-2-[3-methyl-6-(1,1,2,2,2-pentafluoroethyl)-3H-imidazo[4,5-b]pyridin-2-yl]pyridine (20 mg, 0.03 mmol, 49%) as a yellow residue.

The following compound 24 was synthesized by further reacting compound 25, which was synthesized via the general procedure I and II:

### Synthesis of 1-[5-(ethanesulfonyl)-6-[3-methyl-6-(1,1,2,2,2-pentafluoroethyl)-3H-imidazo[4,5-b]pyridin-2-yl]pyridin-3-yl]-Slambda6-thiaspiro[2.3]hexane-5,5-dione (Compound 24):

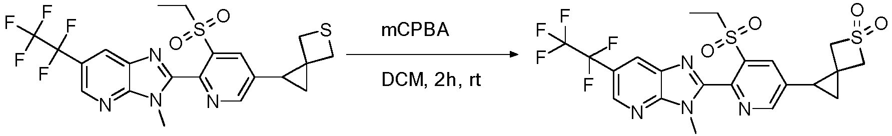

To a stirred solution of 3-(ethanesulfonyl)-2-[3-methyl-6-(1,1,2,2,2-pentafluoroethyl)imidazo[4,5-b]pyridin-2-yl]-5-{5-thiaspiro[2.3]hexan-1-yl}pyridine (1.0 equiv, 21 mg, 0.04 mmol) in DCM (2 mL) was added m-CPBA (2.5 equiv, 17.3 mg, 0.1 mmol) at 0 °C. The resulting mixture was stirred at room temperature for 2 h. The resulting mixture was concentrated under reduced pressure. The crude product was purified by Prep-HPLC with the following conditions (Column: XBride Prep OBD Column 19 x 150mm 8um; Mobile Phase A: water (0.05% NH3.H2O), Mobile Phase B: MeCN; Flow rate: 20 mL/min; Gradient: 30% B to 80% B in 12 min, 75% B; Wave Length: 220 nm) to afford 1-[5-(ethanesulfonyl)-6-[3-methyl-6-(1,1,2,2,2-pentafluoroethyl)-3H-imidazo[4,5-b]pyridin-2-yl]pyridin-3-yl]-5lambda6-thiaspiro[2.3]hexane-5,5-dione (10 mg, 0.02 mmol, 45%) as a white solid.

The following compounds 119 and 120 were synthesized by further reacting compound 15, which was synthesized via the general procedure I and II:

### Synthesis of 5-{6,6-difluorospiro[3.3]heptan-2-yl}-3-(ethanesulfonyl)-2-[3-methyl-6-(1,1,2,2,2-pentafluoroethyl)-3H-imidazo[4,5-b]pyridin-2-yl]pyridin-1-ium-1-olate and 2-(5-{6,6-difluorospiro[3.3]heptan-2-yl}-3-(ethanesulfonyl)pyridin-2-yl)-3-methyl-6-(1,1,2,2,2-pentafluoroethyl)-3H-imidazo[4,5-b]pyridin-4-ium-4-olate (Compounds 119 and 120):

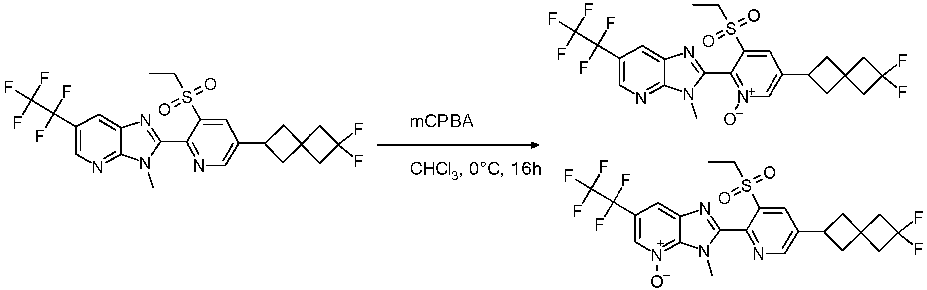

Into a 5 mL vial was added 5-{6,6-difluorospiro[3.3]heptan-2-yl}-3-(ethanesulfonyl)-2-[3-methyl-6-(1,1,2,2,2-pentafluoroethyl)-3H-imidazo[4,5-b]pyridin-2-yl]pyridine (1.0 eq, 76 mg, 0.13 mmol,), mCPBA (1.0 eq, 30 mg, 0.13 mmol), followed by chloroform (1.5 mL). The resulting mixture was stirred overnight at 60 degrees C. before it was diluted with dichloromethane and washed with a saturated NaHCO₃ solution. The crude product was purified by Flash-Prep-HPLC. This resulted in 13.2 mg (17%) of 5-{6,6-difluorospiro[3.3]heptan-2-yl}-3-(ethanesulfonyl)-2-[3-methyl-6-(1,1,2,2,2-pentafluoroethyl)-3H-imidazo[4,5-b]pyridin-2-yl]pyridin-1-ium-1-olate as an off-white solid and 9.8 mg (12%) of 2-(5-{6,6-difluorospiro[3.3]heptan-2-yl}-3-(ethanesulfonyl)pyridin-2-yl)-3-methyl-6-(1,1,2,2,2-pentafluoroethyl)-3H-imidazo[4,5-b]pyridin-4-ium-4-olate as an off-white solid.

### General procedure III for the synthesis of compounds as demonstrated by the synthesis of 2-[5-(ethanesulfonyl)-6-[3-methyl-6-(1,1,2,2,2-pentafluoroethyl)-3H-imidazo[4,5-b]pyridin-2-yl]pyridin-3-yl]-6,6-difluoro-2-azaspiro[3.3]heptane (Compound 28):

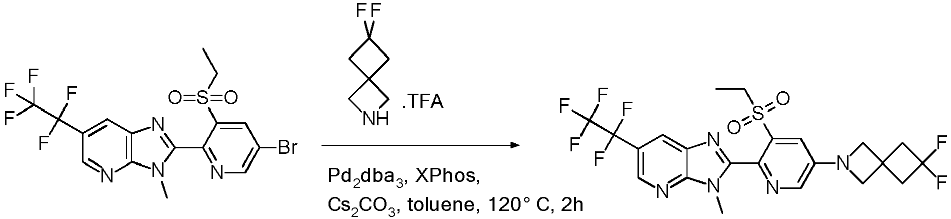

A 2.5 mL microwave vial was charged with 5-bromo-3-(ethanesulfonyl)-2-[3-methyl-6-(1,1,2,2,2-pentafluoroethyl)-3H-imidazo[4,5-b]pyridin-2-yl]pyridine (1.0 eq, 50 mg, 0.1 mmol), cesium carbonate (7.0 eq, 230 mg, 0.7 mmol), commercially available 6,6-difluoro-2-aza-spiro[3.3]heptane trifluoroacetate (1.0 eq, 36 mg, 0.1 mmol) and 1 mL of toluene. The mixture was degassed with argon for 10 min, then tris(dibenzylideneacetone)dipalladium (0.1 eq, 10.7 mg, 0.01 mmol) and 2-dicyclohexylphosphin-2',4',6'-triisopropylbiphenyl (0.3 eq, 12.3 mg, 0.03 mmol) were added and the mixture degassed for additional 5 min and the vial sealed and heated to 120 °C for 2h. The reaction was diluted with water (5 mL) and extracted with DCM (3 x 5 mL). The combined organic layers were dried over anhydrous Na₂SO₄ and concentrated under reduced pressure. The residue was purified by silica gel column chromatography, eluted with cyclohexane / EA (0-10%) to afford 2-[5-(ethanesulfonyl)-6-[3-methyl-6-(1,1,2,2,2-pentafluoroethyl)-3H-imidazo[4,5-b]pyridin-2-yl]pyridin-3-yl]-6,6-difluoro-2-azaspiro[3.3]heptane (35 mg, 0.06 mmol, 63%) as solid.

The following compounds were synthesized employing the general procedure III as described for Compound 28 above analogously by employing commercial starting materials. A person skilled in the art recognizes that the yield of such reaction can be optimized by modifying the source of catalyst, ligand, base and solvent as well as reaction time and temperature. Compounds 26, 27, 29, 30, 31, 32, 33, 34, 35, 36, 37, 38, 39, 40, 41, 42, 46, 47, 48, 49, 50, 51, 52, 53, 54, 55, 56, 57, 58, 59, 62, 63, 64, 65, 66, 67, 68, 69, 70, 72, 73, 74, 75, 76, 77, 78, 79, 80, 81, 82, 84, 85, 86, 87, 88, 90, 91, 92, 93, 94, 95, 96, 97, 99, 100, 101, 102, 103, 104, 106, 107, 108, 109, 110, 112, 113,

The following compounds were synthesized employing the general procedure III but with reactants prepared as described below: compounds 43, 44, 45, 105, 114, 115 and 116.

### Synthesis of 6-methyl-2-azaspiro[3.3]heptane-6-carbonitrile to be used in the synthesis of compound 43:

### Synthesis of tert-butyl 6-cyano-6-methyl-2-azaspiro[3.3]heptane-2-carboxylate:

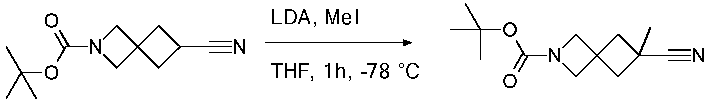

To a stirred solution of tert-butyl 6-cyano-2-azaspiro[3.3]heptane-2-carboxylate (1.0 eq, 2.0 g, 9.0 mmol) in THF (40 mL) were added LDA (in 2 M THF) (18 mL) dropwise at -78°C under a nitrogen atmosphere. The resulting mixture was stirred at -78°C for 30 min under a nitrogen atmosphere. To the above mixture, MeI (1.5 eq, 0.8 mL, 13.5 mmol) was added dropwise over 10 min at -78°C. The resulting mixture was stirred at -78°C for an additional 30 min. The reaction was quenched with sat. NH₄Cl (aq.) at -10°C. The resulting mixture was extracted with EtOAc (3 x 50 mL). The combined organic layers were washed with brine (3 x 50 mL), dried over anhydrous Na₂SO₄. After filtration, the filtrate was concentrated under reduced pressure. The residue was purified by silica gel column chromatography, eluted with PE / THF (1:1) to afford tert-butyl 6-cyano-6-methyl-2-azaspiro[3.3]heptane-2-carboxylate (2.0 g, 7.6 mmol, 85%) as a white solid.

### Synthesis of 6-methyl-2-azaspiro[3.3]heptane-6-carbonitrile

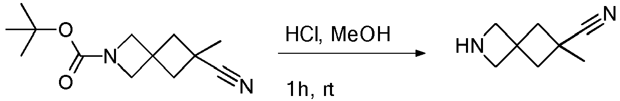

Into a 40mL vial were added tert-butyl 6-cyano-6-methyl-2-azaspiro[3.3]heptane-2-carboxylate (1.0 eq, 500 mg, 2.1 mmol) and HCl in MeOH (10 mL) at room temperature. The resulting mixture was stirred for 1 h at room temperature under nitrogen atmosphere. The resulting mixture was concentrated under reduced pressure. The crude product was used in the next step directly without further purification (general procedure III in the synthesis of compound 43).

The following amine was prepared analogously: 6-methyl-6-(trifluoromethyl)-2-azaspiro[3.3]heptane for the synthesis of compounds 44 and 45.

### Synthesis of 7-methyl-6lambda6-thia-2,7-diazaspiro[3.4]octane-6,6-dione trifluoroacetate to be used in the synthesis of compound 105:

### Synthesis of tert-butyl 7-methyl-6,6-dioxo-6lambda6-thia-2,7-diazaspiro[3.4]octane-2-carboxylate

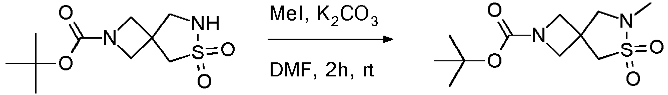

In a 2 mL vial, commercially available tert-butyl 6,6-dioxo-6lambda6-thia-2,7-diazaspiro[3.4]octane-2-carboxylate (1.0 eq, 50 mg, 0.18 mmol) and potassium carbonate (1.0 eq, 25mg, 0.2 mmol) were dissolved in 1 mL DMF. Iodomethane (2 eq, 0.02 mL, 0.4 mmol) was added and the mixture stirred 2 h at rt. The reaction mixture was diluted with water (5 mL) and extracted two times with DCM (5 mL). The combined organic layers were dried over anhydrous Na₂SO₄ and concentrated under reduced pressure to yield tert-butyl 7-methyl-6,6-dioxo-6lambda6-thia-2,7-diazaspiro[3.4]octane-2-carboxylate (40 mg, 79%) as crude oil that was used in the next step without further purification.

### Synthesis of 7-methyl-6lambda6-thia-2,7-diazaspiro[3.4]octane-6,6-dione trifluoroacetate

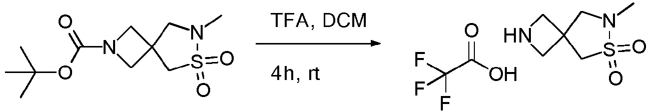

In a 2 mL vial tert-butyl 7-methyl-6,6-dioxo-6lambda6-thia-2,7-diazaspiro[3.4]octane-2-carboxylate (1.0 eq, 40 mg, 0.14 mmol) was dissolved in DCM (1 mL). After addition of 0.04 mL of trifluoroacetic acid the mixture was stirred for four hours at room temperature. The reaction mixture was concentrated to dryness to yield 7-methyl-6lambda6-thia-2,7-diazaspiro[3.4]octane-6,6-dione trifluoroacetate (41 mg, 97%) as a solid residue which was used directly without further purification in the next step (general procedure III in the synthesis of compound 105).

### Synthesis of 6-(ethanesulfonyl)-2-azaspiro[3.3]heptane to be used in the synthesis of compound 115:

### Synthesis of tert-butyl 6-(methanesulfonyloxy)-2-azaspiro[3.3]heptane-2-carboxylate

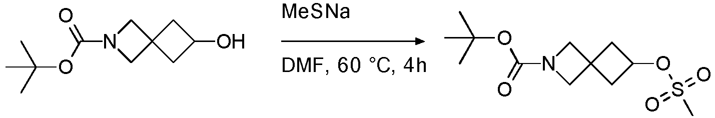

To a stirred solution of tert-butyl 6-methylsulfonyloxy-2-azaspiro[3.3]heptane-2-carboxylate (1.0 eq, 2.0 g, 6.9 mmol) in DMF (40 mL) was added sodium thiomethoxide (2.0 eq, 0.7 g, 13.7 mmol) at room temperature. The resulting mixture was stirred at 60 °C for 4 h. The reaction was quenched with water (50 mL) at room temperature. The resulting mixture was extracted with EtOAc (3 x 100 mL). The combined organic layers were washed with brine (3 x 100 mL), dried over anhydrous Na₂SO₄. After filtration, the filtrate was concentrated under reduced pressure. The residue was purified by silica gel column chromatography, eluted with PE / EA (1:1) to afford tert-butyl 6-methylsulfanyl-2-azaspiro[3.3]heptane-2-carboxylate (1.1 g, 2.6 mmol, 37% yield) as a yellow solid.

### Synthesis of tert-butyl 6-(ethylsulfanyl)-2-azaspiro[3.3]heptane-2-carboxylate

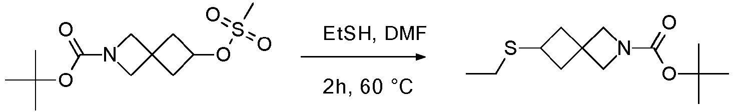

A mixture of tert-butyl 6-methylsulfonyloxy-2-azaspiro[3.3]heptane-2-carboxylate (1.0 eq, 2.0 g, 6.9 mmol) and (ethylsulfanyl)sodium (2.0 eq, 1.2 g, 13.7 mmol) in DMF (100 mL) was stirred at 60 °C for 2 h under a nitrogen atmosphere. The reaction was quenched by the addition of sat. NH₄Cl (aq.) (100 mL) at 0°C. The resulting mixture was extracted with EtOAc (3 x 100 mL). The combined organic layers were washed with water (3 x 50 mL), dried over anhydrous Na₂SO₄. After filtration, the filtrate was concentrated under reduced pressure. The residue was purified by silica gel column chromatography, eluted with PE / THF (1:1) to afford tert-butyl 6-ethylsulfanyl-2-azaspiro[3.3]heptane-2-carboxylate (1.7 g, 6.4 mmol, 93%) as a yellow oil.

### Synthesis of tert-butyl 6-(ethanesulfonyl)-2-azaspiro[3.3]heptane-2-carboxylate

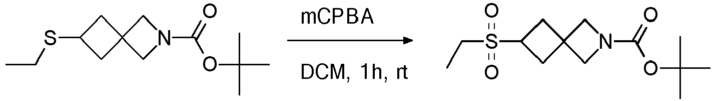

A mixture of tert-butyl 6-ethylsulfanyl-2-azaspiro[3.3]heptane-2-carboxylate (1.0 eq, 1.7 g, 6.6 mmol) and 3-chlorobenzene-1-carboperoxoic acid (2.5 eq, 2.8 g, 16.5 mmol) in DCM (34 mL) was stirred at room temperature for 1 h under a nitrogen atmosphere. The reaction was quenched by the addition of sat. NaHCO₃ (aq.) (40 mL) at room temperature. The resulting mixture was extracted with EtOAc (3 x 30 mL). The combined organic layers were washed with water (3 x 10 mL), dried over anhydrous Na₂SO₄. After filtration, the filtrate was concentrated under reduced pressure. The crude product, tert-butyl 6-ethylsulfonyl-2-azaspiro[3.3]heptane-2-carboxylate (1.5 g, 71%), was used in the next step directly without further purification.

### Synthesis of 6-(ethanesulfonyl)-2-azaspiro[3.3]heptane

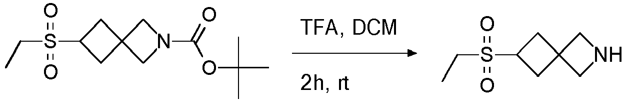

A mixture of tert-butyl 6-ethylsulfonyl-2-azaspiro[3.3]heptane-2-carboxylate (1.0 eq, 1.3 g, 4.5 mmol) and trifluoroacetic acid (6.5mL) in DCM (26 mL) were stirred at room temperature for 2 h under nitrogen atmosphere. The resulting mixture was concentrated under reduced pressure. The crude product 6-ethylsulfonyl-2-azaspiro[3.3]heptane (400 mg, 42% yield) was used in the next step directly without further purification (general procedure III in the synthesis of compound 115).

The reactant 6-methanesulfonyl-2-azaspiro[3.3]heptane used for the synthesis of compound 114 is prepared analogously as described above for 6-ethylsulfonyl-2-azaspiro[3.3]heptane.

### Synthesis of 1-{2,6-diazaspiro[3.3]heptan-2-yl}cyclopropane-1-carbonitrile to be used in the synthesis of compound 116:

### Synthesis of tert-butyl 6-(1-cyanocyclopropyl)-2,6-diazaspiro[3.3]heptane-2-carboxylate

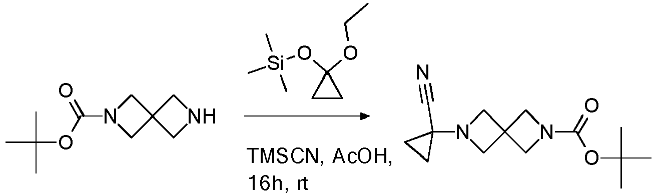

A solution of tert-butyl 2,6-diazaspiro[3.3]heptane-2-carboxylate (1.0 eq, 2.0 g, 10.1 mmol), (1-ethoxycyclopropoxy)-trimethyl-silane (1.0 eq, 1.76 g, 10.1 mmol), trimethylsilylformonitrile (1.0 eq, 1.0 g, 10.1 mmol) and HOAc (20 mL) was stirred at room temperature overnight under nitrogen atmosphere. The resulting mixture was concentrated under reduced pressure. The residue was purified by silica gel column chromatography, eluted with PE / THF (1:1) to afford tert-butyl 2-(1-cyanocyclopropyl)-2,6-diazaspiro[3.3]heptane-6-carboxylate (0.50 g, 1.52 mmol, 15% yield) as a yellow oil.

### Synthesis of 1-{2,6-diazaspiro[3.3]heptan-2-yl}cyclopropane-1-carbonitrile

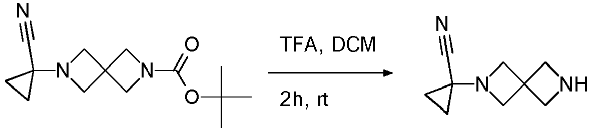

A solution of tert-butyl 2-(1-cyanocyclopropyl)-2,6-diazaspiro[3.3]heptane-6-carboxylate (1.0 eq, 0.5 g, 1.5 mmol) and trifluoroacetic acid (1 mL) in DCM (5 mL) was stirred at room temperature for 2 h under nitrogen atmosphere. The resulting mixture was concentrated under reduced pressure. The crude product was filtered via anion exchange chromatography to obtain 1-(2,6-diazaspiro[3.3]heptan-2-yl)cyclopropanecarbonitrile (0.35 g, 70% yield) as a yellow oil which was used without further purification in the next step (general procedure III in the synthesis of compound 116).

The following compound was synthesized by further modification of a product obtained from the general procedure III:
**Synthesis of 2-{2-[5-(ethanesulfonyl)-6-[3-methyl-6-(1,1,2,2,2-pentafluoroethyl)-3H-imidazo[4,5-b]pyridin-2-yl]pyridin-3-yl]-2-azaspiro[3.3]heptan-6-yl}acetonitrile (Compound 60)**
**Synthesis of 2-{2-[5-(ethanesulfonyl)-6-[3-methyl-6-(1,1,2,2,2-pentafluoroethyl)-3H-imidazo[4,5-b]pyridin-2-yl]pyridin-3-yl]-2-azaspiro[3.3]heptan-6-yl}acetic acid**

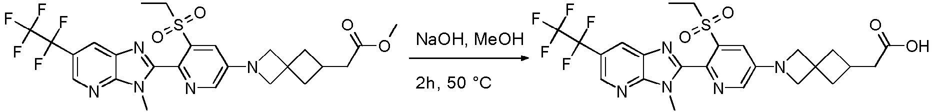

A 5 mL vial was charged with methyl 2-{2-[5-(ethanesulfonyl)-6-[3-methyl-6-(1,1,2,2,2-pentafluoroethyl)-3H-imidazo[4,5-b]pyridin-2-yl]pyridin-3-yl]-2-azaspiro[3.3]heptan-6-yl}acetate (obtained from general procedure III and commercial starting materials) (1.0 eq, 190 mg, 0.3 mmol), 3 mL of methanol and 0.5 mL sodium hydroxide solution 4 M. The mixture was stirred for 2 h at 50°C. The reaction was diluted with DCM (10 mL) and quenched by the addition of 15 mL 1 N HCl. The organic layer was separated by phase separation cartridge and evaporated to yield 2-{2-[5-(ethanesulfonyl)-6-[3-methyl-6-(1,1,2,2,2-pentafluoroethy1)-3H-imidazo[4,5-b]pyridin-2-yl]pyridin-3-yl]-2-azaspiro[3.3]heptan-6-yl;acetic acid (170 mg, 91%) as a yellow residue and used in subsequent steps without further purification.

### Synthesis of 2-{2-[5-(ethanesulfonyl)-6-[3-methyl-6-(1,1,2,2,2-pentafluoroethyl)-3H-imidazo[4,5-b]pyridin-2-yl]pyridin-3-yl]-2-azaspiro[3.3]heptan-6-yl}acetamide

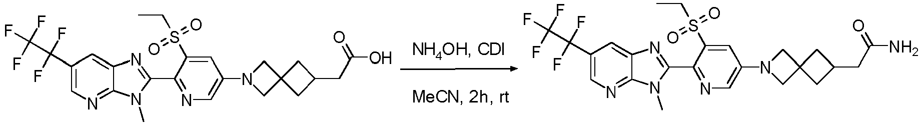

A 5 mL vial was charged with 2-{2-[5-(ethanesulfonyl)-6-[3-methyl-6-(1,1,2,2,2-pentafluoroethyl)-3H-imidazo[4,5-b]pyridin-2-yl]pyridin-3-yl]-2-azaspiro[3.3]heptan-6-yl}acetic acid (1.0 eq, 170 mg, 0.3 mmol), 1,1' Carbonyldiimidazole (1.3 eq, 60 mg, 0.4 mmol) and 3 mL acetonitrile. After stirring for 30 min at 40 °C, the mixture was cooled to 0 °C and a solution of NH₄OH in acetonitrile (5.3 eq, 0.3 mL, 1.5 mmol) was added and the mixture stirred for 2 h at room temperature. The reaction mixture was diluted with DCM (10 mL) and washed with aq. sodium carbonate solution (10%) and sat. brine. The organic layer was separated by phase separation cartridge and evaporated to yield 2-{2-[5-(ethanesulfonyl)-6-[3-methyl-6-(1,1,2,2,2-pentafluoroethyl)-3H-imidazo[4,5-b]pyridin-2-yl]pyridin-3-yl]-2-azaspiro[3.3]heptan-6-yl}acetamide (140 mg, 0.2 mmol, 82%) as a yellow product.

### Synthesis of 2-{2-[5-(ethanesulfonyl)-6-[3-methyl-6-(1,1,2,2,2-pentafluoroethyl)-3H-imidazo[4,5-b]pyridin-2-yl]pyridin-3-yl]-2-azaspiro[3.3]heptan-6-yl}acetonitrile

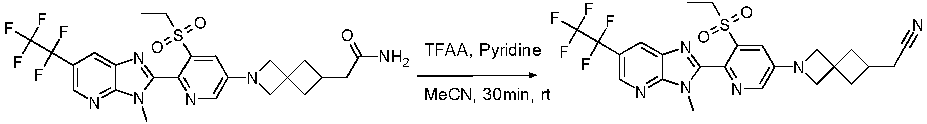

A 5 mL vial was charged with 2-{2-[5-(ethanesulfonyl)-6-[3-methyl-6-(1,1,2,2,2-pentafluoroethyl)-3H-imidazo[4,5-b]pyridin-2-yl]pyridin-3-yl]-2-azaspiro[3.3]heptan-6-yl}acetamide (1.0 eq, 140 mg, 0.2 mmol) and pyridine (0.1 mL) was added. At 0 °C, trifluoroacetic acid anhydride was added dropwise and the mixture stirred for 30 min at room temperature. The mixture was quenched with water (5 mL) and extracted with ethyl acetate. The organic layer was washed with aq. sodium carbonate solution (10%) and brine, filtered over Celite, and evaporated. The residue was purified by silica gel column chromatography, eluted with cyclohexane/EA (0-100%). Product fractions were evaporated and lyophilized from acetonitrile/water to afford 2-{2-[5-(ethanesulfonyl)-6-[3-methyl-6-(1,1,2,2,2-pentafluoroethyl)-3H-imidazo[4,5-b]pyridin-2-y1]pyridin-3-yl]-2-azaspiro[3.3]heptan-6-yl}acetonitrile (78 mg, 0.1 mmol, 62%) as a white solid.

The following compound was synthesized by further modification of a product obtained from the general procedure III:

### Synthesis of 2-{6-[5-(ethanesulfonyl)-6-[3-methyl-6-(1,1,2,2,2-pentafluoroethyl)-3H-imidazo[4,5-b]pyridin-2-yl]pyridin-3-yl]-2,6-diazaspiro[3.3]heptan-2-yl}acetonitrile (Compound 61)

### Synthesis of 2-[5-(ethanesulfonyl)-6-[3-methyl-6-(1,1,2,2,2-pentafluoroethyl)-3H-imidazo[4,5-b]pyridin-2-yl]pyridin-3-yl]-2,6-diazaspiro[3.3]heptane

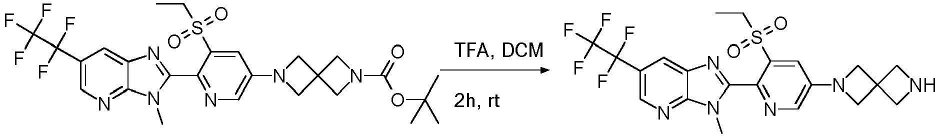

In a 5 mL vial tert-butyl 6-[5-(ethanesulfonyl)-6-[3-methyl-6-(1,1,2,2,2-pentafluoroethyl)-3H-imidazo[4,5-b]pyridin-2-yl]pyridin-3-yl]-2,6-diazaspiro[3.3]heptane-2-carboxylate (obtained from general procedure III and commercial starting materials) (1.0 eq, 165 mg, 0.3 mmol) was dissolved in DCM (3.3 mL). After addition of 0.3 mL of trifluoroacetic acid the mixture was stirred for two hours at room temperature. The reaction mixture was diluted with DCM (10 mL) and washed with aq. sodium carbonate solution (10%) and sat. brine. The organic layer was separated by phase separation cartridge and evaporated to yield 2-[5-(ethanesulfonyl)-6-[3-methyl-6-(1,1,2,2,2-pentafluoroethyl)-3H-imidazo[4,5-b]pyridin-2-yl]pyridin-3-yl]-2,6-diazaspiro[3.3]heptane (130mg, 94%) as yellow residue which was used without further purification in the next step.

### Synthesis of 2-{6-[5-(ethanesulfonyl)-6-[3-methyl-6-(1,1,2,2,2-pentafluoroethyl)-3H-imidazo[4,5-b]pyridin-2-yl]pyridin-3-yl]-2,6-diazaspiro[3.3]heptan-2-yl}acetonitrile

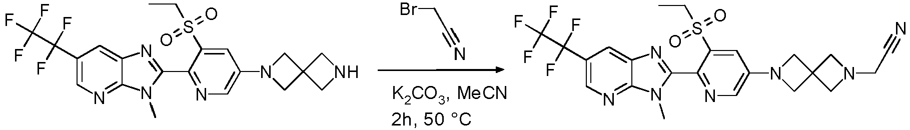

A 5 mL vial was charged with 2-[5-(ethanesulfonyl)-6-[3-methyl-6-(1,1,2,2,2-pentafluoroethyl)-3H-imidazo[4,5-b]pyridin-2-yl]pyridin-3-yl]-2,6-diazaspiro[3.3]heptane (1 equiv, 130mg, 0.25 mmol), potassium carbonate (2.1 eq, 75 mg, 0.55 mmol) and 3.5 mL of acetonitrile. To the mixture was added 2-bromoacetonitrile (1.4 eq, 0.2 mL, 0.35 mmol) and the mixture was stirred for 2 h at 50 °C. After cooling to room temperature, the reaction mixture was diluted with DCM (10 mL) and washed with sat. brine. The organic layer was separated by phase separation cartridge, evaporated and the crude product purification by silica column (cyclohexane:ethyl acetate 0-100%, then 5 CV ethyl acetate:methanol 9:1). The product fractions were evaporated and lyophilized from acetonitrile/water to afford 2-{6-[5-(ethanesulfonyl)-6-[3-methyl-6-(1,1,2,2,2-pentafluoroethyl)-3H-imidazo[4,5-b]pyridin-2-yl]pyridin-3-yl]-2,6-diazaspiro[3.3]heptan-2-yl}acetonitrile (61 mg, 0.11 mmol, 43%) as an off-white solid.

### Synthesis of 2-(ethanesulfonyl)-6-[5-(ethanesulfonyl)-6-[3-methyl-6-(1,1,2,2,2-pentafluoroethyl)-3H-imidazo[4,5-b]pyridin-2-yl]pyridin-3-yl]-2,6-diazaspiro[3.3]heptane (Compound 111):

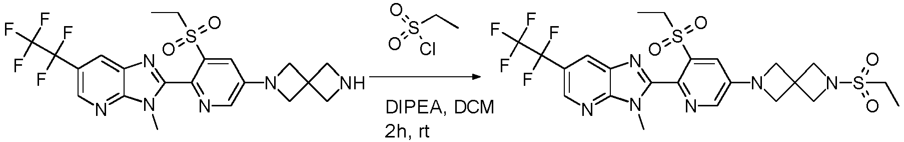

In a 2 mL vial 2-[5-(ethanesulfonyl)-6-[3-methyl-6-(1,1,2,2,2-pentafluoroethyl)-3H-imidazo[4,5-b]pyridin-2-yl]pyridin-3-yl]-2,6-diazaspiro[3.3]heptane (1.0 equiv, 80 mg, 0.15 mmol) and DIPEA (2 equiv, 54 µl, 0.3 mmol) were dissolved in 1 mL DCM. After addition of ethanesulfonyl chloride (1 equiv, 19 µL, 0.15 mmol) the mixture was stirred for 2 h at room temperature. The reaction mixture was evaporated to dryness and directly purified by preparative HPLC (ACN/water/TFA) to afford 2-(ethanesulfonyl)-6-[5-(ethanesulfonyl)-6-[3-methyl-6-(1,1,2,2,2-pentafluoroethyl)-3H-imidazo[4,5-b]pyridin-2-yl]pyridin-3-yl]-2,6-diazaspiro[3.3]heptane (59mg, 0.09 mmol, 62%) as solid.

The following compound was synthesized by further modification of a product obtained from the general procedure III:

### Synthesis of 6-[5-(ethanesulfonyl)-6-[3-methyl-6-(1,1,2,2,2-pentafluoroethyl)-3H-imidazo[4,5-b]pyridin-2-yl]pyridin-3-yl]-2-imino-2lambda6-thia-6-azaspiro[3.3]heptan-2-one (Compound 83):

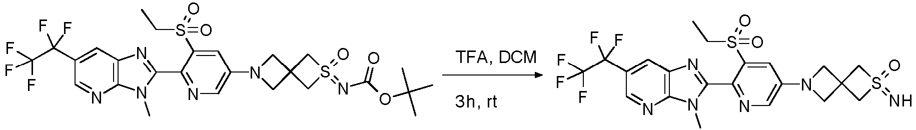

In a 2 mL vial tert-butyl N-{6-[5-(ethanesulfonyl)-6-[3-methyl-6-(1,1,2,2,2-pentafluoroethyl)-3H-imidazo[4,5-b]pyridin-2-yl]pyridin-3-yl]-2-oxo-2lambda6-thia-6-azaspiro[3.3]heptan-2-ylidene}carbamate (obtained from general procedure III and commercial starting materials) (1.0 eq, 78 mg, 0.1 mmol) was dissolved in DCM (1 mL). After addition of 0.07 mL of trifluoroacetic acid the mixture was stirred for 3 hours at room temperature. The reaction mixture was evaporated to dryness and directly purified by preparative HPLC (ACN/water/TFA) to afford 6-[5-(ethanesulfonyl)-6-[3-methyl-6-(1,1,2,2,2-pentafluoroethyl)-3H-imidazo[4,5-b]pyridin-2-yl]pyridin-3-yl]-2-imino-2lambda6-thia-6-azaspiro[3.3]heptan-2-one (65 mg, 0.11 mmol, 98%) as solid.

The following compounds 89, 93a and 93b were synthesized by further reacting compound 92, which was synthesized via the general procedure III:

### Synthesis of 6-[5-(ethanesulfonyl)-6-[3-methyl-6-(1,1,2,2,2-pentafluoroethyl)-3H-imidazo[4,5-b]pyridin-2-yl]pyridin-3-yl]-1lambda6-thia-6-azaspiro[3.3]heptane-1,1-dione (Compound 89):

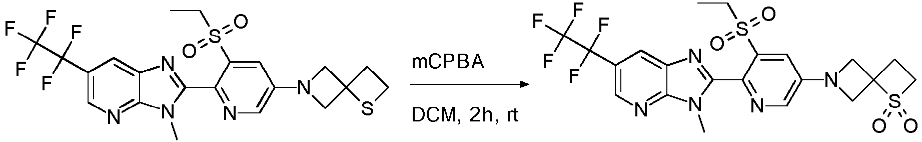

A solution of 6-[5-(ethanesulfonyl)-6-[3-methyl-6-(1,1,2,2,2-pentafluoroethyl)imidazo[4,5-b]pyridin-2-yl]pyridin-3-yl]-1-thia-6-azaspiro[3.3]heptane (1 equiv, 75 mg, 0.14 mmol) (obtained from general procedure III and commercial starting materials) in 2 mL of DCM was treated with m-chlorperbenzoic acid (2.5 equiv, 71.3 mg, 0.35 mmol) under nitrogen atmosphere and stirred at 25 °C for 1 h . The resulting mixture was quenched with 10 ml of 10% Na₂SO₃ and diluted with 10 mL DCM. The organic phase was washed 1 x 10 mL of 10% NaHCO₃, then 1 x 50 mL of brine. The organic phase was collected and dried over anhydrous sodium sulfate and concentrated. The crude product was purified by preparative HPLC (ACN/water/TFA) to afford 6-[5-(ethanesulfonyl)-6-[3-methyl-6-(1,1,2,2,2-pentafluoroethyl)imidazo[4,5-bjpyridin-2-yl]pyridin-3-yl]-1lambda6-thia-6-azaspiro[3.3]heptane-1,1-dione (18 mg) as an off-white solid.

### Synthesis of (1R)-6-[5-(ethanesulfonyl)-6-[3-methyl-6-(1,1,2,2,2-pentafluoroethyl)-3H-imidazo[4,5-b]pyridin-2-yl]pyridin-3-yl]-1-imino-1lambda4-thia-6-azaspiro[3.3]heptan-1-ium-1-olate (Compound 93a) and (1S)-6-[5-(ethanesulfonyl)-6-[3-methyl-6-(1,1,2,2,2-pentafluoroethyl)-3H-imidazo[4,5-b]pyridin-2-yl]pyridin-3-yl]-1-imino-1lambda4-thia-6-azaspiro[3.3]heptan-1-ium-1-olate (Compound 93b):

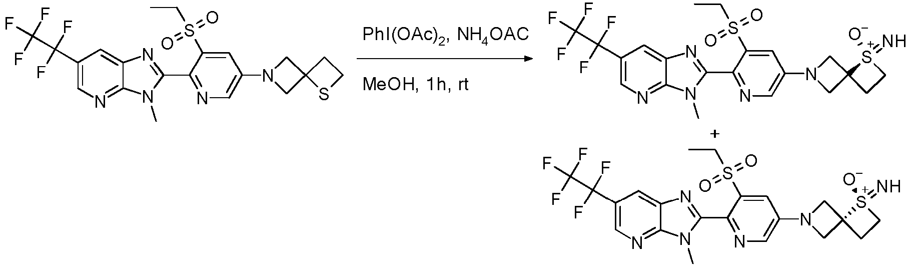

A solution of 6-[5-ethylsulfonyl-6-[3-methyl-6-(1,1,2,2,2-pentafluoroethyl)imidazo[4,5-b]pyridin-2-yl]-3-pyridyl]-1-thia-6-azaspiro[3.3]heptane (1.0 eq, 0.15 g, 0.3 mmol) in MeOH (3mL) was treated with (acetyloxy)(phenyl)-lambda3-iodanyl acetate (2.0 eq, 0.2 g, 0.6 mmol) and ammonium acetate (2.5 eq, 0.05 g, 0.7 mmol) under nitrogen atmosphere. The mixture was stirred at 25 °C for 1h. After evaporation, the residue was purified by silica gel column chromatography, eluted with PE/THF (1:1) to afford 100 mg crude product which was further purified by Prep-HPLC (XBridge Prep OBD C18 Column, 30*150 mm, 5µm; Mobile Phase A: Water(0.05%NH₃H₂O), Mobile Phase B: ACN; Flow rate: 35 mL/min) to afford racemic 6-(5-(ethylsulfonyl)-6-(3-methyl-6-(perfluoroethyl)-3H-imidazo[4,5-b]pyridin-2-yl)pyridin-3-yl)-1-imino-1l6-thia-6-azaspiro[3.3]heptane 1-oxide (60 mg) as an off-white solid. The enantiomers were separated by chiral chromatography using method G to afford of (1R)-6-[5-(ethanesulfonyl)-6-[3-methyl-6-(1,1,2,2,2-pentafluoroethyl)-3H-imidazo[4,5-b]pyridin-2-yl]pyridin-3-yl]-1-imino-1lambda4-thia-6-azaspiro[3.3]heptan-1-ium-1-olate (93a) (17 mg, 10% yield) as an off-white solid and (1S)-6-[5-(ethanesulfonyl)-6-[3-methyl-6-(1,1,2,2,2-pentafluoroethyl)-3H-imidazo[4,5-b]pyridin-2-yl]pyridin-3-yl]-1-imino-1lambda4-thia-6-azaspiro[3.3]heptan-1-ium-1-olate (93b) (13 mg, 8% yield) as an off-white solid.

The following compound was also synthesized with procedures deviating from the general procedures:

### Synthesis of 2-[5-(ethanesulfonyl)-6-[3-methyl-6-(1,1,2,2,2-pentafluoroethyl)-3H-imidazo[4,5-b]pyridin-2-yl]pyridin-3-yl]-6-methyl-6lambda6-thia-2,7-diazaspiro[3.4]oct-6-en-6-one (Compound 117):

### Synthesis of ethyl 3-(chloromethyl)-1-[5-(ethanesulfonyl)-6-[3-methyl-6-(1,1,2,2,2-pentafluoroethyl)-3H-imidazo[4,5-b]pyridin-2-yl]pyridin-3-yl]azetidine-3-carboxylate

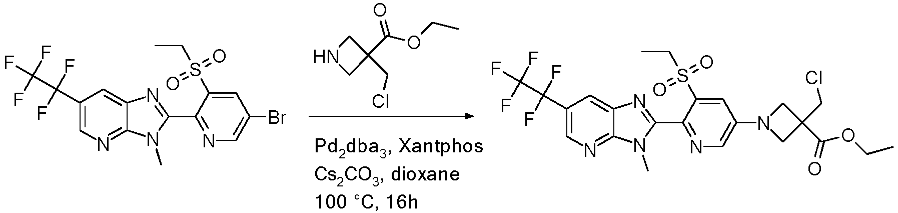

Into a 40 mL vial were added 5-bromo-3-(ethanesulfonyl)-2-[3-methyl-6-(1,1,2,2,2-pentafluoroethyl)imidazo[4,5-b]pyridin-2-yl]pyridine (1 g, 2.0 mmol, 1.0 equiv), ethyl 3-(chloromethyl)azetidine-3-carboxylate (3.0 equiv, 1.1 g, 6.0 mmol), XantPhos (0.4 equiv, 0.5 g, 0.8 mmol), Cs₂CO₃ (3.0 equiv, 2.0 g, 6.0 mmol), and Pd₂(dba)₃ (0.2 equiv, 0.4 g, 0.4 mmol) in dioxane (10 mL). The resulting mixture was stirred at 100 °C overnight under a nitrogen atmosphere. The resulting mixture was concentrated under reduced pressure. The residue was purified by silica gel column chromatography, eluted with PE/EA (1:1) to afford ethyl 3-(chloromethyl)-1-[5-(ethanesulfonyl)-6-[3-methyl-6-(1,1,2,2,2-pentafluoroethyl)imidazo[4,5-b]pyridin-2-yl]pyridin-3-yl]azetidine-3-carboxylate (900 mg, 68%) as a light yellow solid.

### Synthesis of ethyl 1-[5-(ethanesulfonyl)-6-[3-methyl-6-(1,1,2,2,2-pentafluoroethyl)-3H-imidazo[4,5-b]pyridin-2-yl]pyridin-3-yl]-3-[(methylsulfanyl)methyl]azetidine-3-carboxylate

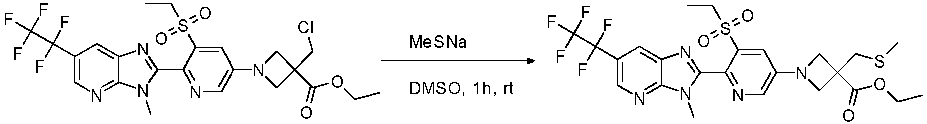

Into a 40 mL vial were added ethyl 3-(chloromethyl)-1-[5-(ethanesulfonyl)-6-[3-methyl-6-(1,1,2,2,2-pentafluoroethyl)imidazo[4,5-b]pyridin-2-yl]pyridin-3-yl]azetidine-3-carboxylate (1 equiv, 900 mg, 1.5 mmol) in DMSO (10 mL). To the above mixture was added sodium thiomethoxide (1.2 equiv, 127 mg, 1.8 mmol) in portions. The resulting mixture was stirred at room temperature for 1h. The reaction was poured into water. The aqueous layer was extracted with EA (3 x 5 mL). The combined organic layers were washed with brine (3 x 5 mL), dried over anhydrous Na₂SO₄. After filtration, the filtrate was concentrated under reduced pressure. The residue was purified by silica gel column chromatography, eluted with PE / EA (1:1) to afford ethyl 1-[5-(ethanesulfonyl)-6-[3-methyl-6-(1,1,2,2,2-pentafluoroethyl)imidazo[4,5-b]pyridin-2-yl]pyridin-3-yl]-3-[(methylsulfanyl)methyl]azetidine-3-carboxylate (450 mg, 44%) as a light yellow solid.

### Synthesis of {1-[5-(ethanesulfonyl)-6-[3-methyl-6-(1,1,2,2,2-pentafluoroethyl)-3H-imidazo[4,5-b]pyridin-2-yl]pyridin-3-yl]-3-[(methylsulfanyl)methyl]azetidin-3-yl}methanol

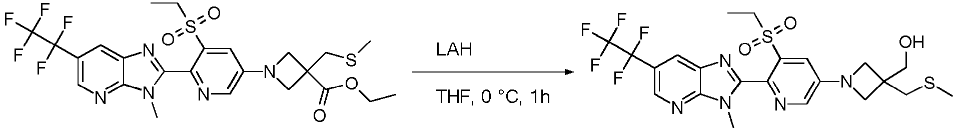

Into a 40 mL vial were added ethyl 1-[5-(ethanesulfonyl)-6-[3-methyl-6-(1,1,2,2,2-pentafluoroethyl)imidazo[4,5-b]pyridin-2-yl]pyridin-3-yl]-3-[(methylsulfanyl)methyl]azetidine-3-carboxylate (1 equiv, 450 mg, 0.7 mmol) in THF (9 mL) at room temperature. To the above mixture was added Lithium aluminum hydride (2.5 M in THF) (0.7 equiv, 0.2 mL, 0.5 mmol,) dropwise at 0°C. The resulting mixture was stirred at 0 °C for additional 1h. The reaction was quenched by the addition of MeOH (2mL) at 0°C. The residue was purified by reversed-phase flash chromatography with the following conditions: column, C18 silica gel; mobile phase, MeCN in Water (0.1% TFA), 10% to 60% gradient in 10 min; detector, UV 254 nm to afford {1-[5-(ethanesulfonyl)-6-[3-methyl-6-(1,1,2,2,2-pentafluoroethyl)imidazo [4,5-b]pyridin-2-yl]pyridin-3-yl]-3-[(methylsulfanyl)methyl] azetidin-3-yl}methanol (300 mg) as a off-white solid.

### Synthesis of {1-[5-(ethanesulfonyl)-6-[3-methyl-6-(1,1,2,2,2-pentafluoroethyl)-3H-imidazo[4,5-b]pyridin-2-yl]pyridin-3-yl]-3-[(methylsulfanyl)methyl]azetidin-3-yl}methyl methanesulfonate

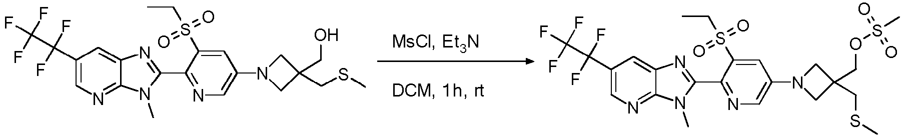

Into a 20 mL vial were added {1-[5-(ethanesulfonyl)-6-[3-methyl-6-(1,1,2,2,2-pentafluoroethyl)imidazo[4,5-b]pyridin-2-yl]pyridin-3-yl]-3-[(methylsulfanyl)methyl]azetidin-3-yl}methanol (1 equiv, 300 mg, 0.5 mmol) and Et₃N (3 equiv, 161 mg, 1.6 mmol) in DCM (3 mL). To the above mixture was added methanesulfonyl chloride (3 equiv, 182 mg, 1.6 mmol) dropwise at 0°C. The resulting mixture was stirred at room temperature for additional 1h. The reaction was poured into water. The aqueous layer was extracted with CH₂Cl₂ (3 x 5 mL). The combined organic layers were washed with brine (1 x 10 mL), dried over anhydrous Na₂SO₄. After filtration, the filtrate was concentrated under reduced pressure to afford {1-[5-(ethanesulfonyl)-6-[3-methyl-6-(1,1,2,2,2-pentafluoroethyl)imidazo [4,5-b]pyridin-2-yl]pyridin-3-yl]-3-[(methylsulfanyl)methyl] azetidin-3-yl}methyl methanesulfonate (250 mg, 62%) as an off-white solid.

### Synthesis of {1-[5-(ethanesulfonyl)-6-[3-methyl-6-(1,1,2,2,2-pentafluoroethyl)-3H-imidazo[4,5-b]pyridin-2-yl]pyridin-3-yl]-3-{[imino(methyl)oxo-lambda6-sulfanyl]methyl}azetidin-3-yl}methyl methanesulfonate

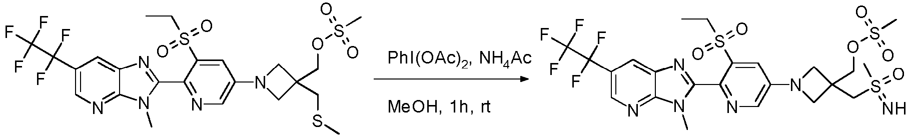

Into a 10 mL vial were added {1-[5-(ethanesulfonyl)-6-[3-methyl-6-(1,1,2,2,2-pentafluoroethyl)imidazo[4,5-b]pyridin-2-yl]pyridin-3-yl]-3-[(methylsulfanyl)methyl]azetidin-3-yl}methyl methanesulfonate (1 equiv, 250 mg, 0.4 mmol,) , (acetyloxy)(phenyl)-1^[3]-iodanyl acetate (1.5 equiv, 188 mg, 0.6 mmol) , ammonium carbamate (1 equiv, 30 mg, 0.4 mmol) and methanol (2.5 mL). The resulting mixture was stirred at room temperature for 1h. The resulting mixture was used in the next step directly without further purification.

### Synthesis of 2-[5-(ethanesulfonyl)-6-[3-methyl-6-(1,1,2,2,2-pentafluoroethyl)-3H-imidazo[4,5-b]pyridin-2-yl]pyridin-3-yl]-6-methyl-6lambda6-thia-2,7-diazaspiro[3.4]oct-6-en-6-one (Compound 117)

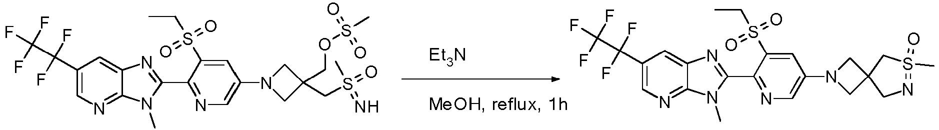

To the reaction mixture of the previous step was added Et₃N (3 equiv, 112 mg, 1.1 mmol) at room temperature. The resulting mixture was stirred at 100 °C for 1 h in a sealed pressure tube. After cooling to room temperature and evaporation of solvents, the crude product was purified by Prep-HPLC with the following conditions (column, C18 silica gel; mobile phase, MeCN in Water (0.1% NH₃.H₂O), 10% to 80% gradient in 10 min; detector, UV 254 nm) to afford 2-[5-(ethanesulfonyl)-6-[3-methyl-6-(1,1,2,2,2-pentafluoroethyl)imidazo[4,5-b]pyridin-2-yl]pyridin-3-yl]-6-methyl-6lambda6-thia-2,7-diazaspiro[3.4]oct-6-en-6-one (29 mg, 13%) as a white solid.

The following compound was also synthesized with procedures deviating from the general procedures:

### Synthesis of 5-[5-(ethanesulfonyl)-6-[3-methyl-6-(1,1,2,2,2-pentafluoroethyl)-3H-imidazo[4,5-b]pyridin-2-yl]pyridin-3-yl]-5-azaspiro[2.4]heptan-6-one (Compound 98):

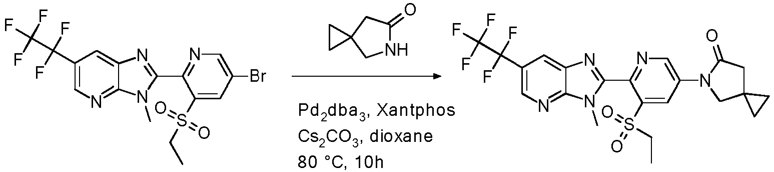

To a stirred mixture of 2-(5-bromo-3-ethylsulfonyl-2-pyridyl)-3-methyl-6-(1,1,2,2,2-pentafluoroethyl)imidazo[4,5-b]pyridine (1.0 eq, 120 mg, 0.24 mmol) and 5-azaspiro[2.4]heptan-6-one (1.2 eq, 32 mg, 0.3 mmol) in 1,4-dioxane (1.5 mL) were added Pd₂(dba)₃ (0.1 eq, 22 mg, 0.02 mmol) , Xantphos (0.2 eq, 28 mg, 0.04 mmol) and Cs₂CO₃ (2.0 eq, 157 mg, 0.5 mmol) under nitrogen atmosphere. The resulting mixture was stirred at 80 °C for 1 h under nitrogen atmosphere. The resulting mixture was concentrated under reduced pressure. The residue was purified by silica gel column chromatography, eluted with PE / EA (3:2) and further lyophilization to afford 5-[5-ethylsulfonyl-6-[3-methyl-6-(1,1,2,2,2-pentafluoroethyl)imidazo[4,5-b]pyridin-2-yl]-3-pyridyl]-5-azaspiro[2.4]heptan-6-one (90 mg, 68% yield) as an off-white solid.

### EXAMPLE 2: Physicochemical characterization of Compounds 1 to 122b

The determination of [M+H]⁺ by LC-MS under acidic chromatographic conditions is done with aqueous gradients containing up to 0.1 % TFA as eluents. Columns used typically include XBridge BEH C18_2.1 x 30 mm and Sunfire C18_2.1 x 30 mm with temperatures at 60°C.

NMR spectra have typically been acquired using Bruker Biospin Avance III HD 400 MHz spectrometer or Bruker Biospin Avance Neo 400 MHz spectrometer. Chemical shifts (δ) are given in [ppm] in reference to tetramethylsilane (TMS) in water as the internal standard. In the absence of the internal standard, the values of chemical shifts are given according to the signals of residual solvents, which have been set for ¹H-NMR: CHCl₃ (7.26 ppm), DMSO (2.50 ppm).

For several examples the ¹H-NMR data are presented in the form of ¹H-NMR peak lists. Each signal peak is characterized first by the δ value in ppm, followed by the signal intensity enclosed in round brackets. The pairs of δ value-signal intensity numbers for different signal peaks are listed with separation from one another by semicolons. The format for a typical peak list is as follows: δ₁ (intensity₁), δ₂ (intensity₂), ....δₙ (intensityₙ). The intensity of shift signals correlates with the height of the signals in a printed example of an NMR spectrum in cm. This shows the true ratios of the signal intensities. For broad signals, the middle of the signal or several peaks and their relative intensity may be displayed relative to the spectrum's most intense signal. The chemical shifts of¹H-NMR spectrums are typically calibrated using tetramethylsilane and/or the solvent's chemical shift, especially when the spectra are measured in DMSO. As such, the tetramethylsilane peak may or may not appear in NMR peak lists. Moreover, similar to traditional ¹H-NMR printouts, they might display solvent signals, signals of compound stereoisomers provided by the invention, and/or impurity peaks. The peaks of stereoisomers of compounds and/or impurity peaks usually exhibit a lower average intensity than the compound peaks (for instance, with a purity of > 90%). These stereoisomers and/or impurities might be characteristic of the specific preparation process. Their peaks can assist in identifying the replication of our preparation process, referencing "by-product fingerprints". A person proficient in the field can calculate the peaks of the target compounds using known methods (MestreC, ACD simulation, and empirically evaluated expected values), and if necessary, isolate the target compound peaks, possibly using additional intensity filters. This isolation process would resemble the peak picking used in traditional ¹H-NMR interpretation. More information about ¹H-NMR peak lists can be found in the Research Disclosure Database under Number 564025 (Questel).

| **Compound** | **ESI-MS (m/z)** | **¹H-NMR data** |
|---|---|---|
| **1** | 519 [M+H]⁺ | (400 MHz, Chloroform-d, *ppm)* δ 9.20 (s, 1H), 8.77 (d, *J* = 1.8 Hz, 1H), 8.72 (d, *J* = 2.1 Hz, 1H), 8.35 (s, 1H), 3.91 (d, *J* = 22.8 Hz, 4H), 3.16 (dd, *J* = 22.4, 14.1 Hz, 2H), 2.61 (t, *J* = 13.6 Hz, 2H), 1.42 (t, *J* = 7.4 Hz, 3H), 0.80-0.76 (m, 4H) |
| **2** | 569 [M+H]⁺ | (400 MHz, DMSO-*d*_{6,} *ppm*) δ 9.25 (d, *J* = 2.0 Hz, 1H), 8.85 (d, *J* = 2.0 Hz, 1H), 8.66 (d, *J* = 2.1 Hz, 1H), 8.48 (d, *J* = 2.1 Hz, 1H), 3.86 (q, *J* = 7.3 Hz, 2H), 3.80 (s, 3H), 3.17-3.04 (m, 2H), 2.96-2.73 (m, 6H), 1.23 (t, *J* = 7.3 Hz, 3H) |
| **3** | 540 [M+H]⁺ | (400.1 MHz, d6-DMSO): δ=8.9880 (2.3); 8.9835 (2.5); 8.8153 (2.0); 8.8105 (2.1); 8.6085 (2.1); 8.6037 (2.1); 8.3090 (2.3); 8.3080 (2.3); 8.3040 (2.3); 7.2157 (0.4); 7.0879 (0.4); 6.9602 (0.4); 4.8811 (0.2); 3.8236 (0.9); 3.8051 (3.2); 3.7866 (3.3); 3.7680 (1.2); 3.7579 (16.0); 3.7386 (0.7); 3.7163 (1.0); 3.6940 (0.6); 3.6721 (0.2); 3.3336 (0.3); 3.3133 (0.9); 3.2922 (1.5); 3.2719 (1.0); 3.2509 (0.3); 3.1736 (0.3); 2.6653 (0.4); 2.6573 (0.4); 2.6434 (0.4); 2.6369 (0.8); 2.6297 (0.6); 2.6144 (0.8); 2.6087 (0.7); 2.5891 (1.1); 2.5797 (0.4); 2.5756 (0.4); 2.5667 (1.1); 2.5637 (1.1); 2.5509 (0.4); 2.5421 (0.6); 2.5303 (0.4); 2.5120 (2.3); 2.5075 (5.3); 2.5030 (6.5); 2.4983 (4.5); 2.4937 (2.3); 2.4886 (1.1); 2.4800 (0.7); 2.4600 (0.6); 2.4336 (0.4); 2.4256 (0.3); 2.4118 (0.4); 2.4044 (0.9); 2.3965 (0.6); 2.3799 (1.0); 2.3760 (1.1); 2.3509 (1.7); 2.3369 (1.2); 2.3254 (1.7); 2.3175 (1.2); 2.3079 (0.7); 2.2980 (0.6); 2.2880 (0.6); 1.2232 (3.3); 1.2047 (7.7); 1.1862 (3.3); -0.0004 (1.3) |
| **3a** | 540 [M+H]⁺ | |
| **3b** | 540 [M+H]⁺ | |
| 4 | 536 [M+H]⁺ | (400.1 MHz, d6-DMSO): δ=8.9137 (2.2); 8.9089 (2.2); 8.8276 (2.1); 8.8229 (2.2); 8.6282 (2.2); 8.6234 (2.1); 8.1575 (2.2); 8.1526 (2.2); 7.2037 (0.2); 7.0760 (0.3); 6.9482 (0.2); 4.1536 (0.9); 3.9653 (0.2); 3.9091 (0.2); 3.8907 (0.5); 3.8724 (0.8); 3.8552 (1.5); 3.8476 (0.5); 3.8367 (1.5); 3.8291 (1.5); 3.8181 (0.5); 3.8107 (1.6); 3.7895 (16.0); 3.7763 (1.0); 3.7569 (0.2); 3.2675 (1.2); 3.2338 (1.1); 3.1709 (0.6); 2.9447 (0.2); 2.7852 (0.1); 2.5230 (0.1); 2.5097 (5.3); 2.5052 (11.5); 2.5006 (16.1); 2.4960 (11.5); 2.4914 (5.4); 2.4599 (0.3); 2.4444 (0.4); 2.4366 (0.5); 2.4242 (0.8); 2.4054 (0.8); 2.3907 (0.5); 2.3786 (0.2); 2.3616 (0.2); 2.3274 (0.1); 2.3225 (0.1); 2.3107 (0.2); 2.3023 (0.1); 2.2774 (0.5); 2.2674 (0.4); 2.2614 (0.3); 2.2507 (0.3); 2.2432 (0.2); 2.2286 (0.2); 2.2116 (0.3); 2.2044 (0.4); 2.1964 (0.3); 2.1877 (0.7); 2.1712 (0.9); 2.1554 (0.8); 2.1340 (0.5); 2.1155 (0.3); 2.1116 (0.3); 2.1024 (0.2); 2.0883 (0.2); 2.0772 (0.3); 2.0713 (0.4); 2.0597 (0.6); 2.0477 (0.5); 2.0361 (0.4); 2.0282 (0.2); 2.0208 (0.2); 2.0102 (0.2); 1.9894 (0.1); 1.9566 (0.2); 1.2166 (3.2); 1.2057 (0.6); 1.1981 (7.3); 1.1873 (0.4); 1.1796 (3.1); 0.0078 (0.1); -0.0004 (4.4); -0.0087 (0.2) |
| **5** | 536 [M+H]⁺ | (400.1 MHz, d6-DMSO): δ=8.8206 (2.9); 8.8153 (4.7); 8.8100 (2.4); 8.6071 (2.2); 8.6023 (2.2); 8.1213 (2.7); 8.1161 (2.7); 4.2453 (0.4); 4.0358 (0.1); 3.9693 (0.2); 3.9455 (0.1); 3.8791 (0.2); 3.8606 (0.5); 3.8423 (0.7); 3.8252 (1.4); 3.8125 (0.5); 3.8067 (1.4); 3.7944 (1.4); 3.7883 (0.6); 3.7755 (2.3); 3.7698 (16.0); 3.7589 (1.0); 3.7406 (0.5); 3.7221 (0.2); 3.1717 (0.4); 3.0247 (0.1); 2.9920 (0.5); 2.9592 (0.6); 2.9451 (0.2); 2.9263 (0.3); 2.8932 (0.5); 2.8678 (0.7); 2.8579 (0.9); 2.8301 (1.0); 2.8205 (0.5); 2.7944 (0.5); 2.7860 (0.1); 2.6658 (0.2); 2.6323 (0.1); 2.5965 (0.1); 2.5852 (0.8); 2.5646 (1.1); 2.5476 (0.9); 2.5406 (0.2); 2.5374 (0.1); 2.5242 (0.3); 2.5103 (3.9); 2.5058 (8.2); 2.5012 (11.5); 2.4966 (8.4); 2.4921 (4.3); 2.4605 (0.6); 2.4281 (0.2); 2.4231 (0.2); 1.5629 (0.2); 1.5474 (2.8); 1.5320 (1.6); 1.5257 (1.6); 1.5113 (0.3); 1.2211 (3.3); 1.2027 (7.5); 1.1842 (3.2); 0.3488 (0.1); -0.0004 (3.0); - 0.0087 (0.1) |
| **6** | 529 [M+H]⁺ | (400.1 MHz, d6-DMSO): δ=9.0077 (2.4); 9.0034 (2.5); 8.8163 (2.1); 8.8116 (2.2); 8.6109 (2.2); 8.6061 (2.2); 8.3061 (2.3); 8.3022 (2.4); 3.9403 (0.8); 3.8566 (0.4); 3.8461 (0.4); 3.8336 (1.1); 3.8228 (1.0); 3.8151 (3.4); 3.7967 (3.8); 3.7781 (1.9); 3.7646 (16.0); 3.7472 (0.3); 3.1729 (0.4); 2.5112 (2.3); 2.5067 (4.9); 2.5021 (6.8); 2.4975 (4.9); 2.4930 (2.3); 2.4072 (0.4); 2.4029 (1.1); 2.3965 (0.9); 2.3813 (1.8); 2.3745 (2.0); 2.3696 (0.7); 2.3595 (1.0); 2.3531 (1.5); 2.3487 (0.6); 2.2349 (0.6); 2.2304 (1.5); 2.2239 (1.0); 2.2067 (1.9); 2.2010 (1.8); 2.1838 (0.8); 2.1775 (1.1); 1.8170 (1.2); 1.7997 (2.7); 1.7819 (1.9); 1.6621 (0.4); 1.6447 (1.1); 1.6286 (1.9); 1.6143 (2.7); 1.6087 |
| | | (2.2); 1.5981 (2.4); 1.5797 (1.0); 1.5742 (0.9); 1.5670 (1.1); 1.5599 (1.7); 1.5519 (0.7); 1.5431 (1.1); 1.5318 (0.6); 1.5269 (0.4); 1.5226 (0.5); 1.5117 (0.2); 1.5037 (0.2); 1.2258 (3.4); 1.2074 (7.7); 1.1888 (3.3); -0.0004 (1.7) |
| **7** | 487 [M+H]⁺ | (400.0 MHz, d6-DMSO): δ=8.9108 (2.9); 8.9056 (3.0); 8.8136 (2.1); 8.8089 (2.2); 8.6071 (2.3); 8.6023 (2.2); 8.1520 (3.0); 8.1468 (3.0); 3.8818 (0.1); 3.8634 (0.5); 3.8451 (0.8); 3.8281 (1.4); 3.8189 (0.2); 3.8095 (1.6); 3.8006 (0.2); 3.7902 (1.7); 3.7817 (0.4); 3.7715 (1.9); 3.7624 (16.0); 3.7550 (1.4); 3.7363 (0.6); 3.7179 (0.2); 3.3052 (2.5); 2.7120 (0.8); 2.7012 (1.0); 2.6922 (1.0); 2.6812 (0.9); 2.5115 (2.5); 2.5069 (5.3); 2.5024 (7.3); 2.4978 (5.2); 2.4933 (2.4); 1.8083 (0.8); 1.7969 (0.9); 1.7883 (0.9); 1.7771 (0.8); 1.4093 (1.0); 1.3982 (1.8); 1.3872 (0.9); 1.2344 (2.3); 1.2192 (3.5); 1.2007 (7.6); 1.1822 (3.3); 1.0958 (0.4); 1.0828 (0.9); 1.0614 (5.3); 1.0477 (0.5); 1.0357 (0.1); 1.0301 (0.1); 0.8693 (0.1); 0.8527 (0.3); 0.8352 (0.1); 0.8036 (0.1); 0.7724 (0.9); 0.7586 (0.5); 0.7497 (0.5); -0.0004 (0.9) |
| **7a** | 487 [M+H]⁺ | |
| **7b** | 487 [M+H]⁺ | |
| **8** | 534 [M+H]⁺ | (400.0 MHz, d6-DMSO): δ=8.9919 (2.6); 8.9870 (2.7); 8.8169 (2.2); 8.8122 (2.4); 8.6129 (2.3); 8.6082 (2.3); 8.3087 (2.5); 8.3039 (2.6); 5.1284 (0.2); 5.1116 (0.6); 5.0948 (1.0); 5.0780 (0.7); 5.0613 (0.2); 4.9888 (0.2); 4.9720 (0.6); 4.9552 (1.0); 4.9384 (0.7); 4.9217 (0.2); 4.1934 (0.4); 3.9335 (0.1); 3.8586 (0.1); 3.8263 (1.0); 3.8079 (3.4); 3.7894 (3.9); 3.7706 (2.4); 3.7577 (16.0); 3.7263 (0.4); 3.1718 (0.2); 2.7306 (0.2); 2.7156 (0.4); 2.7123 (0.4); 2.6999 (0.5); 2.6822 (0.6); 2.6702 (0.5); 2.6519 (0.3); 2.5332 (1.0); 2.5255 (0.8); 2.5111 (4.5); 2.5064 (7.5); 2.5021 (9.9); 2.4975 (6.9); 2.4928 (3.6); 2.4832 (1.8); 2.4673 (0.4); 2.4480 (0.4); 2.4360 (0.6); 2.4166 (1.4); 2.4076 (0.7); 2.3927 (1.2); 2.3865 (1.1); 2.3682 (0.4); 2.3622 (0.7); 2.3548 (0.9); 2.3497 (0.9); 2.3322 (1.4); 2.3251 (1.1); 2.3026 (0.8); 2.2969 (0.6); 2.2794 (0.4); 2.2670 (0.4); 2.2504 (0.4); 2.2248 (0.5); 2.2079 (0.5); 2.1947 (0.4); 2.1778 (0.4); 2.1698 (0.5); 2.1529 (0.5); 2.1397 (0.4); 2.1229 (0.4); 1.2219 (3.5); 1.2035 (7.8); 1.1849 (3.4); -0.0004 (0.6) |
| **8a** | 534 [M+H]⁺ | |
| **8b** | 534 [M+H]⁺ | |
| **9** | 501 [M+H]⁺ | (400.0 MHz, d6-DMSO): δ=8.9625 (0.3); 8.9575 (0.3); 8.8228 (2.8); 8.8175 (3.3); 8.8136 (2.6); 8.8084 (2.4); 8.6178 (0.3); 8.6125 (0.4); 8.6053 (2.2); 8.6005 (2.2); 8.3317 (0.3); 8.3269 (0.3); 7.9555 (2.7); 7.9503 (2.7); 5.3684 (0.1); 4.1723 (0.6); 3.9405 (0.2); 3.9354 (0.2); 3.8894 (0.2); 3.8709 (0.6); 3.8527 (0.9); 3.8355 (1.4); 3.8171 (1.3); 3.8047 (0.5); 3.7984 (0.5); 3.7952 (0.5); 3.7862 (0.6); 3.7763 (1.4); 3.7650 (16.0); 3.7408 (1.0); 3.7225 (0.7); 3.7042 (0.2); 3.5121 (0.3); 3.4938 (0.2); 3.1711 (0.3); 2.7418 (0.2); 2.7184 (0.2); 2.6973 (0.2); 2.6753 (0.1); 2.6709 (0.1); 2.5106 (3.9); 2.5061 (8.6); 2.5016 (12.0); 2.4970 (8.6); 2.4925 (4.1); 2.3918 (0.2); 2.3759 (0.3); 2.3647 (0.5); 2.3436 (0.5); 2.3285 (0.3); 2.3204 (0.3); 2.3128 (0.8); 2.2982 (1.0); 2.2908 (1.0); 2.2761 (0.9); 2.2395 (0.4); 2.2100 (0.9); 2.1905 (1.0); 2.1830 (0.8); 2.1706 (0.7); 2.1538 (0.3); 2.1453 (0.3); 2.1341 (0.5); 2.1208 (0.4); 2.1089 (0.4); 2.0958 (0.4); 2.0629 (0.2); 2.0019 (0.1); 1.9815 (0.3); 1.9754 (0.1); 1.9620 (0.6); 1.9572 (0.6); 1.9442 (0.6); 1.9341 (1.4); 1.9233 (1.4); 1.9141 (0.9); 1.9098 (0.9); 1.8997 (0.3); 1.8855 (0.1); 1.3967 (0.8); 1.3829 (1.1); 1.3746 (0.9); 1.3609 (0.9); 1.2977 (1.0); 1.2835 (1.7); 1.2694 (0.8); 1.2210 (0.5); 1.2076 (3.4); 1.2028 (1.5); 1.1891 (7.4); 1.1706 (3.2); -0.0004 (0.5) |
| **10** | 569 [M+H]⁺ | (400 MHz, DMSO-*d*_{6,} *ppm*) δ 9.24 (d, *J* = 2.1 Hz, 1H), 8.85 (d, *J* = 2.1 Hz, 1H), 8.66 (d, *J* = 2.1 Hz, 1H), 8.45 (d, *J* = 2.1 Hz, 1H), 3.93-3.84 (m, 2H), 3.82 (s, 3H), 3.17 (q, *J* = 12.7 Hz, 1H), 2.98-2.90 (m, 1H), 2.82-2.62 (m, 2H), 2.41-2.29 (m, 3H), 2.29-2.21 (m, 1H), 1.23 (t, *J* = 7.3 Hz, 3H) |
| **11** | 558 [M+H]⁺ | (400.1 MHz, d6-DMSO): δ=9.2094 (2.7); 8.8349 (2.5); 8.8307 (2.7); 8.6386 (2.7); 8.6344 (2.6); 8.6204 (0.1); 8.4411 (2.6); 8.4368 (2.6); 8.1322 (0.1); 7.0750 (0.1); 4.1826 (1.0); 3.9667 (0.2); 3.8770 (1.1); 3.8585 (3.6); 3.8400 (3.7); 3.8214 (1.2); 3.7910 (16.0); 3.7743 (0.7); 3.3701 (0.3); 3.3493 (1.1); 3.3285 (1.7); 3.3076 (1.2); 3.2869 (0.4); 3.0545 (0.3); 3.0438 (0.6); 3.0289 (0.6); 3.0146 (0.8); 3.0109 (0.8); 2.9987 (0.9); 2.9899 (0.9); 2.9773 (0.5); 2.9731 (0.6); 2.9589 (0.7); 2.9547 (0.7); 2.9437 (0.7); 2.9345 (0.4); 2.8545 (0.4); 2.8422 (0.6); 2.8297 (0.7); 2.8204 (0.7); 2.8079 (0.5); 2.7961 (0.7); 2.7877 (0.8); 2.7750 (0.7); 2.7657 (0.7); 2.7530 (0.5); 2.7397 (0.5); 2.7276 (0.3); 2.6938 (0.4); 2.6862 (0.4); 2.6648 (1.2); 2.6578 (0.8); 2.6434 (0.8); 2.6359 (0.8); 2.6232 (0.2); 2.6013 (1.3); 2.5812 (1.2); 2.5726 (0.8); 2.5509 (0.8); 2.5405 (0.4); 2.5191 (1.4); 2.5050 (10.7); 2.5007 (14.9); 2.4966 (11.4); 2.4757 (1.5); 2.4555 (1.4); 2.4470 (0.7); 2.4267 (0.6); 2.3277 (0.1); 1.2387 (3.7); 1.2203 (8.0); 1.2018 (3.6); -0.0004 (1.9) |
| **11a** | 558 [M+H]⁺ | |
| **11b** | 558 [M+H]⁺ | |
| **12** | 537 [M+H]⁺ | (400.0 MHz, d6-DMSO): δ=9.1092 (1.8); 9.1044 (1.9); 9.0708 (1.9); 9.0660 (2.0); 8.8244 (3.1); 8.8197 (3.4); 8.6239 (3.3); 8.6191 (3.3); 8.4389 (3.6); 8.4339 (3.7); 4.3591 (0.4); 4.1421 (0.1); 4.1234 (0.2); 4.1043 (0.5); 4.0822 (0.8); 4.0635 (0.5); 4.0419 (0.3); 4.0215 (0.6); 3.9998 (0.9); 3.9781 (0.7); 3.9567 (0.2); 3.8415 (1.3); 3.8230 (4.6); 3.8045 (4.7); 3.7860 (1.6); 3.7750 (16.0); 3.7729 (15.3); 3.1711 (0.3); 2.7625 (0.7); 2.7565 (0.5); 2.7298 (1.2); 2.7069 (1.4); 2.6781 (1.0); 2.6736 (1.0); 2.6507 (0.8); 2.6150 (1.2); 2.5959 (1.4); 2.5867 (1.3); 2.5654 (1.4); 2.5536 (0.7); 2.5453 (0.4); 2.5402 (0.5); 2.5322 (0.5); 2.5239 (0.4); 2.5194 (0.3); 2.5106 (5.2); 2.5060 (11.4); 2.5015 (16.1); 2.4969 (11.5); 2.4923 (5.4); 2.3283 (0.1); 1.6110 (0.9); 1.5895 (2.3); 1.5677 (1.1); 1.5214 (1.2); 1.4996 (2.6); 1.4777 (1.4); 1.3622 (0.1); 1.2384 (5.0); 1.2199 (11.3); 1.2014 (4.9); -0.0004 (1.7) |
| **12a** | 537 [M+H]⁺ | (400.0 MHz, d6-DMSO): δ=9.1082 (1.0); 9.1037 (1.1); 8.8243 (0.9); 8.8195 (1.0); 8.6235 (1.0); 8.6187 (0.9); 8.4377 (1.0); 8.4366 (1.0); 8.4325 (1.1); 4.0207 (0.3); 3.9990 (0.5); 3.9774 (0.4); 3.8408 (0.4); 3.8223 (1.5); 3.8038 (1.5); 3.7853 (0.5); 3.7714 (7.2); 3.3014 (16.0); 2.7616 (0.4); 2.7557 (0.3); 2.7386 (0.5); 2.7290 (0.7); 2.7123 (0.3); 2.7063 (0.6); 2.5939 (0.2); 2.5859 (0.4); 2.5781 (0.3); 2.5719 (0.3); 2.5649 (0.5); 2.5585 (0.5); 2.5527 (0.4); 2.5443 (0.2); 2.5393 (0.3); 2.5315 (0.3); 2.5230 (0.2); 2.5095 (2.6); 2.5049 (5.5); 2.5003 (7.7); 2.4957 (5.4); 2.4911 (2.5); 2.3383 (0.1); 2.3360 (0.2); 1.5213 (0.7); 1.4995 (1.5); 1.4776 (0.8); 1.2377 (1.6); 1.2193 (3.5); 1.2007 (1.5); -0.0004 (3.0) |
| **12b** | 537 [M+H]⁺ | (400.0 MHz, d6-DMSO): δ=9.0702 (1.3); 9.0656 (1.4); 8.8245 (1.2); 8.8198 (1.3); 8.6245 (1.2); 8.6197 (1.2); 8.4388 (1.3); 8.4380 (1.3); 8.4338 (1.4); 4.1227 (0.1); 4.1040 (0.3); 4.0817 (0.5); 4.0628 (0.3); 4.0405 (0.1); 3.8402 (0.5); 3.8217 (1.8); 3.8032 (1.9); 3.7846 (0.7); 3.7743 (9.2); 3.2996 (16.0); 2.7056 (0.3); 2.7010 (0.3); 2.6778 (0.7); 2.6732 (0.7); 2.6502 (0.6); 2.6146 (0.8); 2.5961 (0.9); 2.5833 (0.5); 2.5645 (0.4); 2.5098 (2.8); 2.5052 (6.1); 2.5006 (8.5); 2.4960 (6.1); 2.4915 (2.8); 1.6110 (0.7); 1.5895 (1.6); 1.5677 (0.8); 1.2373 (2.0); 1.2188 (4.5); 1.2002 (1.9); 0.0078 (0.1); -0.0004 (4.0); - 0.0087 (0.1) |
| **13** | 533 [M+H]⁺ | (400.1 MHz, d6-DMSO): δ=9.2765 (0.1); 9.2711 (0.1); 9.2048 (1.8); 9.2019 (2.0); 9.1996 (2.1); 9.1968 (1.8); 8.8352 (2.0); 8.8304 (2.2); 8.6394 (2.1); 8.6345 (2.0); 8.4872 (0.1); 8.4818 (0.1); 8.4371 (2.0); 8.4348 (2.1); 8.4319 (2.1); 8.4296 (1.9); 5.9547 (0.1); 3.8820 (0.9); 3.8635 (3.2); 3.8450 (3.4); 3.8264 (1.1); 3.8176 (0.2); 3.7935 (16.0); 3.4410 (0.1); 3.3549 (8.7); 3.1707 (0.3); 2.9547 (0.1); 2.9456 (1.0); 2.9376 (0.4); 2.9189 (0.5); 2.9103 (1.6); 2.9017 (0.3); 2.8953 (1.0); 2.8872 (0.4); 2.8686 (0.5); 2.8600 (1.6); 2.8515 (0.2); 2.7847 (0.2); 2.7761 (1.6); 2.7676 (0.5); 2.7490 (0.4); 2.7409 (1.0); 2.7296 (0.3); 2.7204 (1.6); 2.7118 (0.5); 2.6932 (0.4); 2.6851 (1.0); 2.6757 (0.2); 2.6692 (0.1); 2.5394 (0.1); 2.5226 (0.2); 2.5094 (4.9); 2.5049 (10.6); 2.5003 (14.7); 2.4957 (10.4); 2.4911 (4.8); 2.2365 (1.0); 2.2182 (2.0); 2.1991 (1.3); 2.0972 (1.0); 2.0792 (2.1); 2.0601 (1.5); 1.8989 (0.5); 1.8846 (0.9); 1.8796 (1.6); 1.8617 (2.2); 1.8468 (0.7); 1.8413 (1.1); 1.8253 (0.3); 1.8234 (0.3); 1.2353 (3.4); 1.2278 (0.5); 1.2246 (0.5); 1.2169 (7.7); 1.2096 (0.7); 1.1983 (3.3); -0.0004 (0.4) |
| **14** | 551 [M+H]⁺ | (400.1 MHz, d6-DMSO): δ=11.9094 (0.1); 9.1166 (0.3); 9.0952 (2.4); 9.0903 (2.5); 8.8289 (2.1); 8.8243 (2.2); 8.6237 (2.2); 8.6189 (2.2); 8.3980 (2.4); 8.3931 (2.4); 8.3012 (0.1); 4.2983 (0.2); 3.9647 (0.5); 3.8799 (1.9); 3.8619 (2.3); 3.8447 (3.3); 3.8267 (3.7); 3.8088 (2.7); 3.7985 (1.5); 3.7891 (16.0); 3.7774 (1.0); 3.7741 (1.1); 3.7551 (0.5); 3.7496 (0.6); 3.6625 (0.5); 3.6408 (1.0); 3.6184 (0.6); 3.1716 (0.5); 3.1224 (0.2); 3.1001 (0.2); 3.0881 (0.3); 3.0811 (0.2); 3.0659 (0.3); 3.0574 (0.4); 3.0467 (0.3); 3.0345 (0.3); 3.0244 (0.3); 3.0153 (0.3); 2.9924 (0.4); 2.9730 (0.3); 2.9686 (0.4); 2.9581 (0.4); 2.9501 (0.4); 2.9394 (0.5); 2.9359 (0.5); 2.9243 (0.3); 2.9169 (0.4); 2.9077 (0.5); 2.9016 (0.3); 2.8959 (0.3); 2.8829 (0.2); 2.5102 (4.1); 2.5057 (8.6); 2.5011 (11.9); 2.4966 (8.5); 2.4921 (4.1); 2.4170 (0.2); 2.4067 (0.3); 2.3959 (0.4); 2.3860 (0.5); 2.3758 (0.5); 2.3658 (0.5); 2.3549 (0.4); 2.3448 (0.3); 2.2236 (0.2); 2.2006 (0.5); 2.1761 (0.5); 2.1460 (0.4); 2.1347 (0.4); 2.1247 (0.5); 2.1145 (0.5); 2.1056 (0.4); 2.0946 (0.4); 2.0849 (0.2); 1.9336 (0.1); 1.9155 (0.1); 1.8324 (0.2); 1.8115 (0.3); 1.8050 (0.2); 1.7907 (0.5); 1.7849 (0.5); 1.7636 (0.7); 1.7432 (0.5); 1.7223 (0.2); 1.7102 (0.2); 1.6977 (0.3); 1.6869 (0.4); 1.6749 (0.6); 1.6630 (0.5); 1.6519 (0.4); 1.6478 (0.4); 1.6364 (0.3); 1.6248 (0.2); 1.6128 (0.1); 1.5675 (0.3); 1.5462 (0.5); 1.5249 (0.4); 1.5037 (0.2); 1.4269 (0.1); 1.3858 (0.2); 1.2342 (3.2); 1.2157 (7.1); 1.1972 (3.2); 1.1854 (0.2); 1.1798 (0.2); 1.1618 (0.2); 0.8571 (0.3); 0.8430 (0.3); 0.8380 (0.4); 0.8194 (0.2); -0.0004 (0.2) |
| **14a** | 551 [M+H]⁺ | |
| **14b** | 551 [M+H]⁺ | |
| **15** | 551 [M+H]⁺ | (400.1 MHz, d6-DMSO): δ=9.0162 (2.3); 9.0152 (2.2); 9.0111 (2.4); 8.8172 (1.9); 8.8124 (2.0); 8.6116 (2.1); 8.6067 (2.0); 8.3399 (2.2); 8.3386 (2.2); 8.3347 (2.3); 8.3336 (2.1); 4.0733 (0.4); 4.0676 (0.4); 3.9359 (0.2); 3.8430 (0.2); 3.8259 (1.0); 3.8076 (3.2); 3.7985 (1.2); 3.7891 (3.3); 3.7707 (1.3); 3.7602 (16.0); 3.1717 (0.2); 2.8540 (0.6); 2.8476 (0.7); 2.8225 (1.2); 2.8187 (1.3); 2.8160 (1.4); 2.7907 (0.6); 2.7876 (0.7); 2.7846 (0.7); 2.6594 (0.7); 2.6531 (0.7); 2.6242 (2.1); 2.6005 (1.4); 2.5946 (2.2); 2.5896 (1.3); 2.5803 (1.0); 2.5737 (1.3); 2.5691 (0.5); 2.5235 (0.1); 2.5100 (3.8); 2.5057 (7.2); 2.5011 (10.1); 2.4965 (6.7); 2.4918 (3.3); 2.4844 (2.1); 2.4780 (1.7); 2.4608 (0.7); 2.4542 (0.9); 1.2258 (3.3); 1.2073 (7.7); 1.1888 (3.2) |
| **16** | 552 [M+H]⁺ | (400.0 MHz, d6-DMSO): δ=9.0558 (2.3); 9.0516 (2.5); 8.7918 (6.6); 8.3866 (2.3); 8.3854 (2.3); 8.3816 (2.4); 4.3983 (0.4); 4.1101 (0.1); 4.0415 (0.2); 3.9332 (16.0); 3.8606 (0.2); 3.8381 (0.6); 3.8160 (1.0); 3.7935 (0.6); 3.7705 (1.1); 3.7519 (3.3); 3.7333 (3.3); 3.7149 (1.0); 3.1716 (0.4); 2.9456 (0.2); 2.8591 (0.6); 2.8528 (0.8); 2.8276 (1.3); 2.8213 (1.5); 2.7960 (0.7); 2.7897 (0.8); 2.6644 (0.8); 2.6583 (0.7); 2.6298 (2.2); 2.6062 (1.5); 2.6003 (2.4); 2.5954 (1.4); 2.5861 (1.0); 2.5795 (1.4); 2.5750 (0.6); 2.5308 (0.6); 2.5259 (1.6); 2.5194 (1.1); 2.5113 (3.0); 2.5067 (6.9); 2.5021 (10.7); 2.4975 (7.4); 2.4933 (3.6); 2.4784 (0.7); 2.4720 (0.9); 2.3342 (0.1); 2.3287 (0.1); 1.9578 (0.2); 1.2281 (3.5); 1.2096 (8.0); 1.1910 (3.4); 1.1654 (0.1); 1.1517 (0.1); - 0.0004 (0.2) |
| **17** | 538 [M+H]⁺ | (400.1 MHz, d6-DMSO): δ=9.1459 (1.8); 9.1413 (1.9); 9.1077 (1.9); 9.1031 (2.0); 8.7964 (8.0); 8.4794 (3.0); 8.4771 (3.3); 8.4747 (2.9); 7.9780 (0.1); 7.9717 (0.2); 7.9682 (0.2); 7.9594 (0.2); 4.1499 (0.1); 4.1424 (0.2); 4.1338 (0.2); 4.1212 (0.6); 4.1010 (0.8); 4.0804 (0.5); 4.0659 (0.3); 4.0602 (0.3); 4.0389 (0.6); 4.0172 (0.9); 3.9955 (0.6); 3.9738 (0.2); 3.9466 (16.0); 3.9444 (14.9); 3.7816 (1.5); 3.7631 (5.1); 3.7446 (5.1); 3.7261 (1.6); 3.5784 (4.8); 3.4801 (0.3); 3.4576 (0.2); 3.1713 (0.8); 2.9452 (0.3); 2.8798 (0.2); 2.8614 (0.2); 2.7855 (0.2); 2.7677 (0.7); 2.7618 (0.5); 2.7400 (0.9); 2.7351 (1.2); 2.7122 (1.3); 2.6813 (1.0); 2.6768 (1.1); 2.6705 (0.7); 2.6541 (0.9); 2.6377 (1.3); 2.6189 (1.4); 2.6048 (1.2); 2.5962 (0.7); 2.5841 (1.0); 2.5770 (0.8); 2.5711 (0.7); 2.5628 (0.4); 2.5573 (0.4); 2.5499 (0.5); 2.5408 (0.3); 2.5235 (0.2); 2.5102 (5.4); 2.5056 (11.6); 2.5011 (16.2); 2.4965 (11.5); 2.4919 (5.4); 2.4603 (0.1); 2.4355 (0.2); 2.4115 (0.2); 2.4031 (0.1); 2.3960 (0.2); 2.3768 (0.2); 2.3334 (0.2); 2.3281 (0.1); 1.9569 (0.2); 1.6164 (0.9); 1.5949 (2.3); 1.5731 (1.1); 1.5249 (1.2); 1.5031 (2.5); 1.4812 (1.3); 1.4181 (0.1); 1.4137 (0.1); 1.3915 (0.2); 1.3827 (0.1); 1.3693 (0.1); 1.2401 (5.4); 1.2216 (12.4); 1.2031 (5.3); 1.1898 (0.7); 1.1766 (0.1); 1.1713 (0.3); 1.1514 (0.2); 1.1479 (0.3); 1.1313 (0.3); 1.0635 (0.2); 1.0479 (0.2); 0.8882 (0.2) |
| **17a** | 538 [M+H]⁺ | (400.1 MHz, d6-DMSO): δ=9.1455 (0.6); 9.1406 (0.6); 8.7957 (1.6); 8.4803 (0.6); 8.4793 (0.6); 8.4753 (0.6); 4.0380 (0.2); 4.0163 (0.3); 3.9947 (0.2); 3.9429 (3.8); 3.7808 (0.2); 3.7623 (0.8); 3.7438 (0.8); 3.7253 (0.2); 3.2966 (16.0); 2.7669 (0.2); 2.7611 (0.1); 2.7394 (0.3); 2.7343 (0.4); 2.7176 (0.2); 2.7116 (0.3); 2.6115 (0.1); 2.6035 (0.2); 2.5957 (0.2); 2.5899 (0.2); 2.5825 (0.3); 2.5761 (0.2); 2.5702 (0.2); 2.5619 (0.1); 2.5566 (0.1); 2.5491 (0.2); 2.5092 (1.6); 2.5047 (3.5); 2.5001 (4.8); 2.4955 (3.4); 2.4910 (1.6); 1.5247 (0.4); 1.5029 (0.8); 1.4810 (0.4); 1.2393 (0.9); 1.2208 (1.9); 1.2022 (0.8); - 0.0004 (0.3) |
| **17b** | 538 [M+H]⁺ | (400.1 MHz, d6-DMSO): δ=9.1069 (0.6); 9.1024 (0.6); 8.7971 (1.5); 8.4766 (0.6); 8.4725 (0.6); 4.1222 (0.2); 4.1001 (0.2); 4.0800 (0.1); 3.9455 (3.7); 3.7801 (0.2); 3.7617 (0.8); 3.7431 (0.8); 3.7247 (0.2); 3.2966 (16.0); 2.7086 (0.1); 2.6761 (0.3); 2.6697 (0.2); 2.6534 (0.3); 2.6367 (0.4); 2.6179 (0.4); 2.6057 (0.2); 2.5868 (0.2); 2.5092 (1.7); 2.5047 (3.6); 2.5001 (4.9); 2.4956 (3.6); 2.4911 (1.7); 1.6161 (0.3); 1.5946 (0.7); 1.5728 (0.3); 1.2389 (0.8); 1.2205 (1.9); 1.2019 (0.8); -0.0004 (0.3) |
| **18** | 555 [M+H]⁺ | (400 MHz, DMSO-*d*_{6,} *ppm*) δ 9.24 (d, *J* = 2.1 Hz, 1H), 8.85 (d, *J* = 2.1 Hz, 1H), 8.67 (d, *J* = 2.0 Hz, 1H), 8.53 (d, *J* = 2.1 Hz, 1H), 3.87 (q, *J* = 7.4 Hz, 2H), 3.82 (s, 3H), 3.16-2.95 (m, 4H), 1.75 (t, *J* = 8.6 Hz, 2H), 1.24 (t, *J* = 7.4 Hz, 3H) |
| **19** | 555 [M+H]⁺ | (400 MHz, DMSO-*d*₆*_{,} ppm*) δ 9.33 (d, *J* = 2.2, 1H), 8.85 (d, *J* = 2.0 Hz, 1H), 8.67 (d, *J =* 2.1 Hz, 1H), 8.55 (d, *J* = 2.1 Hz, 1H), 3.87 (q, *J* = 7.4 Hz, 2H), 3.81 (s, 3H), 3.14 -2.95 (m, 4H), 1.71 (t, *J* = 8.8 Hz, 2H), 1.24 (t, *J* = 7.4 Hz, 3H) |
| **20** | 558 [M+H]⁺ | (400 MHz, DMSO-*d*_{6,} *ppm*) δ 9.02 (d, *J* = 2.0 Hz, 1H), 8.83 (d, *J* = 2.0 Hz, 1H), 8.63 (d, *J* = 2.1 Hz, 1H), 8.36 (d, *J* = 2.1 Hz, 1H), 4.31 (s, 1H), 4.09 (s, 1H), 4.03 (s, 1H), 3.88-3.65 (m, 7H), 2.76-2.61 (m, 2H), 2.58-2.52 (m, 1H), 1.77 (d, *J* = 8.4 Hz, 3H), 1.21 (t, *J* = 7.4 Hz, 3H) |
| **21** | 594 [M+H]⁺ | (400 MHz, DMSO-*d₆,ppm*) δ 9.03 (d, *J* = 2.0 Hz, 1H), 8.83 (d, *J* = 2.0 Hz, 1H), 8.63 (d, *J* = 2.0 Hz, 1H), 8.36 (d, *J* = 2.1 Hz, 1H), 4.07 (s, 2H), 3.90-3.65 (m, 8H), 3.01 (s, 3H), 2.77 -2.64 (m, 2H), 2.56-2.52 (m, 2H), 1.21 (t, *J* = 7.4 Hz, 3H) |
| **22** | 516 [M+H]⁺ | (400 MHz, DMSO-*d₆,ppm*) δ 9.00 (d, *J* = 2.1 Hz, 1H), 8.83 (d, *J* = 2.0 Hz, 1H), 8.63 (d, *J* = 2.1 Hz, 1H), 8.32 (s, 1H), 3.84-3.76 (m, 7H), 3.64 (br, 3H), 2.74-2.59 (m, 2H), 2.38 (br, 2H), 1.21 (t, *J* = 7.4 Hz, 4H) |
| **23** | 488 [M+H]⁺ | |
| **23a** | 488 [M+H]⁺ | (400.0 MHz, d6-DMSO): δ=8.9426 (0.3); 8.9375 (0.3); 8.7838 (0.8); 8.1824 (0.3); 8.1772 (0.3); 3.9343 (1.8); 3.7628 (0.2); 3.7442 (0.2); 3.7408 (0.2); 3.7223 (0.2); 3.5675 (1.1); 3.2989 (16.0); 2.7205 (0.1); 2.7115 (0.1); 2.5089 (1.6); 2.5044 (3.5); 2.4998 (4.9); 2.4952 (3.4); 2.4906 (1.6); 1.4412 (0.1); 1.4301 (0.2); 1.2211 (0.4); 1.2027 (0.9); 1.1841 (0.4); 1.0679 (0.6); -0.0004 (0.2) |
| **23b** | 488 [M+H]⁺ | (400.0 MHz, d6-DMSO): δ=8.9428 (0.5); 8.9376 (0.5); 8.7839 (1.2); 8.1827 (0.5); 8.1775 (0.5); 3.9607 (0.1); 3.9346 (2.8); 3.7803 (0.1); 3.7631 (0.3); 3.7445 (0.3); 3.7411 (0.3); 3.7226 (0.3); 3.7053 (0.1); 3.5676 (2.3); 3.2990 (16.0); 2.7315 (0.1); 2.7207 (0.2); 2.7117 (0.2); 2.7008 (0.2); 2.5091 (1.7); 2.5046 (3.7); 2.5000 (5.2); 2.4954 (3.6); 2.4908 (1.7); 1.8256 (0.1); 1.8143 (0.2); 1.8057 (0.1); 1.7944 (0.1); 1.4413 (0.2); 1.4302 (0.3); 1.4192 (0.1); 1.2214 (0.6); 1.2029 (1.4); 1.1844 (0.6); 1.0916 (0.1); 1.0867 (0.1); 1.0680 (1.0); 0.7832 (0.2); -0.0004 (0.2) |
| **24** | 551 [M+H]⁺ | (400 MHz, Chloroform-d, *ppm)* δ 8.85 (d, *J* = 2.1 Hz, 1H), 8.76 (d, *J* = 1.9 Hz, 1H), 8.34 (s, 1H), 8.14 (d, *J* = 2.1 Hz, 1H), 4.43 (d, *J* = 13.2 Hz, 1H), 4.35 (d, *J* = 13.1 Hz, 1H), 4.21 (d, *J* = 13.1 Hz, 1H), 3.96-3.84 (m, 4H), 3.75 (d, *J* = 12.9 Hz, 1H), 2.65 (dd, *J* = 9.0, 6.5 Hz, 1H), 1.84 (dd, *J* = 9.1, 6.6 Hz, 1H), 1.66 (t, *J* = 6.5 Hz, 1H), 1.40 (t, *J* = 7.4 Hz, 3H) |
| **24a** | 551 [M+H]⁺ | |
| **24b** | 551 [M+H]⁺ | |
| **25** | 519 [M+H]⁺ | (400 MHz, Chloroform-*d*, *ppm*) δ 8.73(d, *J* = 2.0 Hz, 1H), 8.70 (d, *J* = 2.1 Hz, 1H), 8.29 (d, *J* = 2.0 Hz, 1H), 8.02 (d, *J* = 2.2 Hz, 1H), 3.91 (s, 3H), 3.98-3.76 (m, 2H), 3.57 (d, *J* = 8.9 Hz, 1H), 3.45 (d, *J* = 9.0 Hz, 1H), 3.31 (d, *J* = 9.0 Hz, 1H), 3.10 (d, *J* = 9.0 Hz, 1H), 2.23 (dd, *J* = 9.2, 6.4 Hz, 1H), 1.58-1.51 (m, 1H), 1.37 (q, *J* = 7.0, 6.6 Hz, 4H) |
| **26** | 622 [M+Na]⁺ | (400.1 MHz, d6-DMSO): δ=8.7648 (0.2); 8.7600 (0.2); 8.5334 (0.2); 8.5286 (0.2); 8.2261 (0.2); 8.2194 (0.3); 7.2721 (0.2); 7.2654 (0.2); 6.5688 (0.5); 4.1910 (0.5); 4.1524 (0.4); 3.7966 (0.3); 3.7780 (0.3); 3.7192 (1.4); 3.2964 (16.0); 2.7379 (0.2); 2.7015 (0.2); 2.5087 (0.9); 2.5042 (1.9); 2.4996 (2.6); 2.4950 (1.9); 2.4905 (1.0); 2.4534 (0.1); 1.2169 (0.3); 1.1984 (0.7); 1.1799 (0.3) |
| **27** | 546 [M+H]⁺ | (400.1 MHz, d6-DMSO): δ=8.7626 (0.2); 8.7579 (0.2); 8.5314 (0.2); 8.5267 (0.2); 8.1985 (0.3); 8.1918 (0.3); 7.2515 (0.3); 7.2448 (0.3); 4.9451 (0.5); 4.1553 (0.5); 4.0940 (0.5); 3.7970 (0.3); 3.7785 (0.3); 3.7197 (1.4); 3.2975 (16.0); 2.5087 (0.8); 2.5042 (1.7); 2.4996 (2.3); 2.4951 (1.6); 2.4907 (0.8); 2.2748 (0.7); 1.2236 (0.9); 1.2156 (0.4); 1.1970 (0.7); 1.1785 (0.3) |
| **28** | 552 [M+H]⁺ | (400.1 MHz, d6-DMSO): δ=8.7680 (0.3); 8.7633 (0.3); 8.5377 (0.3); 8.5328 (0.3); 8.2535 (0.4); 8.2468 (0.4); 7.2979 (0.4); 7.2911 (0.4); 4.2497 (2.0); 3.8229 (0.1); 3.8044 (0.4); 3.7859 (0.4); 3.7674 (0.1); 3.7247 (2.2); 3.2955 (16.0); 2.9626 (0.4); 2.9315 (0.8); 2.9004 (0.4); 2.5090 (0.9); 2.5044 (2.0); 2.4998 (2.7); 2.4952 (1.9); 2.4907 (0.9); 1.9868 (0.3); 1.2207 (0.5); 1.2023 (1.1); 1.1927 (0.1); 1.1837 (0.5); 1.1749 (0.2) |
| **29** | 552 [M+H]⁺ | (400.1 MHz, d6-DMSO): δ=8.7711 (0.1); 8.7664 (0.1); 8.5439 (0.1); 8.5391 (0.1); 8.3109 (0.1); 8.3042 (0.1); 7.3714 (0.1); 7.3647 (0.1); 4.3980 (0.1); 4.3753 (0.1); 4.1108 (0.1); 4.0885 (0.1); 4.0392 (0.0); 4.0214 (0.0); 3.8198 (0.0); 3.8013 (0.1); 3.7827 (0.1); 3.7642 (0.0); 3.7265 (0.4); 3.3440 (0.0); 3.3049 (16.0); 3.2680 (0.0); 2.5883 (0.0); 2.5766 (0.0); 2.5665 (0.0); 2.5564 (0.0); 2.5442 (0.0); 2.5341 (0.0); 2.5228 (0.0); 2.5180 (0.0); 2.5093 (0.3); 2.5047 (0.7); 2.5001 (1.0); 2.4955 (0.7); 2.4910 (0.3); 2.1431 (0.0); 2.1368 (0.0); 2.1213 (0.0); 2.1007 (0.0); 1.9866 (0.0); 1.3981 (0.1); 1.2354 (0.0); 1.2261 (0.1); 1.2076 (0.2); 1.1891 (0.1); 1.1749 (0.0); 1.1571 (0.0); -0.0004 (0.1) |
| **30** | 552 [M+H]⁺ | (400.0 MHz, d6-DMSO): δ=8.7697 (0.4); 8.7648 (0.4); 8.5447 (0.4); 8.5398 (0.4); 8.4137 (0.5); 8.4067 (0.5); 7.4106 (0.5); 7.4036 (0.5); 4.0389 (0.2); 4.0211 (0.2); 3.8404 (0.2); 3.8218 (0.6); 3.8032 (0.6); 3.7848 (0.2); 3.7397 (3.3); 3.7200 (0.1); 3.7143 (0.1); 3.6999 (0.1); 3.6879 (0.2); 3.6657 (0.4); 3.6505 (0.1); 3.6414 (0.2); 3.6285 (0.2); 3.2995 (16.0); 2.5092 (1.6); 2.5046 (3.6); 2.5000 (5.1); 2.4954 (3.6); 2.4908 (1.7); 2.2990 (0.1); 2.2822 (0.1); 1.9872 (0.8); 1.7578 (0.1); 1.7443 (0.1); 1.7349 (0.2); 1.7236 (0.1); 1.7178 (0.1); 1.7034 (0.1); 1.3978 (0.5); 1.2344 (0.7); 1.2159 (1.6); 1.1974 (0.7); 1.1926 (0.3); 1.1748 (0.5); 1.1570 (0.2); -0.0004 (0.2) |
| **31** | 538 [M+H]⁺ | (400.0 MHz, d6-DMSO): δ=8.7766 (0.6); 8.7718 (0.7); 8.5535 (0.7); 8.5486 (0.7); 8.3459 (0.9); 8.3391 (1.0); 7.4081 (0.9); 7.4014 (1.0); 4.3512 (0.5); 4.3300 (1.2); 4.2956 (0.7); 4.2744 (0.3); 3.8276 (0.3); 3.8090 (1.0); 3.7905 (1.0); 3.7720 (0.3); 3.7354 (5.1); 3.2992 (16.0); 2.5092 (2.4); 2.5046 (5.2); 2.5000 (7.2); 2.4954 (5.1); 2.4909 (2.4); 1.9873 (0.4); 1.8720 (0.3); 1.8498 (0.7); 1.8272 (0.3); 1.3978 (0.1); 1.2317 (1.1); 1.2132 (2.5); 1.1946 (1.0); 1.1748 (0.2); 1.1570 (0.1); -0.0004 (2.6) |
| **32** | 502 [M+H]⁺ | (400.1 MHz, d6-DMSO): δ=8.7757 (1.0); 8.7709 (1.0); 8.5475 (1.1); 8.5426 (1.0); 8.2604 (1.5); 8.2536 (1.5); 7.3033 (1.5); 7.2965 (1.5); 4.2264 (7.6); 3.8403 (0.4); 3.8218 (1.6); 3.8033 (1.6); 3.7848 (0.5); 3.7438 (8.2); 3.3040 (16.0); 2.5166 (3.0); 2.5121 (6.4); 2.5075 (9.0); 2.5028 (6.4); 2.4983 (3.0); 2.0776 (1.8); 1.3493 (0.4); 1.2338 (1.7); 1.2153 (3.9); 1.1968 (1.6); 0.7491 (6.9) |
| **33** | 518 [M+H]⁺ | (400.1 MHz, d6-DMSO): δ=8.7664 (0.4); 8.7617 (0.4); 8.5351 (0.4); 8.5302 (0.4); 8.2396 (0.6); 8.2328 (0.6); 7.2897 (0.6); 7.2829 (0.6); 4.7654 (2.9); 4.5638 (0.1); 4.3150 (3.0); 4.0391 (0.3); 4.0213 (0.3); 3.8209 (0.2); 3.8024 (0.6); 3.7839 (0.7); 3.7654 (0.2); 3.7188 (3.3); 3.2919 (16.0); 2.5084 (2.4); 2.5038 (5.0); 2.4993 (7.0); 2.4947 (5.0); 2.4901 (2.3); 2.2994 (0.1); 1.9866 (1.2); 1.2363 (0.2); 1.2180 (0.7); 1.1995 (1.6); 1.1925 (0.4); 1.1810 (0.7); 1.1747 (0.7); 1.1569 (0.3); 0.0078 (0.2); -0.0004 (5.4); -0.0087 (0.2) |
| **34** | 516 [M+H]⁺ | (400.0 MHz, d6-DMSO): δ=8.7645 (0.4); 8.7598 (0.4); 8.5350 (0.4); 8.5303 (0.4); 8.2027 (0.5); 8.1960 (0.5); 7.2485 (0.5); 7.2417 (0.5); 4.1055 (2.5); 3.8217 (0.1); 3.8032 (0.5); 3.7847 (0.5); 3.7662 (0.2); 3.7210 (2.6); 3.3151 (6.6); 3.3058 (10.6); 3.3017 (16.0); 2.5092 (2.2); 2.5047 (4.8); 2.5001 (6.6); 2.4955 (4.7); 2.4910 (2.2); 2.2587 (0.5); 2.2399 (0.9); 2.2208 (0.6); 1.8626 (0.2); 1.8444 (0.3); 1.8250 (0.2); 1.3980 (0.5); 1.2166 (0.6); 1.1981 (1.3); 1.1796 (0.5) |
| **35** | 546 [M+H]⁺ | (400.0 MHz, d6-DMSO): δ=8.7666 (0.2); 8.7619 (0.2); 8.5372 (0.2); 8.5324 (0.2); 8.2317 (0.3); 8.2249 (0.3); 7.2808 (0.3); 7.2740 (0.3); 4.0387 (0.1); 4.0209 (0.1); 3.9112 (1.5); 3.8127 (0.3); 3.7941 (0.3); 3.7756 (0.1); 3.7303 (1.7); 3.5914 (0.3); 3.5795 (0.4); 3.5670 (0.3); 3.3162 (7.0); 3.3085 (10.6); 3.3013 (16.0); 2.5091 (2.2); 2.5046 (4.7); 2.5000 (6.5); 2.4955 (4.6); 2.4909 (2.2); 1.9870 (0.6); 1.8235 (0.3); 1.8109 (0.4); 1.7980 (0.3); 1.2256 (0.4); 1.2072 (0.8); 1.1922 (0.2); 1.1887 (0.4); 1.1746 (0.3); 1.1568 (0.2); -0.0004 (0.2) |
| **36** | 532 [M+H]+ | (400.1 MHz, d6-DMSO): δ=8.7685 (0.9); 8.7638 (1.0); 8.5390 (1.0); 8.5343 (1.0); 8.2544 (1.3); 8.2476 (1.3); 7.3042 (1.3); 7.2975 (1.3); 4.1463 (0.1); 4.1266 (6.1); 4.1071 (0.2); 3.8680 (4.1); 3.8276 (0.4); 3.8091 (1.4); 3.7906 (1.5); 3.7828 (1.1); 3.7720 (0.6); 3.7653 (2.2); 3.7479 (1.1); 3.7291 (7.0); 3.2959 (16.0); 2.5087 (3.2); 2.5042 (7.0); 2.4996 (9.7); 2.4951 (7.0); 2.4906 (3.4); 2.2339 (0.9); 2.2165 (1.8); 2.1990 (0.9); 1.2336 (0.2); 1.2248 (1.5); 1.2063 (3.4); 1.1878 (1.4); -0.0005 (0.4) |
| **37** | 532 [M+H]⁺ | (400.1 MHz, d6-DMSO): δ=8.7624 (2.1); 8.7579 (2.3); 8.5324 (2.2); 8.5279 (2.2); 8.3679 (2.0); 8.3610 (2.2); 8.3304 (0.2); 8.3235 (0.2); 7.3740 (2.1); 7.3670 (2.3); 7.3598 (0.4); 7.3364 (0.2); 7.3295 (0.2); 4.6587 (3.0); 4.6436 (4.1); 4.5706 (4.1); 4.5555 (3.1); 4.5340 (0.2); 4.4796 (0.4); 4.4716 (0.4); 3.9714 (0.1); 3.9239 (0.2); 3.9097 (0.3); 3.8948 (0.3); 3.8362 (1.4); 3.8177 (3.5); 3.7992 (3.8); 3.7807 (2.1); 3.7733 (1.7); 3.7582 (6.5); 3.7344 (16.0); 3.7169 (2.4); 3.6079 (5.1); 3.5362 (1.0); 3.5164 (1.7); 3.4991 (2.7); 3.4903 (1.4); 3.4821 (1.6); 3.4558 (0.8); 3.4473 (0.8); 3.4353 (0.7); 3.4204 (0.3); 3.3077 (0.5); 3.1697 (0.1); 2.6733 (0.1); 2.6687 (0.2); 2.6640 (0.1); 2.5387 (0.2); 2.5360 (0.1); 2.5221 (0.3); 2.5173 (0.4); 2.5088 (8.7); 2.5042 (18.7); 2.4996 (26.0); 2.4950 (18.5); 2.4905 (8.7); 2.3618 (1.3); 2.3447 (2.7); 2.3271 (1.4); 2.0691 (1.4); 2.0293 (0.2); 2.0125 (0.2); 1.9358 (0.1); 1.9180 (0.2); 1.9000 (0.1); 1.2344 (3.6); 1.2160 (7.6); 1.1975 (3.3) |
| **38** | 532 [M+H]⁺ | (400.1 MHz, d6-DMSO):δ=8.7775 (1.8); 8.7727 (2.0); 8.7078 (1.8); 8.7008 (1.9); 8.5537 (2.0); 8.5488 (1.9); 8.1684 (1.8); 8.1613 (1.7); 5.1714 (2.7); 5.1519 (2.9); 4.7139 (3.0); 4.6943 (2.8); 3.9631 (0.2); 3.9332 (0.1); 3.9101 (0.2); 3.8748 (1.0); 3.8563 (3.1); 3.8378 (3.3); 3.8193 (1.5); 3.7877 (16.0); 3.7683 (2.6); 3.7447 (3.4); 3.6079 (0.3); 3.5578 (1.3); 3.5414 (2.6); 3.5248 (1.3); 3.4898 (0.1); 2.6686 (0.1); 2.5387 (0.1); 2.5360 (0.1); 2.5221 (0.2); 2.5174 (0.3); 2.5088 (6.1); 2.5042 (13.1); 2.4996 (18.3); 2.4950 (12.8); 2.4905 (5.9); 2.4291 (1.3); 2.4118 (2.8); 2.3945 (1.5); 2.3265 (0.1); 2.0695 (3.2); 1.9262 (0.3); 1.9095 (1.2); 1.8926 (1.8); 1.8757 (1.1); 1.8587 (0.3); 1.2508 (2.9); 1.2324 (7.0); 1.2138 (2.8); 1.2034 (0.2) |
| **39** | 555 [M+H]⁺ | (400 MHz, Chloroform-d, *ppm)* δ 9.09 (s, 1H), 8.31 (d, *J* = 12.7 Hz, 2H), 8.08 (s, 1H), 7.50 (s, 1H), 7.28 (s, 4H), 5.18 (t, *J* = 13.9 Hz, 2H), 4.28 (s, 2H), 4.16 (s, 2H), 3.79 (q, *J* = 7.4 Hz, 2H), 2.93 (d, *J* = 12.7 Hz, 2H), 2.45 (d, *J* = 12.7 Hz, 2H), 1.57(s, 3H), 1.39 (t, *J* = 7.3 Hz, 3H) |
| **40** | 586 [M+H]⁺ | (400.0 MHz, d6-DMSO):δ=8.7801 (0.4); 8.7753 (0.4); 8.5530 (0.4); 8.5482 (0.4); 8.3239 (0.5); 8.3171 (0.6); 7.3796 (0.5); 7.3729 (0.6); 5.3953 (0.2); 5.3758 (0.2); 4.9348 (0.2); 4.9176 (0.4); 4.8745 (0.4); 4.8573 (0.2); 4.5496 (0.2); 4.5275 (0.3); 4.4775 (0.3); 4.4554 (0.2); 4.3418 (0.3); 4.3274 (0.4); 4.0646 (0.2); 4.0468 (0.5); 4.0290 (0.5); 4.0112 (0.2); 3.8269 (0.1); 3.8085 (0.5); 3.7899 (0.5); 3.7714 (0.2); 3.7292 (3.0); 3.3086 (16.0); 2.5171 (1.4); 2.5125 (3.0); 2.5080 (4.2); 2.5034 (3.0); 2.4988 (1.4); 1.9951 (2.2); 1.4057 (0.2); 1.2301 (0.6); 1.2116 (1.4); 1.2004 (0.7); 1.1931 (0.6); 1.1826 (1.2); 1.1648 (0.6) |
| | 630 [M+HCOO]⁻ | |
| **41** | 546 [M+H]⁺ | (400.0 MHz, d6-DMSO): δ=8.7760 (0.3); 8.7712 (0.3); 8.5486 (0.3); 8.5438 (0.3); 8.2723 (0.2); 8.2659 (0.2); 7.3013 (0.2); 7.2948 (0.3); 4.0468 (0.1); 4.0290 (0.1); 4.0193 (0.2); 4.0111 (0.1); 4.0011 (0.3); 3.9823 (0.2); 3.9494 (0.1); 3.9381 (0.2); 3.9206 (0.2); 3.9097 (0.2); 3.8719 (0.1); 3.8534 (0.4); 3.8349 (0.4); 3.8164 (0.1); 3.7543 (2.3); 3.4174 (0.1); 3.3883 (0.3); 3.3607 (0.1); 3.3081 (16.0); 2.5171 (1.5); 2.5125 (3.3); 2.5079 (4.6); 2.5033 (3.3); 2.4988 (1.5); 2.3928 (0.2); 2.3744 (0.3); 2.3558 (0.2); 2.2873 (0.2); 2.2754 (0.2); 1.9952 (0.5); 1.8726 (0.3); 1.8414 (0.2); 1.4059 (0.1); 1.2434 (0.2); 1.2380 (0.5); 1.2195 (1.1); 1.2007 (0.6); 1.1826 (0.3); 1.1649 (0.2) |
| **42** | 552 [M+H]⁺ | (400.0 MHz, d6-DMSO): δ=8.7778 (0.4); 8.7730 (0.4); 8.5552 (0.4); 8.5504 (0.4); 8.3056 (0.4); 8.2988 (0.5); 7.3875 (0.4); 7.3807 (0.4); 4.0333 (0.2); 4.0153 (0.4); 3.9972 (0.2); 3.8540 (0.2); 3.8355 (0.5); 3.8170 (0.5); 3.7985 (0.2); 3.7573 (2.7); 3.5640 (0.2); 3.5298 (0.2); 3.2982 (16.0); 2.9741 (0.1); 2.9662 (0.1); 2.9574 (0.1); 2.9511 (0.1); 2.7107 (0.2); 2.6929 (0.4); 2.6744 (0.2); 2.5086 (2.9); 2.5041 (6.2); 2.4995 (8.7); 2.4950 (6.2); 2.4904 (2.9); 1.9870 (0.1); 1.2353 (0.6); 1.2169 (1.3); 1.1983 (0.6); -0.0004 (0.6) |
| **43** | 555 [M+H]⁺ | (400 MHz, Chloroform-d, *ppm)* δ 9.45 (s, 1H), 8.79 (s, 1H), 8.10 (s, 1H), 7.89 (s, 1H), 7.44(s, 1H), 4.42 (s, 2H), 4.20 (s, 2H), 3.74 (q, *J* = 7.4 Hz, 2H), 2.93 (d, *J* = 12.7 Hz, 2H), 2.45 (d, *J* = 12.7 Hz, 2H), 1.58(s, 3H), 1.39 (t, *J* = 7.3 Hz, 3H) |
| **44** | 598 [M+H]⁺ | (300 MHz, Chloroform-d, *ppm)* δ 9.10 (s, 1H), 8.38 (d, *J* = 2.4 Hz, 2H), 8.08 (d, *J =* 2.7 Hz, 1H), 7.54 (d, *J* = 2.7 Hz, 1H), 5.19 (t, *J* = 13.8 Hz, 2H), 4.19 (s, 4H), 3.80 (q, *J* = 7.5 Hz, 2H), 2.73 (d, *J* = 13.5 Hz, 2H), 2.25 (d, *J* = 13.5 Hz, 2H), 1.42-1.37 (m, 6H) |
| **45** | 584 [M+H]⁺ | (300 MHz, Chloroform-d, *ppm)* δ 9.42 (s, 1H), 8.76 (s, 1H), 8.09 (s, 1H), 7.86 (d, *J* = 2.7 Hz, 1H), 7.43 (d, *J* = 2.7 Hz, 1H), 4.18 (d, *J* = 1.8 Hz, 4H), 3.71 (q, *J* = 7.5 Hz, 2H), 2.73 (d, *J* = 13.8 Hz, 2H), 2.25 (d, *J* = 13.5 Hz, 2H), 1.44-1.33 (m, 6H) |
| **46** | 542 [M+H]⁺ | (400.0 MHz, d6-DMSO): δ=8.7045 (1.4); 8.2446 (0.6); 8.2380 (0.7); 7.2836 (0.6); 7.2769 (0.7); 4.1894 (1.3); 4.1815 (1.3); 4.0109 (0.2); 3.9123 (3.5); 3.8041 (0.2); 3.7855 (0.7); 3.7670 (0.7); 3.7484 (0.2); 3.3570 (0.2); 3.3360 (0.3); 3.3152 (0.2); 3.2982 (16.0); 2.7177 (0.2); 2.7133 (0.1); 2.6862 (0.4); 2.6643 (0.4); 2.6339 (0.4); 2.6142 (0.4); 2.6079 (0.3); 2.6023 (0.2); 2.5827 (0.2); 2.5222 (0.1); 2.5089 (3.1); 2.5043 (6.6); 2.4998 (9.1); 2.4952 (6.5); 2.4907 (3.0); 1.9871 (0.3); 1.2504 (0.2); 1.2297 (0.8); 1.2112 (1.8); 1.1926 (0.9); 1.1746 (0.2); -0.0004 (1.5) |
| **47** | 547 [M+H]⁺ | (400.0 MHz, d6-DMSO): δ=8.1820 (0.2); 8.1752 (0.2); 7.3133 (0.4); 7.2196 (0.2); 7.2128 (0.2); 4.1368 (0.5); 4.1265 (0.5); 4.0385 (0.1); 4.0207 (0.1); 3.8047 (1.3); 3.6922 (0.3); 3.6737 (0.3); 3.3237 (0.1); 3.2992 (16.0); 3.2886 (0.1); 2.6729 (0.1); 2.6685 (0.2); 2.6465 (0.1); 2.6151 (0.1); 2.5953 (0.1); 2.5086 (1.5); 2.5041 (3.3); 2.4995 (4.5); 2.4949 (3.2); 2.4903 (1.5); 1.9869 (0.5); 1.2448 (0.2); 1.1923 (0.2); 1.1786 (0.4); 1.1746 (0.4); 1.1602 (0.8); 1.1417 (0.3); 0.9714 (0.1); -0.0004 (0.5) |
| **48** | 541 [M+H]⁺ | (400.0 MHz, d6-DMSO): δ=9.2358 (0.4); 8.4494 (0.6); 8.4478 (0.6); 8.1314 (0.4); 8.1246 (0.4); 8.1044 (0.5); 8.1015 (0.5); 7.2945 (0.4); 7.2878 (0.4); 5.7696 (0.1); 5.7493 (2.6); 5.7323 (0.3); 5.6948 (0.1); 4.0815 (0.8); 4.0699 (0.8); 3.9620 (0.1); 3.9435 (0.4); 3.9249 (0.4); 3.9063 (0.1); 3.3466 (0.1); 3.3255 (0.2); 3.3025 (16.0); 2.6987 (0.1); 2.6673 (0.2); 2.6453 (0.2); 2.6109 (0.2); 2.5911 (0.2); 2.5848 (0.2); 2.5793 (0.1); 2.5596 (0.1); 2.5091 (1.3); 2.5045 (2.9); 2.4999 (4.0); 2.4953 (2.9); 2.4908 (1.3); 1.9077 (0.2); 1.2737 (0.5); 1.2552 (1.1); 1.2479 (0.3); 1.2366 (0.5); -0.0004 (0.1) |
| **49** | 504 [M+H]⁺ | (400.1 MHz, d6-DMSO):δ=8.6734 (1.1); 8.4957 (0.5); 8.3886 (0.2); 8.3843 (0.2); 8.3678 (0.4); 8.3634 (0.4); 8.3056 (0.5); 8.2848 (0.3); 8.0545 (0.8); 8.0474 (0.8); 7.2953 (0.8); 7.2882 (0.8); 4.1240 (1.7); 4.1190 (1.7); 3.3454 (0.3); 3.3243 (0.5); 3.2970 (16.0); 2.7053 (0.2); 2.7008 (0.1); 2.6833 (0.3); 2.6780 (0.3); 2.6737 (0.5); 2.6519 (0.4); 2.6139 (0.4); 2.5943 (0.4); 2.5879 (0.3); 2.5823 (0.2); 2.5671 (0.1); 2.5628 (0.2); 2.5168 (0.1); 2.5083 (2.4); 2.5038 (5.2); 2.4992 (7.3); 2.4946 (5.3); 2.4900 (2.5); 2.0694 (0.5); 1.1326 (0.9); 1.1142 (2.1); 1.0957 (0.9); 0.0078 (0.1); -0.0004 (4.0); -0.0087 (0.2) |
| **50** | 527 [M+H]⁺ | (400.0 MHz, d6-DMSO):δ=8.1800 (0.5); 8.1733 (0.5); 7.7982 (0.9); 7.7001 (1.0); 7.2317 (0.5); 7.2249 (0.5); 4.1369 (1.1); 4.1265 (1.1); 4.0387 (0.2); 4.0210 (0.2); 3.8027 (0.2); 3.7842 (0.5); 3.7657 (0.6); 3.7473 (0.2); 3.6178 (2.7); 3.3489 (0.2); 3.3278 (0.3); 3.3032 (16.0); 3.1696 (0.1); 2.7056 (0.1); 2.7012 (0.1); 2.6834 (0.2); 2.6783 (0.2); 2.6740 (0.3); 2.6521 (0.3); 2.6200 (0.3); 2.6002 (0.3); 2.5940 (0.2); 2.5885 (0.2); 2.5687 (0.1); 2.5090 (1.2); 2.5045 (2.6); 2.4999 (3.6); 2.4954 (2.5); 2.4908 (1.2); 1.9871 (1.0); 1.2475 (0.3); 1.1924 (0.3); 1.1863 (0.6); 1.1746 (0.6); 1.1679 (1.4); 1.1569 (0.3); 1.1493 (0.6); -0.0004 (0.2) |
| **51** | 527 [M+H]⁺ | (400.0 MHz, d6-DMSO): δ=8.7760 (0.4); 8.7713 (0.4); 8.5520 (0.4); 8.5472 (0.4); 8.3533 (0.5); 8.3466 (0.6); 7.3992 (0.5); 7.3924 (0.5); 5.7500 (1.1); 4.3878 (0.2); 4.3665 (0.4); 4.3329 (0.4); 4.3117 (0.2); 4.2990 (0.1); 4.2777 (0.4); 4.2599 (0.4); 4.2386 (0.1); 4.0396 (0.1); 4.0218 (0.1); 3.8338 (0.2); 3.8153 (0.6); 3.7967 (0.6); 3.7782 (0.2); 3.7407 (2.9); 3.3058 (16.0); 2.5103 (0.7); 2.5058 (1.5); 2.5012 (2.2); 2.4966 (1.5); 2.4921 (0.7); 2.2154 (0.2); 2.2005 (0.2); 2.1923 (0.2); 2.1775 (0.2); 1.9879 (0.4); 1.5287 (0.2); 1.5142 (0.2); 1.5057 (0.2); 1.4910 (0.2); 1.4690 (0.2); 1.4543 (0.4); 1.4396 (0.1); 1.2346 (0.7); 1.2161 (1.4); 1.1976 (0.6); 1.1934 (0.2); 1.1753 (0.3); 1.1575 (0.1) |
| | 571 [M+HCOO]⁻ | |
| **51a** | 527 [M+H]⁺ | (400.0 MHz, d6-DMSO): δ=8.7759 (0.5); 8.7711 (0.5); 8.5522 (0.5); 8.5474 (0.5); 8.3530 (0.7); 8.3463 (0.7); 7.3982 (0.7); 7.3915 (0.7); 4.3871 (0.2); 4.3659 (0.5); 4.3321 (0.5); 4.3109 (0.2); 4.2983 (0.2); 4.2771 (0.5); 4.2593 (0.5); 4.2381 (0.2); 3.8330 (0.2); 3.8144 (0.7); 3.7958 (0.7); 3.7774 (0.2); 3.7397 (3.6); 3.3004 (16.0); 2.5094 (1.5); 2.5048 (3.2); 2.5003 (4.5); 2.4957 (3.2); 2.4911 (1.4); 2.2152 (0.2); 2.2003 (0.3); 2.1922 (0.2); 2.1773 (0.2); 1.5280 (0.2); 1.5135 (0.3); 1.5050 (0.2); 1.4904 (0.3); 1.4686 (0.3); 1.4539 (0.4); 1.4391 (0.2); 1.2338 (0.8); 1.2153 (1.8); 1.1967 (0.7); -0.0004 (1.7) |
| **51b** | 527 [M+H]⁺ | (400.0 MHz, d6-DMSO): δ=8.7759 (0.6); 8.7712 (0.6); 8.5521 (0.6); 8.5473 (0.6); 8.3531 (0.8); 8.3464 (0.8); 7.3985 (0.8); 7.3917 (0.8); 4.3873 (0.3); 4.3660 (0.5); 4.3323 (0.6); 4.3111 (0.3); 4.2984 (0.2); 4.2772 (0.6); 4.2594 (0.6); 4.2382 (0.2); 3.8331 (0.2); 3.8146 (0.8); 3.7960 (0.8); 3.7776 (0.2); 3.7399 (4.1); 3.3010 (16.0); 2.5095 (1.4); 2.5050 (3.0); 2.5004 (4.2); 2.4959 (3.0); 2.4913 (1.4); 2.2153 (0.2); 2.2004 (0.3); 2.1922 (0.3); 2.1774 (0.3); 1.5282 (0.2); 1.5136 (0.3); 1.5052 (0.2); 1.4905 (0.3); 1.4687 (0.3); 1.4540 (0.5); 1.4392 (0.2); 1.2339 (0.9); 1.2155 (2.0); 1.1969 (0.9); -0.0004 (1.4) |
| **52** | 495 [M+H]⁺ | (400.0 MHz, d6-DMSO): δ=8.1701 (2.9); 8.1633 (3.1); 7.8444 (0.1); 7.8003 (5.3); 7.7021 (5.5); 7.2599 (0.1); 7.2245 (3.0); 7.2177 (3.0); 5.1522 (0.1); 5.1361 (0.4); 5.1198 (0.7); 5.1034 (0.4); 5.0871 (0.1); 5.0130 (0.2); 4.9962 (0.5); 4.9798 (0.7); 4.9633 (0.5); 4.9468 (0.1); 4.1274 (6.1); 4.1040 (6.3); 4.0566 (0.2); 4.0387 (0.5); 4.0210 (0.5); 4.0031 (0.2); 3.9705 (0.1); 3.9545 (0.2); 3.9385 (0.2); 3.9214 (0.1); 3.8036 (0.9); 3.7852 (3.2); 3.7666 (3.3); 3.7482 (1.0); 3.6997 (0.2); 3.6549 (0.3); 3.6473 (0.2); 3.6411 (0.3); 3.6197 (16.0); 3.4844 (1.9); 3.1695 (0.2); 2.7364 (0.4); 2.7290 (0.4); 2.7195 (0.6); 2.7124 (0.9); 2.7036 (0.9); 2.6960 (0.8); 2.6868 (1.0); 2.6799 (0.8); 2.6692 (0.5); 2.6635 (0.6); 2.5390 (1.7); 2.5223 (0.2); 2.5089 (7.0); 2.5043 (15.3); 2.4998 (21.5); 2.4952 (15.4); 2.4907 (7.4); 2.4698 (0.7); 2.4609 (0.6); 2.4521 (0.6); 2.4434 (0.4); 2.4356 (0.7); 2.4307 (0.8); 2.4229 (0.5); 2.4146 (0.6); 2.4056 (0.6); 2.3971 (0.5); 2.3886 (0.4); 2.3813 (0.5); 2.3356 (0.1); 2.3311 (0.1); 2.3267 (0.2); 2.3220 (0.1); 1.9871 (1.7); 1.9077 (0.1); 1.2524 (0.2); 1.2356 (0.5); 1.2161 (0.1); 1.1923 (0.8); 1.1859 (3.5); 1.1746 (1.5); 1.1675 (8.0); 1.1568 (0.9); 1.1490 (3.4); 1.0898 (0.2); 1.0721 (0.2); -0.0004 (3.3); - 0.0087 (0.1) |
| **53** | 538 [M+H]⁺ | (400.1 MHz, d6-DMSO): δ=9.2500 (0.2); 8.7275 (0.0); 8.4604 (0.3); 8.4586 (0.3); 8.2494 (0.2); 8.2426 (0.2); 8.1820 (0.0); 8.1791 (0.0); 8.1273 (0.3); 8.1243 (0.3); 7.4271 (0.0); 7.4190 (0.2); 7.4122 (0.2); 5.7744 (0.1); 5.7371 (0.1); 5.6996 (0.1); 4.2726 (0.1); 4.2524 (0.3); 4.2143 (0.2); 4.1941 (0.1); 4.0704 (0.0); 4.0572 (0.0); 3.9703 (0.1); 3.9517 (0.2); 3.9332 (0.2); 3.9146 (0.1); 3.5675 (1.3); 3.2971 (16.0); 3.2570 (0.0); 3.1765 (0.1); 3.1633 (0.1); 2.6685 (0.0); 2.5385 (0.0); 2.5219 (0.0); 2.5171 (0.0); 2.5086 (0.7); 2.5040 (1.4); 2.4994 (2.0); 2.4948 (1.4); 2.4903 (0.6); 2.3263 (0.0); 1.8413 (0.1); 1.8192 (0.2); 1.7966 (0.1); 1.3982 (0.0); 1.3031 (0.0); 1.2888 (0.2); 1.2702 (0.6); 1.2516 (0.2); 1.2365 (0.0); -0.0004 (0.1) |
| **54** | 502 [M+H]⁺ | (400.1 MHz, d6-DMSO): δ=9.2439 (1.0); 8.4535 (1.6); 8.4518 (1.6); 8.1607 (1.1); 8.1539 (1.2); 8.1182 (1.5); 8.1152 (1.5); 7.3275 (1.2); 7.3207 (1.2); 5.7713 (0.3); 5.7339 (0.7); 5.6964 (0.4); 4.1407 (5.9); 3.9727 (0.3); 3.9541 (1.2); 3.9355 (1.2); 3.9170 (0.4); 3.2941 (16.0); 2.5087 (1.6); 2.5041 (3.5); 2.4995 (4.9); 2.4949 (3.5); 2.4904 (1.6); 1.9868 (0.3); 1.2826 (1.3); 1.2640 (3.0); 1.2454 (1.3); 1.1748 (0.2); 0.7207 (5.2); 0.4544 (0.2); 0.0078 (0.1); - 0.0004 (3.8); -0.0087 (0.1) |
| **55** | 595 [M+H]⁺ | (400.1 MHz, d6-DMSO): δ=9.2500 (0.2); 8.7275 (0.0); 8.4604 (0.3); 8.4586 (0.3); 8.2494 (0.2); 8.2426 (0.2); 8.1820 (0.0); 8.1791 (0.0); 8.1273 (0.3); 8.1243 (0.3); 7.4271 (0.0); 7.4190 (0.2); 7.4122 (0.2); 5.7744 (0.1); 5.7371 (0.1); 5.6996 (0.1); 4.2726 (0.1); 4.2524 (0.3); 4.2143 (0.2); 4.1941 (0.1); 4.0704 (0.0); 4.0572 (0.0); 3.9703 (0.1); 3.9517 (0.2); 3.9332 (0.2); 3.9146 (0.1); 3.5675 (1.3); 3.2971 (16.0); 3.2570 (0.0); 3.1765 (0.1); 3.1633 (0.1); 2.6685 (0.0); 2.5385 (0.0); 2.5219 (0.0); 2.5171 (0.0); 2.5086 (0.7); 2.5040 (1.4); 2.4994 (2.0); 2.4948 (1.4); 2.4903 (0.6); 2.3263 (0.0); 1.8413 (0.1); 1.8192 (0.2); 1.7966 (0.1); 1.3982 (0.0); 1.3031 (0.0); 1.2888 (0.2); 1.2702 (0.6); 1.2516 (0.2); 1.2365 (0.0); -0.0004 (0.1) |
| **56** | 540 [M+H]⁺ | (400.0 MHz, d6-DMSO): δ=8.1717 (0.5); 8.1649 (0.4); 7.3138 (0.7); 7.2109 (0.5); 7.2041 (0.4); 5.1156 (0.1); 4.9756 (0.1); 4.1275 (0.9); 4.1040 (0.9); 4.0386 (0.2); 4.0208 (0.2); 3.8065 (2.3); 3.7128 (0.1); 3.6942 (0.5); 3.6757 (0.5); 3.6573 (0.1); 3.2992 (16.0); 3.0708 (0.1); 2.7072 (0.1); 2.6984 (0.1); 2.6905 (0.1); 2.6817 (0.2); 2.6741 (0.1); 2.5087 (1.6); 2.5042 (3.5); 2.4996 (4.9); 2.4951 (3.5); 2.4906 (1.6); 2.4652 (0.1); 2.4264 (0.1); 1.9870 (1.0); 1.1924 (0.3); 1.1789 (0.8); 1.1747 (0.8); 1.1604 (1.4); 1.1420 (0.5); 0.9709 (0.2); -0.0004 (1.6) |
| **57** | 516 [M+H]⁺ | (400.1 MHz, d6-DMSO): δ=9.2368 (1.9); 8.4477 (3.1); 8.4460 (3.1); 8.1146 (2.2); 8.1078 (2.5); 8.1049 (3.2); 8.1018 (3.0); 7.7784 (0.1); 7.4758 (0.1); 7.4624 (0.2); 7.4580 (0.2); 7.4122 (0.1); 7.4048 (0.1); 7.3770 (0.1); 7.3367 (0.1); 7.2906 (2.2); 7.2839 (2.2); 7.2738 (0.1); 7.2546 (0.1); 5.7675 (0.7); 5.7301 (1.4); 5.6926 (0.7); 4.0185 (10.7); 3.9625 (0.6); 3.9440 (2.3); 3.9254 (2.3); 3.9068 (0.7); 3.5684 (7.4); 3.2975 (16.0); 2.9242 (0.2); 2.9090 (0.1); 2.5095 (1.5); 2.5050 (3.2); 2.5004 (4.5); 2.4958 (3.2); 2.4912 (1.5); 2.2427 (2.0); 2.2239 (3.8); 2.2048 (2.5); 1.9951 (0.2); 1.9759 (0.1); 1.8774 (0.3); 1.8575 (0.9); 1.8394 (1.3); 1.8200 (0.7); 1.8016 (0.2); 1.2762 (2.4); 1.2577 (5.6); 1.2391 (2.5); -0.0004 (1.6) |
| **58** | 546 [M+H]⁺ | (400.1 MHz, d6-DMSO): δ=9.2358 (1.4); 8.4469 (2.1); 8.4458 (2.2); 8.1113 (1.5); 8.1043 (3.2); 8.1013 (2.1); 7.2852 (1.5); 7.2785 (1.5); 5.7669 (0.5); 5.7295 (1.0); 5.6920 (0.5); 4.0494 (3.1); 4.0398 (0.5); 4.0219 (0.4); 4.0028 (3.1); 3.9665 (0.2); 3.9608 (0.5); 3.9422 (1.5); 3.9236 (1.6); 3.9051 (0.5); 3.8424 (0.1); 3.8253 (0.5); 3.8083 (0.8); 3.7912 (0.5); 3.7742 (0.1); 3.3134 (0.1); 3.2956 (16.0); 3.1401 (10.2); 3.0685 (0.1); 3.0622 (0.2); 2.5571 (0.5); 2.5500 (0.4); 2.5403 (0.6); 2.5328 (0.7); 2.5252 (0.6); 2.5088 (2.1); 2.5045 (3.5); 2.4999 (4.6); 2.4954 (3.2); 2.4909 (1.5); 2.1240 (0.6); 2.1167 (0.4); 2.1066 (0.6); 2.0993 (0.6); 2.0921 (0.5); 2.0818 (0.4); 2.0747 (0.5); 1.9870 (1.5); 1.2740 (1.7); 1.2555 (3.7); 1.2369 (1.6); 1.1927 (0.4); 1.1750 (0.8); 1.1572 (0.4); -0.0004 (1.7) |
| **59** | 534 [M+H]⁺ | (400.0 MHz, d6-DMSO): δ=9.2415 (6.1); 8.4520 (9.9); 8.1227 (6.0); 8.1160 (6.3); 8.1086 (8.1); 8.1060 (7.8); 7.2906 (6.1); 7.2839 (6.1); 5.7718 (2.1); 5.7344 (4.6); 5.6968 (2.3); 5.2971 (0.1); 5.1503 (0.4); 5.1341 (1.1); 5.1177 (1.7); 5.1013 (1.1); 5.0847 (0.5); 5.0108 (0.5); 4.9943 (1.3); 4.9779 (1.9); 4.9614 (1.4); 4.9452 (0.7); 4.8338 (0.6); 4.7564 (0.5); 4.6074 (0.3); 4.5076 (0.2); 4.4788 (0.2); 4.3373 (0.1); 4.2511 (0.1); 4.2272 (0.1); 4.0715 (13.9); 4.0491 (14.1); 3.9597 (2.0); 3.9411 (6.7); 3.9225 (6.8); 3.9040 (2.1); 3.8645 (0.1); 3.1698 (0.5); 2.7291 (0.9); 2.7215 (0.8); 2.7120 (1.3); 2.7053 (2.0); 2.6964 (1.8); 2.6887 (1.8); 2.6795 (2.2); 2.6734 (1.8); 2.6642 (1.1); 2.6560 (1.1); 2.5389 (0.3); 2.5090 (13.4); 2.5045 (28.5); 2.5000 (39.4); 2.4955 (28.1); 2.4911 (13.3); 2.4772 (1.5); 2.4694 (1.1); 2.4610 (1.3); 2.4522 (1.2); 2.4435 (1.1); 2.4348 (0.9); 2.4268 (1.4); 2.4222 (1.6); 2.4141 (1.0); 2.4059 (1.3); 2.3969 (1.2); 2.3886 (1.1); 2.3799 (0.8); 2.3725 (0.9); 2.3315 (0.2); 2.3270 (0.3); 2.3221 (0.2); 1.2727 (7.2); 1.2542 (16.0); 1.2356 (7.1); 0.1458 (0.1); 0.0076 (0.7); -0.0004 (22.1); -0.0086 (0.8) |
| **60** | 555 [M+H]⁺ | (400.0 MHz, d6-DMSO): δ=8.7658 (1.0); 8.7611 (1.1); 8.5366 (1.1); 8.5318 (1.1); 8.2035 (1.4); 8.1968 (1.4); 7.2527 (1.4); 7.2459 (1.4); 4.1811 (2.8); 4.0739 (2.8); 3.8241 (0.4); 3.8057 (1.5); 3.7871 (1.6); 3.7687 (0.5); 3.7223 (7.5); 3.3009 (16.0); 2.6524 (1.5); 2.6363 (2.1); 2.5622 (0.1); 2.5429 (0.3); 2.5261 (0.3); 2.5099 (2.1); 2.5054 (4.4); 2.5008 (5.8); 2.4963 (4.2); 2.4917 (2.1); 2.4698 (0.6); 2.4541 (0.3); 2.4446 (0.6); 2.4395 (0.6); 2.4268 (0.3); 2.4190 (0.4); 2.0908 (0.5); 2.0721 (0.7); 2.0683 (0.6); 2.0603 (0.5); 2.0412 (0.5); 1.2183 (1.6); 1.1998 (3.7); 1.1813 (1.7); 1.1675 (0.2); -0.0004 (1.5) |
| **61** | 556 [M+H]⁺ | (400.1 MHz, d6-DMSO): δ=8.7664 (1.1); 8.7617 (1.1); 8.5349 (1.1); 8.5301 (1.1); 8.2385 (1.4); 8.2318 (1.5); 7.2928 (1.5); 7.2860 (1.4); 4.2441 (6.7); 3.8211 (0.4); 3.8026 (1.5); 3.7840 (1.6); 3.7656 (0.5); 3.7330 (0.2); 3.7212 (7.9); 3.6453 (4.0); 3.4903 (6.0); 3.2945 (16.0); 2.5095 (2.0); 2.5050 (4.1); 2.5004 (5.7); 2.4958 (4.0); 2.4913 (1.8); 1.2360 (0.4); 1.2203 (1.7); 1.2018 (3.8); 1.1833 (1.6); -0.0004 (0.2) |
| **62** | 535 [M+H]⁺ | (400.1 MHz, d6-DMSO): δ=8.7071 (1.3); 8.2401 (0.6); 8.2334 (0.6); 7.2814 (0.6); 7.2747 (0.6); 5.1356 (0.1); 4.9957 (0.1); 4.1899 (1.1); 4.1655 (1.1); 3.9225 (3.2); 3.8129 (0.2); 3.7944 (0.6); 3.7759 (0.7); 3.7574 (0.2); 3.3057 (16.0); 2.7406 (0.1); 2.7333 (0.2); 2.7244 (0.2); 2.7167 (0.2); 2.7077 (0.2); 2.7006 (0.1); 2.5170 (1.1); 2.5124 (2.5); 2.5078 (3.5); 2.5032 (2.4); 2.4987 (1.2); 2.4834 (0.1); 2.4584 (0.1); 2.4533 (0.1); 2.4371 (0.1); 2.0777 (0.5); 1.2384 (0.7); 1.2199 (1.6); 1.2014 (0.7) |
| **63** | 527 [M+H]⁺ | (400.1 MHz, d6-DMSO): δ=9.4560 (3.1); 9.2499 (3.9); 9.2481 (4.0); 8.3147 (3.5); 8.0521 (2.8); 8.0451 (2.9); 7.3283 (2.9); 7.3213 (2.9); 4.1572 (6.8); 4.1515 (6.9); 3.7472 (1.0); 3.7288 (3.4); 3.7102 (3.4); 3.6918 (1.0); 3.3952 (11.7); 3.3758 (9.3); 3.3543 (16.0); 3.3298 (2.5); 3.3090 (1.2); 3.2881 (0.4); 3.1705 (1.4); 2.7116 (0.8); 2.7073 (0.6); 2.6894 (1.1); 2.6844 (1.4); 2.6801 (1.9); 2.6583 (1.6); 2.6257 (1.8); 2.6060 (1.8); 2.5998 (1.2); 2.5943 (1.0); 2.5746 (0.9); 2.5393 (0.2); 2.5226 (0.2); 2.5092 (6.4); 2.5047 (13.8); 2.5002 (19.2); 2.4956 (13.8); 2.4911 (6.6); 2.3271 (0.1); 2.0696 (0.2); 1.2261 (3.6); 1.2077 (8.1); 1.1891 (3.5); -0.0004 (0.3) |
| **64** | 497 [M+H]⁺ | (400.1 MHz, d6-DMSO): δ=8.6744 (0.4); 8.4992 (0.2); 8.4972 (0.2); 8.3888 (0.1); 8.3846 (0.1); 8.3681 (0.1); 8.3637 (0.1); 8.3061 (0.2); 8.2854 (0.1); 8.0435 (0.3); 8.0363 (0.3); 7.2822 (0.3); 7.2750 (0.3); 5.1185 (0.1); 4.9786 (0.1); 4.1167 (0.5); 4.0929 (0.5); 4.0390 (0.1); 4.0212 (0.1); 3.3108 (6.3); 3.2986 (16.0); 3.1764 (0.2); 3.1633 (0.1); 2.7123 (0.1); 2.7034 (0.1); 2.6956 (0.1); 2.6867 (0.1); 2.6794 (0.1); 2.6690 (0.1); 2.6636 (0.1); 2.5089 (1.1); 2.5043 (2.3); 2.4998 (3.3); 2.4952 (2.3); 2.4906 (1.1); 2.4658 (0.1); 2.4318 (0.1); 2.4269 (0.1); 2.4106 (0.1); 1.9865 (0.2); 1.3981 (0.1); 1.1925 (0.1); 1.1747 (0.1); 1.1569 (0.1); 1.1328 (0.3); 1.1144 (0.7); 1.0959 (0.3); -0.0004 (0.1) |
| **65** | 539 [M+H]⁺ | (400.1 MHz, d6-DMSO): δ=8.7175 (0.8); 8.3916 (0.1); 8.3686 (0.3); 8.3619 (0.4); 7.4318 (0.4); 7.4251 (0.3); 4.3724 (0.2); 4.3509 (0.5); 4.3371 (0.1); 4.3180 (0.3); 4.2975 (0.1); 4.0392 (0.1); 4.0251 (0.2); 4.0216 (0.1); 3.9252 (1.9); 3.8040 (0.1); 3.7855 (0.4); 3.7669 (0.4); 3.7485 (0.1); 3.2937 (16.0); 2.5087 (1.3); 2.5042 (2.7); 2.4996 (3.7); 2.4950 (2.6); 2.4905 (1.2); 1.9867 (0.2); 1.8811 (0.1); 1.8590 (0.3); 1.8363 (0.1); 1.2435 (0.4); 1.2362 (0.1); 1.2250 (1.0); 1.2063 (0.5); 1.1927 (0.1); 1.1867 (0.1); 1.1749 (0.1); 1.1571 (0.1); -0.0004 (0.1) |
| **66** | 503 [M+H]⁺ | (400.1 MHz, d6-DMSO): δ=8.7052 (0.9); 8.3832 (0.1); 8.2765 (0.4); 8.2698 (0.4); 7.3166 (0.4); 7.3098 (0.4); 4.2413 (2.1); 4.2274 (0.2); 4.0396 (0.1); 4.0299 (0.2); 4.0219 (0.1); 3.9305 (2.2); 3.8996 (0.1); 3.8183 (0.1); 3.7998 (0.4); 3.7813 (0.4); 3.7628 (0.1); 3.6971 (0.1); 3.2970 (16.0); 2.5096 (0.7); 2.5051 (1.5); 2.5005 (2.1); 2.4959 (1.5); 2.4913 (0.7); 1.9872 (0.2); 1.2404 (0.5); 1.2220 (1.1); 1.2033 (0.5); 1.1931 (0.1); 1.1830 (0.1); 1.1753 (0.1); 1.1575 (0.1); 0.7489 (1.9) |
| **67** | 547 [M+H]⁺ | (400.1 MHz, d6-DMSO): δ=8.6978 (2.3); 8.3758 (0.2); 8.2223 (1.0); 8.2156 (1.1); 7.2667 (1.1); 7.2600 (1.0); 4.1601 (2.0); 4.1462 (0.2); 4.1155 (1.9); 4.1019 (0.2); 4.0398 (0.2); 4.0219 (0.2); 4.0144 (0.4); 3.9468 (0.2); 3.9152 (5.7); 3.8977 (0.1); 3.8339 (0.3); 3.8170 (0.6); 3.8055 (0.4); 3.8000 (0.4); 3.7870 (1.2); 3.7685 (1.2); 3.7500 (0.4); 3.2939 (16.0); 3.1462 (7.9); 3.0773 (0.1); 2.5782 (0.3); 2.5710 (0.3); 2.5615 (0.4); 2.5538 (0.5); 2.5462 (0.4); 2.5368 (0.3); 2.5295 (0.4); 2.5185 (0.1); 2.5096 (1.6); 2.5050 (3.5); 2.5004 (4.9); 2.4959 (3.4); 2.4913 (1.6); 2.1465 (0.4); 2.1392 (0.3); 2.1293 (0.4); 2.1218 (0.5); 2.1145 (0.4); 2.1045 (0.3); 2.0973 (0.4); 1.9873 (0.6); 1.2307 (1.3); 1.2228 (0.2); 1.2122 (2.9); 1.2044 (0.2); 1.1935 (1.4); 1.1753 (0.4); 1.1576 (0.2) |
| **68** | 546 [M+H]⁺ | (400.0 MHz, d6-DMSO): δ=8.7650 (0.2); 8.7604 (0.3); 8.5358 (0.3); 8.5311 (0.3); 8.2000 (0.3); 8.1933 (0.3); 7.2463 (0.3); 7.2396 (0.3); 4.1356 (0.7); 4.0900 (0.7); 3.8307 (0.1); 3.8210 (0.1); 3.8137 (0.2); 3.8026 (0.4); 3.7970 (0.2); 3.7840 (0.4); 3.7746 (0.1); 3.7656 (0.1); 3.7206 (1.7); 3.3022 (16.0); 3.1437 (2.2); 3.0749 (0.1); 2.5729 (0.1); 2.5562 (0.1); 2.5485 (0.1); 2.5408 (0.1); 2.5239 (0.1); 2.5092 (1.0); 2.5047 (2.1); 2.5002 (3.0); 2.4957 (2.1); 2.4912 (1.0); 2.1397 (0.1); 2.1225 (0.1); 2.1151 (0.1); 2.1078 (0.1); 2.0905 (0.1); 1.9873 (0.2); 1.2156 (0.4); 1.1971 (0.9); 1.1786 (0.4) |
| **69** | 541 [M+H]⁺ | (400.1 MHz, d6-DMSO): δ=8.7699 (0.4); 8.7652 (0.4); 8.5421 (0.5); 8.5373 (0.4); 8.2957 (0.6); 8.2890 (0.6); 7.3367 (0.6); 7.3299 (0.6); 4.3987 (0.3); 4.3769 (0.3); 4.2548 (0.3); 4.2326 (0.2); 4.2176 (0.2); 4.1950 (0.3); 4.1141 (0.3); 4.0919 (0.2); 3.8281 (0.2); 3.8096 (0.6); 3.7910 (0.6); 3.7727 (0.2); 3.7293 (3.2); 3.7162 (0.3); 3.6958 (0.2); 3.2946 (16.0); 2.5092 (1.3); 2.5046 (2.7); 2.5000 (3.7); 2.4954 (2.6); 2.4909 (1.2); 2.3457 (0.2); 2.3314 (0.1); 2.3221 (0.3); 2.3122 (0.1); 2.2997 (0.2); 2.2920 (0.1); 2.2862 (0.1); 2.2823 (0.1); 2.2763 (0.1); 2.2693 (0.1); 2.2645 (0.1); 2.2540 (0.1); 2.1574 (0.1); 2.1366 (0.2); 2.1321 (0.1); 2.1095 (0.1); 2.0701 (0.2); 1.2267 (0.7); 1.2082 (1.5); 1.1897 (0.7); -0.0004 (0.1) |
| **70** | 596 [M+H]⁺ | (400.0 MHz, d6-DMSO): δ=8.7096 (3.5); 8.2846 (1.5); 8.2780 (1.6); 7.3250 (1.6); 7.3184 (1.6); 4.3312 (7.2); 4.1348 (7.2); 3.9168 (8.3); 3.8117 (0.5); 3.7932 (1.7); 3.7747 (1.7); 3.7562 (0.5); 3.3133 (16.0); 3.1702 (0.3); 3.0306 (8.8); 2.5098 (2.7); 2.5053 (5.7); 2.5008 (7.9); 2.4962 (5.6); 2.4917 (2.6); 2.0716 (1.5); 1.2355 (1.8); 1.2170 (4.1); 1.1985 (1.8); -0.0004 (1.0) |
| **72** | 520 [M+H]⁺ | (400.0 MHz, d6-DMSO): δ=9.6226 (5.9); 8.4717 (9.0); 8.3130 (6.1); 8.1909 (0.4); 8.1371 (5.9); 8.1303 (6.1); 7.2774 (6.0); 7.2706 (6.0); 5.1476 (0.2); 5.1311 (0.9); 5.1147 (1.4); 5.0982 (0.9); 5.0820 (0.2); 5.0080 (0.2); 4.9912 (0.9); 4.9748 (1.4); 4.9584 (1.0); 4.9420 (0.3); 4.1543 (0.2); 4.1283 (0.6); 4.0875 (13.2); 4.0646 (14.1); 4.0431 (7.2); 4.0245 (7.4); 4.0060 (2.8); 3.9377 (1.3); 3.1697 (1.0); 3.0936 (0.2); 2.7271 (0.9); 2.7198 (0.8); 2.7101 (1.3); 2.7032 (2.0); 2.6942 (1.8); 2.6867 (1.7); 2.6776 (2.2); 2.6704 (1.8); 2.6640 (1.0); 2.6538 (1.1); 2.5390 (0.2); 2.5225 (0.4); 2.5091 (14.3); 2.5046 (30.9); 2.5001 (43.2); 2.4955 (31.0); 2.4910 (14.7); 2.4761 (1.5); 2.4681 (1.1); 2.4601 (1.3); 2.4511 (1.2); 2.4426 (1.2); 2.4336 (0.9); 2.4259 (1.4); 2.4211 (1.6); 2.4131 (1.0); 2.4047 (1.3); 2.3960 (1.2); 2.3875 (1.1); 2.3789 (0.8); 2.3714 (0.9); 2.3312 (0.2); 2.3269 (0.3); 2.3225 (0.2); 1.9872 (2.3); 1.3979 (1.0); 1.2398 (7.6); 1.2214 (16.0); 1.2028 (7.0); 1.1927 (0.9); 1.1748 (1.3); 1.1570 (0.7); 1.0323 (0.2); 1.0142 (0.4); 0.9956 (0.2); 0.0077 (0.6); -0.0004 (20.2); - 0.0087 (0.7) |
| **73** | 487 [M+H]⁺ | (400.0 MHz, d6-DMSO): δ=9.1624 (0.1); 9.1573 (0.1); 8.8368 (0.4); 8.8189 (0.4); 8.7132 (0.2); 8.4529 (0.9); 8.4027 (0.1); 8.3976 (0.1); 8.1575 (0.6); 8.1507 (0.6); 8.0687 (0.5); 8.0405 (0.1); 8.0336 (0.1); 7.3160 (0.6); 7.3093 (0.6); 7.2786 (0.1); 7.2715 (0.1); 7.1928 (0.3); 7.1750 (0.3); 4.1414 (4.1); 4.1167 (0.6); 4.1025 (0.5); 4.0569 (0.1); 4.0391 (0.3); 4.0213 (0.3); 3.8967 (0.1); 3.8781 (0.1); 3.3007 (16.0); 2.5093 (1.9); 2.5048 (4.1); 2.5003 (5.7); 2.4957 (4.1); 2.4913 (1.9); 1.9874 (1.1); 1.3977 (0.2); 1.2524 (0.7); 1.2340 (1.5); 1.2154 (0.7); 1.2074 (0.3); 1.1926 (0.4); 1.1749 (0.6); 1.1571 (0.3); 1.1033 (0.1); 1.0848 (0.2); 1.0662 (0.1); 0.7176 (3.9); 0.6998 (0.5); -0.0004 (0.3) |
| **74** | 531 [M+H]⁺ | (400.0 MHz, d6-DMSO): δ=8.8286 (0.5); 8.8107 (0.5); 8.4405 (1.5); 8.1081 (0.9); 8.1013 (0.9); 8.0625 (0.7); 7.8177 (0.1); 7.8108 (0.1); 7.7915 (0.1); 7.7870 (0.1); 7.7779 (0.1); 7.4765 (0.1); 7.4710 (0.1); 7.4634 (0.2); 7.4588 (0.2); 7.3775 (0.2); 7.3372 (0.1); 7.2728 (1.0); 7.2660 (1.0); 7.1883 (0.4); 7.1702 (0.4); 4.1436 (0.3); 4.1251 (1.0); 4.1066 (1.0); 4.0882 (0.3); 4.0521 (2.0); 4.0052 (2.0); 3.8211 (0.3); 3.8040 (0.5); 3.7870 (0.3); 3.3008 (16.0); 3.1766 (0.1); 3.1635 (0.1); 3.1373 (6.4); 2.5532 (0.3); 2.5462 (0.2); 2.5365 (0.4); 2.5288 (0.4); 2.5213 (0.4); 2.5093 (2.1); 2.5047 (4.4); 2.5002 (5.7); 2.4957 (4.0); 2.4913 (1.9); 2.1197 (0.4); 2.1124 (0.2); 2.1023 (0.4); 2.0950 (0.4); 2.0878 (0.3); 2.0776 (0.2); 2.0705 (0.3); 1.2424 (1.1); 1.2239 (2.5); 1.2054 (1.1); -0.0004 (0.7) |
| **75** | 580 [M+H]⁺ | (400.0 MHz, d6-DMSO): δ=8.8333 (0.8); 8.8154 (0.8); 8.4530 (2.2); 8.1621 (1.3); 8.1553 (1.4); 8.0693 (1.0); 7.3163 (1.4); 7.3095 (1.4); 7.1951 (0.5); 7.1768 (0.5); 4.2265 (6.6); 4.1523 (0.4); 4.1339 (1.6); 4.1152 (7.9); 4.0970 (0.6); 3.3072 (16.0); 3.1695 (0.3); 3.0205 (7.8); 2.5223 (0.1); 2.5089 (3.9); 2.5044 (8.4); 2.4999 (11.7); 2.4954 (8.4); 2.4909 (3.9); 1.2474 (1.6); 1.2289 (3.6); 1.2103 (1.6); -0.0004 (1.2) |
| **76** | 558 [M+H]⁺ | (400.1 MHz, d6-DMSO): δ=8.6793 (4.1); 8.6786 (4.1); 8.5006 (1.9); 8.4985 (1.9); 8.3907 (0.8); 8.3864 (0.8); 8.3698 (1.4); 8.3656 (1.3); 8.3085 (1.8); 8.2877 (1.1); 8.0932 (2.8); 8.0861 (2.8); 7.3362 (2.8); 7.3290 (2.8); 4.2672 (11.8); 4.1201 (11.9); 4.0811 (0.1); 3.9305 (0.1); 3.8904 (0.1); 3.7573 (1.3); 3.3381 (0.9); 3.3234 (0.9); 3.1708 (0.5); 3.0412 (0.1); 3.0224 (16.0); 2.5227 (0.1); 2.5179 (0.2); 2.5094 (3.5); 2.5048 (7.6); 2.5002 (10.6); 2.4956 (7.4); 2.4910 (3.4); 2.0698 (0.2); 1.1398 (3.4); 1.1214 (7.7); 1.1029 (3.3); 0.0078 (0.2); -0.0004 (5.7); -0.0088 (0.2) |
| **77** | 601 [M+H]⁺ | (400.1 MHz, d6-DMSO): δ=8.2195 (2.8); 8.2127 (2.8); 7.3131 (4.3); 7.2592 (2.8); 7.2524 (2.8); 4.2779 (12.7); 4.1154 (12.6); 3.8747 (0.2); 3.8096 (14.5); 3.7182 (0.8); 3.6997 (3.0); 3.6812 (3.0); 3.6627 (0.9); 3.4274 (16.0); 3.1696 (0.8); 3.0995 (0.2); 3.0810 (0.5); 3.0720 (0.7); 3.0629 (0.5); 3.0438 (0.2); 3.0183 (16.0); 2.6727 (0.1); 2.6682 (0.2); 2.6639 (0.1); 2.5382 (0.2); 2.5217 (0.3); 2.5084 (9.1); 2.5039 (19.5); 2.4993 (27.1); 2.4947 (19.3); 2.4902 (9.0); 2.3308 (0.1); 2.3262 (0.2); 2.3215 (0.1); 2.0693 (0.9); 1.2000 (0.4); 1.1841 (4.5); 1.1658 (8.8); 1.1473 (3.5); 1.1260 (0.1); 0.9723 (1.5); 0.0078 (0.2); - 0.0004 (4.9); -0.0088 (0.2) |
| **78** | 556 [M+H]⁺ | (400.1 MHz, d6-DMSO): δ= 8.2166 (0.5); 8.2098 (0.5); 7.8000 (0.9); 7.7008 (1.0); 7.2704 (0.5); 7.2636 (0.5); 4.2786 (2.3); 4.1220 (2.2); 4.0391 (0.2); 4.0213 (0.2); 3.8104 (0.1); 3.7919 (0.5); 3.7734 (0.5); 3.7550 (0.2); 3.6224 (2.8); 3.2967 (16.0); 3.0221 (3.0); 2.5087 (0.8); 2.5042 (1.8); 2.4996 (2.5); 2.4950 (1.8); 2.4904 (0.8); 1.9867 (1.1); 1.1925 (0.8); 1.1742 (1.7); 1.1566 (0.6); 1.1555 (0.6); -0.0004 (0.1) |
| **79** | 581 [M+H]⁺ | (400.0 MHz, d6-DMSO): δ= 9.3023 (1.3); 9.2843 (1.3); 8.4847 (1.6); 8.2068 (2.0); 8.2001 (2.1); 7.5388 (0.8); 7.5348 (0.8); 7.5208 (0.8); 7.5168 (0.8); 7.2917 (2.1); 7.2850 (2.1); 4.2792 (9.7); 4.1310 (9.6); 4.0848 (0.1); 4.0716 (0.1); 3.9171 (0.7); 3.8986 (2.3); 3.8801 (2.3); 3.8616 (0.7); 3.3116 (16.0); 3.1850 (0.3); 3.1719 (0.3); 3.0327 (11.6); 2.8972 (0.3); 2.7387 (0.3); 2.5171 (1.4); 2.5126 (3.0); 2.5081 (4.2); 2.5036 (3.1); 2.4991 (1.5); 1.2574 (2.4); 1.2389 (5.5); 1.2204 (2.4) |
| **80** | 488 [M+H]⁺ | (400.1 MHz, d6-DMSO): δ=9.2955 (0.3); 9.2776 (0.3); 8.4779 (0.4); 8.4759 (0.3); 8.1925 (0.5); 8.1857 (0.6); 7.5298 (0.2); 7.5256 (0.2); 7.5119 (0.2); 7.5076 (0.2); 7.2792 (0.6); 7.2723 (0.6); 4.1841 (2.9); 3.9117 (0.2); 3.8932 (0.6); 3.8747 (0.6); 3.8562 (0.2); 3.2936 (16.0); 2.5087 (1.3); 2.5042 (2.8); 2.4996 (4.0); 2.4949 (2.8); 2.4904 (1.3); 1.9867 (0.2); 1.2535 (0.6); 1.2350 (1.5); 1.2165 (0.6); 1.1748 (0.1); 0.7314 (2.6); -0.0004 (1.8) |
| **81** | 581 [M+H]⁺ | (400.1 MHz, d6-DMSO): δ= 9.4615 (0.3); 9.4594 (0.3); 9.2615 (0.5); 9.2593 (0.4); 8.3216 (0.2); 8.3190 (0.4); 8.3163 (0.2); 8.0919 (0.3); 8.0849 (0.4); 7.3657 (0.3); 7.3587 (0.3); 4.2996 (1.4); 4.1278 (1.4); 3.7385 (0.4); 3.7199 (0.4); 3.7015 (0.1); 3.2919 (16.0); 3.0248 (1.9); 2.5167 (0.1); 2.5082 (2.2); 2.5037 (4.8); 2.4991 (6.7); 2.4945 (4.7); 2.4899 (2.1); 1.9864 (0.2); 1.2319 (0.4); 1.2134 (0.9); 1.1949 (0.4); 1.1746 (0.1); -0.0004 (2.2) |
| 82 | 566 [M+H]⁺ | (400 MHz, DMSO*-d*₆*, ppm*) δ 8.78 (d, *J* = 2.0 Hz, 1H), 8.56 (d, *J* = 2.0 Hz, 1H), 8.31 (d, *J* = 2.8 Hz, 1H), 7.35 (d, *J* = 2.8 Hz, 1H), 4.56 (s, 4H), 4.40 (s, 4H), 3.81 (q, *J* = 7.6 Hz, 2H), 3.74 (s, 3H), 1.21 (t, *J* = 7.4 Hz, 3H) |
| **83** | 565 [M+H]⁺ | (400.1 MHz, d6-DMSO): δ= 8.7702 (0.2); 8.7654 (0.3); 8.5405 (0.3); 8.5357 (0.3); 8.2923 (0.3); 8.2856 (0.4); 7.3316 (0.4); 7.3248 (0.4); 5.7476 (0.5); 4.5099 (0.2); 4.3734 (0.2); 4.3642 (0.7); 4.3408 (1.0); 4.2727 (0.4); 4.2657 (0.1); 4.2392 (0.2); 3.8222 (0.1); 3.8037 (0.4); 3.7852 (0.4); 3.7667 (0.1); 3.7260 (1.9); 3.2963 (16.0); 2.5091 (0.8); 2.5046 (1.7); 2.5000 (2.3); 2.4954 (1.6); 2.4908 (0.7); 1.2670 (0.2); 1.2233 (0.4); 1.2048 (0.9); 1.1863 (0.4); - 0.0004 (0.5) |
| | 609 [M+HCOO]⁻ | |
| **84** | 581 [M+H]⁺ | (400.1 MHz, d6-DMSO): δ= 9.6255 (0.1); 9.6227 (0.1); 8.4790 (0.2); 8.3143 (0.1); 8.1803 (0.1); 8.1735 (0.1); 7.3160 (0.1); 7.3092 (0.1); 4.2398 (0.5); 4.1156 (0.5); 4.0477 (0.1); 4.0292 (0.1); 3.2950 (16.0); 3.0187 (0.7); 2.5084 (0.8); 2.5038 (1.7); 2.4992 (2.4); 2.4946 (1.7); 2.4900 (0.8); 1.2440 (0.1); 1.2255 (0.3); 1.2070 (0.1); -0.0004 (0.4) |
| **85** | 524 [M+H]⁺ | (400.1 MHz, d6-DMSO): δ= 9.4692 (0.3); 9.4672 (0.3); 9.2804 (0.4); 9.2782 (0.4); 8.3300 (0.2); 8.3275 (0.3); 8.3249 (0.2); 8.1773 (0.3); 8.1702 (0.3); 7.4734 (0.3); 7.4664 (0.3); 4.3461 (0.2); 4.3249 (0.4); 4.2893 (0.2); 4.2682 (0.1); 3.7438 (0.3); 3.7252 (0.3); 3.7068 (0.1); 3.2949 (16.0); 2.5086 (1.0); 2.5040 (2.1); 2.4994 (3.0); 2.4949 (2.1); 2.4903 (1.0); 1.8496 (0.2); 1.8269 (0.1); 1.2419 (0.4); 1.2234 (0.8); 1.2049 (0.4); -0.0004 (0.4) |
| **86** | 523 [M+H]⁺ | (400.1 MHz, d6-DMSO): δ= 8.8394 (0.3); 8.8215 (0.3); 8.4679 (0.8); 8.2453 (0.6); 8.2385 (0.6); 8.0760 (0.4); 7.4076 (0.6); 7.4007 (0.6); 7.1995 (0.2); 7.1964 (0.2); 7.1816 (0.2); 7.1783 (0.2); 4.2736 (0.4); 4.2532 (0.8); 4.2156 (0.4); 4.1951 (0.2); 4.1536 (0.2); 4.1350 (0.6); 4.1165 (0.6); 4.0980 (0.2); 3.2965 (16.0); 2.5092 (0.9); 2.5046 (2.0); 2.5000 (2.8); 2.4954 (2.0); 2.4908 (0.9); 2.0699 (2.4); 1.8383 (0.2); 1.8161 (0.4); 1.7935 (0.2); 1.2601 (0.7); 1.2416 (1.6); 1.2231 (0.7); -0.0004 (0.7) |
| **87** | 524 [M+H]⁺ | (400.0 MHz, d6-DMSO): δ= 9.3044 (1.4); 9.2864 (1.5); 8.4913 (1.8); 8.4893 (1.8); 8.2842 (2.7); 8.2774 (2.8); 7.5389 (0.9); 7.5348 (1.0); 7.5210 (0.9); 7.5168 (1.0); 7.3825 (2.7); 7.3757 (2.7); 4.3181 (1.7); 4.2973 (3.5); 4.2600 (2.0); 4.2391 (1.0); 3.9066 (0.8); 3.8881 (3.0); 3.8696 (3.0); 3.8511 (0.9); 3.3718 (16.0); 3.1694 (1.4); 2.6736 (0.1); 2.6690 (0.2); 2.6645 (0.1); 2.5391 (0.1); 2.5363 (0.1); 2.5224 (0.2); 2.5091 (8.4); 2.5045 (18.1); 2.4999 (25.3); 2.4953 (17.9); 2.4908 (8.2); 2.3314 (0.1); 2.3267 (0.1); 2.3223 (0.1); 1.8563 (0.8); 1.8341 (2.0); 1.8115 (1.0); 1.2583 (3.1); 1.2398 (7.3); 1.2213 (3.1); 0.0078 (0.2); -0.0004 (6.8); -0.0087 (0.2) |
| **88** | 534 [M+H]⁺ | (400.0 MHz, d6-DMSO): δ= 8.7668 (1.1); 8.7621 (1.2); 8.5378 (1.2); 8.5330 (1.2); 8.2120 (1.6); 8.2052 (1.6); 7.2565 (1.6); 7.2498 (1.6); 5.1418 (0.2); 5.1254 (0.4); 5.1090 (0.2); 5.0019 (0.2); 4.9855 (0.4); 4.9690 (0.2); 4.1579 (3.0); 4.1344 (3.1); 4.0580 (0.4); 4.0402 (1.1); 4.0224 (1.1); 4.0046 (0.4); 3.8253 (0.5); 3.8069 (1.7); 3.7883 (1.7); 3.7698 (0.5); 3.7243 (8.7); 3.3035 (16.0); 2.7446 (0.2); 2.7371 (0.2); 2.7277 (0.3); 2.7206 (0.5); 2.7116 (0.4); 2.7040 (0.4); 2.6949 (0.5); 2.6880 (0.4); 2.6790 (0.2); 2.6711 (0.3); 2.5110 (1.3); 2.5064 (2.9); 2.5018 (4.0); 2.4972 (3.0); 2.4927 (1.5); 2.4786 (0.3); 2.4696 (0.3); 2.4611 (0.3); 2.4523 (0.2); 2.4447 (0.3); 2.4397 (0.4); 2.4318 (0.2); 2.4235 (0.3); 2.4145 (0.3); 2.4060 (0.2); 2.3976 (0.2); 2.3900 (0.2); 1.9885 (4.9); 1.3972 (0.1); 1.2355 (0.1); 1.2186 (1.8); 1.2002 (4.2); 1.1935 (1.6); 1.1816 (1.8); 1.1757 (2.8); 1.1579 (1.3); -0.0004 (0.2) |
| **89** | 566 [M+H]⁺ | (300 MHz, DMSO*-d*₆*,ppm*) δ 8.78 (s, 1H), 8.57 (s, 1H), 8.40 (s, 1H), 7.46 (d, *J* = 2.7 Hz, 1H), 4.65 (d, *J =* 9.6 Hz, 2H), 4.38 (d, *J* = 9.9 Hz, 2H), 4.17 (t, *J* = 8.7 Hz, 2H), 3.80 (q, *J* = 7.5 Hz, 2H), 3.32 (s, 3H), 2.45 (d, *J* = 8.1 Hz, 1H), 1.22 (t, *J =* 7.5 Hz, 3H) |
| **90** | 463 [M+H]⁺ | (400.0 MHz, d6-DMSO): δ=8.2128 (0.7); 8.2060 (0.8); 7.8036 (1.3); 7.7075 (1.3); 7.2641 (0.7); 7.2573 (0.8); 4.1907 (4.0); 4.0385 (0.2); 4.0207 (0.2); 3.8152 (0.2); 3.7967 (0.8); 3.7782 (0.8); 3.7598 (0.2); 3.6328 (4.0); 3.2990 (16.0); 2.5086 (2.7); 2.5041 (5.8); 2.4995 (8.0); 2.4950 (5.8); 2.4904 (2.7); 1.9869 (0.9); 1.3979 (0.3); 1.1947 (0.9); 1.1762 (2.0); 1.1574 (1.0); 0.7324 (3.6); 0.0078 (0.1); -0.0004 (3.4); -0.0087 (0.1) |
| **91** | 508 [M+H]⁺ | (400.0 MHz, d6-DMSO): δ=8.2144 (0.8); 8.2076 (0.8); 7.3208 (1.1); 7.2517 (0.8); 7.2448 (0.8); 4.1897 (3.9); 4.0564 (0.1); 4.0386 (0.4); 4.0208 (0.4); 4.0030 (0.1); 3.8209 (4.0); 3.7247 (0.2); 3.7061 (0.8); 3.6876 (0.8); 3.6692 (0.2); 3.2988 (16.0); 3.0828 (0.1); 3.0737 (0.2); 3.0647 (0.1); 2.5088 (2.3); 2.5043 (5.0); 2.4997 (7.0); 2.4951 (5.0); 2.4906 (2.3); 1.9870 (1.7); 1.3978 (0.2); 1.2354 (0.1); 1.1923 (0.7); 1.1883 (1.1); 1.1746 (1.3); 1.1700 (2.3); 1.1568 (0.7); 1.1515 (0.9); 0.9751 (0.4); 0.7292 (3.6); -0.0004 (3.2); -0.0087 (0.1) |
| **92** | 534 [M+H]⁺ | (300 MHz, DMSO*-d*₆*,ppm*) δ 8.78 (d, *J* = 2.1 Hz, 1H), 8.55 (d, *J=* 2.1Hz, 1H), 8.24 (d, *J* = 2.7 Hz, 1H), 7.30 (d, *J* = 2.7 Hz, 1H), 4.42 (d, *J* = 9.3 Hz, 2H), 4.31 (d, *J =* 9.3 Hz, 2H), 3.82 (d, *J* = 7.2 Hz, 2H), 3.75 (s, 3H), 3.24 (t, *J =* 7.5 Hz, 2H), 3.10 (t*, J =* 7.5 Hz, 2H), 1.20 (t*, J =* 7.5 Hz, 3H) |
| **93** | 565 [M+H]⁺ | |
| **93a** | 565 [M+H]⁺ | (300 MHz, DMSO*-d*₆*, ppm*) δ 8.78 (d, *J* = 2.1 Hz, 1H), 8.57 (d, *J* = 2.1 Hz, 1H), 8.37 (d, *J* = 2.7 Hz, 1H), 7.41 (d, *J =* 2.7 Hz, 1H), 4.83 (s, 1H), 4.68-4.52 (m, 2H), 4.33 (dd, *J* = 15.3, 9.7 Hz, 2H), 3.87-3.82 (m, 3H), 3.74 (s, 3H), 2.45-2.42 (m, 1H), 1.22 (t, *J =* 7.5 Hz, 3H) |
| **93b** | 565 [M+H]⁺ | (300 MHz, DMSO-d6, ppm) δ 8.78 (d, J = 2.1 Hz, 1H), 8.57 (d, J = 2.1 Hz, 1H), 8.37 (d, *J =* 2.7 Hz, 1H), 7.41 (d, *J =* 2.7 Hz, 1H), 4.83 (s, 1H), 4.63 (d, *J* = 9.3 Hz, 1H), 4.57 (d, *J=* 9.3 Hz, 1H), 4.33 (dd, *J =* 15.3, 9.7 Hz, 2H), 4.03-3.77 (m, 3H), 3.74 (s, 3H), 2.48-2.36 (m, 1H), 1.22 (t, *J* = 7.5 Hz, 3H) |
| **94** | 530 [M+H]⁺ | (300 MHz, DMSO*-d*₆*,ppm*) δ 8.82-8.75 (m, 2H), 8.56 (d, *J* = 2.1 Hz, 1H), 7.75 (d, *J =* 2.7 Hz, 1H), 3.83 (q, *J =* 7.5 Hz, 2H), 3.76 (s, 3H), 3.59 (dd, *J* = 6.6, 4.2 Hz, 4H), 1.51 (dd, *J =* 6.6, 4.2Hz, 4H), 1.22 (t, *J =* 7.4 Hz, 3H), 0.40 (s, 4H) |
| **95** | 580 [M+H]⁺ | (300 MHz, DMSO*-d*₆*,ppm*) δ 8.79 (t, *J* = 2.1 Hz, 2H), 8.57 (d, *J* = 2.1 Hz, 1H), 7.76 (d, *J =* 2.7 Hz, 1H), 3.82 (q, *J* = 7.5 Hz, 2H), 3.75 (s, 3H), 3.50 (t, *J* = 5.7 Hz, 4H), 2.46 (d, *J =* 12.9 Hz, 3H), 1.77 (t, *J =* 5.4 Hz, 4H), 1.22 (t*, J* = 7.5 Hz, 3H) |
| **96** | 544 [M+H]⁺ | (300 MHz, DMSO*-d*₆*,ppm*) δ 8.77 (dd, *J* = 4.2, 2.4 Hz, 2H), 8.56 (d, *J =* 2.1 Hz, 1H), 7.73 (d, *J =* 2.7 Hz, 1H), 3.82 (q*, J =* 7.5 Hz, 2H), 3.75 (s, 3H), 3.45 (t, *J =* 5.7 Hz, 4H), 1.95-1.77 (m, 6H), 1.70 (t, *J* = 5.6 Hz, 4H), 1.21 (t, *J* = 7.4 Hz, 3H) |
| **97** | 566 [M+H]⁺ | (300 MHz, DMSO*-d*₆*,ppm*) δ 8.80 (dd, *J* = 6.3, 2.4 Hz, 2H), 8.57 (d, *J =* 2.1 Hz, 1H), 7.79 (d, *J =* 2.7 Hz, 1H), 3.83 (q, *J =* 7.5 Hz, 2H), 3.76 (s, 3H), 3.67 (q, *J =* 6.6, 4.5 Hz, 2H), 3.56 (td, *J* = 9.0, 8.4, 3.9 Hz, 2H), 1.85-1.77 (m, 2H), 1.69-1.64 (m, 2H), 1.42 (t, *J* = 8.7 Hz, 2H), 1.22 (t, *J* = 7.4 Hz, 3H) |
| **98** | 530 [M+H]⁺ | (300 MHz, DMSO*-d*₆*, ppm*) δ 9.22 (d, *J* = 2.4 Hz, 1H), 8.98 (d, *J* = 2.4 Hz, 1H), 8.83 (d, *J=* 2.1 Hz, 1H), 8.64 (d, *J =* 2.1 Hz, 1H), 3.99 (s, 2H), 3.84 (q, *J* = 7.2 Hz, 2H), 3.77 (s, 3H), 2.71 (s, 2H), 1.20 (dt, *J* = 12.6, 7.2 Hz, 3H), 0.77 (s, 4H) |
| **99** | 502 [M+H]⁺ | (400.0 MHz, d6-DMSO): δ=8.7003 (0.9); 8.6959 (0.9); 8.3230 (0.7); 8.3222 (0.6); 8.3013 (1.3); 8.3004 (1.3); 8.2620 (0.6); 8.2576 (0.6); 8.2403 (0.3); 8.2358 (0.3); 8.2220 (1.2); 8.2153 (1.2); 7.3067 (1.2); 7.3000 (1.2); 4.2490 (5.9); 3.9742 (0.4); 3.9556 (1.4); 3.9371 (1.4); 3.9186 (0.4); 3.3145 (16.0); 3.1697 (0.4); 2.5096 (2.9); 2.5050 (6.3); 2.5004 (8.7); 2.4958 (6.2); 2.4913 (2.9); 1.2989 (1.4); 1.2804 (3.3); 1.2618 (1.4); 1.2336 (0.1); 0.7452 (5.5); - 0.0004 (0.1) |
| **100** | 595 [M+H]⁺ | (400.0 MHz, d6-DMSO): δ=8.7022 (2.1); 8.6977 (2.1); 8.3223 (1.5); 8.3213 (1.5); 8.3006 (3.1); 8.2996 (3.1); 8.2636 (1.5); 8.2591 (1.5); 8.2419 (0.8); 8.2373 (0.8); 8.2288 (2.8); 8.2222 (2.9); 7.3116 (2.8); 7.3049 (2.8); 7.2003 (0.4); 7.0725 (0.4); 6.9447 (0.4); 4.3415 (11.9); 4.1277 (12.0); 3.9674 (0.9); 3.9489 (3.1); 3.9304 (3.2); 3.9119 (0.9); 3.3981 (8.3); 3.1962 (0.1); 3.1699 (1.0); 3.0259 (16.0); 2.6696 (0.1); 2.5396 (0.1); 2.5230 (0.2); 2.5182 (0.3); 2.5097 (6.2); 2.5051 (13.5); 2.5005 (18.8); 2.4959 (13.3); 2.4914 (6.1); 2.3273 (0.1); 1.2922 (3.4); 1.2737 (7.9); 1.2552 (3.3); 1.2401 (0.1); -0.0004 (0.7) |
| **101** | 527 [M+H]⁺ | (400.1 MHz, d6-DMSO): δ=9.2886 (0.4); 9.2707 (0.4); 8.4722 (0.5); 8.4702 (0.5); 8.1619 (0.8); 8.1551 (0.8); 7.5267 (0.3); 7.5225 (0.3); 7.5087 (0.3); 7.5044 (0.3); 7.2476 (0.8); 7.2409 (0.8); 4.1296 (1.6); 4.1196 (1.6); 4.0391 (0.1); 4.0213 (0.1); 3.8988 (0.3); 3.8804 (0.9); 3.8619 (0.9); 3.8434 (0.3); 3.3477 (0.2); 3.3265 (0.3); 3.3055 (0.3); 3.2945 (16.0); 2.7061 (0.2); 2.7015 (0.2); 2.6840 (0.3); 2.6787 (0.4); 2.6744 (0.5); 2.6526 (0.4); 2.6183 (0.4); 2.5986 (0.4); 2.5923 (0.3); 2.5868 (0.3); 2.5713 (0.1); 2.5670 (0.2); 2.5088 (1.5); 2.5042 (3.2); 2.4996 (4.4); 2.4950 (3.1); 2.4905 (1.5); 1.9868 (0.5); 1.2442 (1.0); 1.2346 (0.1); 1.2257 (2.2); 1.2072 (0.9); 1.1925 (0.2); 1.1747 (0.3); 1.1569 (0.2) |
| **102** | 526 [M+H]⁺ | (400.0 MHz, d6-DMSO): δ=8.8703 (2.9); 8.8524 (3.1); 8.4925 (8.2); 8.1375 (5.5); 8.1307 (5.7); 8.0721 (4.2); 7.2864 (6.1); 7.2796 (6.1); 7.2424 (2.2); 7.2243 (2.2); 4.8185 (0.9); 4.4447 (0.2); 4.2731 (0.2); 4.2625 (0.2); 4.2430 (0.1); 4.2244 (0.1); 4.0934 (13.1); 4.0826 (13.4); 4.0697 (6.1); 4.0512 (5.8); 4.0328 (1.8); 3.9821 (0.1); 3.9085 (0.1); 3.8974 (0.1); 3.3642 (0.5); 3.3439 (1.7); 3.3227 (2.7); 3.3022 (1.8); 3.2811 (0.6); 3.1701 (1.5); 2.6962 (1.7); 2.6917 (1.2); 2.6742 (2.3); 2.6692 (3.0); 2.6646 (3.8); 2.6427 (3.2); 2.6094 (3.6); 2.5897 (3.6); 2.5833 (2.4); 2.5779 (2.0); 2.5623 (1.1); 2.5581 (1.7); 2.5396 (0.3); 2.5231 (0.4); 2.5183 (0.6); 2.5096 (13.4); 2.5051 (29.4); 2.5005 (41.3); 2.4959 (29.3); 2.4913 (13.6); 2.3318 (0.2); 2.3274 (0.3); 2.3227 (0.2); 1.2328 (7.0); 1.2143 (16.0); 1.1958 (6.8); 1.1683 (0.1); 1.0499 (0.1); 0.0078 (0.5); -0.0004 (17.6); -0.0088 (0.5) |
| **103** | 527 [M+H]⁺ | (400.1 MHz, d6-DMSO): δ=9.6230 (0.4); 9.6208 (0.4); 8.4704 (0.6); 8.3104 (0.4); 8.1463 (0.4); 8.1395 (0.4); 7.2824 (0.4); 7.2756 (0.4); 4.0981 (0.8); 4.0868 (0.8); 4.0591 (0.1); 4.0406 (0.5); 4.0220 (0.5); 4.0037 (0.1); 3.3439 (0.1); 3.3226 (0.2); 3.2936 (16.0); 2.6973 (0.1); 2.6692 (0.2); 2.6656 (0.3); 2.6438 (0.2); 2.6103 (0.2); 2.5906 (0.2); 2.5843 (0.2); 2.5788 (0.1); 2.5590 (0.1); 2.5087 (1.9); 2.5042 (4.0); 2.4996 (5.5); 2.4950 (3.9); 2.4905 (1.8); 1.9869 (0.1); 1.2467 (0.1); 1.2400 (0.5); 1.2216 (1.1); 1.2030 (0.5); 1.1081 (0.1); 1.0930 (0.1); 0.9480 (0.1); 0.9330 (0.1); 0.0000 (0.1) |
| **104** | 541 [M+H]⁺ | (400.0 MHz, d6-DMSO): δ=8.6953 (1.6); 8.6909 (1.6); 8.3156 (1.1); 8.2939 (2.4); 8.2588 (1.2); 8.2543 (1.1); 8.2371 (0.6); 8.2326 (0.6); 8.1908 (2.1); 8.1842 (2.2); 7.2732 (2.1); 7.2665 (2.1); 4.2000 (4.0); 4.1929 (4.1); 3.9604 (0.7); 3.9418 (2.4); 3.9233 (2.5); 3.9048 (0.7); 3.5595 (0.1); 3.5465 (0.1); 3.4265 (16.0); 3.3696 (0.8); 3.3491 (0.9); 3.3278 (1.1); 3.3074 (0.7); 3.2863 (0.3); 3.1691 (1.9); 2.7113 (0.5); 2.7070 (0.4); 2.6894 (0.7); 2.6843 (0.9); 2.6797 (1.2); 2.6686 (0.4); 2.6580 (1.1); 2.6278 (1.2); 2.6081 (1.2); 2.6018 (0.8); 2.5964 (0.6); 2.5766 (0.6); 2.5388 (0.3); 2.5222 (0.4); 2.5173 (0.7); 2.5088 (14.1); 2.5043 (30.6); 2.4997 (42.6); 2.4951 (30.0); 2.4905 (13.7); 2.3310 (0.2); 2.3264 (0.3); 2.3218 (0.2); 2.0705 (0.2); 1.2875 (2.6); 1.2690 (6.0); 1.2504 (2.5); 0.0078 (0.2); -0.0004 (7.7); -0.0088 (0.2) |
| **105** | 595 [M+H]⁺ | (400.1 MHz, d6-DMSO): δ=8.7716 (0.5); 8.7668 (0.5); 8.5414 (0.5); 8.5366 (0.5); 8.2981 (0.7); 8.2914 (0.7); 7.3411 (0.7); 7.3344 (0.7); 4.2594 (0.5); 4.2383 (0.9); 4.1953 (0.9); 4.1742 (0.5); 3.8245 (0.2); 3.8061 (0.7); 3.7875 (0.7); 3.7691 (0.2); 3.7300 (5.0); 3.5258 (1.8); 3.2935 (16.0); 2.6090 (3.6); 2.5088 (1.4); 2.5043 (3.1); 2.4997 (4.3); 2.4951 (3.1); 2.4905 (1.4); 1.2266 (0.8); 1.2081 (1.7); 1.1896 (0.7); -0.0004 (0.5) |
| **106** | 507 [M+H]⁺ | (400.1 MHz, d6-DMSO): δ=8.1581 (0.4); 8.1513 (0.4); 7.7940 (0.7); 7.6970 (0.8); 7.2170 (0.4); 7.2102 (0.4); 4.1052 (0.8); 4.0595 (0.8); 3.8262 (0.1); 3.8092 (0.2); 3.8005 (0.1); 3.7921 (0.2); 3.7820 (0.4); 3.7635 (0.4); 3.7450 (0.1); 3.6175 (2.2); 3.2945 (16.0); 3.1411 (3.1); 2.5652 (0.1); 2.5580 (0.1); 2.5484 (0.1); 2.5408 (0.2); 2.5333 (0.2); 2.5230 (0.1); 2.5166 (0.2); 2.5084 (1.0); 2.5039 (2.2); 2.4993 (3.0); 2.4947 (2.1); 2.4902 (1.0); 2.1320 (0.2); 2.1247 (0.1); 2.1148 (0.2); 2.1074 (0.2); 2.1001 (0.1); 2.0828 (0.1); 1.9865 (0.4); 1.1925 (0.1); 1.1851 (0.5); 1.1747 (0.3); 1.1666 (1.1); 1.1569 (0.1); 1.1481 (0.5); -0.0004 (0.2) |
| **107** | 532 [M+H]⁺ | (400.1 MHz, d6-DMSO): δ=9.2877 (0.2); 9.2697 (0.2); 8.4705 (0.3); 8.4685 (0.3); 8.1416 (0.5); 8.1349 (0.5); 7.5252 (0.2); 7.5210 (0.2); 7.5073 (0.2); 7.5030 (0.2); 7.2336 (0.5); 7.2268 (0.5); 4.0984 (0.9); 4.0522 (0.9); 3.8993 (0.1); 3.8808 (0.5); 3.8623 (0.5); 3.8438 (0.2); 3.8265 (0.1); 3.8095 (0.2); 3.7924 (0.2); 3.2958 (16.0); 3.1413 (3.6); 2.5656 (0.1); 2.5585 (0.1); 2.5489 (0.2); 2.5412 (0.2); 2.5337 (0.2); 2.5235 (0.1); 2.5170 (0.2); 2.5090 (0.9); 2.5044 (2.0); 2.4998 (2.7); 2.4952 (1.9); 2.4906 (0.9); 2.1312 (0.2); 2.1238 (0.1); 2.1139 (0.2); 2.1065 (0.2); 2.0993 (0.2); 2.0891 (0.1); 2.0819 (0.1); 1.2438 (0.5); 1.2253 (1.3); 1.2068 (0.5) |
| **108** | 541 [M+H]⁺ | (400.0 MHz, d6-DMSO): δ=8.7667 (0.8); 8.7618 (0.9); 8.5370 (0.9); 8.5321 (0.8); 8.2207 (1.2); 8.2139 (1.2); 7.2632 (1.2); 7.2565 (1.2); 4.1667 (2.2); 4.1572 (2.3); 3.8199 (0.3); 3.8014 (1.2); 3.7829 (1.3); 3.7644 (0.4); 3.7202 (6.4); 3.5402 (0.2); 3.4614 (16.0); 3.3743 (0.2); 3.3541 (0.4); 3.3329 (0.5); 3.3125 (0.4); 3.2914 (0.1); 2.7137 (0.3); 2.7093 (0.2); 2.6916 (0.4); 2.6865 (0.5); 2.6821 (0.7); 2.6603 (0.6); 2.6283 (0.6); 2.6086 (0.6); 2.6022 (0.4); 2.5968 (0.3); 2.5770 (0.3); 2.5104 (2.3); 2.5058 (5.1); 2.5012 (7.1); 2.4966 (5.0); 2.4920 (2.3); 2.0708 (0.5); 1.2169 (1.3); 1.1984 (3.1); 1.1799 (1.3); - 0.0004 (0.1) |
| | 585 [M+HCOO]⁻ | |
| **109** | 559 [M+H]⁺ | (400.1 MHz, d6-DMSO): δ=8.7686 (0.8); 8.7639 (0.8); 8.5378 (0.8); 8.5330 (0.8); 8.2501 (1.1); 8.2433 (1.1); 7.2972 (1.1); 7.2905 (1.1); 4.3457 (1.7); 4.2875 (4.8); 4.0670 (1.6); 3.8254 (0.3); 3.8069 (1.2); 3.7884 (1.2); 3.7699 (0.4); 3.7232 (6.1); 3.2987 (16.0); 3.1701 (0.2); 2.5090 (2.8); 2.5044 (6.1); 2.4998 (8.5); 2.4952 (6.1); 2.4906 (2.9); 1.7673 (5.1); 1.2211 (1.2); 1.2027 (2.9); 1.1841 (1.2); 0.8989 (0.1); 0.8812 (0.2) |
| **110** | 595 [M+H]⁺ | (400.0 MHz, d6-DMSO): δ=8.7710 (0.2); 8.7664 (0.2); 8.5422 (0.2); 8.5375 (0.2); 8.2599 (0.2); 8.2532 (0.2); 7.3018 (0.2); 7.2951 (0.2); 4.3072 (1.1); 4.1288 (1.1); 4.0387 (0.1); 4.0209 (0.1); 3.8094 (0.3); 3.7909 (0.3); 3.7732 (0.1); 3.7234 (1.3); 3.3009 (16.0); 3.0270 (1.3); 2.5088 (1.2); 2.5043 (2.5); 2.4998 (3.4); 2.4953 (2.4); 2.4909 (1.1); 1.9870 (0.6); 1.2212 (0.3); 1.2028 (0.6); 1.1924 (0.2); 1.1843 (0.3); 1.1746 (0.3); 1.1568 (0.2); -0.0004 (0.9) |
| **111** | 609 [M+H]⁺ | (400.1 MHz, d6-DMSO): δ=8.7766 (2.1); 8.7718 (2.2); 8.5453 (2.2); 8.5404 (2.1); 8.2627 (2.9); 8.2559 (3.0); 7.3097 (2.9); 7.3029 (2.9); 4.3165 (12.8); 4.1298 (12.9); 3.8331 (0.9); 3.8146 (3.1); 3.7961 (3.1); 3.7776 (0.9); 3.7305 (16.0); 3.5507 (0.1); 3.4123 (13.6); 3.3133 (0.1); 3.1785 (2.0); 3.1616 (4.2); 3.1432 (4.3); 3.1248 (1.3); 2.5304 (0.1); 2.5255 (0.2); 2.5171 (4.4); 2.5125 (9.5); 2.5079 (13.2); 2.5033 (9.4); 2.4987 (4.3); 1.2621 (4.5); 1.2437 (9.9); 1.2287 (3.8); 1.2254 (5.0); 1.2104 (7.7); 1.1918 (3.2) |
| **112** | 532 [M+H]⁺ | (400.1 MHz, d6-DMSO): δ=9.4572 (0.1); 9.4551 (0.1); 9.2465 (0.1); 9.2442 (0.1); 8.3163 (0.1); 8.3137 (0.1); 8.3110 (0.1); 8.0282 (0.1); 8.0211 (0.1); 7.3101 (0.1); 7.3031 (0.1); 4.1276 (0.2); 4.0832 (0.2); 3.8290 (0.0); 3.8121 (0.1); 3.7951 (0.0); 3.7432 (0.0); 3.7247 (0.1); 3.7062 (0.1); 3.6878 (0.0); 3.3675 (0.0); 3.3614 (0.0); 3.3095 (16.0); 3.1432 (0.8); 2.5704 (0.0); 2.5634 (0.0); 2.5537 (0.0); 2.5461 (0.0); 2.5387 (0.0); 2.5287 (0.0); 2.5220 (0.0); 2.5182 (0.0); 2.5093 (0.4); 2.5047 (0.8); 2.5001 (1.2); 2.4955 (0.8); 2.4909 (0.4); 2.1386 (0.0); 2.1313 (0.0); 2.1214 (0.0); 2.1140 (0.0); 2.1067 (0.0); 2.0966 (0.0); 2.0894 (0.0); 1.5658 (0.0); 1.2297 (0.0); 1.2240 (0.1); 1.2056 (0.3); 1.1870 (0.1); -0.0004 (0.2) |
| | 576 [M+HCOO]⁻ | |
| **113** | 532 [M+H]⁺ | (400.0 MHz, d6-DMSO): δ=9.6211 (1.5); 9.4363 (0.1); 8.4692 (2.3); 8.3743 (0.1); 8.3710 (0.2); 8.3110 (1.6); 8.1948 (0.1); 8.1865 (0.4); 8.1271 (1.6); 8.1203 (1.6); 7.3090 (0.1); 7.3021 (0.1); 7.2714 (1.6); 7.2646 (1.6); 4.1064 (0.3); 4.0666 (3.4); 4.0407 (1.8); 4.0212 (4.2); 4.0038 (0.6); 3.8396 (0.1); 3.8225 (0.5); 3.8129 (0.2); 3.8054 (0.8); 3.7962 (0.1); 3.7884 (0.6); 3.7712 (0.1); 3.4309 (16.0); 3.3117 (0.1); 3.1699 (1.1); 3.1381 (11.8); 3.1088 (0.2); 3.0930 (0.2); 3.0905 (0.2); 2.5561 (0.6); 2.5489 (0.5); 2.5394 (0.7); 2.5316 (0.8); 2.5238 (0.8); 2.5095 (4.4); 2.5051 (9.1); 2.5005 (12.3); 2.4959 (8.7); 2.4914 (4.0); 2.1227 (0.6); 2.1154 (0.4); 2.1055 (0.7); 2.0981 (0.7); 2.0907 (0.6); 2.0806 (0.4); 2.0734 (0.5); 1.2393 (1.9); 1.2208 (4.3); 1.2022 (1.8); 1.0325 (0.2); 1.0141 (0.4); 0.9957 (0.2); -0.0004 (0.4) |
| **114** | 594 [M+H]⁺ | (300 MHz, DMSO*-d*₆*, ppm*) δ 8.78 (d, *J =* 2.1 Hz, 1H), 8.56 (d, *J =* 2.1 Hz, 1H), 8.23 (d, *J* = 2.7 Hz, 1H), 7.27 (d, *J* = 2.7 Hz, 1H), 4.19 (s, 2H), 4.14 (s, 2H), 3.94 (q, *J* = 7.8 Hz, 1H), 3.81 (q, *J =* 7.5 Hz, 2H), 3.73 (s, 3H), 2.93 (s, 3H), 2.73-2.54 (m, 4H), 1.21 (t, *J =* 7.5 Hz, 3H) |
| **115** | 608 [M+H]⁺ | (300 MHz, DMSO*-d*₆*,ppm*) δ 8.78 (d, *J* = 2.1 Hz, 1H), 8.56 (d, *J* = 2.1Hz, 1H), 8.23 (d, *J =* 2.7 Hz, 1H), 7.27 (d, *J =* 2.7 Hz, 1H), 4.20 (s, 2H), 4.13 (s, 2H), 3.99 (q, *J =* 7.8 Hz, 1H), 3.81 (q, *J* = 7.5 Hz, 2H), 3.73 (s, 3H), 3.04 (q, *J* = 7.5 Hz, 2H), 2.63 (d, *J* = 7.8 Hz, 4H), 1.21 (td, *J* = 7.5, 1.3 Hz, 6H) |
| **116** | 582 [M+H]⁺ | (300 MHz, Chloroform-*d,ppm*) δ 8.69 (s, 1H), 8.27 (s, 1H), 8.09 (d, *J =* 2.7 Hz, 1H), 7.38 (d, *J* = 2.7 Hz, 1H), 4.27 (s, 4H), 3.91-3.79 (m, 5H), 3.78 (s, 4H), 1.38 (t, *J* = 7.5 Hz, 3H), 1.29-1.19 (m, 2H), 1.12 (s, 2H) |
| **117** | 579 [M+H]⁺ | (300 MHz, DMSO-d6, *ppm*) δ 8.78 (d, *J* = 2.1 Hz, 1H), 8.56 (d, *J =* 2.1 Hz, 1H), 8.28 (d, *J* = 2.7 Hz, 1H), 7.32 (d, *J* = 2.7 Hz, 1H), 4.25 (t, *J* = 8.4 Hz, 2H), 4.11-4.06 (m, 2H), 4.02-3.76 (m, 4H), 3.73 (s, 3H), 3.65-3.60 (m, 2H), 3.15 (s, 3H), 1.21 (t, *J* = 7.4 Hz, 3H) |
| **118** | 473 [M+H]⁺ | (400.0 MHz, d6-DMSO): δ=9.5153 (5.1); 9.5131 (5.6); 9.4182 (7.9); 9.4159 (8.2); 8.7765 (5.9); 8.7711 (6.1); 8.7551 (0.1); 8.3899 (4.1); 8.3873 (6.7); 8.3846 (4.3); 8.2460 (6.0); 8.2406 (6.0); 5.3529 (0.1); 3.9376 (0.7); 3.9192 (1.7); 3.9008 (2.4); 3.8839 (4.1); 3.8654 (4.7); 3.8556 (3.4); 3.8467 (4.0); 3.8372 (6.3); 3.8188 (6.3); 3.8019 (4.0); 3.7835 (2.7); 3.7652 (1.3); 3.1788 (0.4); 3.0467 (0.3); 2.9522 (0.1); 2.7360 (1.6); 2.7252 (2.0); 2.7161 (2.0); 2.7052 (1.8); 2.6828 (0.1); 2.6782 (0.1); 2.5482 (0.1); 2.5317 (0.2); 2.5182 (6.4); 2.5137 (14.0); 2.5091 (19.7); 2.5045 (14.1); 2.4999 (6.6); 2.3360 (0.1); 2.0848 (0.3); 1.8150 (1.6); 1.8036 (1.8); 1.7951 (1.7); 1.7837 (1.6); 1.4209 (1.8); 1.4098 (3.6); 1.3987 (1.8); 1.2650 (7.0); 1.2465 (16.0); 1.2280 (6.9); 1.1127 (0.3); 1.1052 (0.9); 1.0996 (1.0); 1.0924 (2.1); 1.0740 (4.7); 1.0644 (9.0); 1.0506 (1.1); 1.0314 (0.3); 1.0242 (0.2); 0.8347 (0.2); 0.7861 (0.1); 0.7558 (1.7); 0.7447 (1.2); 0.7317 (1.0); 0.7257 (1.0) |
| **118a** | 473 [M+H]⁺ | (400.0 MHz, d6-DMSO): δ=9.5067 (0.7); 9.5044 (0.7); 9.4100 (1.0); 9.4078 (1.1); 8.7684 (0.8); 8.7630 (0.8); 8.3823 (0.5); 8.3797 (0.9); 8.3770 (0.6); 8.2374 (0.8); 8.2320 (0.8); 3.9103 (0.2); 3.8919 (0.2); 3.8750 (0.4); 3.8565 (0.3); 3.8470 (0.1); 3.8380 (0.1); 3.8285 (0.3); 3.8100 (0.4); 3.7930 (0.2); 3.7747 (0.2); 3.3005 (16.0); 2.7281 (0.2); 2.7174 (0.3); 2.7084 (0.3); 2.6974 (0.2); 2.5094 (1.5); 2.5048 (3.3); 2.5003 (4.6); 2.4957 (3.3); 2.4911 (1.5); 1.8071 (0.2); 1.7957 (0.2); 1.7872 (0.2); 1.7758 (0.2); 1.4133 (0.2); 1.4021 (0.5); 1.3910 (0.2); 1.3523 (0.1); 1.2563 (0.9); 1.2378 (2.1); 1.2193 (0.9); 1.0975 (0.1); 1.0917 (0.1); 1.0846 (0.3); 1.0663 (0.6); 1.0565 (1.2); 1.0425 (0.1); 0.7476 (0.2); 0.7364 (0.2); 0.7233 (0.1); 0.7173 (0.1); -0.0004 (0.5) |
| **118b** | 473 [M+H]⁺ | (400.0 MHz, d6-DMSO): δ=9.5070 (1.2); 9.5048 (1.3); 9.4103 (2.0); 9.4080 (2.0); 8.7686 (1.5); 8.7631 (1.5); 8.3825 (1.0); 8.3798 (1.6); 8.3770 (1.1); 8.2376 (1.5); 8.2322 (1.5); 7.2037 (0.3); 7.0759 (0.3); 6.9481 (0.3); 3.9105 (0.3); 3.8922 (0.4); 3.8753 (0.7); 3.8567 (0.7); 3.8472 (0.2); 3.8383 (0.2); 3.8287 (0.7); 3.8102 (0.7); 3.7933 (0.4); 3.7749 (0.3); 3.3072 (16.0); 2.7283 (0.4); 2.7175 (0.5); 2.7085 (0.5); 2.6975 (0.4); 2.5183 (0.1); 2.5097 (2.5); 2.5051 (5.4); 2.5005 (7.6); 2.4959 (5.4); 2.4913 (2.5); 1.8072 (0.4); 1.7958 (0.4); 1.7873 (0.4); 1.7759 (0.4); 1.4133 (0.5); 1.4022 (0.9); 1.3911 (0.4); 1.3525 (0.2); 1.2566 (1.8); 1.2381 (4.1); 1.2195 (1.7); 1.0976 (0.2); 1.0917 (0.2); 1.0846 (0.5); 1.0663 (1.1); 1.0566 (2.2); 1.0427 (0.3); 0.7479 (0.4); 0.7366 (0.3); 0.7235 (0.2); 0.7175 (0.2); -0.0004 (0.9) |
| **119** | 584 [M+NH₄]⁺ | (400.1 MHz, d6-DMSO): δ=8.8365 (2.2); 8.8319 (2.3); 8.7894 (2.8); 8.7879 (2.8); 8.6499 (2.3); 8.6452 (2.2); 7.8479 (2.8); 7.8456 (2.8); 4.1161 (0.2); 3.7864 (0.2); 3.7707 (0.3); 3.7521 (0.9); 3.7438 (0.2); 3.7339 (1.7); 3.7205 (16.0); 3.6981 (1.4); 3.6908 (0.9); 3.6798 (0.4); 3.6683 (0.3); 3.6312 (0.1); 3.6166 (0.1); 3.6010 (0.4); 3.5825 (1.3); 3.5640 (1.6); 3.5466 (1.5); 3.5401 (1.0); 3.5281 (2.1); 3.5001 (6.6); 3.1793 (0.5); 2.8305 (0.7); 2.8247 (0.9); 2.7991 (1.5); 2.7954 (1.6); 2.7930 (1.7); 2.7645 (0.8); 2.7616 (0.8); 2.6521 (0.8); 2.6466 (0.8); 2.6205 (1.7); 2.6148 (1.6); 2.5887 (0.9); 2.5829 (0.9); 2.5738 (0.8); 2.5681 (0.6); 2.5495 (1.5); 2.5444 (2.0); 2.5293 (1.1); 2.5233 (1.9); 2.5177 (4.0); 2.5131 (7.4); 2.5085 (10.5); 2.5039 (8.1); 2.4996 (4.5); 2.4824 (1.7); 2.4776 (1.9); 2.4529 (0.7); 2.4485 (0.6); 1.2447 (0.4); 1.2332 (0.1); 1.2277 (0.1); 1.2131 (3.4); 1.1947 (7.6); 1.1762 (3.3) |
| **120** | 567 [M+H]⁺, 565 [M-H]⁻ | (400.1 MHz, d6-DMSO): δ=9.0292 (1.9); 9.0245 (2.0); 8.8651 (0.2); 8.8602 (0.2); 8.8366 (0.2); 8.8321 (0.3); 8.7890 (0.3); 8.7874 (0.3); 8.7108 (2.1); 8.7084 (2.1); 8.6506 (0.2); 8.6459 (0.2); 8.3470 (1.9); 8.3428 (1.9); 8.2554 (2.0); 7.8473 (0.3); 7.8449 (0.3); 4.1197 (0.2); 4.1005 (11.9); 3.9177 (0.1); 3.9109 (0.3); 3.8922 (0.1); 3.8663 (0.1); 3.8446 (0.2); 3.8288 (0.5); 3.8222 (0.5); 3.8000 (0.8); 3.7777 (0.6); 3.7615 (0.2); 3.7556 (0.2); 3.7334 (0.3); 3.7194 (2.0); 3.7037 (2.6); 3.6852 (2.6); 3.6668 (0.8); 3.6007 (0.1); 3.5821 (0.2); 3.5636 (0.3); 3.5463 (0.3); 3.5278 (0.3); 3.4415 (16.0); 3.1787 (1.0); 2.8573 (0.6); 2.8515 (0.6); 2.8259 (1.2); 2.8224 (1.2); 2.8199 (1.3); 2.7940 (0.8); 2.7648 (0.1); 2.6820 (0.1); 2.6775 (0.2); 2.6728 (0.1); 2.6618 (0.6); 2.6558 (0.7); 2.6241 (2.0); 2.6003 (1.5); 2.5946 (1.9); 2.5804 (0.9); 2.5738 (1.2); 2.5451 (0.3); 2.5306 (0.4); 2.5175 (7.1); 2.5130 (15.0); 2.5085 (19.6); 2.5040 (14.0); 2.4994 (6.4); 2.4886 (1.8); 2.4823 (1.6); 2.4649 (0.6); 2.4584 (0.8); 2.3353 (0.1); 1.2777 (0.1); 1.2707 (0.2); 1.2524 (0.4); 1.2446 (0.6); 1.2342 (0.2); 1.2250 (0.2); 1.2151 (2.8); 1.1967 (6.3); 1.1782 (2.7) |

### EXAMPLE 3: Screening method to test contact activity of compounds of formula (I), (Ia) and/or (Ib) against adult Ctenocephalides felis

Compounds of formulae (I), (Ia) and/or (Ib) dissolved in 100% DMSO are diluted in acetone to a desired concentration. The resulting formulation is used to coat the fibers of a 0.5-inch-long piece of pipe cleaner placed in a glass scintillation vial. The vial is capped with a rubber gasket and filter paper insert. After the acetone has evaporated, ten adult *Ctenocephalides felis* fleas are added to each vial. The vials are then incubated at 22°C, 80% relative humidity, and 12 hours of light/12 hours of dark until a visual evaluation for mortality is performed at 72 hours post-treatment. Compound efficacy at a given dose is expressed as percentage mortality and adjusted to remove background mortality observed in control vials containing DMSO only. A dose response series of treated vials is implemented to determine EC₅₀ values. Compounds 4, 9, 10, 14, 22, 29, 37, 38, 42, 44, 45, 52, 55, 57, 62, 64, 65, 66, 69, 77, 78, 81, 99, 100, 117 exhibit EC₅₀ values of between 100 µM and 10 µM. Compounds 1, 2, 3, 3a, 3b, 5, 6, 7, 7a, 7b, 8, 8a, 8b, 12, 12a, 12b, 13, 15, 16, 17, 17a, 17b, 18, 19, 20, 21, 23a, 23b, 24, 25, 26, 27, 28, 30, 31, 32, 34, 35, 36, 39, 40, 41, 43, 46, 47, 48, 49, 50, 51, 51a, 51b, 53, 54, 56, 58, 59, 60, 61, 63, 67, 68, 70, 72, 73, 74, 75, 76, 79, 80, 82, 83, 84, 85, 86, 87, 88, 89, 90, 91, 92, 93a, 93b, 94, 95, 96, 97, 98, 101, 102, 103, 104, 105, 106, 107, 108, 109, 110, 111, 112, 113, 114, 115, 116 exhibit EC₅₀ values of less than 10 µM.

### EXAMPLE 4: Screening method to test contact activity of compounds of formula (I), (Ia) and/or (Ib) against adult Rhipicephalus sanguineus

Compounds of formulae (I), (Ia) and/or (Ib) formulated in 100% DMSO are diluted in a solution containing acetone and Triton X-100 (0.02%). The resulting formulation is used to coat the inner walls of glass scintillation vials and the filter paper covering the cap of the vial. Once dried, ten adult *R. sanguineus* ticks are added to the vials. The vials are incubated at 24°C, 95% relative humidity, and 12 hours of light/12 hours of dark until evaluation. Ticks are visually evaluated for mortality at 48 hours post-treatment. Compound efficacy at a given dose is expressed as percentage mortality. A dose response series is implemented to determine EC₅₀ values. Compounds 2, 3, 3b, 4, 5, 6, 8, 8a, 9, 10, 12, 13, 14, 15, 18, 19, 20, 21, 22, 26, 27, 28, 29, 30, 31, 32, 33, 34, 37, 38, 40, 41, 42, 44, 45, 46, 47, 48, 49, 50, 51, 51a, 51b, 52, 53, 55, 56, 58, 60, 61, 62, 63, 64, 65, 66, 67, 68, 69, 73, 75, 76, 77, 78, 79, 81, 82, 83, 84, 89, 90, 92, 93a, 93b, 94, 95, 96, 97, 101, 102, 103, 104, 105, 106, 108, 109, 110, 111, 114, 115, 116, 117 exhibit EC₅₀ values of between 100 µM and 10 µM. Compounds 1, 3, 3a, 7, 7a, 7b, 8b, 11, 12a, 12b, 16, 17, 17a, 17b, 23a, 23b, 24, 25, 35, 36, 39, 43, 54, 57, 59, 72, 74, 80, 85, 86, 87, 88, 91, 98, 99, 100, 107, 112, 113 exhibit EC₅₀ values of less than 10 µM.

### EXAMPLE 5: Screening method to test ingestion activity of compounds of formula (I), (Ia) and/or (Ib) against adult Ctenocephalides felis

Compounds of formulae (I), (Ia) and/or (Ib) dissolved in 100% DMSO are added to bovine blood and offered via an artificial membrane feeding system to adult *C. felis* fleas. The motility of fleas is then visually inspected 48h post treatment. Efficacy is expressed in % motility reduction compared to controls containing blood treated with DMSO only. A dose response series is implemented to determine EC₅₀ values. Compounds 12b, 13, 16, 17, 17a, 17b, 22, 37, 38, 41, 42, 46, 49, 50, 52, 53, 54, 65, 66, 73, 85, 93a, 97, 99, 100, 104, 106 exhibit EC₅₀ values of between 10 µM and 1 µM. Compounds 1, 2, 3, 3a, 3b, 5, 7, 7a, 7b, 8, 8a, 8b, 11, 12, 12a, 15, 18, 19, 20, 21, 23a, 23b, 24, 25, 26, 27, 28, 29, 30, 31, 32, 34, 35, 36, 39, 40, 43, 47, 48, 51, 51a, 51b, 55, 56, 57, 58, 59, 60, 61, 63, 67, 68, 69, 70, 72, 74, 75, 77, 78, 79, 80, 81, 82, 83, 84, 86, 87, 88, 89, 90, 91, 92, 93b, 94, 95, 96, 98, 101, 102, 103, 105, 107, 108, 109, 110, 111, 112, 113, 114, 115, 116, 117 exhibit EC₈₀ values of less than 1 µM.

### REFERENCES

The following references, to the extent that they provide exemplary procedural or other details supplementary to those set forth herein, are specifically incorporated herein by reference.
(1) WO 2015/071180
(2) WO 2016/030229
(3) WO 2016/039441
(4) WO 2016/071214
(5) WO 2018/206348
(6) WO 2019/219689
(7) WO 2019/229089
(8) WO 2019/234158
(9) WO 2020/084075
(10) WO 2020/141136
(11) WO 2021/033141
(12) WO 2021/085370
(13) WO 2022/253841
(14) WO 2023/036934
(15) WO 2024/175557
(16) WO 2024/175558
(17) WO 2024/175559

## Claims

1. A compound of formula (I) or formula (Ia) or formula (Ib) 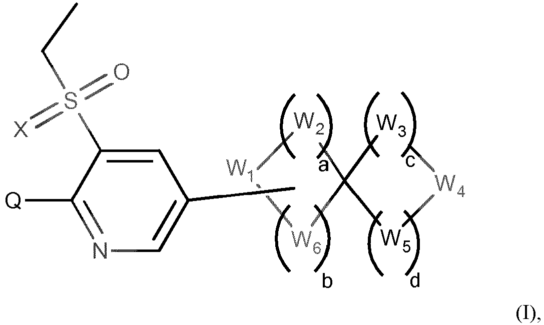 or 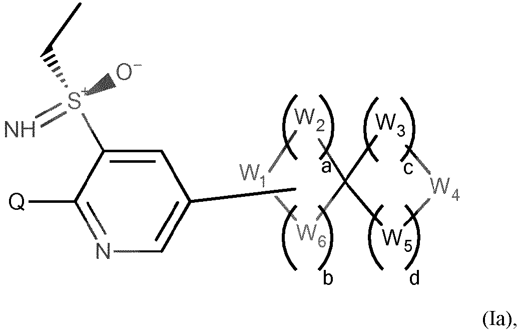 or 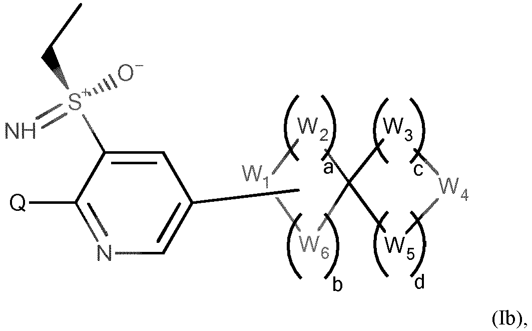 wherein:
X independently is O or NH, preferably O;
W₁ independently is C-Rₐ, if the central pyridine ring is attached via W₁, or C-R_{b}R_{c}, or N, if the central pyridine ring is attached via W₁, or N-R_{d};
W₂, W₆, if present, in each occurrence independently from each other are C-Rₑ, if the central pyridine ring is attached via a W₂ or a W₆, or C-R_{f}R_{g}, or N, if the central pyridine ring is attached via a W₂ or a W₆, or N-Rₕ;
W₃, W₅, if present, in each occurrence independently from each other are C-RᵢRⱼ, or (N=), or N-Rₖ, or O, or S(O)ᵤ, or S(O)ᵤ-R₁, or S(O)=N-Rₘ;
W₄ independently is C-RₙRₒ, or (N=), or N-Rₚ, or O, or S(O)ᵤ, or S(O)ᵤ-Rₚₚ, or S(O)=N-Rₚₚₚ;
a, b, c, d in each occurrence are independently from each other 0, 1, or 2, with the proviso that a+b and c+d independently from each other are 1, 2, 3 or 4;
u in each occurrence independently is 0, 1 or 2;
Rₐ, R_{b}, R_{c}, Rₐ, Rₑ, R_{f}, R_{g}, Rₕ, Rᵢ, Rⱼ, Rₖ, Rₗ, Rᵤ, Rₙ, Rₒ, Rₚ, Rₚₚ, Rₚₚₚ, if present, in each occurrence independently from each other are hydrogen; halogen, such as F, Cl, Br, I; C₁-C₆-alkyl, such as CH₃; C₂-C₆-alkenyl, such as CH=CH₂; C₂-C₆-alkynyl, such as CCH; C₁-C₆-haloalkyl, such as CF₃, CHF₂, CH₂-CF₃, CH₂-CHF₂, CH₂-CH₂F, CF₂-CH₃, CH₂F, CF₂Cl, CF₂-CF₃, CF₂-CHF₂; C₂-C₆-haloalkenyl, such as CF=CH₂; C₂-C₆-haloalkynyl, such as CC-CHF₂; C(O)-(C₁-C₆-alkyl), such as C(O)-methyl; C(O)H; C(O)-(C₁-C₆-haloalkyl), such as C(O)-CF₂Cl; C(O)-(C₁-C₆-haloalkenyl), such as C(O)-CH=CHF; C(O)-(C₁-C₆-haloalkynyl), such as C(O)-CC-CHF₂; C(O)-N(Rₓ)(R_{y}), such as C(O)-NH₂; C(O)-N(CH₃)₂; C(O)-(C₃-C₈-cycloalkyl), such as C(O)-cyclopropyl; C(O)-C₃-C₈-cycloalkyl substituted with C₁-C₆-alkyl, such as C(O)-cyclopropyl-methyl; C(O)-C₃-C₈-cycloalkyl substituted with C₁-C₆-haloalkyl, such as C(O)-cyclopropyl-CH₂F; C(O)-C₃-C₈-cycloalkyl substituted with C₁-C₆-alkoxy, such as C(O)-cyclopropyl-OCH₃; C(O)-C₃-C₈-cycloalkyl substituted with C₁-C₆-haloalkoxy, such as C(O)-cyclopropyl-OCH₂F; C(S)-(C₁-C₆-alkyl), such as C(S)-methyl; C(S)-(C₁-C₆-haloalkyl), such as C(S)-CF₂Cl; C(S)-(C₁-C₆-haloalkenyl), such as C(S)-CH=CHF; C(S)-(C₁-C₆-haloalkynyl), such as C(S)-CC-CHF₂; C(S)-N(Rₓ)(R_{y}), such as C(S)-NH₂; C(S)-N(CH₃)₂; C(S)-(C₃-C₈-cycloalkyl), such as C(S)-cyclopropyl; C(S)-C₃-C₈-cycloalkyl substituted with C₁-C₆-alkyl, such as C(S)-cyclopropyl-methyl; C(S)-C₃-C₈-cycloalkyl substituted with C₁-C₆-haloalkyl, such as C(S)-cyclopropyl-CH₂F; C(S)-C₃-C₈-cycloalkyl substituted with C₁-C₆-alkoxy, such as C(S)-cyclopropyl-OCH₃; C(S)-C₃-C₈-cycloalkyl substituted with C₁-C₆-haloalkoxy, such as C(S)-cyclopropyl-OCH₂F; C₁-C₆-alkoxy, such as methoxy, ethoxy, propoxy; C₁-C₆-haloalkoxy, such as O-CF₃; C₃-C₈-cycloalkyl, such as cyclopropyl; (C₃-C₈-cycloalkyl)-OH, such as cyclopropyl-OH; (C₃-C₈-cycloalkyl)-CN, such as cyclopropyl-CN; (C₃-C₈-cycloalkyl)-C(O)ORᵥ; (C₃-C₈-cycloalkyl)-(C₁-C₆-haloalkyl); such as cyclopropyl-CH₂F; (C₃-C₈-cycloalkyl)-(C₁-C₆-haloalkenyl); such as cyclopropyl-CH=CHF; (C₃-C₈-cycloalkyl)-(C₁-C₆-haloalkynyl); such as cyclopropyl-CC-CHF₂; C₃-C₈-cycloalkoxy, such as cyclopropyloxy; C₃-C₈-cycloalkyl substituted with C₁-C₆-haloalkoxy, such as cyclopropyl-OCH₂F; OH; CN; C(O)ORᵥ; (C₁-C₆-alkyl)-OH, such as methyl-OH; (C₁-C₆-alkyl)-CN, such as methyl-CN; (C₁-C₆-alkyl)-C(O)ORᵥ; S(O)ᵣ-(C₁-C₆-alkyl), such as S-CH₃, S(O)-CH₃, S(O)₂-CH₃, S-ethyl, S(O)-ethyl, S(O)₂-ethyl; S(O)ₛ-(C₁-C₆-haloalkyl), such as S-CF₃, S(O)-CF₃, S(O)₂-CF₃; S(O)ₜ-R_{w}; S(O)₂-N(Rₓ)(R_{y}); S(O)-N(Rₓ)-C(O)-R_{y}, such as S(O)-NH-C(O)-CH₃; S(O)-(R_{w})=N-R_{z}; Si(Rₓ)(R_{y})(R_{z}); SH; SF₅; NO₂; N(Rₓ)(R_{y}), such as NH₂, NH-CH₃, N(CH₃)₂; S(O)(=N-Rₓ)-N(R_{y})(R_{z});
C-R_{b}R_{c}, C-R_{f}R_{g}, C-RᵢRⱼ, C-RₙRₒ in each occurrence independently from each other can also form C=O;
Q is selected from the group consisting of: Q1, Q2, Q3, wherein:
Q1 is an unsubstituted or substituted monocyclic, preferably 5- or 6-membered, heteroaryl or heterocyclyl containing one, two or three heteroatoms, which are each independently selected from the group consisting of N, O, S, S(O), S(O)₂; wherein preferably Q1 if substituted is substituted with one, two, three or four substituents each independently selected from the group consisting of: halogen, such as F, Cl, Br, I; C₁-C₆-alkyl, such as CH₃; C₂-C₆-alkenyl, such as CH=CH₂; C₂-C₆-alkynyl, such as CCH; C₁-C₆-haloalkyl, such as CF₃, CHF₂, CH₂-CF₃, CH₂-CHF₂, CH₂-CH₂F, CF₂-CH₃, CH₂F, CF₂Cl, CF₂-CF₃, CF₂-CHF₂; C₂-C₆-haloalkenyl, such as CF=CH₂; C₂-C₆-haloalkynyl, such as CC-CHF₂; C(O)-(C₁-C₆-alkyl), such as C(O)-methyl; C(O)H; C(O)-(C₁-C₆-haloalkyl), such as C(O)-CF₂Cl; C(O)-(C₁-C₆-haloalkenyl), such as C(O)-CH=CHF; C(O)-(C₁-C₆-haloalkynyl), such as C(O)-CC-CHF₂; C(O)-N(Rₓ)(R_{y}), such as C(O)-NH₂; C(O)-N(CH₃)₂; C(O)-(C₃-C₈-cycloalkyl), such as C(O)-cyclopropyl; C(O)-C₃-C₈-cycloalkyl substituted with C₁-C₆-alkyl, such as C(O)-cyclopropyl-methyl; C(O)-C₃-C₈-cycloalkyl substituted with C₁-C₆-haloalkyl, such as C(O)-cyclopropyl-CH₂F; C(O)-C₃-C₈-cycloalkyl substituted with C₁-C₆-alkoxy, such as C(O)-cyclopropyl-OCH₃; C(O)-C₃-C₈-cycloalkyl substituted with C₁-C₆-haloalkoxy, such as C(O)-cyclopropyl-OCH₂F; C(S)-(C₁-C₆-alkyl), such as C(S)-methyl; C(S)-(C₁-C₆-haloalkyl), such as C(S)-CF₂Cl; C(S)-(C₁-C₆-haloalkenyl), such as C(S)-CH=CHF; C(S)-(C₁-C₆-haloalkynyl), such as C(S)-CC-CHF₂; C(S)-N(Rₓ)(R_{y}), such as C(S)-NH₂; C(S)-N(CH₃)₂; C(S)-(C₃-C₈-cycloalkyl), such as C(S)-cyclopropyl; C(S)-C₃-C₈-cycloalkyl substituted with C₁-C₆-alkyl, such as C(S)-cyclopropyl-methyl; C(S)-C₃-C₈-cycloalkyl substituted with C1-C6-haloalkyl, such as C(S)-cyclopropyl-CH₂F; C(S)-C₃-C₈-cycloalkyl substituted with C₁-C₆-alkoxy, such as C(S)-cyclopropyl-OCH₃; C(S)-C₃-C₈-cycloalkyl substituted with C₁-C₆-haloalkoxy, such as C(S)-cyclopropyl-OCH₂F; C₁-C₆-alkoxy, such as methoxy, ethoxy, propoxy; C₁-C₆-haloalkoxy, such as O-CF₃; C₃-C₈-cycloalkyl, such as cyclopropyl; (C₃-C₈-cycloalkyl)-OH, such as cyclopropyl-OH; (C₃-C₈-cycloalkyl)-CN, such as cyclopropyl-CN; (C₃-C₈-cycloalkyl)-C(O)ORᵥ; (C₃-C₈-cycloalkyl)-(C₁-C₆-haloalkyl); such as cyclopropyl-CH₂F; (C₃-C₈-cycloalkyl)-(C₁-C₆-haloalkenyl); such as cyclopropyl-CH=CHF; (C₃-C₈-cycloalkyl)-(C₁-C₆-haloalkynyl); such as cyclopropyl-CC-CHF₂; C₃-C₈-cycloalkoxy, such as cyclopropyloxy; C₃-C₈-cycloalkyl substituted with C₁-C₆-haloalkoxy, such as cyclopropyl-OCH₂F; OH; CN; C(O)ORᵥ; (C₁-C₆-alkyl)-OH, such as methyl-OH; (C₁-C₆-alkyl)-CN, such as methyl-CN; (C₁-C₆-alkyl)-C(O)ORᵥ; S(O)ᵣ-(C₁-C₆-alkyl), such as S-CH₃, S(O)-CH₃, S(O)₂-CH₃, S-ethyl, S(O)-ethyl, S(O)₂-ethyl; S(O)ₛ-(C₁-C₆-haloalkyl), such as S-CF₃, S(O)-CF₃, S(O)₂-CF₃; S(O)ₜ-R_{w}; S(O)₂-N(Rₓ)(R_{y}); S(O)-N(Rₓ)-C(O)-R_{y}, such as S(O)-NH-C(O)-CH₃; S(O)-(R_{w})=N-R_{z}; Si(Rₓ)(R_{y})(R_{z}); SH; SF₅; NO₂; N(Rₓ)(R_{y}), such as NH₂, NH-CH₃, N(CH₃)₂; S(O)(=N-Rₓ)-N(R_{y})(R_{z});
Q2 is an unsubstituted or substituted fused bicyclic, preferably 9- or 10-membered, heteroaryl or heterocyclyl containing one, two, three, four or five heteroatoms, which are each independently selected from the group consisting of N, O, S, S(O), S(O)₂; wherein preferably Q2 if substituted is substituted with one, two, three, four, five or six substituents each independently selected from the group consisting of: halogen, such as F, Cl, Br, I; C₁-C₆-alkyl, such as CH₃; C₂-C₆-alkenyl, such as CH=CH₂; C₂-C₆-alkynyl, such as CCH; C₁-C₆-haloalkyl, such as CF₃, CHF₂, CH₂-CF₃, CH₂-CHF₂, CH₂-CH₂F, CF₂-CH₃, CH₂F, CF₂Cl, CF₂-CF₃, CF₂-CHF₂; C₂-C₆-haloalkenyl, such as CF=CH₂; C₂-C₆-haloalkynyl, such as CC-CHF₂; C(O)-(C₁-C₆-alkyl), such as C(O)-methyl; C(O)H; C(O)-(C₁-C₆-haloalkyl), such as C(O)-CF₂Cl; C(O)-(C₁-C₆-haloalkenyl), such as C(O)-CH=CHF; C(O)-(C₁-C₆-haloalkynyl), such as C(O)-CC-CHF₂; C(O)-N(Rₓ)(R_{y}), such as C(O)-NH₂; C(O)-N(CH₃)₂; C(O)-(C₃-C₈-cycloalkyl), such as C(O)-cyclopropyl; C(O)-C₃-C₈-cycloalkyl substituted with C₁-C₆-alkyl, such as C(O)-cyclopropyl-methyl; C(O)-C₃-C₈-cycloalkyl substituted with C₁-C₆-haloalkyl, such as C(O)-cyclopropyl-CH₂F; C(O)-C₃-C₈-cycloalkyl substituted with C₁-C₆-alkoxy, such as C(O)-cyclopropyl-OCH₃; C(O)-C₃-C₈-cycloalkyl substituted with C₁-C₆-haloalkoxy, such as C(O)-cyclopropyl-OCH₂F; C(S)-(C₁-C₆-alkyl), such as C(S)-methyl; C(S)-(C₁-C₆-haloalkyl), such as C(S)-CF₂Cl; C(S)-(C₁-C₆-haloalkenyl), such as C(S)-CH=CHF; C(S)-(C₁-C₆-haloalkynyl), such as C(S)-CC-CHF₂; C(S)-N(Rₓ)(R_{y}), such as C(S)-NH₂; C(S)-N(CH₃)₂; C(S)-(C₃-C₈-cycloalkyl), such as C(S)-cyclopropyl; C(S)-C₃-C₈-cycloalkyl substituted with C₁-C₆-alkyl, such as C(S)-cyclopropyl-methyl; C(S)-C₃-C₈-cycloalkyl substituted with C₁-C₆-haloalkyl, such as C(S)-cyclopropyl-CH₂F; C(S)-C₃-C₈-cycloalkyl substituted with C₁-C₆-alkoxy, such as C(S)-cyclopropyl-OCH₃; C(S)-C₃-C₈-cycloalkyl substituted with C₁-C₆-haloalkoxy, such as C(S)-cyclopropyl-OCH₂F; C₁-C₆-alkoxy, such as methoxy, ethoxy, propoxy; C₁-C₆-haloalkoxy, such as O-CF₃; C₃-C₈-cycloalkyl, such as cyclopropyl; (C₃-C₈-cycloalkyl)-OH, such as cyclopropyl-OH; (C₃-C₈-cycloalkyl)-CN, such as cyclopropyl-CN; (C₃-C₈-cycloalkyl)-C(O)ORᵥ; (C₃-C₈-cycloalkyl)-(C₁-C₆-haloalkyl); such as cyclopropyl-CH₂F; (C₃-C₈-cycloalkyl)-(C₁-C₆-haloalkenyl); such as cyclopropyl-CH=CHF; (C₃-C₈-cycloalkyl)-(C₁-C₆-haloalkynyl); such as cyclopropyl-CC-CHF₂; C₃-C₈-cycloalkoxy, such as cyclopropyloxy; C₃-C₈-cycloalkyl substituted with C₁-C₆-haloalkoxy, such as cyclopropyl-OCH₂F; OH; CN; C(O)ORᵥ; (C₁-C₆-alkyl)-OH, such as methyl-OH; (C₁-C₆-alkyl)-CN, such as methyl-CN; (C₁-C₆-alkyl)-C(O)ORᵥ; S(O)ᵣ-(C₁-C₆-alkyl), such as S-CH₃, S(O)-CH₃, S(O)₂-CH₃, S-ethyl, S(O)-ethyl, S(O)₂-ethyl; S(O)ₛ-(C₁-C₆-haloalkyl), such as S-CF₃, S(O)-CF₃, S(O)₂-CF₃; S(O)ₜ-R_{w}; S(O)₂-N(Rₓ)(R_{y}); S(O)-N(Rₓ)-C(O)-R_{y}, such as S(O)-NH-C(O)-CH₃; S(O)-(R_{w})=N-R_{z}; Si(Rₓ)(R_{y})(R_{z}); SH; SF₅; NO₂; N(Rₓ)(R_{y}), such as NH₂, NH-CH₃, N(CH₃)₂; S(O)(=N-Rₓ)-N(R_{y})(R_{z});
Q3 is an unsubstituted or substituted fused tricyclic, preferably 12- or 13-membered, heteroaryl or heterocyclyl containing one, two, three, four, five, six or seven heteroatoms, which are each independently selected from the group consisting of N, O, S, S(O), S(O)₂; wherein preferably Q3 if substituted is substituted with one, two, three, four, five, six, seven or eight substituents each independently selected from the group consisting of: halogen, such as F, Cl, Br, I; C₁-C₆-alkyl, such as CH₃; C₂-C₆-alkenyl, such as CH=CH₂; C₂-C₆-alkynyl, such as CCH; C₁-C₆-haloalkyl, such as CF₃, CHF₂, CH₂-CF₃, CH₂-CHF₂, CH₂-CH₂F, CF₂-CH₃, CH₂F, CF₂Cl, CF₂-CF₃, CF₂-CHF₂; C₂-C₆-haloalkenyl, such as CF=CH₂; C₂-C₆-haloalkynyl, such as CC-CHF₂; C(O)-(C₁-C₆-alkyl), such as C(O)-methyl; C(O)H; C(O)-(C₁-C₆-haloalkyl), such as C(O)-CF₂Cl; C(O)-(C₁-C₆-haloalkenyl), such as C(O)-CH=CHF; C(O)-(C₁-C₆-haloalkynyl), such as C(O)-CC-CHF₂; C(O)-N(Rₓ)(R_{y}), such as C(O)-NH₂; C(O)-N(CH₃)₂; C(O)-(C₃-C₈-cycloalkyl), such as C(O)-cyclopropyl; C(O)-C₃-C₈-cycloalkyl substituted with C₁-C₆-alkyl, such as C(O)-cyclopropyl-methyl; C(O)-C₃-C₈-cycloalkyl substituted with C₁-C₆-haloalkyl, such as C(O)-cyclopropyl-CH₂F; C(O)-C₃-C₈-cycloalkyl substituted with C₁-C₆-alkoxy, such as C(O)-cyclopropyl-OCH₃; C(O)-C₃-C₈-cycloalkyl substituted with C₁-C₆-haloalkoxy, such as C(O)-cyclopropyl-OCH₂F; C(S)-(C₁-C₆-alkyl), such as C(S)-methyl; C(S)-(C₁-C₆-haloalkyl), such as C(S)-CF₂Cl; C(S)-(C₁-C₆-haloalkenyl), such as C(S)-CH=CHF; C(S)-(C₁-C₆-haloalkynyl), such as C(S)-CC-CHF₂; C(S)-N(Rₓ)(R_{y}), such as C(S)-NH₂; C(S)-N(CH₃)₂; C(S)-(C₃-C₈-cycloalkyl), such as C(S)-cyclopropyl; C(S)-C₃-C₈-cycloalkyl substituted with C₁-C₆-alkyl, such as C(S)-cyclopropyl-methyl; C(S)-C₃-C₈-cycloalkyl substituted with C₁-C₆-haloalkyl, such as C(S)-cyclopropyl-CH₂F; C(S)-C₃-C₈-cycloalkyl substituted with C₁-C₆-alkoxy, such as C(S)-cyclopropyl-OCH₃; C(S)-C₃-C₈-cycloalkyl substituted with C₁-C₆-haloalkoxy, such as C(S)-cyclopropyl-OCH₂F; C₁-C₆-alkoxy, such as methoxy, ethoxy, propoxy; C₁-C₆-haloalkoxy, such as O-CF₃; C₃-C₈-cycloalkyl, such as cyclopropyl; (C₃-C₈-cycloalkyl)-OH, such as cyclopropyl-OH; (C₃-C₈-cycloalkyl)-CN, such as cyclopropyl-CN; (C₃-C₈-cycloalkyl)-C(O)ORᵥ; (C₃-C₈-cycloalkyl)-(C₁-C₆-haloalkyl); such as cyclopropyl-CH₂F; (C₃-C₈-cycloalkyl)-(C₁-C₆-haloalkenyl); such as cyclopropyl-CH=CHF; (C₃-C₈-cycloalkyl)-(C₁-C₆-haloalkynyl); such as cyclopropyl-CC-CHF₂; C₃-C₈-cycloalkoxy, such as cyclopropyloxy; C₃-C₈-cycloalkyl substituted with C₁-C₆-haloalkoxy, such as cyclopropyl-OCH₂F; OH; CN; C(O)ORᵥ; (C₁-C₆-alkyl)-OH, such as methyl-OH; (C₁-C₆-alkyl)-CN, such as methyl-CN; (C₁-C₆-alkyl)-C(O)ORᵥ; S(O)ᵣ-(C₁-C₆-alkyl), such as S-CH₃, S(O)-CH₃, S(O)₂-CH₃, S-ethyl, S(O)-ethyl, S(O)₂-ethyl; S(O)ₛ-(C₁-C₆-haloalkyl), such as S-CF₃, S(O)-CF₃, S(O)₂-CF₃; S(O)ₜ-R_{w}; S(O)₂-N(Rₓ)(R_{y}); S(O)-N(Rₓ)-C(O)-R_{y}, such as S(O)-NH-C(O)-CH₃; S(O)-(R_{w})=N-R_{z}; Si(Rₓ)(R_{y})(R_{z}); SH; SF₅; NO₂; N(Rₓ)(R_{y}), such as NH₂, NH-CH₃, N(CH₃)₂; S(O)(=N-Rₓ)-N(R_{y})(R_{z});
Rᵥ, R_{w}, Rₓ, R_{y}, R_{z} in each occurrence are independently from each other hydrogen; halogen, such as F, Cl, Br, I; C₁-C₆-alkyl, such as methyl, ethyl; C₂-C₆-alkenyl, such as CH=CH₂; C₂-C₆-alkynyl, such as CCH; C₁-C₆-haloalkyl, such as CF₃; CHF₂, CH₂-CF₃, CH₂-CHF₂, CH₂-CH₂F, CF₂-CH₃, CH₂F, CF₂Cl, CF₂-CF₃, CF₂-CHF₂; C₂-C₆-haloalkenyl, such as CF=CH₂; C₂-C₆-haloalkynyl, such as CC-CHF₂; C₁-C₆-haloalkyloxy, such as O-CF₃; C₃-C₈-cycloalkyl, such as cyclopropyl; (C₃-C₈-cycloalkyl)-OH, such as cyclopropyl-OH; (C₃-C₈-cycloalkyl)-CN, such as cyclopropyl-CN; (C₃-C₈-cycloalkyl)-C(O)OH; (C₃-C₈-cycloalkyl)-(C₁-C₆-haloalkyl); such as cyclopropyl-CH₂F; (C₃-C₈-cycloalkyl)-(C₁-C₆-haloalkenyl); such as cyclopropyl-CH=CHF; (C₃-C₈-cycloalkyl)-(C₁-C₆-haloalkynyl); such as cyclopropyl-CC-CHF₂; C₃-C₈-cycloalkoxy, such as cyclopropyloxy;
r, s, t in each occurrence independently from each other are 0, 1 or 2;
optionally, wherein at least one nitrogen atom is an N-oxide;
optionally, but preferably in case at least one nitrogen atom is an N-oxide, the at least one nitrogen atom being an N-oxide comprises the one of the central pyridine moiety;
optionally, but preferably in case at least one nitrogen atom is an N-oxide, the at least one nitrogen atom being an N-oxide is comprised within a six-membered heterocyclic moiety containing one or more nitrogen atoms of any one of Q1, Q2, Q3;
optionally, but preferably in case at least one nitrogen atom is an N-oxide, with the proviso that if X is present and is NH, the NH nitrogen atom is not an N-oxide;
optionally, but preferably in case at least one nitrogen atom is an N-oxide, with the further proviso that, if possible, and if any of Q1, Q2, Q3 is substituted, the at least one nitrogen atom being an N-oxide is not present in any potential substituents of any of Q1, Q2, Q3;
or a salt thereof.

2. The compound according to claim 1, wherein 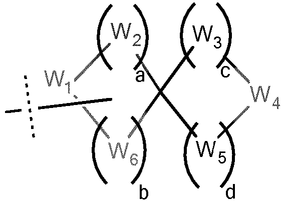 is independently selected from the group consisting of: 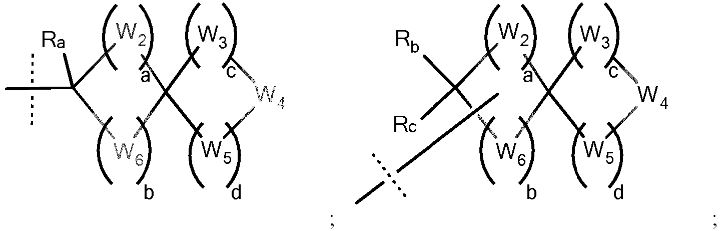 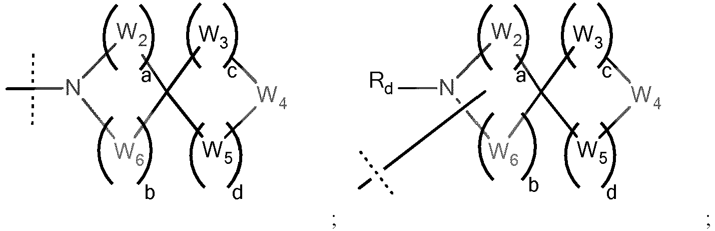 or a salt thereof.

3. The compound according to any one of claims 1 to 2, wherein Q is Q1, Q2 or Q3, which independently are selected from the group consisting of: 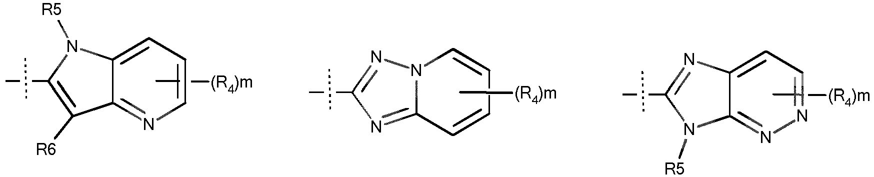 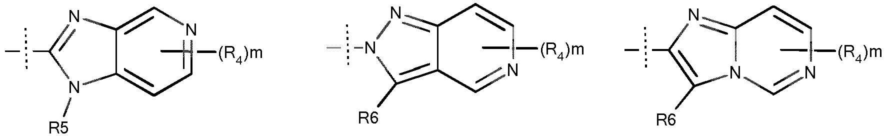 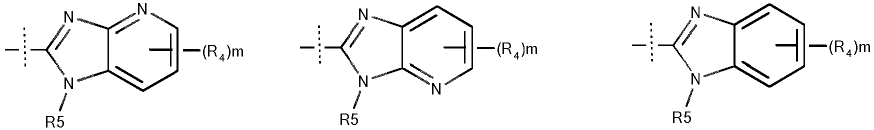 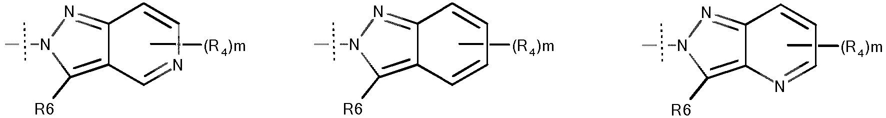 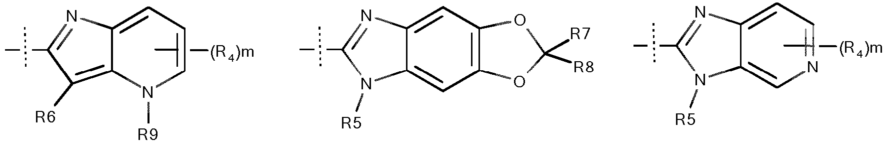 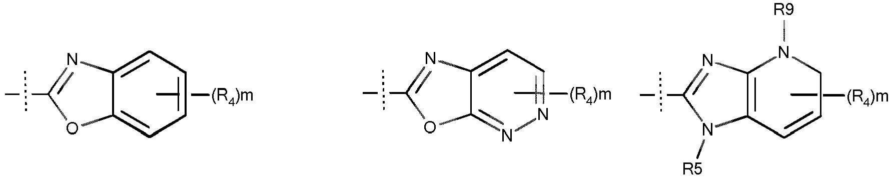 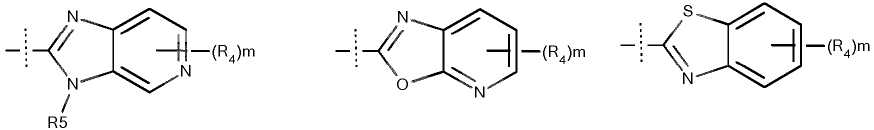 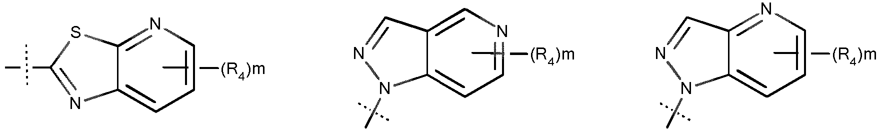 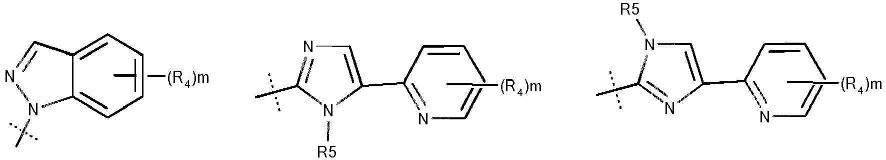 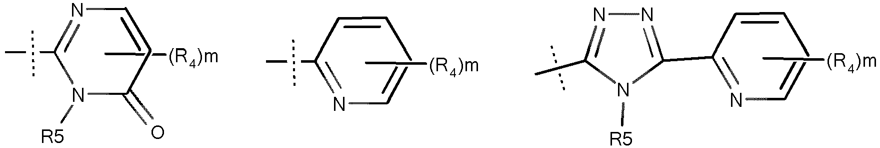 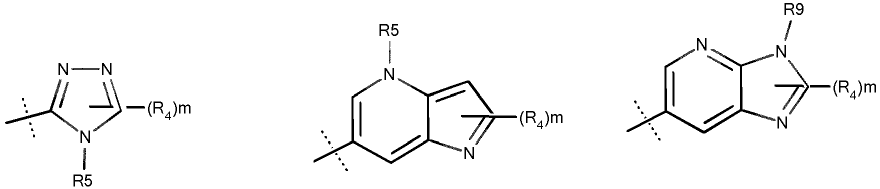 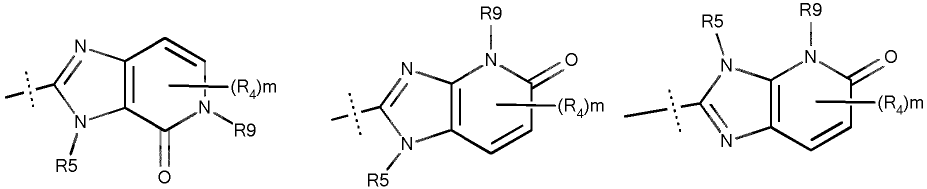 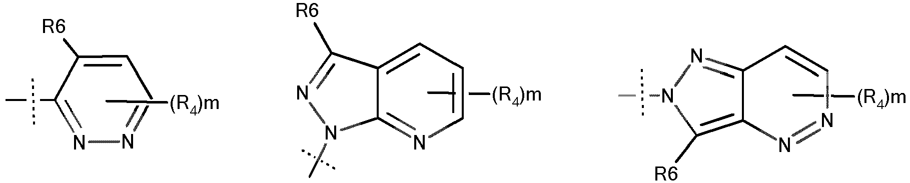 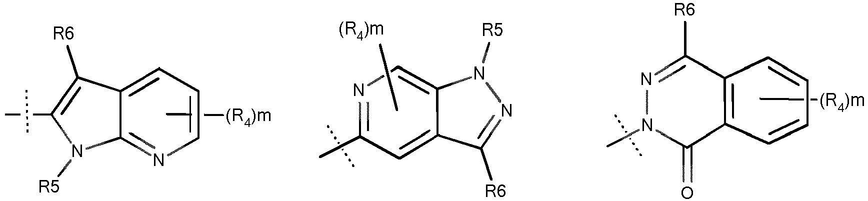 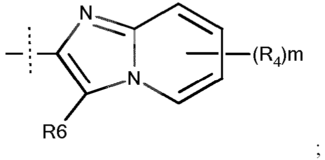 wherein:
R₄ in each occurrence independently is C₁-C₆-haloalkyl, such as CF₃, C₂F₅; CH₂-CF₂-CF₃; C₂-C₆-haloalkenyl, such as CH=CF-CF₃; C₁-C₆-haloalkoxy, such as O-CH₂-CF₃, O-CH₂-C₂F₅; S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; wherein R₄, if present once or more times, in each occurrence is independently attached to a C atom; and/or two R₄, if present, can form =O (provided that in such case there is no unsaturation at the corresponding ring C atom);
R5, R9 in each occurrence are independently hydrogen; C₁-C₆-alkyl, such as methyl, ethyl, isopropyl; C₂-C₆-alkenyl, such as ethenyl; C₁-C₆-haloalkyl, such as CF₃, CH₂-CHF₂, CH₂-CF₃, CHF₂, CH₂-CF₂-CF₃; C₃-C₈-cycloalkyl, such as cyclopropyl; C₃-C₈-cycloalkyl-alkyl, such as cyclopropyl-methyl; C₃-C₈-cycloalkyl substituted with one or more haloalkyl, such as cyclopropyl substituted with one or more CF₃; C₃-C₈-cycloalkyl substituted with one or more halogens, such as cyclopropyl substituted with one or more F;
C(O)-(C₁-C₆-alkyl), such as C(O)-methyl; heterocyclyl, such as aziridinyl, oxetanyl; S(O)ₚ-(C₁-C₆-alkyl),
such as S-methyl, S(O)-methyl; S(O)₂-methyl; S(O)_{q}-(C₁-C₆-haloalkyl), such as S-CF₃, S(O)-CF₃, S(O)₂-CF₃;
R6, R7, R8 in each occurrence independently are hydrogen; halogen, such as F, Cl, Br, I;
m is 0, 1 or 2;
p, q in each occurrence independently are 0, 1 or 2;
or a salt thereof.

4. The compound according to any one of claims 1 to 3, wherein Q is Q1, Q2 or Q3, which independently are selected from the group consisting of: 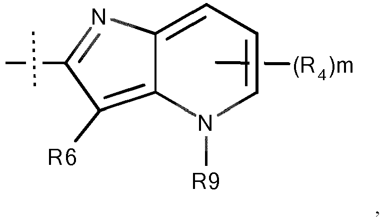 wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₃, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CHF₂" or "CF₂-CH₃";
R9 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, CH₂-CF₂-CF₃; C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R9 is "CHF₂" or "CH₂-CHF₂" or "ethyl" or "CH₂-cyclopropyl";
R6 is independently hydrogen, halogen, such as F, Cl, Br, I; in particular R6 is "hydrogen" or "F";
m is 0, 1 or 2, in particular m is 1;
p, q in each occurrence independently are 0, 1 or 2; 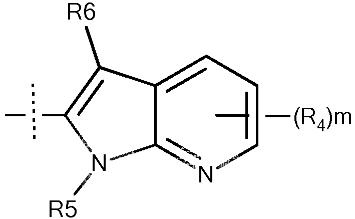 wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₃, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₂CF₃" or "CF₂-CH₃";
R5 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, CH₂-CF₂-CF₃; C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R5 is "hydrogen" or "methyl";
R6 is independently hydrogen, halogen, such as F, Cl, Br, I; in particular R6 is "hydrogen";
m is 0, 1 or 2, in particular m is 1;
p, q in each occurrence independently are 0, 1 or 2; 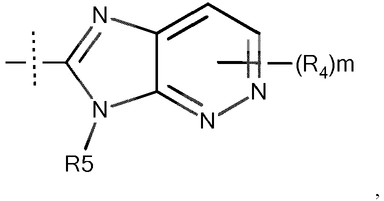 wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₃, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CHF₂" or "CF₂-cyclopropyl" or "CF₂-CF₃" or "CF₂-CH₂-CH₃" or "S-CF₃" or "S(O)₂-CF₃" or "CF₂-CH₃";
R5 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, CH₂-CF₂-CF₃; C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R5 is "methyl";
m is 0, 1 or 2, in particular m is 1;
p, q in each occurrence independently are 0, 1 or 2; 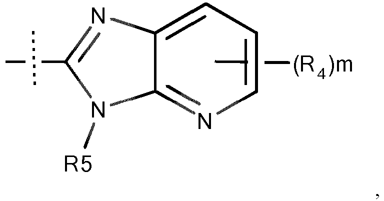 wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₃, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CHF₂" or "CF₂-CF₃" or "CF₂-cyclopropyl" or "CF₂-CH₂-CH₃" or "S(O)₂-CF₃" or "CF₂-CH₃";
R5 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, CH₂-CF₂-CF₃; C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R5 is "methyl";
m is 0, 1 or 2, in particular m is 1;
p, q in each occurrence independently are 0, 1 or 2; 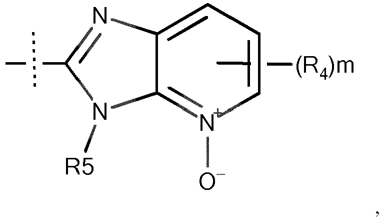 wherein:
R₄ is independently C₁-C₆-haloalkyl, such as CF₃, C₂F₅, C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CHF₂" or "CF₂-CF₃" or "CF₂-cyclopropyl" or "CF₂-CH₂-CH₃" or "S(O)₂-CF₃" or "CF₂-CH₃";
R5 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R5 is "methyl";
m is 0, 1 or 2; in particular m is 1;
p, q in each occurrence independently are 0, 1 or 2; 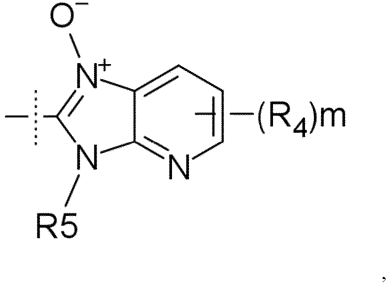 wherein:
R₄ is independently C₁-C₆-haloalkyl, such as CF₃, C₂F₅, C₂-C₆-haloalkenyl, such as CH=CF-CF₃, C₁-C₆-haloalkoxy, such as O-CH₂-CF₃, O-CH₂-C₂F₃, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CHF₂" or "CF₂-CF₃" or "CF₂-cyclopropyl" or "CF₂-CH₂-CH₃" or "S(O)₂-CF₃" or "CF₂-CH₃";
R5 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R5 is "methyl"; m is 0, 1 or 2; in particular m is 1;
p, q in each occurrence independently are 0, 1 or 2; 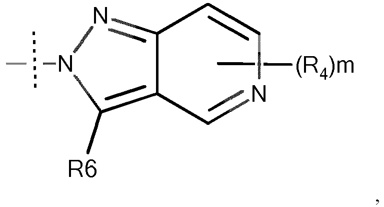 wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CF₂-CF₃" or "S-CF₃" or "S(O)-CF₃" or "S(O)₂-CF₃" or "CF₂-CH₃";
R6 is independently hydrogen, halogen, such as F, Cl, Br, I; in particular R6 is "hydrogen" or "F";
m is 0, 1 or 2, in particular m is 1;
p, q in each occurrence independently are 0, 1 or 2; 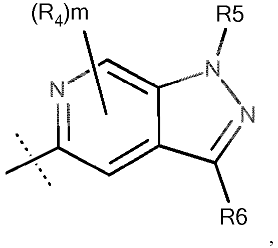 wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CF₂-CF₃" or "S-CF₃" or "S(O)-CF₃" or "S(O)₂-CF₃" or "CF₂-CH₃";
R5 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, CH₂-CF₂-CF₃; C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R5 is "methyl" or "CH₂-CF₂-CF₃";
R6 is independently hydrogen, halogen, such as F, Cl, Br, I; in particular R6 is "hydrogen" or "F";
m is 0, 1 or 2, in particular m is 1;
p, q in each occurrence independently are 0, 1 or 2; 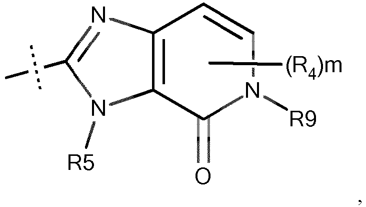 wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₃, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CHF₂" or "CF₂-CH₃";
R5 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, CH₂-CF₂-CF₃; C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R5 is "methyl" or "CH₂-CF₂-CF₃";
R9 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, CH₂-CF₂-CF₃; C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R9 is "CHF₂" or "CH₂-CHF₂" or "ethyl" or "cyclopropyl" or "CH₂-cyclopropyl";
R6 is independently hydrogen, halogen, such as F, Cl, Br, I; in particular R6 is "hydrogen" or "F";
m is 0, 1 or 2, in particular m is 1;
p, q in each occurrence independently are 0, 1 or 2; 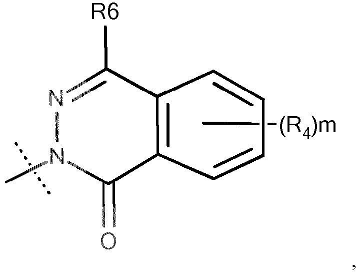 wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CF₂-CF₃" or "S-CF₃" or "S(O)-CF₃" or "S(O)₂-CF₃" or "CF₂-CH₃";
R6 is independently hydrogen, halogen, such as F, Cl, Br, I; in particular R6 is "hydrogen" or "F";
m is 0, 1 or 2, in particular m is 1;
p, q in each occurrence independently are 0, 1 or 2; 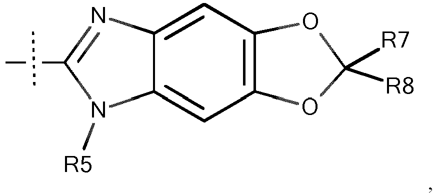 wherein:
R5 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, CH₂-CF₂-CF₃; C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R5 is "methyl" or "CH₂-CF₂-CF₃";
R7, R8 independently are hydrogen; halogen, such as F, Cl, Br, I; in particular R7, R8 are both "F";
p, q in each occurrence independently are 0, 1 or 2; 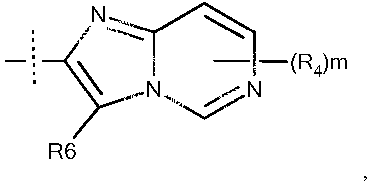 wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CF₂-CF₃" or "S-CF₃" or "S(O)-CF₃" or "S(O)₂-CF₃" or "CF₂-CH₃";
R6 is independently hydrogen, halogen, such as F, Cl, Br, I; in particular R6 is "hydrogen" or "F";
m is 0, 1 or 2, in particular m is 1;
p, q in each occurrence independently are 0, 1 or 2; 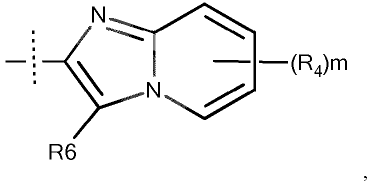 wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CF₂-CF₃" or "S-CF₃" or "S(O)-CF₃" or "S(O)₂-CF₃" or "CF₂-CH₃";
R6 is independently hydrogen, halogen, such as F, Cl, Br, I; in particular R6 is "hydrogen" or "F";
m is 0, 1 or 2, in particular m is 1;
p, q in each occurrence independently are 0, 1 or 2; 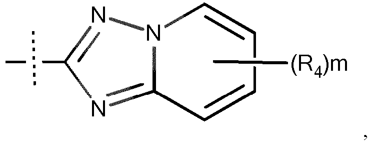 wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CF₂-CF₃" or "S-CF₃" or "S(O)-CF₃" or "S(O)₂-CF₃" or "CF₂-CH₃";
m is 0, 1 or 2, in particular m is 1;
p, q in each occurrence independently are 0, 1 or 2; 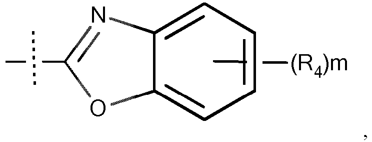 wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CF₂-CF₃" or "S-CF₃" or "S(O)-CF₃" or "S(O)₂-CF₃" or "CF₂-CH₃";
m is 0, 1 or 2, in particular m is 1;
p, q in each occurrence independently are 0, 1 or 2;
or a salt thereof.

5. The compound according to any one of claims 1 to 4, wherein the optional at least one nitrogen atom being an N-oxide comprises the one of the central pyridine moiety selected from the group consisting of formula (II) or formula (IIa) or formula (IIb): 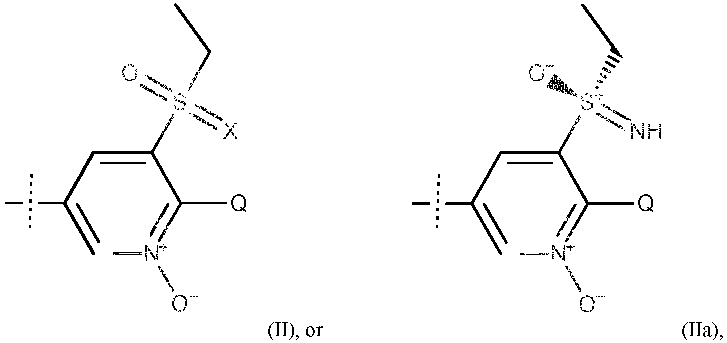 or 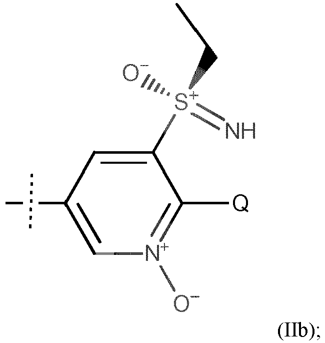 wherein X and Q independently are as claimed in any one of the preceding claims;
or a salt thereof.

6. A compound of formula (I) or formula (Ia) or formula (Ib), preferably according to any one of claims 1 to 5, 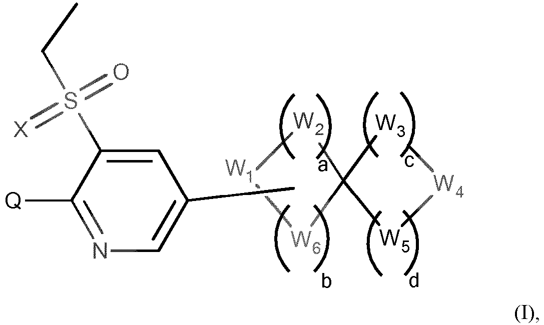 or 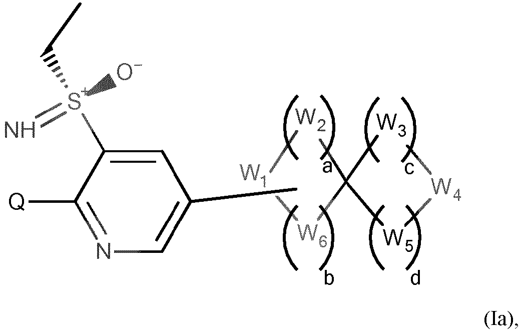 or 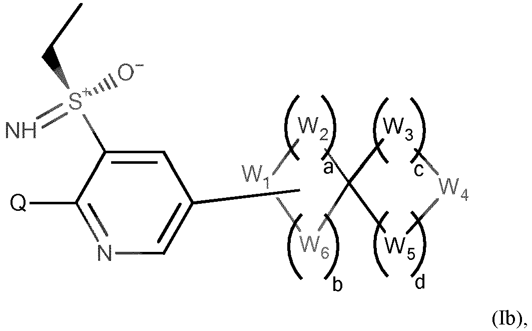 wherein:
X inpendently is O or NH, preferably O;
W₁ independently is C-Rₐ, if the central pyridine ring is attached via W₁, or C-R_{b}R_{c}, or N, if the central pyridine ring is attached via W₁, or N-R_{d};
W₂, W₆, if present, in each occurrence independently from each other are C-Rₑ, if the central pyridine ring is attached via a W₂ or a W₆, or C-R_{f}R_{g}, or N, if the central pyridine ring is attached via a W₂ or a W₆, or N-Rₕ;
W₃, W₅, if present, in each occurrence independently from each other are C-RᵢRⱼ, or (N=), or N-Rₖ, or O, or S(O)ᵤ, or S(O)ᵤ-R₁, or S(O)=N-Rₘ;
W₄ independently is C-RₙRₒ, or (N=), or N-Rₚ, or O, or S(O)ᵤ, or S(O)ᵤ-Rₚₚ, or S(O)=N-Rₚₚₚ;
a, b, c, d in each occurrence are independently from each other 0, 1, or 2, with the proviso that a+b and c+d independently from each other are 1, 2, 3 or 4;
u in each occurrence independently is 0, 1 or 2;
Rₐ, R_{b}, R_{c}, R_{d}, Rₑ, R_{f}, R_{g}, Rₕ, Rᵢ, Rⱼ, Rₖ, Rᵢ, Rᵤ, Rₙ, Rₒ, Rₚ, Rₚₚ, Rₚₚₚ, if present, in each occurrence independently from each other are hydrogen; halogen, such as F, Cl, Br, I; C₁-C₆-alkyl, such as CH₃; C₂-C₆-alkenyl, such as CH=CH₂; C₂-C₆-alkynyl, such as CCH; C₁-C₆-haloalkyl, such as CF₃, CHF₂, CH₂-CF₃, CH₂-CHF₂, CH₂-CH₂F, CF₂-CH₃, CH₂F, CF₂Cl, CF₂-CF₃, CF₂-CHF₂; C₂-C₆-haloalkenyl, such as CF=CH₂; C₂-C₆-haloalkynyl, such as CC-CHF₂; C(O)-(C₁-C₆-alkyl), such as C(O)-methyl; C(O)H; C(O)-(C₁-C₆-haloalkyl), such as C(O)-CF₂Cl; C(O)-(C₁-C₆-haloalkenyl), such as C(O)-CH=CHF; C(O)-(C₁-C₆-haloalkynyl), such as C(O)-CC-CHF₂; C(O)-N(Rₓ)(R_{y}), such as C(O)-NH₂, C(O)-N(CH₃)₂; C(O)-(C₃-C₈-cycloalkyl), such as C(O)-cyclopropyl; C(O)-C₃-C₈-cycloalkyl substituted with C₁-C₆-alkyl, such as C(O)-cyclopropyl-methyl; C(O)-C₃-C₈-cycloalkyl substituted with C₁-C₆-haloalkyl, such as C(O)-cyclopropyl-CH₂F; C(O)-C₃-C₈-cycloalkyl substituted with C₁-C₆-alkoxy, such as C(O)-cyclopropyl-OCH₃; C(O)-C₃-C₈-cycloalkyl substituted with C₁-C₆-haloalkoxy, such as C(O)-cyclopropyl-OCH₂F; C(S)-(C₁-C₆-alkyl), such as C(S)-methyl; C(S)-(C₁-C₆-haloalkyl), such as C(S)-CF₂Cl; C(S)-(C₁-C₆-haloalkenyl), such as C(S)-CH=CHF; C(S)-(C₁-C₆-haloalkynyl), such as C(S)-CC-CHF₂; C(S)-N(Rₓ)(R_{y}), such as C(S)-NH₂, C(S)-N(CH₃)₂; C(S)-(C₃-C₈-cycloalkyl), such as C(S)-cyclopropyl; C(S)-C₃-C₈-cycloalkyl substituted with C₁-C₆-alkyl, such as C(S)-cyclopropyl-methyl; C(S)-C₃-C₈-cycloalkyl substituted with C₁-C₆-haloalkyl, such as C(S)-cyclopropyl-CH₂F; C(S)-C₃-C₈-cycloalkyl substituted with C₁-C₆-alkoxy, such as C(S)-cyclopropyl-OCH₃; C(S)-C₃-C₈-cycloalkyl substituted with C₁-C₆-haloalkoxy, such as C(S)-cyclopropyl-OCH₂F; C₁-C₆-alkoxy, such as methoxy, ethoxy, propoxy; C₁-C₆-haloalkoxy, such as O-CF₃; C₃-C₈-cycloalkyl, such as cyclopropyl; (C₃-C₈-cycloalkyl)-OH, such as cyclopropyl-OH; (C₃-C₈-cycloalkyl)-CN, such as cyclopropyl-CN; (C₃-C₈-cycloalkyl)-C(O)ORᵥ; (C₃-C₈-cycloalkyl)-(C₁-C₆-haloalkyl); such as cyclopropyl-CH₂F; (C₃-C₈-cycloalkyl)-(C₁-C₆-haloalkenyl); such as cyclopropyl-CH=CHF; (C₃-C₈-cycloalkyl)-(C₁-C₆-haloalkynyl); such as cyclopropyl-CC-CHF₂; C₃-C₈-cycloalkoxy, such as cyclopropyloxy; C₃-C₈-cycloalkyl substituted with C₁-C₆-haloalkoxy, such as cyclopropyl-OCH₂F; OH; CN; C(O)ORᵥ; (C₁-C₆-alkyl)-OH, such as methyl-OH; (C₁-C₆-alkyl)-CN, such as methyl-CN; (C₁-C₆-alkyl)-C(O)ORᵥ; S(O)ᵣ-(C₁-C₆-alkyl), such as S-CH₃, S(O)-CH₃, S(O)₂-CH₃, S-ethyl, S(O)-ethyl, S(O)₂-ethyl; S(O)ₛ-(C₁-C₆-haloalkyl), such as S-CF₃, S(O)-CF₃, S(O)₂-CF₃; S(O)ₜ-R_{w}; S(O)₂-N(Rₓ)(R_{y}); S(O)-(R_{w})=N-R_{z}; Si(Rₓ)(R_{y})(R_{z}); SH; SF₅; NO₂; N(Rₓ)(R_{y}), such as NH₂, NH-CH₃, N(CH₃)₂; S(O)(=N-Rₓ)-N(R_{y})(R_{z});
C-R_{b}R_{c}, C-R_{f}R_{g}, C-RᵢRⱼ, C-RₙRₒ in each occurrence independently from each other can form C=O;
r, s, t in each occurrence independently from each other are 0, 1 or 2;
Rᵥ, R_{w}, Rₓ, R_{y}, R_{z} in each occurrence are independently from each other hydrogen; halogen, such as F, Cl, Br, I; C₁-C₆-alkyl, such as methyl, ethyl; C₂-C₆-alkenyl, such as CH=CH₂; C₂-C₆-alkynyl, such as CCH; C₁-C₆-haloalkyl, such as CF₃; CHF₂, CH₂-CF₃, CH₂-CHF₂, CH₂-CH₂F, CF₂-CH₃, CH₂F, CF₂Cl, CF₂-CF₃, CF₂-CHF₂; C₂-C₆-haloalkenyl, such as CF=CH₂; C₂-C₆-haloalkynyl, such as CC-CHF₂; C₁-C₆-haloalkyloxy, such as O-CF₃; C₃-C₈-cycloalkyl, such as cyclopropyl; (C₃-C₈-cycloalkyl)-OH, such as cyclopropyl-OH; (C₃-C₈-cycloalkyl)-CN, such as cyclopropyl-CN; (C₃-C₈-cycloalkyl)-C(O)OH; (C₃-C₈-cycloalkyl)-(C₁-C₆-haloalkyl); such as cyclopropyl-CH₂F; (C₃-C₈-cycloalkyl)-(C₁-C₆-haloalkenyl); such as cyclopropyl-CH=CHF; (C₃-C₈-cycloalkyl)-(C₁-C₆-haloalkynyl); such as cyclopropyl-CC-CHF₂; C₃-C₈-cycloalkoxy, such as cyclopropyloxy;
optionally, wherein at least one nitrogen atom is an N-oxide;
optionally, but preferably in case at least one nitrogen atom is an N-oxide, the at least one nitrogen atom being an N-oxide comprises the one of the central pyridine moiety;
optionally, but preferably in case at least one nitrogen atom is an N-oxide, the at least one nitrogen atom being an N-oxide is comprised within a six-membered heterocyclic moiety containing one or more nitrogen atoms of any one of Q;
optionally, but preferably in case at least one nitrogen atom is an N-oxide, with the proviso that if X is present and is NH, the NH nitrogen atom is not an N-oxide;
optionally, but preferably in case at least one nitrogen atom is an N-oxide, with the further proviso that, if possible, and if any of Q is substituted, the at least one nitrogen atom being an N-oxide is not present in any potential substituents of any of Q;
and 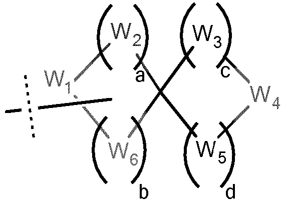 is 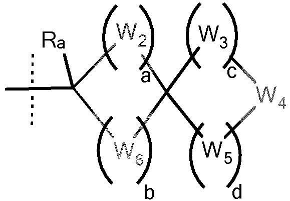 or 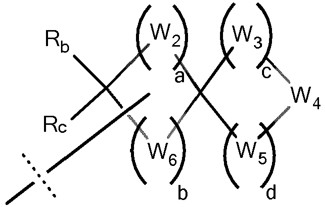 or 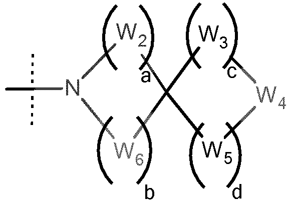 or 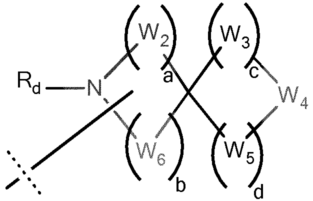 and
Q is 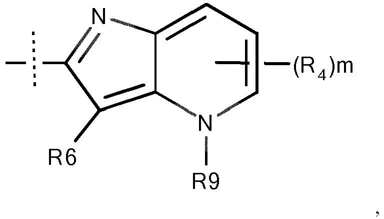 wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₃, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CHF₂" or "CF₂-CH₃";
R9 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, CH₂-CF₂-CF₃; C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R9 is "CHF₂" or "CH₂-CHF₂" or "ethyl" or "CH₂-cyclopropyl";
R6 is independently hydrogen, halogen, such as F, Cl, Br, I; in particular R6 is "hydrogen" or "F";
m is 0, 1 or 2, in particular m is 1;
p, q in each occurrence independently are 0, 1 or 2;
or Q is 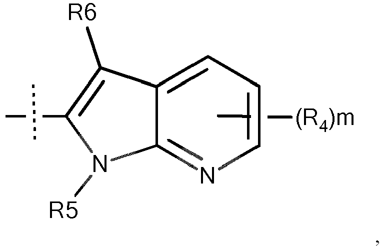
wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₃, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₂CF₃" or "CF₂-CH₃";
R5 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, CH₂-CF₂-CF₃; C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R5 is "hydrogen" or "methyl";
R6 is independently hydrogen, halogen, such as F, Cl, Br, I; in particular R6 is "hydrogen";
m is 0, 1 or 2, in particular m is 1;
p, q in each occurrence independently are 0, 1 or 2;
or Q is 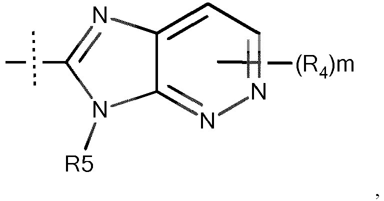
wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₃, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CHF₂" or "CF₂-cyclopropyl" or "CF₂-CF₃" or "CF₂-CH₂-CH₃" or "S-CF₃" or "S(O)₂-CF₃" or "CF₂-CH₃";
R5 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, CH₂-CF₂-CF₃; C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R5 is "methyl";
m is 0, 1 or 2, in particular m is 1;
p, q in each occurrence independently are 0, 1 or 2;
or Q is 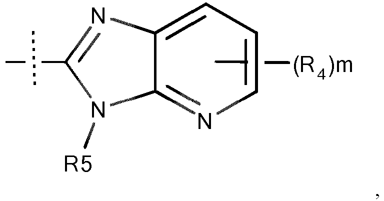
wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₃, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CHF₂" or "CF₂-CF₃" or "CF₂-cyclopropyl" or "CF₂-CH₂-CH₃" or "S(O)₂-CF₃" or "CF₂-CH₃";
R5 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, CH₂-CF₂-CF₃; C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R5 is "methyl";
m is 0, 1 or 2, in particular m is 1;
p, q in each occurrence independently are 0, 1 or 2;
or Q is 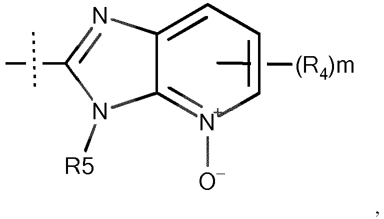
wherein:
R₄ in each occurrence is independently C₁-C₆-haloalkyl, such as CF₃, C₂F₅, C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CHF₂" or "CF₂-CF₃" or "CF₂-cyclopropyl" or "CF₂-CH₂-CH₃" or "S(O)₂-CF₃" or "CF₂-CH₃";
R5 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R5 is "methyl";
m is 0, 1 or 2; in particular m is 1;
p, q in each occurrence independently are 0, 1 or 2;
or Q is 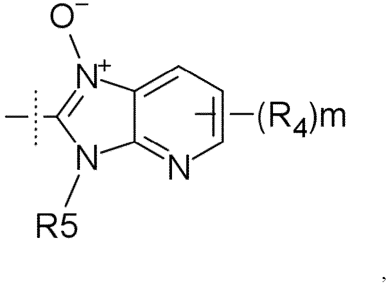
wherein:
R₄ in each occurrence is independently C₁-C₆-haloalkyl, such as CF₃, C₂F₅, C₂-C₆-haloalkenyl, such as CH=CF-CF₃, C₁-C₆-haloalkoxy, such as O-CH₂-CF₃, O-CH₂-C₂F₃, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CHF₂" or "CF₂-CF₃" or "CF₂-cyclopropyl" or "CF₂-CH₂-CH₃" or "S(O)₂-CF₃" or "CF₂-CH₃";
R5 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R5 is "methyl";
m is 0, 1 or 2; in particular m is 1;
p, q in each occurrence independently are 0, 1 or 2;
or Q is 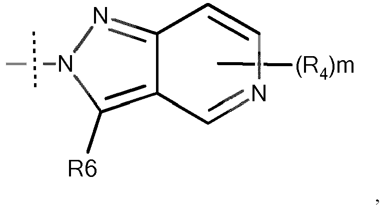
wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CF₂-CF₃" or "S-CF₃" or "S(O)-CF₃" or "S(O)₂-CF₃" or "CF₂-CH₃";
R6 is independently hydrogen, halogen, such as F, Cl, Br, I; in particular R6 is "hydrogen" or "F";
m is 0, 1 or 2, in particular m is 1;
p, q in each occurrence independently are 0, 1 or 2;
or Q is 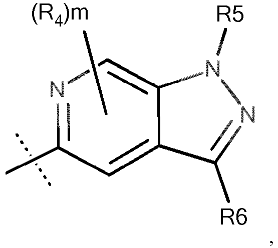
wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CF₂-CF₃" or "S-CF₃" or "S(O)-CF₃" or "S(O)₂-CF₃" or "CF₂-CH₃";
R5 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, CH₂-CF₂-CF₃; C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R5 is "methyl" or "CH₂-CF₂-CF₃";
R6 is independently hydrogen, halogen, such as F, Cl, Br, I; in particular R6 is "hydrogen" or "F";
m is 0, 1 or 2, in particular m is 1;
p, q in each occurrence independently are 0, 1 or 2;
or Q is 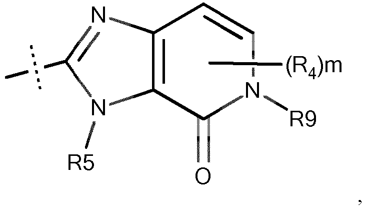
wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₃, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CHF₂" or "CF₂-CH₃";
R5 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, CH₂-CF₂-CF₃; C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R5 is "methyl" or "CH₂-CF₂-CF₃";
R9 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, CH₂-CF₂-CF₃; C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R9 is "CHF₂" or "CH₂-CHF₂" or "ethyl" or "cyclopropyl" or "CH₂-cyclopropyl";
R6 is independently hydrogen, halogen, such as F, Cl, Br, I; in particular R6 is "hydrogen" or "F";
m is 0, 1 or 2, in particular m is 1;
p, q in each occurrence independently are 0, 1 or 2;
or Q is 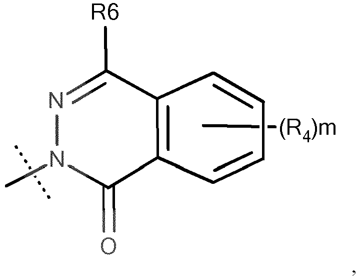
wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CF₂-CF₃" or "S-CF₃" or "S(O)-CF₃" or "S(O)₂-CF₃" or "CF₂-CH₃";
R6 is independently hydrogen, halogen, such as F, Cl, Br, I; in particular R6 is "hydrogen" or "F";
m is 0, 1 or 2, in particular m is 1;
p, q in each occurrence independently are 0, 1 or 2;
or Q is 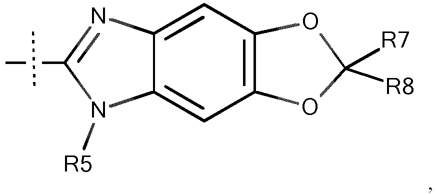
wherein:
R5 is independently hydrogen, C₁-C₆-alkyl, such as methyl, ethyl, isopropyl, C₂-C₆-alkenyl, such as ethenyl, C₁-C₆-haloalkyl, such as CF₃, CH₂-CHF₂, CH₂-CF₃, CHF₂, CH₂-CF₂-CF₃; C₃-C₈-cycloalkyl, such as cyclopropyl, C₃-C₈-cycloalkyl-alkyl, such as cyclopropyl-methyl, C₃-C₈-cycloalkyl substituted with one or more haloalkyl, such as cyclopropyl substituted with one or more CF₃, C₃-C₈-cycloalkyl substituted with one or more halogens, such as cyclopropyl substituted with one or more F, C(O)-(C₁-C₆-alkyl), such as C(O)-methyl, heterocyclyl, such as aziridinyl, oxetanyl, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃; in particular R5 is "methyl" or "CH₂-CF₂-CF₃";
R7, R8 independently are hydrogen; halogen, such as F, Cl, Br, I; in particular R7, R8 are both "F";
p, q in each occurrence independently are 0, 1 or 2;
or Q is 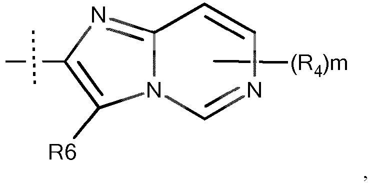
wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CF₂-CF₃" or "S-CF₃" or "S(O)-CF₃" or "S(O)₂-CF₃" or "CF₂-CH₃";
R6 is independently hydrogen, halogen, such as F, Cl, Br, I; in particular R6 is "hydrogen" or "F";
m is 0, 1 or 2, in particular m is 1;
p, q in each occurrence independently are 0, 1 or 2;
or Q is 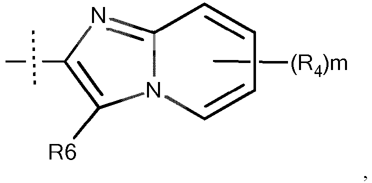
wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CF₂-CF₃" or "S-CF₃" or "S(O)-CF₃" or "S(O)₂-CF₃" or "CF₂-CH₃";
R6 is independently hydrogen, halogen, such as F, Cl, Br, I; in particular R6 is "hydrogen" or "F";
m is 0, 1 or 2, in particular m is 1;
p, q in each occurrence independently are 0, 1 or 2;
or Q is 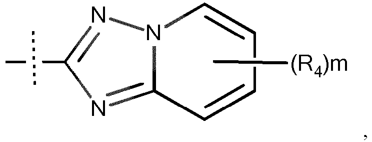
wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CF₂-CF₃" or "S-CF₃" or "S(O)-CF₃" or "S(O)₂-CF₃" or "CF₂-CH₃";
m is 0, 1 or 2, in particular m is 1;
p, q in each occurrence independently are 0, 1 or 2;
or Q is 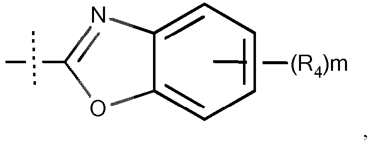
wherein:
R₄ in each occurrence is independently C₁-C₆-haloalkyl, such as CF₃, C₂F₅, CH₂-CF₂-CF₃; C₂-C₆-haloalkenyl, such as CH=CF-CF₃, C₁-C₆-haloalkoxy, such as O-CH₂-CF₃, O-CH₂-C₂F₅, S(O)ₚ-(C₁-C₆-alkyl), such as S-methyl, S(O)-methyl; S(O)₂-methyl; S(O)_{q}-(C₁-C₆-haloalkyl), such as S-CF₃, S(O)-CF₃, S(O)₂-CF₃, wherein R₄, if present once or more times, in each occurrence is independently attached to a C atom; and/or two R₄, if present, can form =O (provided that in such case there is no unsaturation at the corresponding ring C atom); in particular R₄ is "CF₃" or "CF₂-CF₃" or "S-CF₃" or "S(O)-CF₃" or "S(O)₂-CF₃" or "CF₂-CH₃";
m is 0, 1 or 2, in particular m is 1;
p, q in each occurrence independently are 0, 1 or 2;
and/or
wherein the optional at least one nitrogen atom being an N-oxide comprises the one of the central pyridine moiety selected from the group consisting of formula (II) or formula (IIa) or formula (IIb): 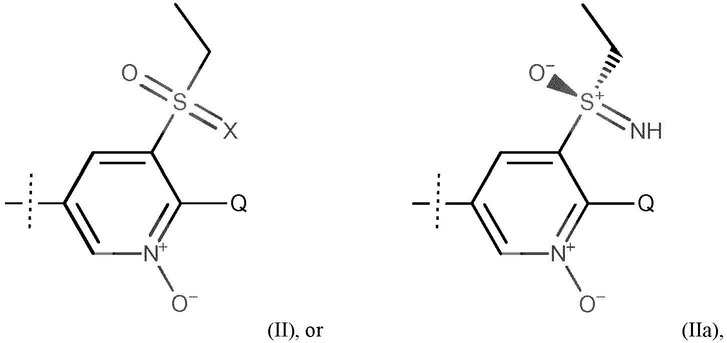 or 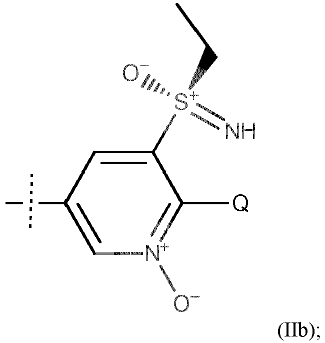
wherein X and Q independently are as defined in this claim, preferably are as claimed in any one of the preceding claims;
or a salt thereof.

7. A compound of formula (I) or formula (Ia) or formula (Ib), preferably according to any one of claims 1 to 6, wherein the compound is selected from the group consisting of:
| | |
|---|---|
| **1** | 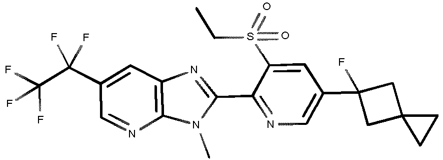 |
| **2** | 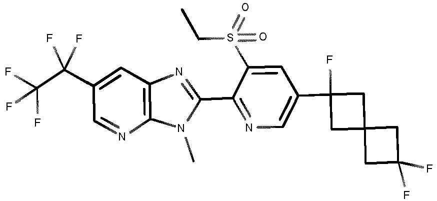 |
| **3** | 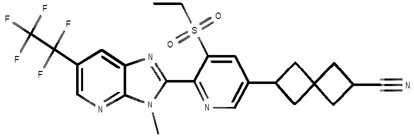 |
| **3a** | 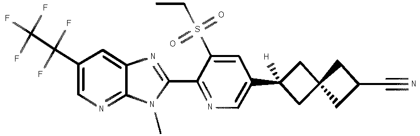 |
| **3b** | 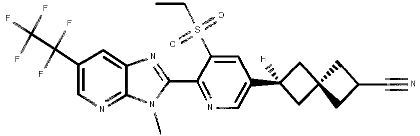 |
| **4** | 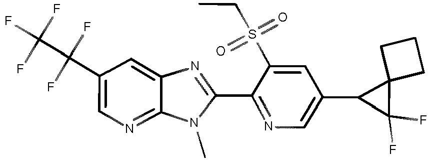 |
| **5** | 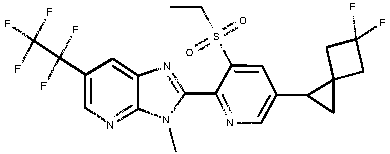 |
| **6** | 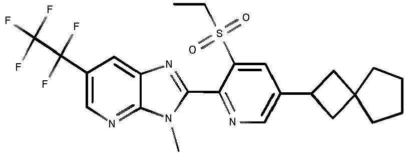 |
| **7** | 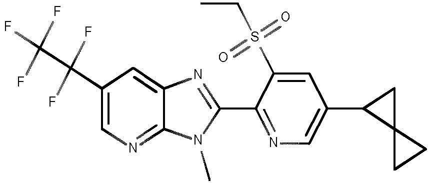 |
| **7a** | 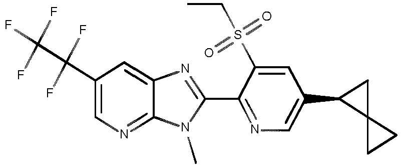 |
| **7b** | 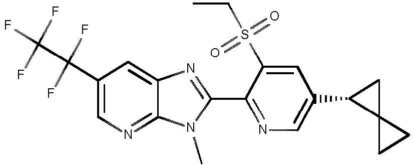 |
| **8** | 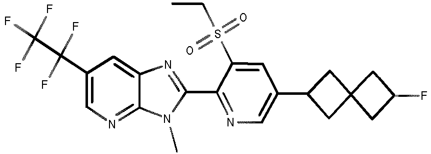 |
| **8a** | 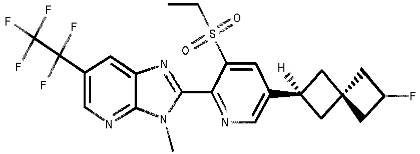 |
| **8b** | 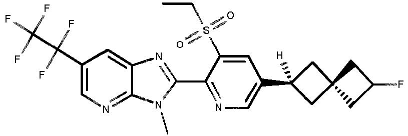 |
| **9** | 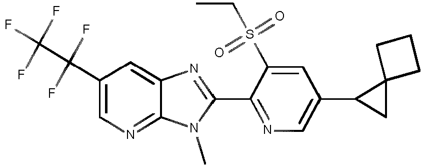 |
| **10** | 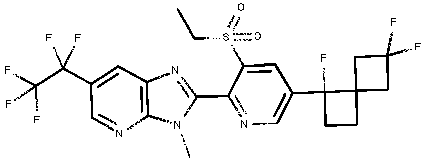 |
| **11** | 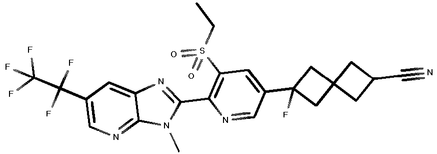 |
| **11a** | 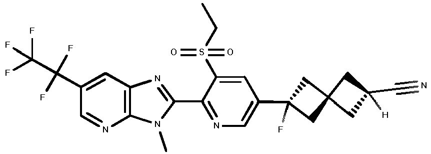 |
| **11b** | 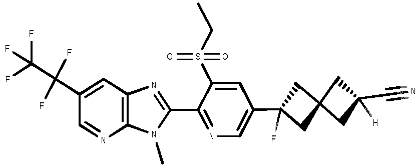 |
| **12** | 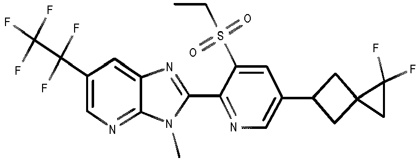 |
| **12a** | 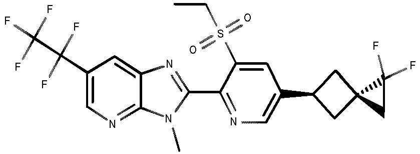 |
| **12b** | 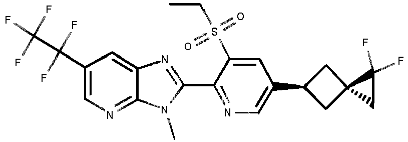 |
| **13** | 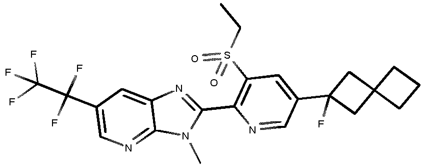 |
| **14** | 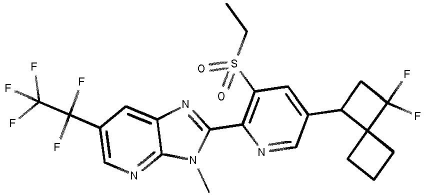 |
| **14a** | 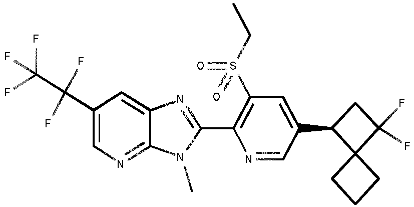 |
| **14b** | 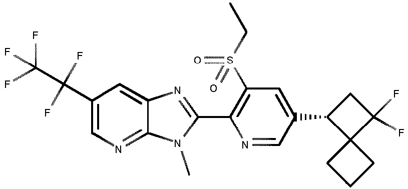 |
| **15** | 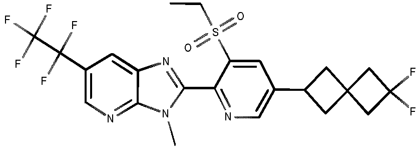 |
| **16** | 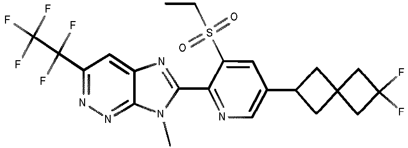 |
| **17** | 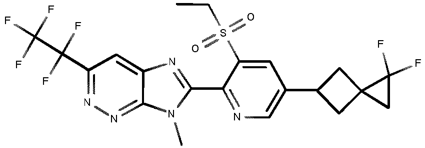 |
| **17a** | 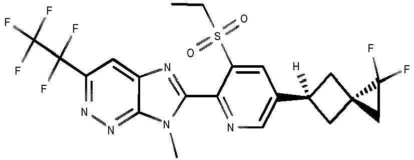 |
| **17b** | 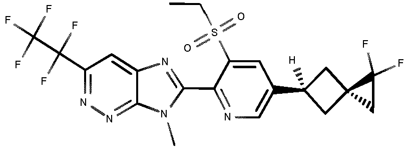 |
| **18** | 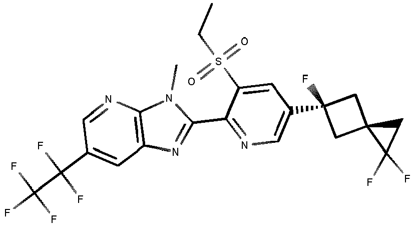 |
| **19** | 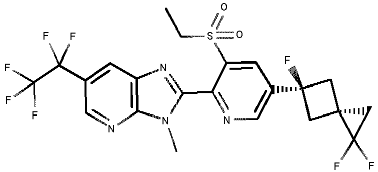 |
| **20** | 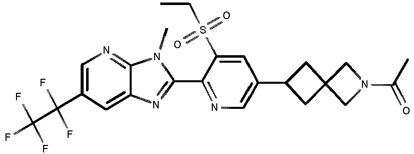 |
| **21** | 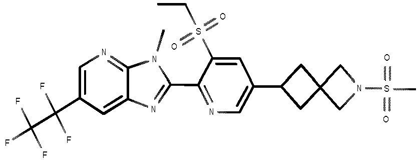 |
| **22** | 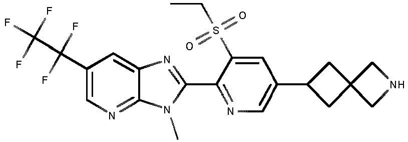 |
| **23** | 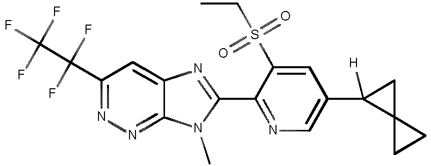 |
| **23a** | 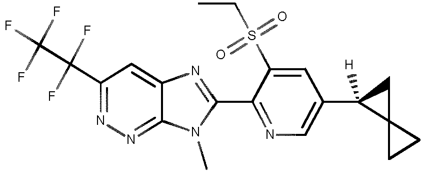 |
| **23b** | 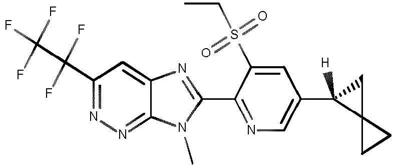 |
| **24** | 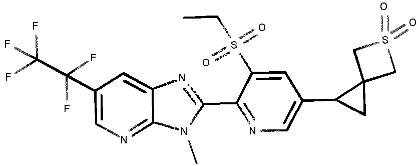 |
| **24a** | 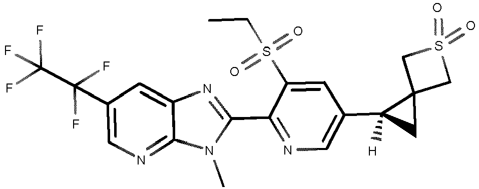 |
| **24b** | 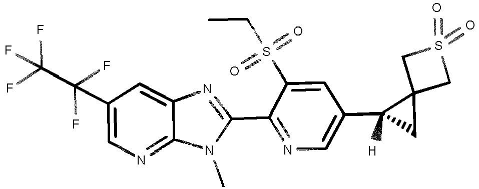 |
| **25** | 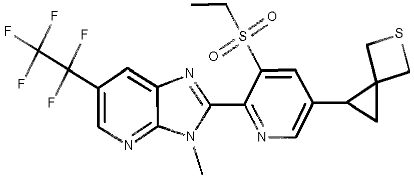 |
| **26** | 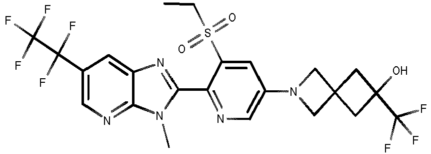 |
| **27** | 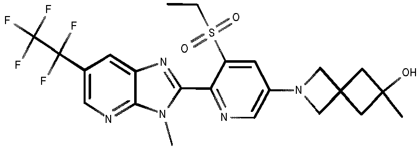 |
| **28** | 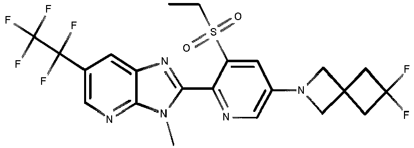 |
| **29** | 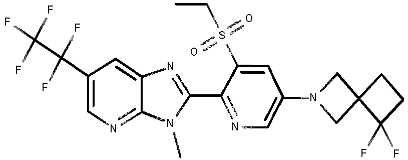 |
| **30** | 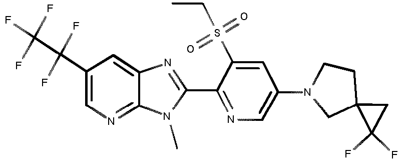 |
| **31** | 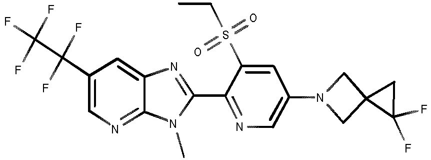 |
| **32** | 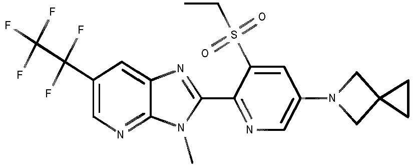 |
| **33** | 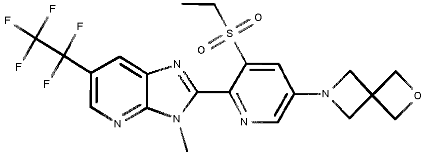 |
| **34** | 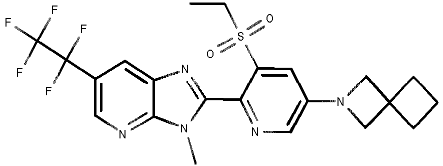 |
| **35** | 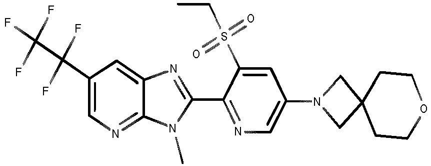 |
| **36** | 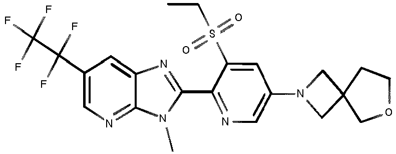 |
| **37** | 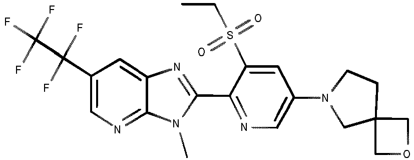 |
| **38** | 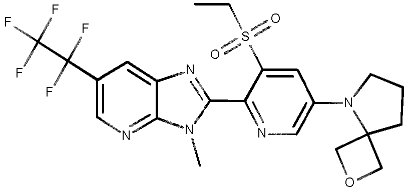 |
| **39** | 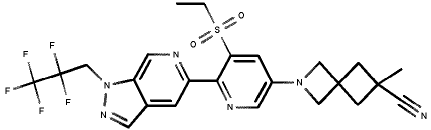 |
| **40** | 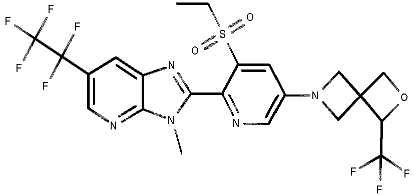 |
| **41** | 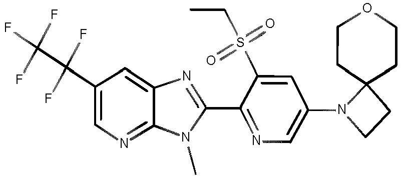 |
| **42** | 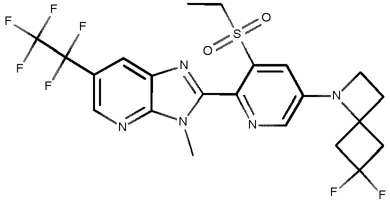 |
| **43** | 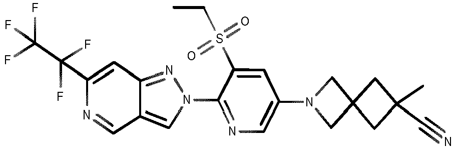 |
| **44** | 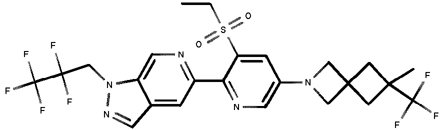 |
| **45** | 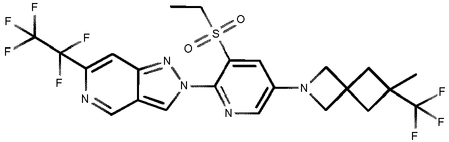 |
| **46** | 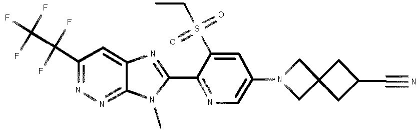 |
| **47** | 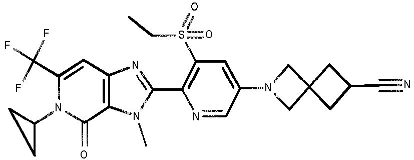 |
| **48** | 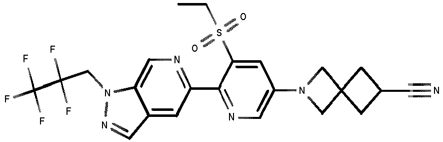 |
| **49** | 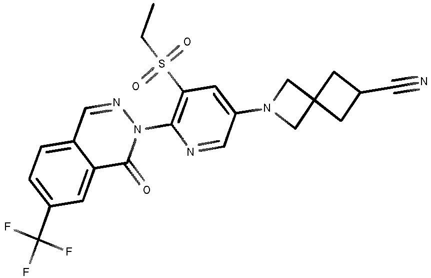 |
| **50** | 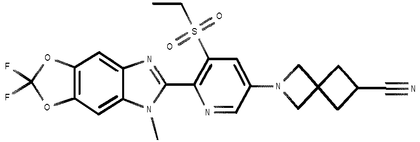 |
| **51** | 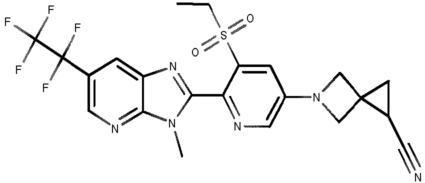 |
| **51a** | 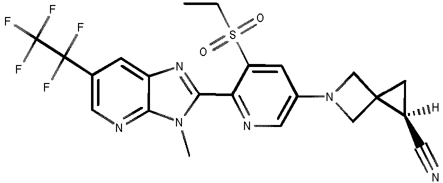 |
| **51b** | 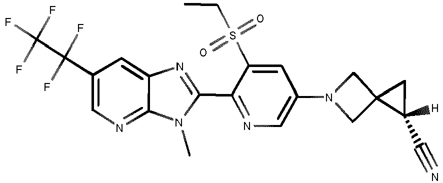 |
| **52** | 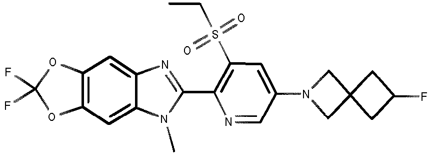 |
| **53** | 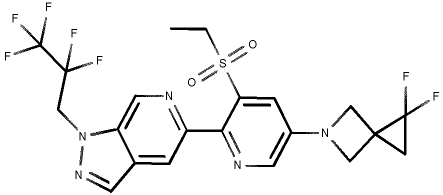 |
| **54** | 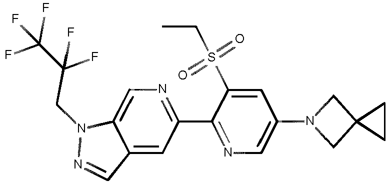 |
| **55** | 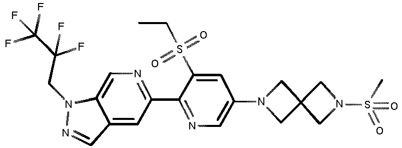 |
| **56** | 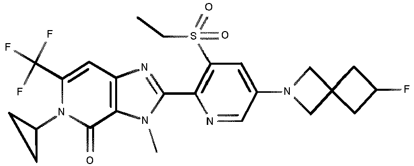 |
| **57** | 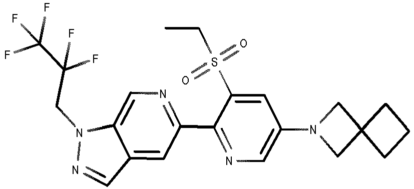 |
| **58** | 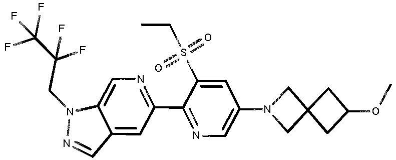 |
| **59** | 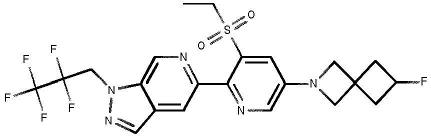 |
| **60** | 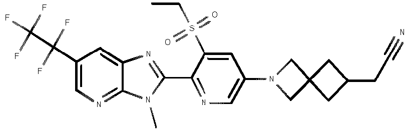 |
| **61** | 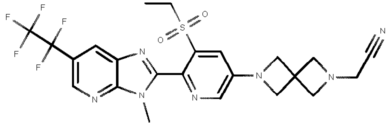 |
| **62** | 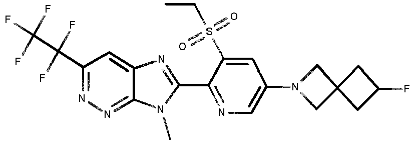 |
| **63** | 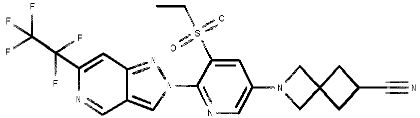 |
| **64** | 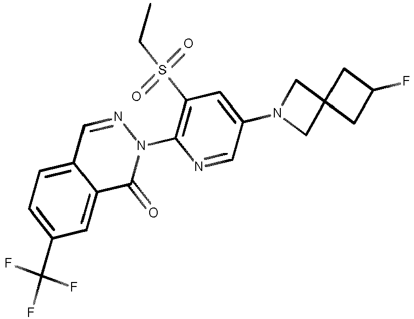 |
| **65** | 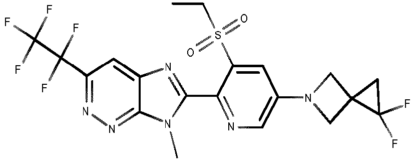 |
| **66** | 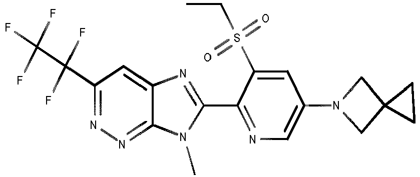 |
| **67** | 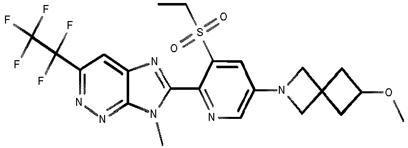 |
| **68** | 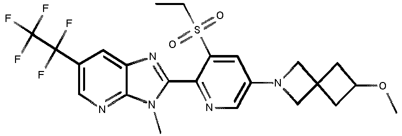 |
| **69** | 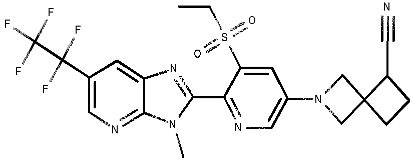 |
| **70** | 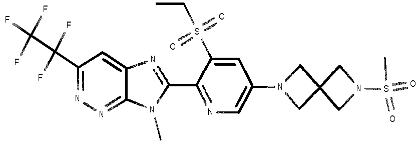 |
| **72** | 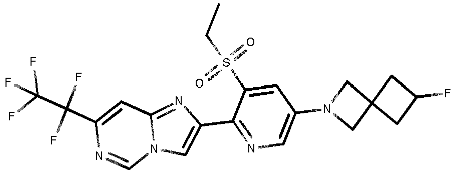 |
| **73** | 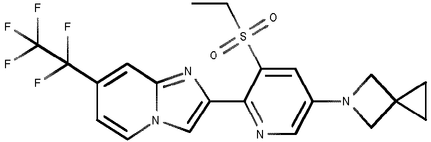 |
| **74** | 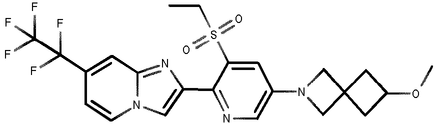 |
| **75** | 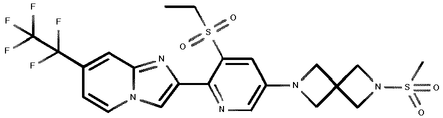 |
| **76** | 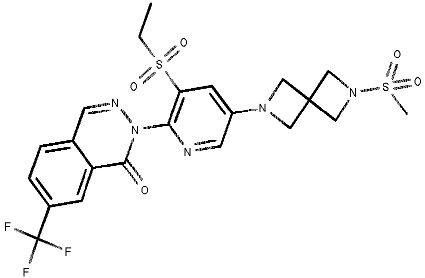 |
| **77** | 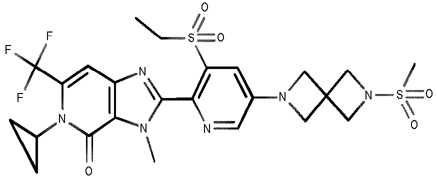 |
| **78** | 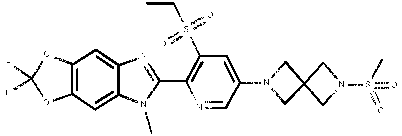 |
| **79** | 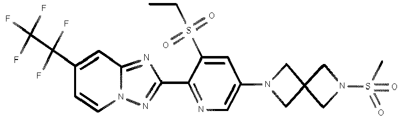 |
| **80** | 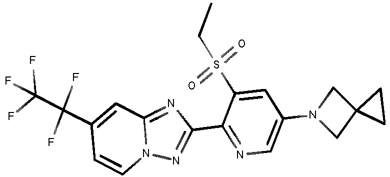 |
| **81** | 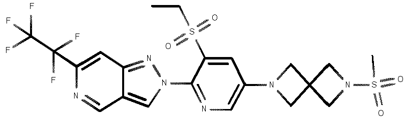 |
| **82** | 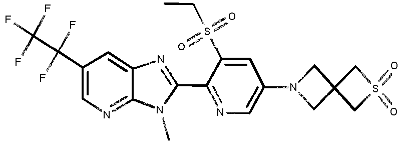 |
| **83** | 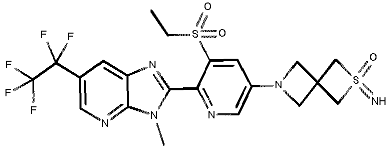 |
| **84** | 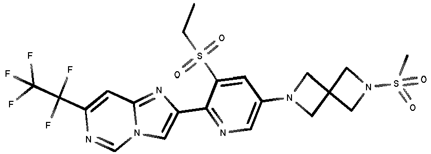 |
| **85** | 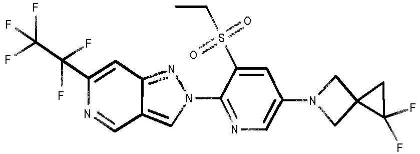 |
| **86** | 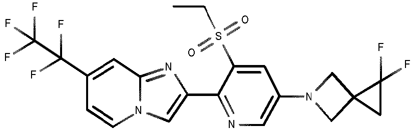 |
| **87** | 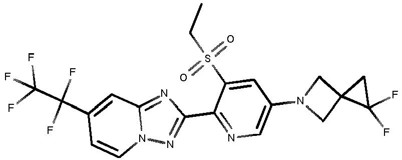 |
| **88** | 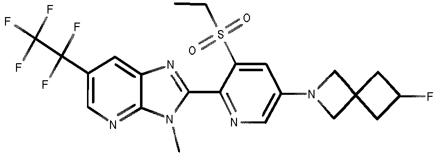 |
| **89** | 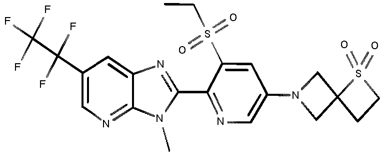 |
| **90** | 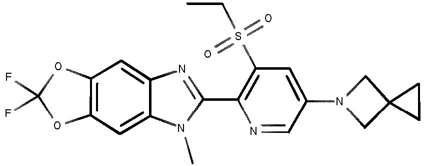 |
| **91** | 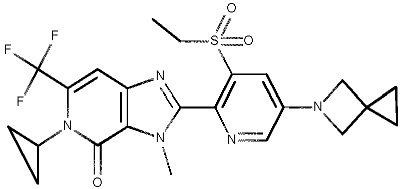 |
| **92** | 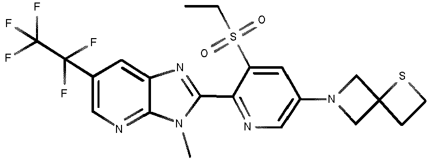 |
| **93** | 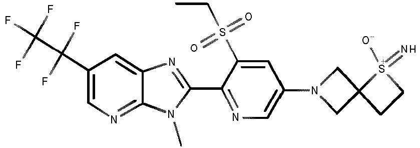 |
| **93a** | 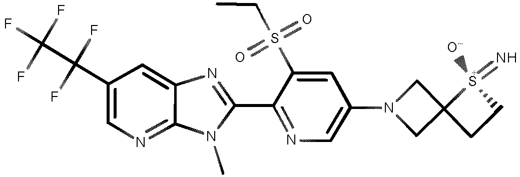 |
| **93b** | 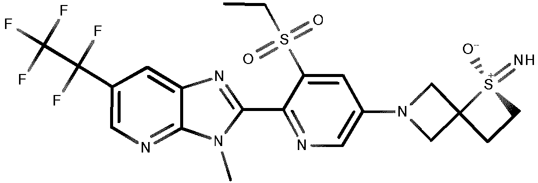 |
| **94** | 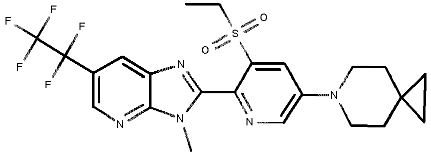 |
| **95** | 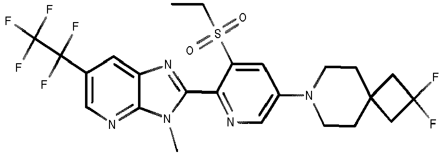 |
| **96** | 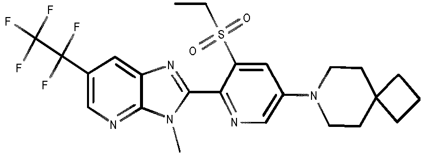 |
| **97** | 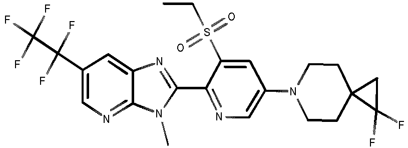 |
| **98** | 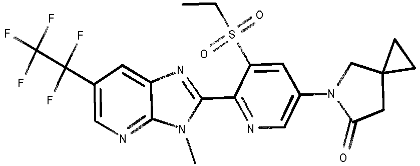 |
| **99** | 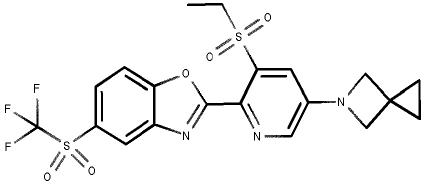 |
| **100** | 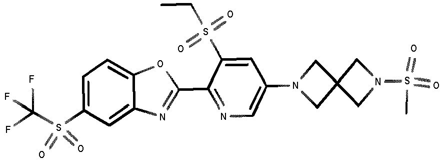 |
| **101** | 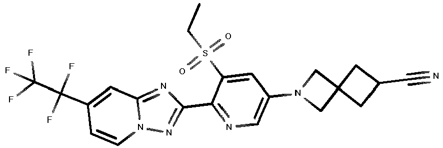 |
| **102** | 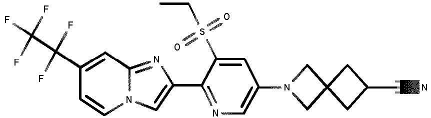 |
| **103** | 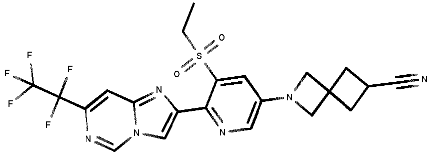 |
| **104** | 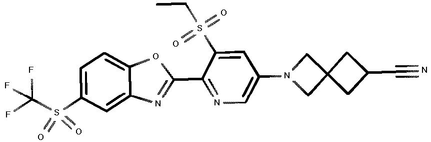 |
| **105** | 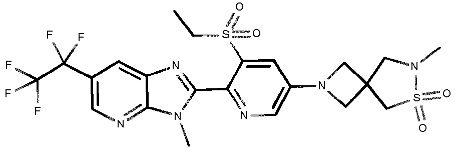 |
| **106** | 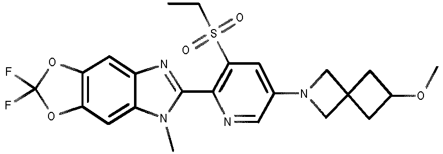 |
| **107** | 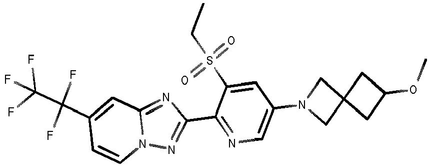 |
| **108** | 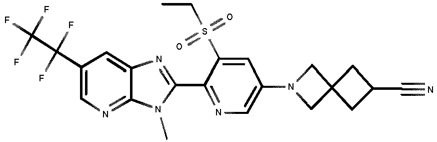 |
| **109** | 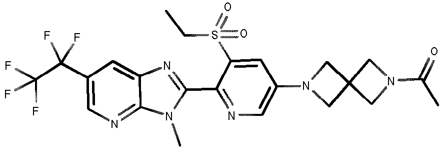 |
| **110** | 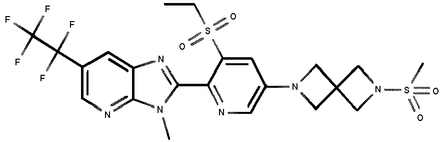 |
| **111** | 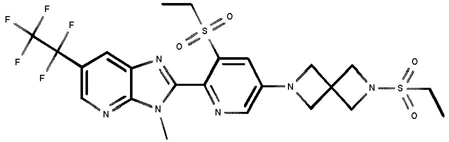 |
| **112** | 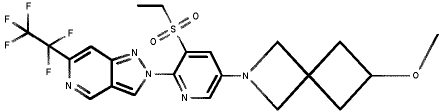 |
| **113** | 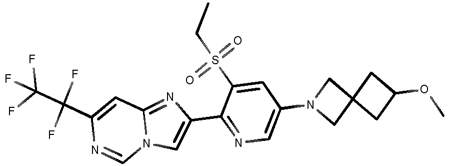 |
| **114** | 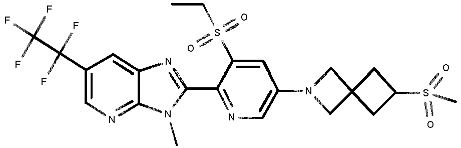 |
| **115** | 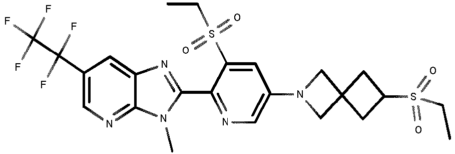 |
| **116** | 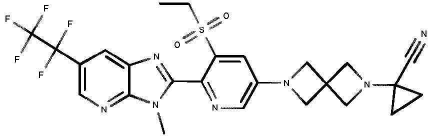 |
| **117** | 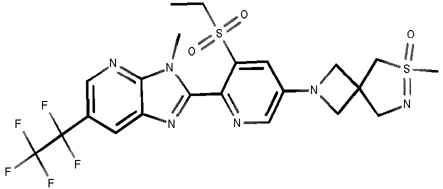 |
| **118** | 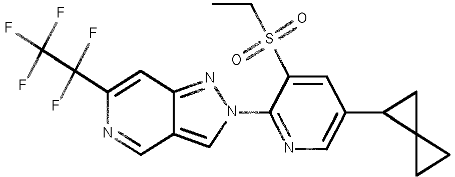 |
| **118a** | 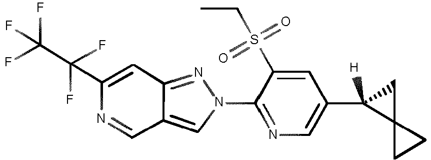 |
| **118b** | 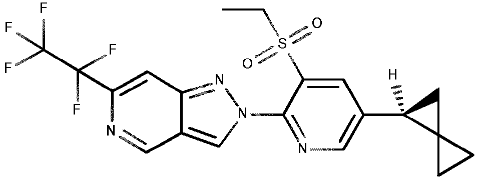 |
| **119** | 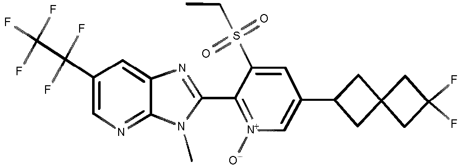 |
| **120** | 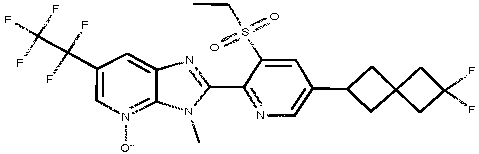 |
| **121** | 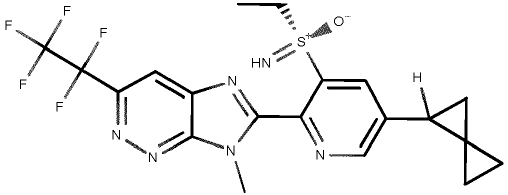 |
| **121a** | 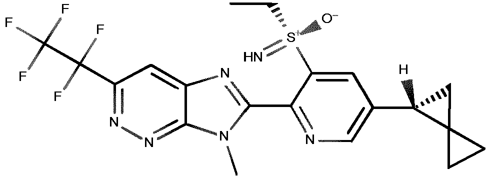 |
| **121b** | 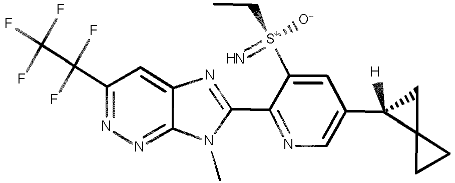 |
| **121c** | 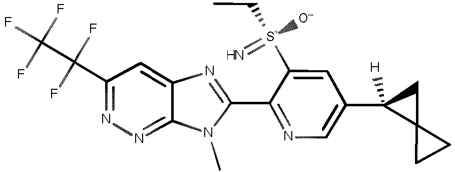 |
| **121d** | 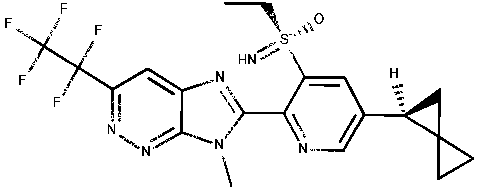 |
| **122** | 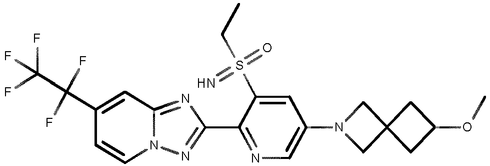 |
| **122a** | 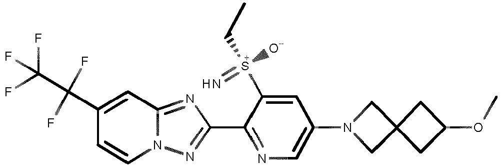 |
| **122b** | 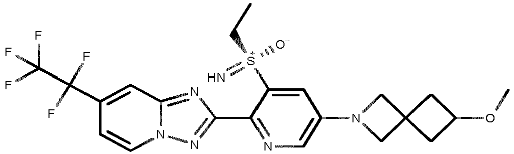 |
or a pharmaceutically acceptable salt thereof.

8. A pharmaceutical composition comprising one or more compound(s) of formula (I), (Ia) and/or (Ib) according to any one of claims 1 to 7 or a pharmaceutically acceptable salt thereof and one or more pharmaceutically acceptable excipient(s).

9. The pharmaceutical composition according to claim 8 further comprising one or more additional pharmaceutically active agent(s).

10. A compound of formula (I), (Ia) or (Ib) according to any one of claims 1 to 7 or a pharmaceutically acceptable salt thereof or a pharmaceutical composition according to any one of claims 8 to 9 for use as a medicament, preferably for use as an antiparasitic medicament.

11. A compound of formula (I), (Ia) or (Ib) according to any one of claims 1 to 7 or a pharmaceutically acceptable salt thereof or a pharmaceutical composition according to any one of claims 8 to 9 for use in a method of treatment, prevention and/or control of a parasitic infection and/or infestation in/on an animal, preferably of an ectoparasitic infestation on an animal, more preferably of an infestation of fleas and/or ticks on an animal.

12. Use of a compound of formula (I), (Ia) or (Ib) according to any one of claims 1 to 7 or a salt thereof or a composition comprising one or more compound(s) of formula (I), (Ia) or (Ib) according to any one of claims 1 to 7 or a salt thereof as a crop protection agent / for crop protection.
